# EUROPEAN PATENT APPLICATION

(11) **EP 4 786 466 A1**
(43) Date of publication of application: **05.08.2026**
(21) Application number: 24889077.4
(22) Date of filing: 05.11.2024
(51) Int. Cl.: C07D 403/12, A61K 31/517, A61P 35/00, C07D 239/95, C07D 417/12, C07D 417/14, C07D 403/14, C07D 471/04, C07D 487/04, C07D 491/113

(54) **QUINAZOLINE DERIVATIVE, SALT THEREOF, AND PHARMACEUTICAL COMPOSITION COMPRISING SAME AS ACTIVE INGREDIENT**

(30) Priority: 08.11.2023 KR 20230153780
(71) Applicant: Korea Research Institute of Chemical Technology, Daejeon 34114 (KR); Korea Institute of Radiological & Medical Sciences, Seoul 01812 (KR)
(72) Inventor: JEONG, Kwan Young, Daejeon 34114 (KR); LEE, Kyu Myung, Daejeon 34114 (KR); KIM, Jae Sung, Namyangju-si, Gyeonggi-do (KR); JUN, Kyoungho, Seoul 01815 (KR); KIM, Ji In, Daejeon 34127 (KR)
(74) Representative: Gassner, Birgitta
(86) International application number: PCT/KR2024/017235
(87) International publication number: WO 2025/100889

(57) **Abstract**

The present invention relates to a quinazoline derivative, a salt thereof, and a pharmaceutical composition containing the same as an active ingredient, which can exhibit an effect of preventing or treating cancer by inhibiting MASTL activity, and in particular can exhibit an effective anticancer effect in cancers where MASTL is overexpressed, such as breast cancer.

## Description

### BACKGROUND

### [Technical Field]

The present invention relates to quinazoline derivatives, salts thereof, and a pharmaceutical composition having MASTL inhibitory activity comprising the same as an active ingredient.

### [Background Art]

Cancer cells proliferate abnormally because they cannot regulate the cell cycle due to problems with the regulation of mitotic kinases or checkpoints compared to normal cells, so targeting the cell cycle of cancer cells is emerging as a strategy for anticancer treatment. However, since mitotic inhibitors can cause side effects if they affect the mitosis of normal cells, it is important to target factors that are overexpressed in cancer cells.

MASTL (Microtubule-associated serine/threonine kinase-like), also known as Greatwall kinase (GWL), belongs to the AGC family of serine/threonine protein kinases. MASTL regulates the cell division cycle and is associated with various cancers, including breast, gastric, colon, and liver cancer.

When MASTL is overexpressed in cancer, cell proliferation and migration of cancer cells increase. In particular, MASTL is overexpressed in breast cancer, and it is known that knockout of MASTL inhibits the proliferation of breast cancer cells, so most MASTL research to date has been related to breast cancer.

Breast cancer accounts for 31% of cancer-related deaths in women, making effective targeted therapy important, and inhibition of MASTL has been shown to be effective not only for general breast cancer but also for triple-negative breast cancer (TNBC). Triple-negative breast cancer refers to a form of breast cancer in which estrogen receptors (ER), progesterone receptors (PR), and HER-2 receptors (human epidermal growth factor 2-receptor), which are important for cell growth and characteristics, are absent, and it accounts for 15-20% of all breast cancers. Triple-negative breast cancer presents challenges such as low responsiveness to hormone therapy or targeted anticancer drugs used for general breast cancer, rapid cancer growth, and metastasis, resulting in a low survival rate; therefore, the development of treatments is crucial.

MASTL regulates mitotic progression through the direct phosphorylation of α-endosulfin (ENSA) and cAMP-regulated phosphoprotein 19 (ARPP19). When MASTL, activated by CDK-cyclin at the start of mitosis, phosphorylates ENSA and ARPP19, the activity of the tumor suppressor protein phosphatase 2A complex (PP2A-B55) decreases by more than 10 times, and conversely, when mitosis ends, MASTL becomes inactivated, and ARPP19 and ENSA are also dephosphorylated, reactivating PP2A.

Protein phosphatase 2A (PP2A) is a major mammalian serine-threonine phosphatase and a tumor suppressor, with its activity inhibited in most tumors. Since mutations in the PP2A subunit gene can induce or cause the recurrence of breast cancer, the PP2A-B55 complex may play a role in suppressing tumors in breast cancer. Furthermore, it has been revealed that a decrease in MASTL induces apoptosis and lowers cell viability in breast cancer cell lines, and it has been reported that inhibiting MASTL increases PP2A activity and can enhance the radiosensitivity of cancer cells. Therefore, inhibition of MASTL activity can reduce tumor growth and metastasis in breast cancer by increasing PP2A activity, and a good anticancer effect can be expected when combined with radiation therapy.

To date, AT13148, GKI-1, and MKI-1 have been reported as MASTL inhibitors. AT13148 is a multi-AGC kinase inhibitor with an IC₅₀ value of 5.9 µM in MASTL. GKI-1 (Greatwall Kinase Inhibitor-1) is a compound derived from studies of AT13148 and the MASTL kinase domain, with an IC₅₀ value of 4.9 µM in MASTL. It was confirmed that GKI-1 inhibits MASTL *in vitro* and delays mitotic progression by reducing phosphorylated ENSA. MKI-1 (MASTL Kinase Inhibitor-1) is a compound derived through *in silico* screening and has an IC₅₀ value of 9.9 µM in MASTL. It also exhibits antitumor activity and radiosensitivity by increasing PP2A activity *in vitro* and *in vivo* and destabilizing c-Myc.

All three compounds developed to date exhibit activity in the µM range, resulting in low MASTL inhibitory effects. Since most of them show general inhibitory effects on the AGC kinase family, there is a need to develop compounds that can selectively inhibit only MASTL.

For related literature, refer to Cory A. Ocasio, et al., A first generation inhibitor of human Greatwall kinase, enabled by structural and functional characterization of a minimal kinase domain construct. Oncotarget 2016, 7 (44).

### [DETAILED DESCRIPTION OF THE INVENTION]

### [Summary]

The present invention aims to provide a compound inhibiting MASTL activity or a pharmaceutical composition comprising the same as an active ingredient.

### [Technical solution]

To achieve the above objective, the present invention provides a compound represented by Formula1, a stereoisomer thereof, a hydrate thereof, a solvate thereof, or a salt thereof.

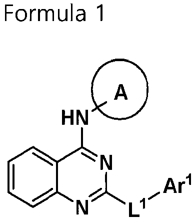

The compound, a stereoisomer thereof, a hydrate thereof, a solvate thereof, or a salt thereof inhibit MASTL activity.

Additionally, in another aspect, the present invention provides a pharmaceutical composition for preventing or treating cancer comprising, as an active ingredient, a compound represented by Formula1, a stereoisomer thereof, a hydrate thereof, a solvate thereof, or a salt thereof. The composition is characterized by inhibiting MASTL, and the cancer includes gastric cancer, breast cancer, uterine cancer, colon cancer, lung cancer, colorectal cancer, pancreatic cancer, liver cancer, or prostate cancer.

Additionally, in another aspect, the present invention provides a method for preventing or treating cancer comprising the step of administering the pharmaceutical composition for cancer prevention or treatment to a mammal other than a human.

Additionally, in another aspect, the present invention provides a health functional food composition for preventing or improving cancer comprising, as an active ingredient, a compound represented by Formula1, a stereoisomer thereof, a hydrate thereof, a solvate thereof, or a salt thereof.

Additionally, in another aspect, the present invention provides a PROTAC compound or a pharmaceutically acceptable salt thereof comprising a target protein ligand, a linker, and an E3 enzyme ligand, wherein the target protein ligand is a compound represented by Formula 1.

### [Effects of the Invention]

The compound according to the present invention or the composition comprising the same as an active ingredient can exhibit an anticancer effect by inhibiting MASTL activity, and in particular, can exhibit an effective anticancer effect in breast cancer in which MASTL is overexpressed.

### [DETAILED DESCRIPTION]

The present invention will be described in detail below with reference to embodiments thereof. In describing the present invention, if it is determined that a detailed description of related known components or functions may obscure the essence of the invention, such detailed description will be omitted.

As used in this specification and the appended claims, unless otherwise noted, the meanings of the following terms are as follows:
The term "halogen" as used in the present invention is fluorine (F), bromine (Br), chlorine (Cl), or iodine (I) unless otherwise noted.

The terms "alkyl" or "alkyl group" used in the present invention refer to aliphatic hydrocarbon radicals, and refer to radicals of saturated aliphatic functional groups including straight-chain alkyl groups, branched-chain alkyl groups, cycloalkyl (alicyclic) groups, alkyl-substituted cycloalkyl groups, and cycloalkyl-substituted alkyl groups. For example, C₁~C₆ alkyls are aliphatic hydrocarbons having 1 to 6 carbon atoms, including methyl, ethyl, propyl, n-butyl, n-pentyl, n-hexyl, isopropyl, isobutyl, sec-butyl, tert-butyl, neopentyl, isopentyl, etc. As an example, cyclic alkyl may be used interchangeably with "cycloalkyl" in this specification, and may include, but are not limited to, cyclopropyl, cyclobutyl, cyclopentyl, cyclohexyl, cycloheptyl, or cyclooctyl.

The term "alkenyl group" as used in the present invention refers to a group in which at least two carbon atoms are connected by at least one carbon-carbon double bond, and includes, but is not limited to, straight-chain or side-chain groups.

The terms "alkoxyl group" or "alkoxy group" used in the present invention refer to radicals in which a hydrogen atom of a hydroxyl group is substituted with an alkyl group, for example, C₁-C₆ alkoxy groups include all of methoxy, ethoxy, propoxy, n-butoxy, n-pentyloxy, isopropoxy, sec-butoxy, tert-butoxy, neopentyloxy, isopentyloxy, etc.

In the present invention, "haloalkyl" may mean a straight-chain or branched-chain alkyl (hydrocarbon) having a carbon atom substituted with one or more halos as defined herein. Examples of said haloalkyls include, but are not limited to, methyl, ethyl, propyl, isopropyl, isobutyl, or n-butyl independently substituted with one or more halogens, e.g., F, Cl, Br, or I.

In the present invention, "heterocyclic ring" may mean a ring containing 1 to 5 heteroatoms selected from N, O and S as ring-forming atoms, and may be saturated or partially unsaturated. Here, if unsaturated, it may be referred to as a heterocycloalkene. Unless otherwise noted, heterocycloalkyls may be single rings or multiple rings such as spiro rings, bridged rings, or fused rings. Additionally, "3 to 12 atomic heterocyclic group" may mean a heterocyclic group comprising 3 to 12 ring-forming atoms, and as examples, pyrrolidine, piperidine, imidazolidine, pyrazolidine, butyrolactam, valerolactam, imidazolidinone, hydantoin, dioxolane, phthalimide, piperidine, pyrimidine 2,4(1H,3H)-dione, 1,4-dioxane, morpholine, thiomorpholine, thiomorpholine-S-oxide, thiomorpholine S,S-oxide, piperazine, pyran, pyridone, 3-pyrroline, thiopyran, pyrone, tetrahydrofuran, tetrahydrothiophene, quinuclidine, tropane, 2-azaspyro[3,3]heptane, (1R,5S)-3-azabicyclo[3.2.1]octane, (1s,4s)-2-azabicyclo[2.2.2]octane, or (1R,4R)-2-oxa-5-azabicyclo[2.2.2]octane, etc., Examples of saturated 'heterocyclic groups' include thiophene, purine, pyrrole, pyrazol, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine, pyrazolopyridine, N-arylcarbazole, N-heteroarylcarbazole, N-alkylcarbazole, benzoxazole, benzimidazole, benzothiazole, Benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, and dibenzofuran are examples, but are not limited to these.

The terms "aryl group" or "arylene group" used in the present invention refer to a single-ring or multi-ring aromatic group and include an aromatic ring formed by an adjacent substituent participating in bonding or reaction. For example, aryl groups may be exemplified by, but are not limited to, phenyl groups, naphthalene, fluorene, anthracene, phenanthrene, bibenzene, terbenzene, quaterbenzene, quinquebenzene, sexibenzene, triphenylene, pyrene, benzofluoranthene, chrysene, etc.

The term "aliphatic ring" as used in this invention refers to an aliphatic hydrocarbon ring.

The term "aromatic ring" as used in this invention refers to an aromatic system composed of hydrocarbons containing one or more rings, examples of which include benzene and naphthalene.

Additionally, the definitions described in the present invention may be added to form chemically related combinations, such as "arylalkyl," "alkylcarbonyl," "arylcarbonyl," etc. When the term "alkyl" is used as a suffix, as in the other terms below, "phenylalkyl" or "hydroxyalkyl," it means an alkyl group substituted with a substituent selected from another clearly named group. Thus, for example, "phenylalkyl" means an alkyl group having a phenyl substituent, and thus includes benzyl, phenylethyl, and biphenyl. "alkylaminoalkyl" means an alkyl group having an alkylamino substituent.

Hereinafter, a compound according to one aspect of the present invention and a pharmaceutical composition comprising the same will be described.

The present invention provides a compound represented by Formula 1, a stereoisomer thereof, a hydrate thereof, a solvate thereof, or a salt thereof. 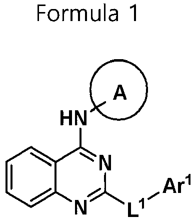 wherein:
L¹ is a single bond; -NH-; or a C₂-C₃₀ heterocyclic group containing at least one N;
Ar¹ is selected from the group consisting of hydrogen; a C₁-C₃₀ alkyl group; a C₁-C₃₀ hydroxyalkyl group; a C₆-C₃₀ aryl group; a C₇-C₃₀ arylalkyl group; a C₂-C₃₀ heterocyclic group containing at least one N; -S(=O)₂-R'; and -C(=O)-NH-R'; and said Ar¹ may be optionally substituted with one or more R¹s.

Ring A is a C₆-C₃₀ aryl group; a -(CH₂)ₙ-aryl group; or a C₂-C₃₀ heterocyclic group containing at least one N; and the A ring can be optionally substituted with one or more R²s.
n is an integer of 1 to 10,
R' is a C₁-C₃₀ alkyl group; a C₃-C₃₀ cycloalkyl group; a C₇-C₃₀ arylalkyl group; a C₆-C₃₀ aryl group; or a C₂-C₃₀ heterocyclic group;
R¹ is independently selected from the group consisting of hydrogen; halogen; hydroxyl group; oxo group; -(CH₂)n-cyano group; C₃-C₃₀ cycloalkyl group; C₁-C₃₀ alkyl group substituted or unsubstituted with halogen; C₁-C₃₀ alkoxy group; -L'- C₂-C₃₀ heterocyclic group; -L'-C(=O)-NH₂; -L'-C(=O)O-R^{a}; -L'-S(=O)₂-R^{a}; and -S(=O)₂-NR^{b}R^{c};
R² are independently selected from the group consisting of hydrogen; halogen; hydroxyl group; amino group; oxo group; C₃-C₃₀ cycloalkyl group; C₁-C₃₀ alkyl group; C₂-C₃₀ alkenyl group; C₁-C₃₀ alkoxy group; -NH-C(=O)O-R^{d}; -S(=O)₂-R^{d}; -S(=O)₂-NR^{e}R^{f}; and -O-C₇-C₃₀ arylalkyl group; or multiple adjacent R²s may bond to each other to form a ring.
L' is a single bond; or C₁-C₁₀ alkylene group;
R^{a}, R^{b}, R^{c}, R^{d}, R^{e} and R^{f} are independently hydrogen; a C₁-C₁₀ alkyl group; a C₃-C₃₀ cycloalkyl group; a C₆-C₁₂ aryl group substituted or unsubstituted with a halogen; a C₃-C₃₀ heterocyclic group containing at least one N; or a C₁-C₁₀ hydroxyalkyl group.

Additionally, the present invention provides a compound represented by Formula 1-1, a stereoisomer thereof, a hydrate thereof, a solvate thereof, or a salt thereof. 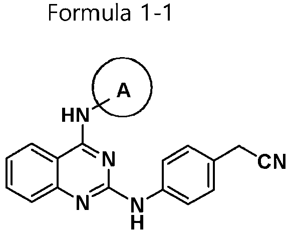 wherein:
Ring A is a substituent represented by any one of Formulas A-1 to A-5, 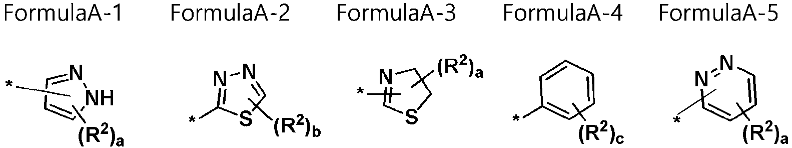 wherein:
* indicates a position to be bonded,
R² is the same as defined in Formula 1,
a is an integer of 0 to 3, b is an integer of 0 to 2, and c is an integer of 0 to 5.

Additionally, the present invention provides a compound represented by Formula 2-1, a stereoisomer thereof, a hydrate thereof, a solvate thereof, or a salt thereof, wherein Formula1 is represented by Formula 2-1. 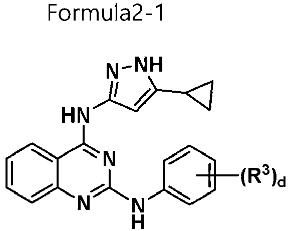 wherein:
R³ is independently hydrogen; cyano group; C₃-C₃₀ cycloalkyl group; C₁-C₃₀ alkoxy group; -L'-C₂-C₃₀ heterocyclic group;-L"-C(=O)-NH₂; -L"-C(=O)O-R^{a}; -L'-S(=O)₂-R^{a}; and -S(=O)₂-NR^{b}R^{c};
Except where the R³ is entirely hydrogen,
the L" is a C₁-C₁₀ alkylene group;
d is an integer of 0 to 5, and
L', R^{a}, R^{b}, and R^{c} are the same as defined in Formula 1.

Additionally, the present invention provides a compound represented by any one of the Formulas 2-2 to 2-6, a stereoisomer thereof, a hydrate thereof, a solvate thereof, or a salt thereof, wherein Formula 1 is represented by any one of the following formulas 2-2 to 2-6. 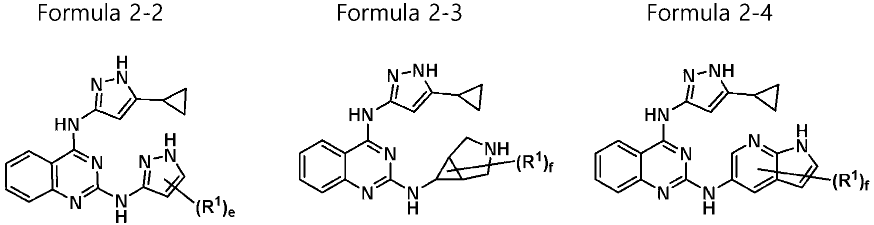 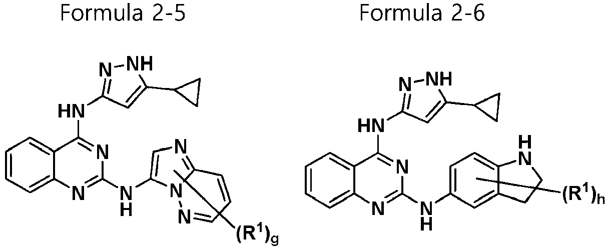 wherein:
R¹ is the same as defined in Formula 1,
e is an integer of 0 to 3, f is an integer of 0 to 5, g is an integer of 0 to 4, and h is an integer of 0 to 6.

Additionally, the present invention provides a compound represented by any one of the following formulas 3-1 to 3-9, a stereoisomer thereof, a hydrate thereof, a solvate thereof, or a salt thereof, wherein Formula1 is represented by any one of the following formulas 3-1 to 3-9. 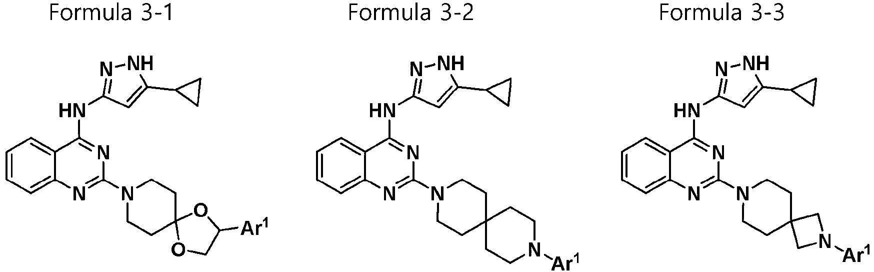 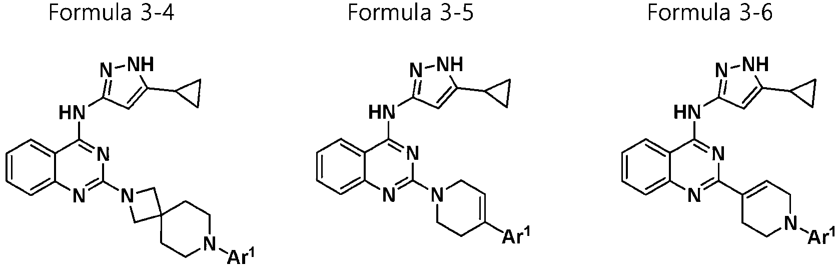 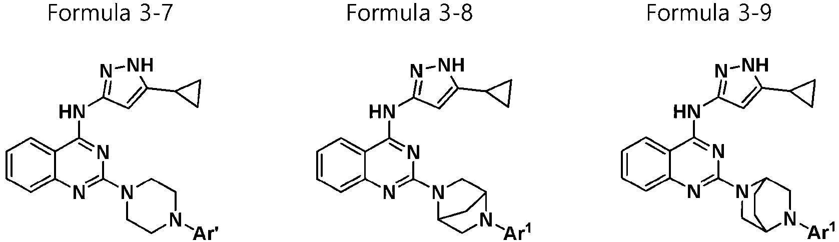 wherein:
Ar' is selected from the group consisting of a C₁-C₃₀ hydroxyalkyl group; a C₆-C₃₀ aryl group; a C₇-C₃₀ arylalkyl group; and a C₂-C₃₀ heterocyclic group containing at least one N; -S(=O)₂-R'; and -C(=O)-NH-R'; and the Ar' can be optionally substituted with one or more R¹s,
Ar¹, R¹, and R' are the same as defined in Formula 1.

Specifically, Formula 1 may be represented by any one of the following compounds but is not limited thereto.

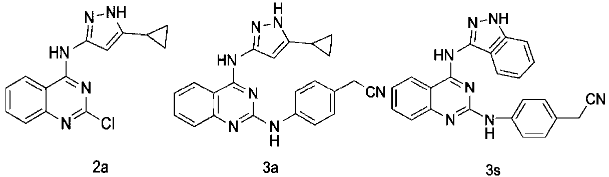

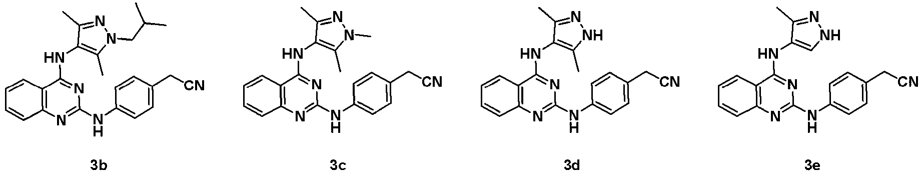

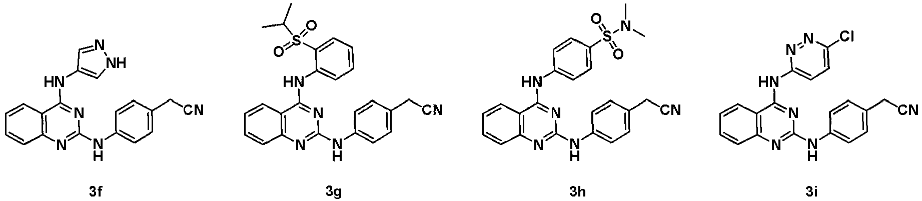

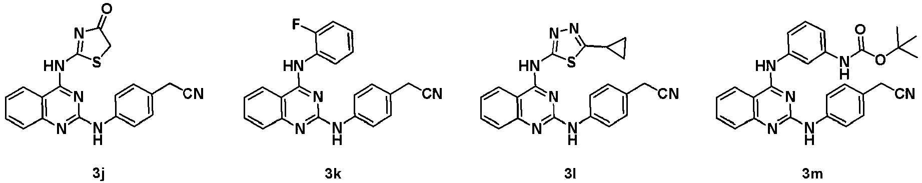

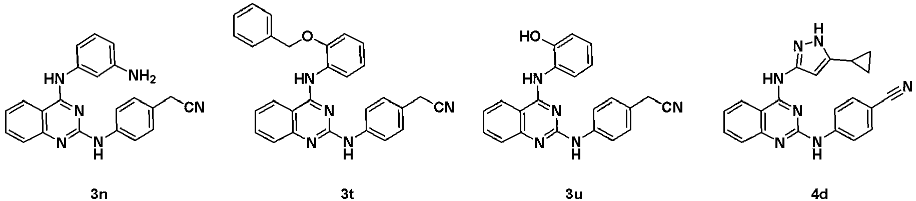

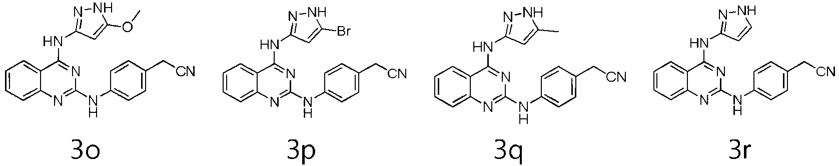

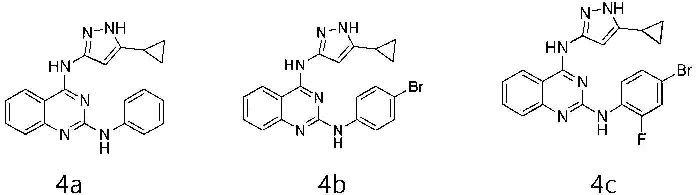

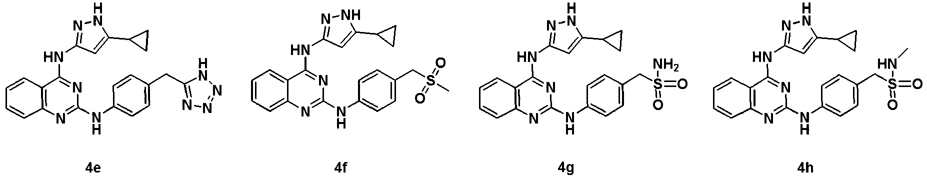

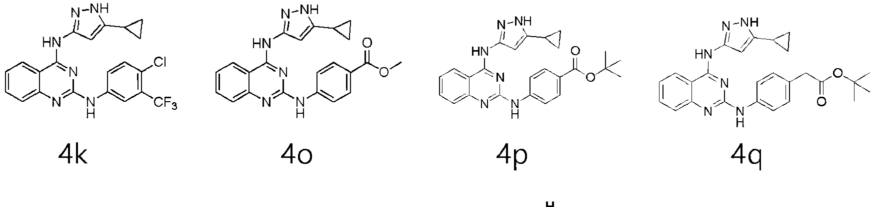

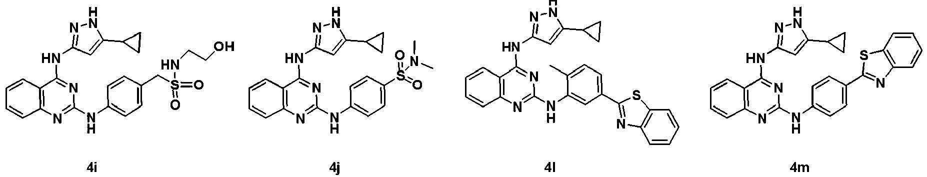

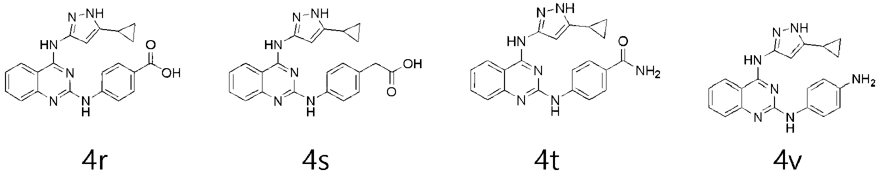

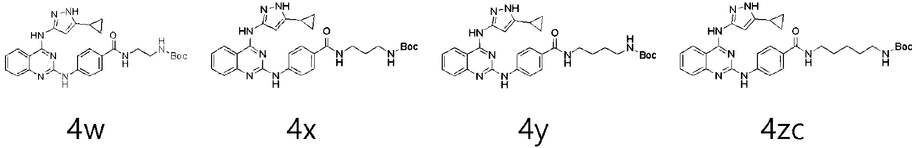

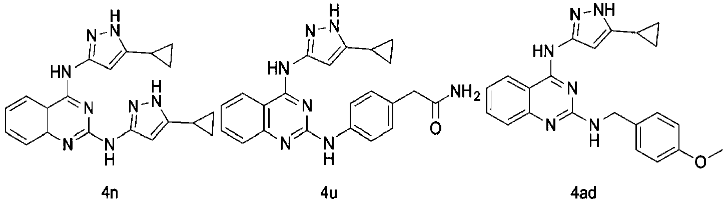

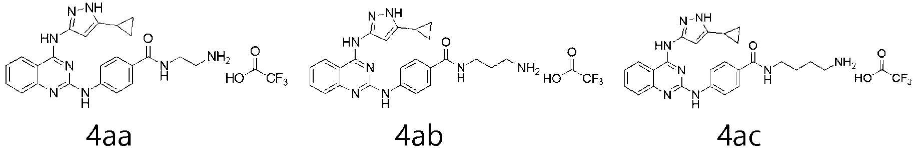

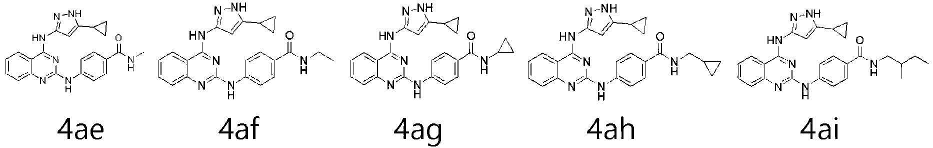

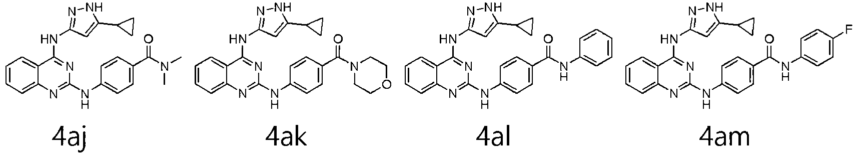

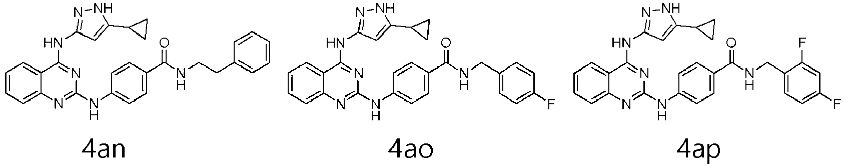

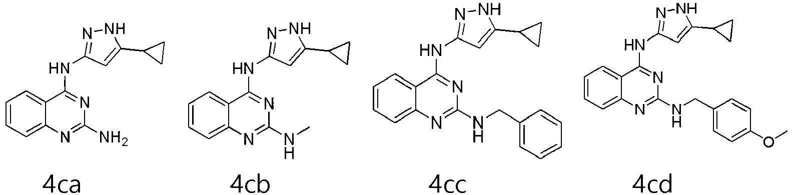

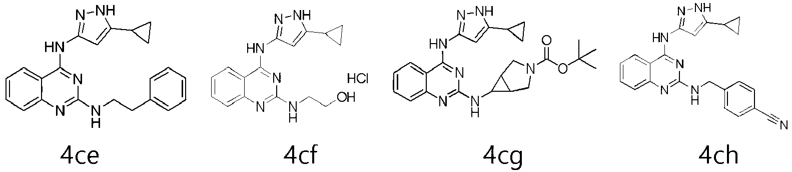

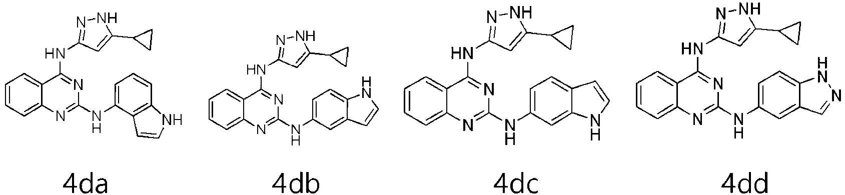

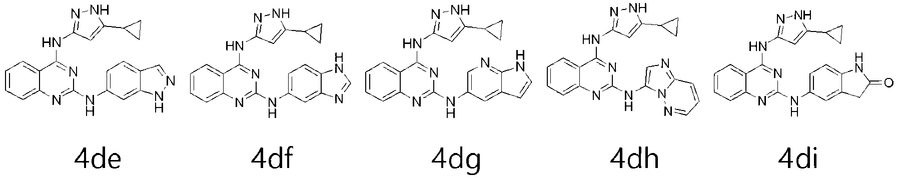

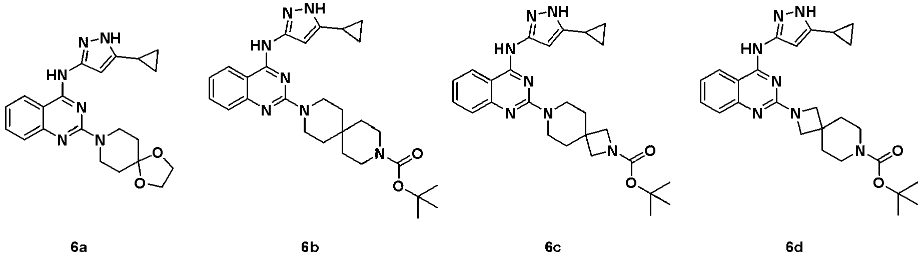

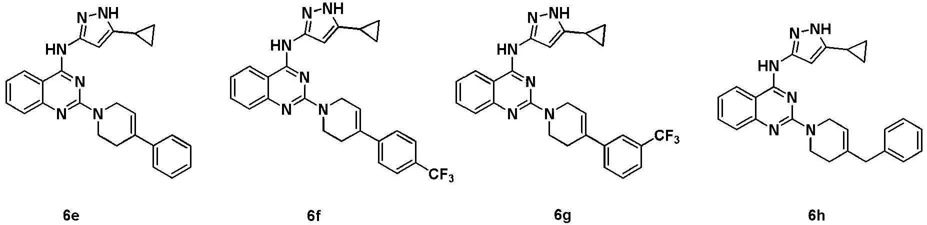

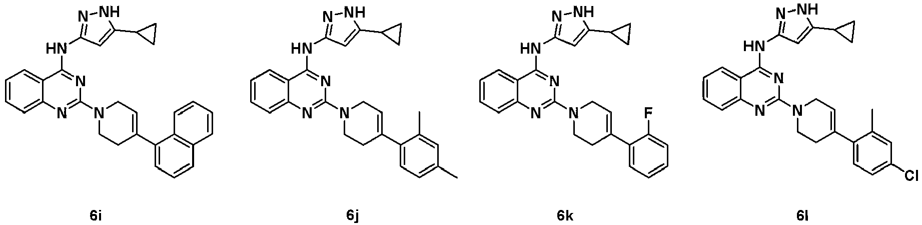

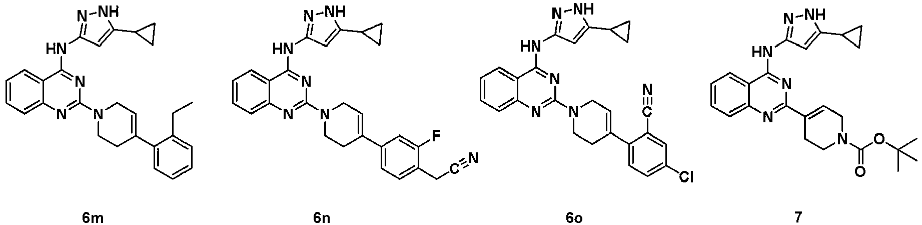

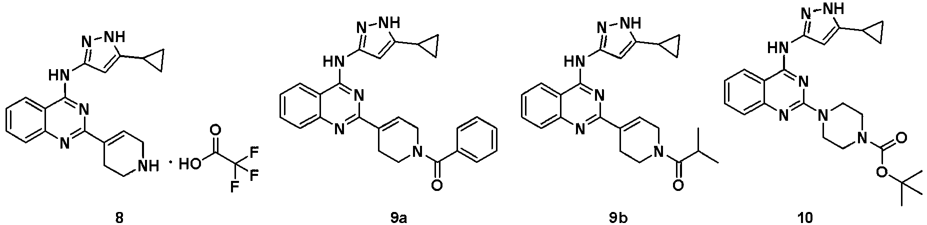

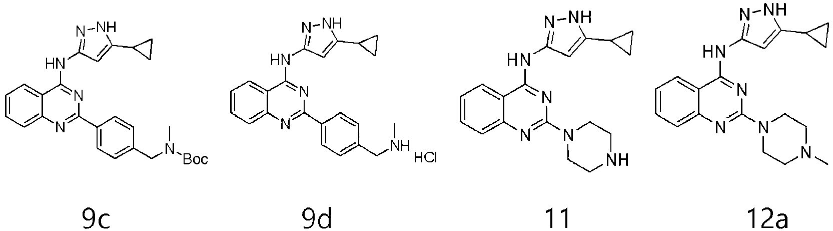

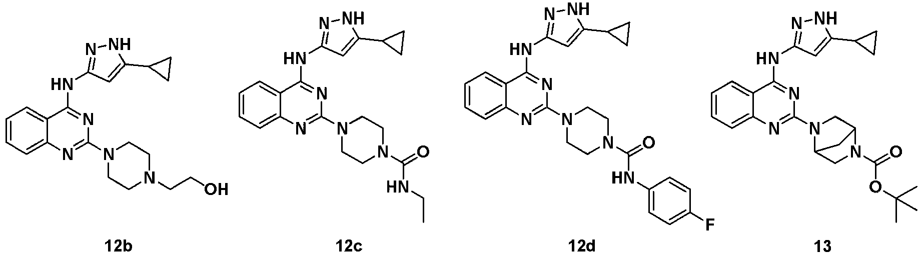

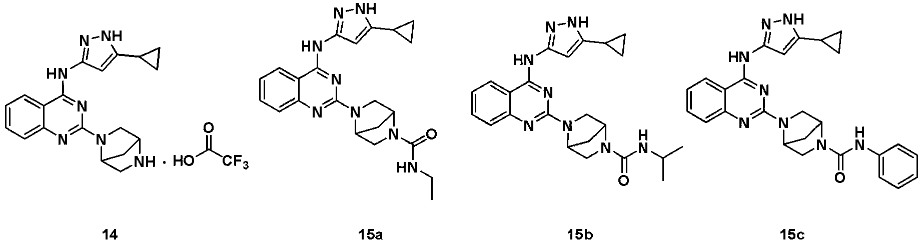

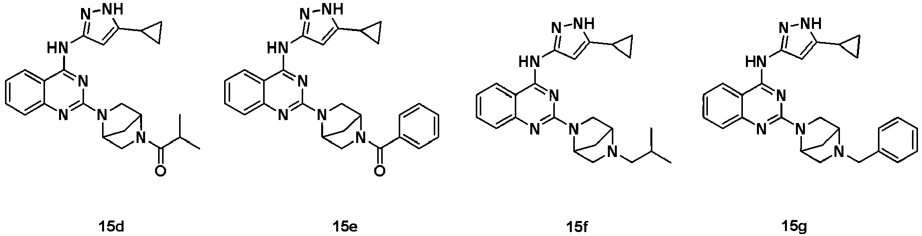

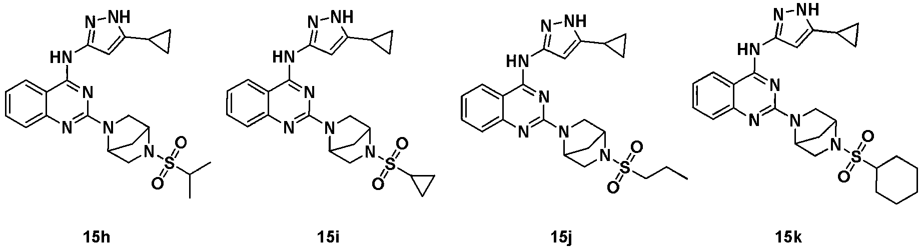

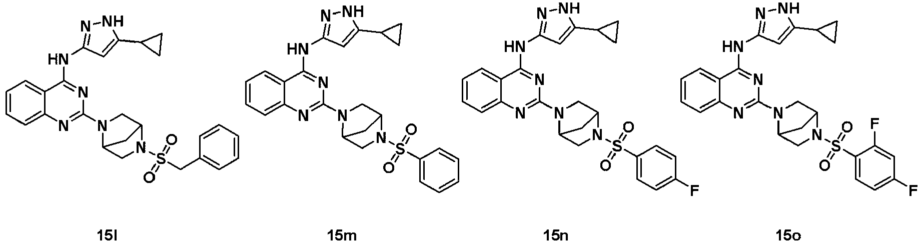

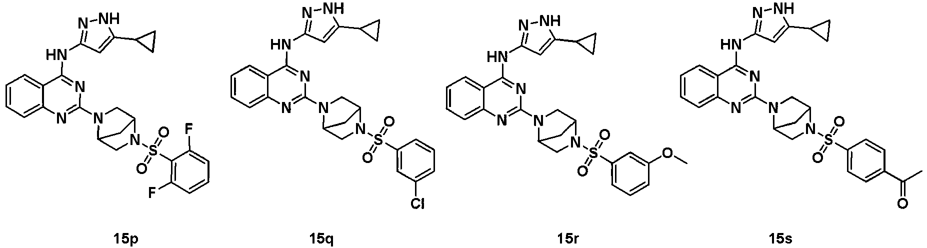

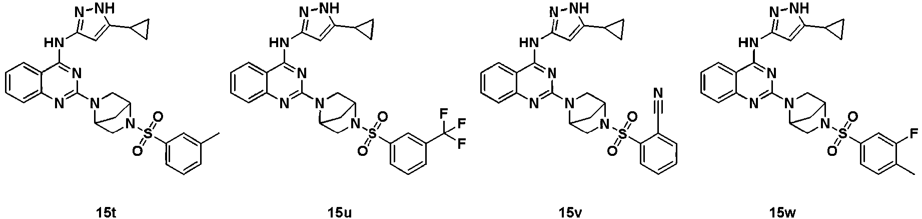

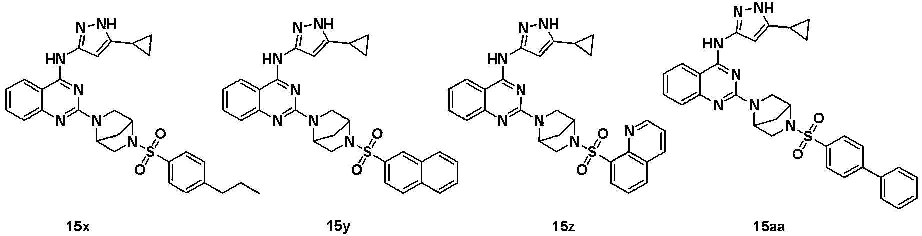

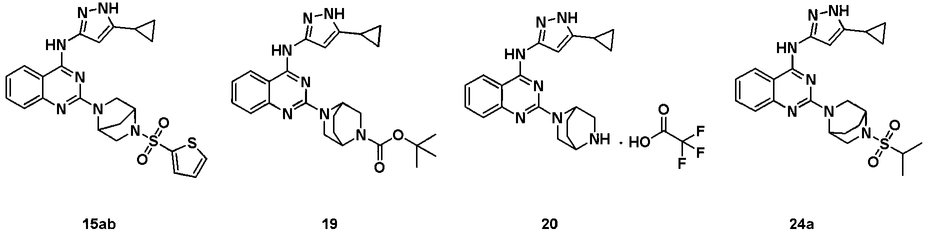

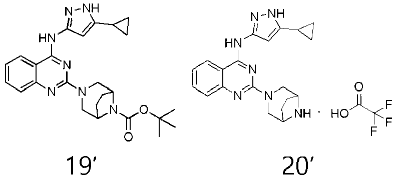

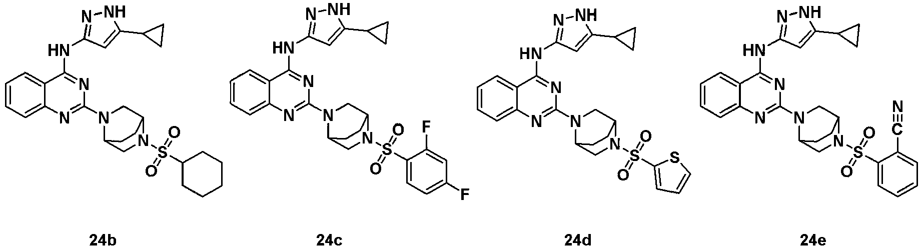

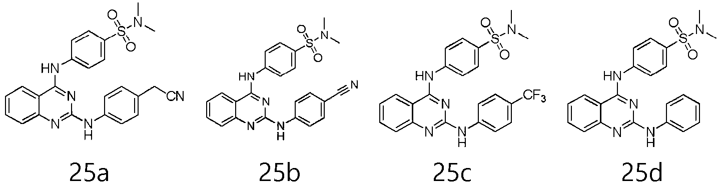

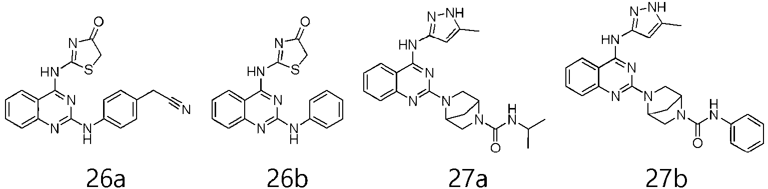

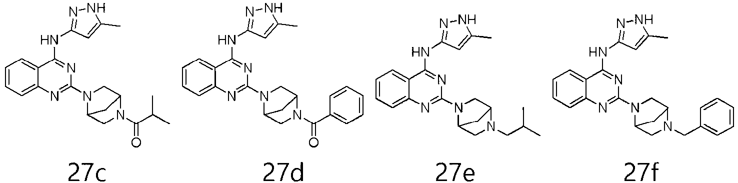

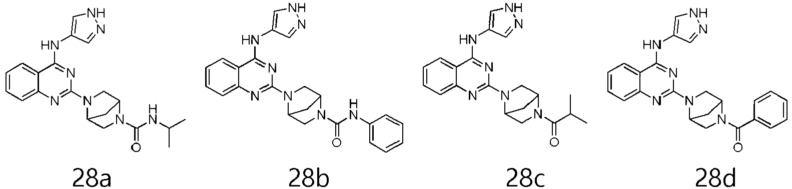

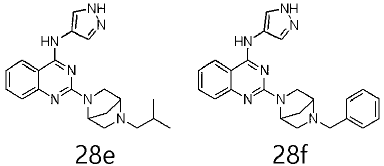

The compound, a stereoisomer thereof, a hydrate thereof, a solvate thereof, or a salt thereof, according to the present invention inhibit MASTL (microtubule-associated serine/threonine kinase-like) activity.

Additionally, in another aspect, the present invention provides a pharmaceutical composition for the prevention or treatment of cancer comprising a compound, a stereoisomer thereof, a hydrate thereof, a solvate thereof, or a salt thereof as an active ingredient.

The composition has the effect of inhibiting MASTL (microtubule-associated serine/threonine kinase-like).

Additionally, the cancers may include, but are not limited to, stomach cancer, breast cancer, uterine cancer, colon cancer, colorectal cancer, pancreatic cancer, liver cancer, or prostate cancer.

Additionally, in another aspect, the present invention provides a method for preventing or treating cancer comprising the step of administering a composition to a mammal other than a human.

Additionally, in another aspect, the present invention provides a health functional food composition for preventing or improving cancer comprising the compound, a stereoisomer thereof, a hydrate thereof, a solvate thereof, or a pharmaceutically acceptable salt thereof as an active ingredient.

PROTACs (Proteolysis Targeting Chimeras) technology is a type of Targeted Protein Degradation (TPD) technology that utilizes intracellular protein degradation systems to specifically degrade and remove disease-causing proteins. PROTACs are double-bond molecules that bind to disease-causing proteins, induce proteasomes to degrade the proteins, and ultimately lead to selective protein hydrolysis.

That is, the E3 enzyme (E3 ligase) can attach a label called ubiquitin to a target protein, and the target protein with this ubiquitin label is degraded by the proteasome. When the target protein ligand of the PROTAC compound binds to the target protein, the E3 enzyme is positioned very close to the target protein by the PROTAC compound, thereby creating an environment in which the target protein can be removed by the proteasome.

PROTACs consist of a binder that binds to a target protein, a binder that binds to an E3 ubiquitin ligase, and a linker that acts as an intermediate connecting the two. By binding to two proteins, PROTACs bring the target protein to an E3 ligase, inducing tagging-specifically ubiquitination-of the target protein for subsequent degradation by the proteasome. Because the target protein is degraded through binding, it offers the ease of access to a wide range of diseases, and because the target protein is degraded at the source, it can overcome the problem of resistance.

Ubiquitination involves three steps of activation, conjugation, and ligation performed by a ubiquitin-activating enzyme (E1), a ubiquitin-conjugating enzyme (E2), and a ubiquitin ligase (E3). As a result of this sequential cascade, ubiquitin is covalently bonded to a target protein. The ubiquitinated protein is eventually degraded by a proteasome.

Since the core of these PROTACs technology is the ability to accurately mediate the ubiquitination of target proteins through E3 enzymes and ligands, the discovery of E3 enzyme ligands and target protein ligands suitable for target proteins, as well as the design and synthesis of bifunctional compounds, are required.

In another aspect, the present invention provides a PROTAC compound or a pharmaceutically acceptable salt thereof comprising a target protein ligand, a linker, and an E3 enzyme ligand, wherein the target protein ligand is a compound represented by Formula 1.

The compound represented by Formula 1 according to the present invention can be used as a target protein ligand in a PROTAC compound comprising a target protein ligand, a linker, and an E3 enzyme ligand, or in a pharmaceutically acceptable salt thereof.

At this time, the target protein may preferably be a MASTL protein, and a PROTAC compound may bind to the MASTL protein and induce its degradation, thereby achieving a therapeutic effect on diseases of MASTL overexpression, and, for example, may have a preventive or therapeutic effect on breast cancer.

In the PROTAC compound according to the present invention, the compound of Formula 1 and the linker can be connected by a covalent bond.

In the PROTAC compound according to the present invention, the linker is covalently connected to the target protein ligand and the E3 enzyme ligand, respectively, thereby connecting the target protein ligand and the E3 enzyme ligand. A person skilled in the art will understand that some substituents may be detached during the covalent reaction process at the sites of the linker, the target protein ligand, and the E3 enzyme ligand that are covalently connected to each other.

Additionally, in the present invention, the pharmaceutical composition may include a pharmaceutically acceptable carrier or a pharmaceutically acceptable excipient in addition to the compound according to the present invention.

In the present invention, "pharmaceutically acceptable" means that it is commonly used in the pharmaceutical field and does not stimulate a living organism upon administration, nor does it impair the biological activity and properties of the administered compound.

Specifically, the carrier is one that is commonly used in formulations and may be, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, or mineral oil. Excipients such as cocoa butter and suppository wax, coloring agents, coating agents, sweeteners, flavoring agents, and fragrances may also be present in the composition.

The excipients include any and all solvents, inert diluents, dispersants and/or granulators, surfactants and/or emulsifiers, disintegrants, binders, preservatives, buffers, lubricants and/or oils suitable for the desired specific dosage form. Excipients such as cocoa butter and suppository waxes, coloring agents, coating agents, sweeteners, flavoring agents and fragrances may also be present in the composition.

Exemplary diluents include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dried starch, corn starch, powdered sugar, and combinations thereof.

Exemplary preservatives include, but are not limited to, antioxidants, chelating agents, antimicrobial preservatives, antifungal preservatives, alcohol preservatives, acidic preservatives, and other preservatives.

Exemplary antioxidants include, but are not limited to, alpha-tocopherol, ascorbic acid, ascorbyl palmitate, butylated hydroxyanisole, butylated hydroxytoluene, monothioglycerol, potassium metabisulfite, propionic acid, propyl gallate, sodium ascorbate, sodium bisulfite, sodium iodide, sodium metabisulfite, sodium nitrite, sodium sulfite, and sodium thiosulfate.

Exemplary chelating agents include ethylenediaminetetraacetic acid (EDTA) and its salts and hydrates (e.g., sodium edetate, disodium edetate, trisodium edetate, disodium calcium edetate, dipotassium edetate, etc.), citric acid and its salts and hydrates (e.g., citric acid monohydrate), fumaric acid and its salts and hydrates, malic acid and its salts and hydrates, phosphoric acid and its salts and hydrates, and tartaric acid and its salts and hydrates. Exemplary antimicrobial preservatives include, but are not limited to, benzalkonium chloride, benzetonium chloride, benzyl alcohol, bronopol, cetrimide, cetylpyridinium chloride, chlorhexidine, chlorobutanol, chlorocresol, chloroxylenol, cresol, ethyl alcohol, glycerin, hexatidine, imidourea, phenol, phenoxyethanol, phenylethyl alcohol, phenylmercury nitrate, propylene glycol, and thimerosal.

Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid, but are not limited thereto.

Exemplary alcohol preservatives include, but are not limited to, ethanol, polyethylene glycol, phenol, phenolic compounds, bisphenol, chlorobutanol, hydroxybenzoate, and phenylethyl alcohol.

Exemplary acidic preservatives include, but are not limited to, vitamin A, vitamin C, vitamin E, beta-carotene, citric acid, acetic acid, dehydroacetic acid, ascorbic acid, sorbic acid, and phytic acid.

Other preservatives include, but are not limited to, tocopherol, tocopherol acetate, deteroxime mesylate, cetrimide, butylated hydroxyanisole (BHA), butylated hydroxytoluene (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl.

Exemplary buffers include citrate buffer solution, acetate buffer solution, phosphate buffer solution, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentacarbonate, disaccharide, phosphoric acid, tricalcium phosphate, calcium hydroxyphosphate, potassium acetate, potassium chloride, potassium gluconate, potassium mixture, dipotassium phosphate, monopotassium phosphate, potassium phosphate mixture, sodium acetate, sodium bicarbonate, sodium chloride, sodium citrate, sodium lactate, disodium phosphate, monopotassium phosphate, sodium phosphate mixture, tromethamine, magnesium hydroxide, aluminum hydroxide, alginate, Pyrogen-free water, isotonic saline, Ringer's solution, ethyl alcohol, etc., and combinations thereof are included, but not limited to.

The composition of the present invention may be administered orally or parenterally. When administering parenterally, it may be administered intravenously, intra-arterially, subcutaneously, intramuscularly, intra-articularly, intra-synovially, intraretinally, intrahepatically, intralesionally, intracranially, or locally. Specifically, the composition of the present invention may be formulated as an injectable and administered intravenously but is not limited thereto.

Additionally, the composition may have various forms, including, but not limited to, liquid, suspension, paste, powder, concentrate, granulated powder for mixing to an appropriate concentration, or solid form, according to conventional methods. For example, the composition of the present invention may be prepared in the form of an injectable, a transdermal formulation, an intubation formulation, an oral formulation, or a rectal formulation.

Suitable dosages of the composition of the present invention can be prescribed in various ways depending on factors such as the formulation method, mode of administration, patient's age, body weight, sex, pathological condition, food, time of administration, route of administration, excretion rate, and response sensitivity.

Additionally, in the present invention, the health functional food composition can be easily utilized as a food effective for preventing or improving cancer, such as a main ingredient, auxiliary ingredient, food additive, functional food, or beverage.

Additionally, in the present invention, the health functional food composition may include a food-grade acceptable food additive in addition to the compound according to the present invention, and may further include a suitable carrier, excipient, and diluent commonly used in the manufacture of functional foods.

In this document, the term "food" refers to a natural product or processed product containing one or more nutrients, preferably one that has undergone a certain degree of processing to become ready for direct consumption, and includes, in the conventional sense, food, food additives, functional foods, and beverages.

The term "functional food" above refers to a group of foods to which added value has been imparted by using physical, biochemical, or biotechnological methods to enable the function of the food to act or manifest for a specific purpose, or to foods processed by designing them to sufficiently express in vivo regulatory functions regarding the regulation of biological defense rhythms, disease prevention, and recovery possessed by the food composition; specifically, it may be a health functional food. The above functional food may include food science-acceptable food additives and may further include appropriate carriers, excipients, and diluents commonly used in the manufacture of functional foods.

The term "beverage" above refers to a general term for anything consumed to quench thirst or enjoy taste, and includes functional beverages. Aside from containing the composition for the prevention and improvement of cancer as an essential ingredient in the indicated proportions, the beverage has no special restrictions on other ingredients and may contain various flavoring agents or natural carbohydrates, etc., as additional ingredients, just like ordinary beverages.

Foods to which the health functional food composition according to the present invention can be added include, for example, various types of food, beverages, chewing gum, tea, vitamin complexes, functional foods, etc. Additionally, in the present invention, food includes, but is not limited to, special nutritional foods (e.g., infant formula, baby food, etc.), processed meat products, fish products, tofu products, jelly products, noodles (e.g., ramen, noodles, etc.), bread products, health supplements, seasoning foods (e.g., soy sauce, soybean paste, red pepper paste, mixed sauce, etc.), sauces, confectionery products (e.g., snacks), candies, chocolates, chewing gum, ice cream, dairy products (e.g., fermented milk, cheese, etc.), other processed foods, kimchi, pickled foods (various types of kimchi, pickled vegetables, etc.), beverages (e.g., fruit beverages, vegetable beverages, soy milk, fermented beverages, etc.), and natural seasonings (e.g., ramen soup mix). The food, beverage, or food additive can be manufactured by conventional manufacturing methods.

Furthermore, in addition to what is described above, a food containing the health functional food composition having the cancer prevention or improvement effect of the present invention may contain various nutritional supplements, vitamins, minerals (electrolytes), flavoring agents such as synthetic flavoring agents and natural flavoring agents, coloring agents and fillers (cheese, chocolate, etc.), pectic acid and its salts, alginic acid and its salts, organic acids, protective colloidal thickeners, pH adjusters, stabilizers, preservatives, glycerin, alcohol, carbonating agents used in carbonated beverages, etc., and the ingredients may be used independently or in combination.

Hereinafter, examples of synthesis and experimental examples of the compound represented by Formula 1 of the present invention will be specifically described, but the present invention is not limited to the following examples.

### [Synthesis example]

The compound represented by Formula 1-1 according to the present invention can be prepared according to the Reaction Scheme 1, but is not limited thereto. 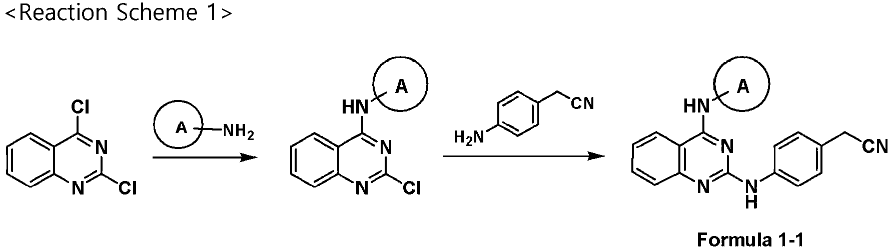 wherein, Ring A is the same as defined above.

### 2-chloro-M-(5-cyclopropyl-1G-pyrazol-3-yl)quinazolin-4-amine (2a)

To a stirring solution of 2,4-dichloro-quniazoline (4.00 g, 20.10 mmol) and DIPEA (2.63 mL, 15.07 mmol) in MeCN (50 mL) in a round bottom glass flask at room temperature, a solution of 5-cyclopropyl-1H-pyrazol-3-amine (2.72 g, 22.11 mmol) and DIPEA (2.63 mL, 15.07 mmol) in MeCN (50 mL) was added dropwise. After 24 hours an equal volume of water was added and the mixture stirred for 2 hours at room temperature. The product was recovered by filtration and washed in a sequential manner with approximately 100 mL of the cold MeCN and EtOAc. After drying, a white powder compound **2a** was recovered. (4.18 g, 73% yield.)

¹H NMR (300 MHz, DMSO-*d*₆) δ 12.35 (s, 1H), 10.79 (s, 1H), 8.64 (d, *J* = 8.3 Hz, 1H), 7.85 (t, *J* = 7.7 Hz, 1H), 7.69 (d, *J* = 8.3 Hz, 1H), 7.58 (t, *J* = 7.6 Hz, 1H), 6.50 (s, 1H), 4.04 (s, 1H), 3.31 (s, 1H), 1.96 (dt, *J* = 8.5, 3.6 Hz, 1H), 1.10 **-** 0.85 (m, 2H), 0.85 **-** 0.64 (m, 2H).

### 2-(4-((4-((5-cyclopropyl-1G-pyrazol-3-yl)amino)quinazolin-2-yl)amino)phenyl)acetonitrile (3a)

Compound 2a (1.50 g, 5.25 mmol) was dissolved in *tert-*butanol (15 mL), and a solution of 4-aminobenzonitrile (1.04 g, 7.87 mmol) in tert-butanol (5 mL) was added to the mixture. 37% HCl in water (0.100 mL) was added and the reaction was heated at 110°C while stirring. After 6 h, the solvent was evaporated. The product was recovered by filtration washed with MeOH to afford compound 3a as a white solid. (1.79 g, 89% yield.)

¹H NMR (300 MHz, DMSO-*d*₆) δ 11.52 (s, 1H), 10.58 (s, 1H), 8.67 (d, *J* = 8.3 Hz, 1H), 7.87 (t, *J* = 7.7 Hz, 1H), 7.64 (d, *J* = 8.3 Hz, 1H), 7.58 (d, *J* = 8.2 Hz, 2H), 7.50 (d, *J* = 7.7 Hz, 1H), 7.48 - 7.38 (m, 2H), 6.19 (s, 1H), 4.10 (s, 2H), 1.87 (td, *J* = 8.5, 4.3 Hz, 1H), 1.07 - 0.81 (m, 2H), 0.72 - 0.48 (m, 2H). MS (ESI) m/z calcd for C₂₂H₁₉N₇ [M⁺], 381.4 ; found, 382.2 [M⁺ + H⁺].

### 2-(4-((4-((1-isobutyl-3,5-dimethyl-1G-pyrazol-4-yl)amino)quinazolin-2-yl)amino)phenyl)acetonitrile (3b)

### 1) 2-chloro-N-(1-isobutyl-3,5-dimethyl-1H-pyrazol-4-yl)quinazolin-4-amine (2b)

To a stirring solution of 1-isobutyl-3,5-dimethyl-1*H*-pyrazol-4-amine (200 mg, 1.00 mmol) in MeCN (10 mL) in a round bottom glass flask at room temperature, 2,4-dichloro-quniazoline (295 mg, 1.48 mmol) and DIPEA (0.263 mL, 1.51 mmol) was added. After overnight an equal volume of water was added and the mixture stirred for 2 h at room temperature. The product was recovered by filtration and washed with water. After drying, a white solid compound **2b** was recovered. (345 mg, 104% yield.)

¹H NMR (300 MHz, DMSO-d₆) δ 9.78 (s, 1H), 8.47 (d, *J* = 8.0 Hz, 1H), 7.92 - 7.80 (m, 1H), 7.68 (d, *J* = 7.8 Hz, 1H), 7.61 (t, *J* = 7.6 Hz, 1H), 3.81 (d, *J* = 7.3 Hz, 2H), 2.12 (s, 4H), 2.03 (s, 3H), 0.89 (d, *J* = 6.7 Hz, 6H).

### 2) 3b

Compound 2b (70 mg, 0.21 mmol) was dissolved in *t*-butanol (2 mL), and a soluton of 4-aminobenzyl cyanide (42 mg, 0.32 mmol) in *t*-butanol (1 mL) was added to the mixture. 37% HCl in water (0.010 mL) was added and the reaction was heated at 110°C while stirring. After 4 h, a yellow solid compound 3b was recovered by filtration washing with ethyl acetate. (91 mg, 101% yield.)

¹H NMR (300 MHz, DMSO-d₆) δ 12.91 (s, 1H), 10.83 (s, 1H), 10.62 (s, 1H), 8.58 (d, *J* = 8.2 Hz, 1H), 7.91 (t, *J* = 7.5 Hz, 1H), 7.61 (d, *J* = 8.3 Hz, 1H), 7.56 (t, *J* = 7.7 Hz, 2H), 7.45 (d, *J* = 8.5 Hz, 2H), 7.22 (d, *J* = 8.3 Hz, 2H), 4.02 (s, 2H), 3.84 (d, *J* = 7.3 Hz, 2H), 2.25 **-** 2.12 (m, 2H), 2.12 (s, 3H), 2.04 (s, 3H), 0.88 (d, *J* = 6.7 Hz, 6H). ¹³C NMR (101 MHz, DMSO-d₆) δ 161.45, 151.75, 143.06, 139.64, 137.05, 136.28, 135.23, 128.96, 127.43, 125.49, 125.19, 119.65, 118.00, 114.62, 110.67, 56.08, 31.77, 29.60, 22.26, 20.15, 12.19, 9.84. MS (ESI) m/z calcd for C₂₅H₂₇N₇ [M⁺], 425.5 ; found, 426.5 [M⁺ + H⁺].

### 2-(4-((4-((1,3,5-trimethyl-1G-pyrazol-4-yl)amino)quinazolin-2-yl) amino)phenyl)acetonitrile (3c)

### 1) 2-chloro-N-(1,3,5-trimethyl-1H-pyrazol-4-yl)quinazolin-4-amine (2c)

To a stirring solution of 2,4-dichloro-quniazoline (100 mg, 0.50 mmol) and DIPEA (0.066 mL, 0.38 mmol) in MeCN (2 mL) in a round bottom glass flask at room temperature, a solution of 1,3,5-trimethyl-1*H*-pyrazol-4-amine (69 mg, 0.55 mmol) and DIPEA (0.066 mL, 0.38 mmol) in MeCN (2 mL) was added dropwise. After 4 hours an equal volume of water was added and the mixture stirred for 2 h at room temperature. The product was recovered by filtration and washed with water. After drying, a white solid compound **2c** was recovered. (109 mg, 75% yield.)

¹H NMR (300 MHz, DMSO-d₆) δ 9.77 (s, 1H), 8.47 (d, *J* = 8.3 Hz, 1H), 7.92 **-** 7.81 (m, 1H), 7.69 (d, *J* = 8.3 Hz, 1H), 7.62 (t, *J* = 7.6 Hz, 1H), 3.71 (s, 3H), 2.11 (s, 3H), 2.00 (s, 3H). 2) **3c**

Compound **2c** (100 mg, 0.35 mmol) was dissolved in t-butanol (1 mL), and a solution of 4-aminobenzyl cyanide (69 mg, 0.52 mmol) in t-butanol (1 mL) was added to the mixture. 37% HCl in water (0.015 mL) was added and the reaction was heated at 110°C while stirring. After overnight, a white solid compound **3a** was recovered by filtration washing with ethyl acetate. (133 mg, 100% yield.)

¹H NMR (300 MHz, DMSO-d₆) δ 10.84 (s, 1H), 10.64 (s, 1H), 8.60 (d, *J* = 8.2 Hz, 1H), 8.00 **-** 7.82 (m, 1H), 7.62 (d, *J* = 8.3 Hz, 1H), 7.56 (t, *J* = 7.6 Hz, 1H), 7.43 (d, *J* = 8.5 Hz, 2H), 7.26 (d, *J* = 8.3 Hz, 2H), 4.04 (s, 2H), 3.75 (s, 3H), 2.06 (d, *J* = 6.0 Hz, 6H), 1.11 (s, 1H). ¹³C NMR (101 MHz, DMSO-d₆) δ 161.36, 151.79, 142.50, 139.63, 136.94, 136.25, 135.51, 129.05, 127.76, 125.49, 125.24, 119.76, 118.02, 114.87, 110.73, 36.67, 31.78, 22.25, 11.90, 9.93. MS (ESI) m/z calcd for C₂₂H₂₁N₇ [M⁺], 383.5 ; found, 384.4 [M⁺ + H⁺].

### 2-(4-((4-((3,5-dimethyl-1G-pyrazol-4-yl)amino)quinazolin-2-yl)amino)phenyl)acetonitrile (3d)

### 1) 2-chloro-N-(3,5-dimethyl-1H-pyrazol-4-yl)quinazolin-4-amine (2d)

To a stirring solution of 2,4-dichloroquniazoline (100 mg, 0.50 mmol) and DIPEA (0.066 mL, 0.38 mmol) in MeCN (2 mL) in a round bottom glass flask at room temperature, a solution of 3,5-dimethyl-1*H*-pyrazol-4-amine (61 mg, 0.55 mmol) and DIPEA (0.066 mL, 0.38 mmol) in MeCN (2 mL) was added dropwise. After 2 hours an equal volume of water was added and the mixture stirred for 2 h at room temperature. The product was recovered by filtration and washed with water. After drying, a white solid compound 2d was recovered. (114 mg, 83% yield.)

¹H NMR (300 MHz, DMSO-d₆) δ 12.30 (s, 1H), 9.74 (s, 1H), 8.47 (d, *J* = 8.2 Hz, 1H), 7.90 **-** 7.82 (m, 1H), 7.72 **-** 7.65 (m, 1H), 7.65 **-** 7.56 (m, 1H), 4.03 (q, *J* = 7.1 Hz, 1H), 2.07 (s, 6H), 1.99 (s, 1H), 1.18 (t, *J* = 7.1 Hz, 1H).

### 2) 3d

Compound **2d** (70 mg, 0.26 mmol) was dissolved in *t*-butanol (2 mL), and a solution of 4-aminobenzyl cyanide (51 mg, 0.38 mmol) in *t*-butanol (1 mL) was added to the mixture. 37% HCl in water (0.010 mL) was added and the reaction was heated at 110°C while stirring. After 3 h, a white solid compound **3d** was recovered by filtration washing with ethyl acetate. (87 mg, 92% yield.)

¹H NMR (300 MHz, DMSO-d₆) δ 12.73 (s, 1H), 10.78 (s, 1H), 10.60 (s, 1H), 8.57 (d, *J* = 8.2 Hz, 1H), 7.91 (t, *J* = 7.7 Hz, 1H), 7.62 (d, *J* = 8.3 Hz, 1H), 7.56 (t, *J* = 7.7 Hz, 2H), 7.50 **-** 7.41 (m, 2H), 7.25 (d, *J* = 8.2 Hz, 2H), 4.03 (d, *J* = 2.9 Hz, 3H), 2.09 (s, 6H), 1.99 (s, 1H), 1.18 (t, *J* = 7.1 Hz, 1H). ¹³C NMR (101 MHz, DMSO-d₆) δ 170.81, 161.30, 151.80, 139.66, 137.07, 136.22, 129.06, 127.50, 127.50, 125.48, 125.20, 121.41, 119.71, 118.07, 114.56, 110.72, 60.23, 22.27, 21.24, 14.56, 11.01. MS (ESI) m/z calcd for C₂₁H₁₉N₇ [M⁺], 369.4 ; found, 370.4 [M⁺ + H⁺].

### 2-(4-((4-((3-methyl-1G-pyrazol-4-yl)amino)quinazolin-2-yl)amino)phenyl)acetonitrile (3e)

### 1) 2-chloro-N-(3-methyl-1H-pyrazol-4-yl)quinazolin-4-amine (2e)

To a stirring solution of 2,4-dichloro-quniazoline (100 mg, 0.50 mmol) and DIPEA (0.066 mL, 0.38 mmol) in MeCN (2 mL) in a round bottom glass flask at room temperature, a solution of 3-methy-1*H*-pyrazol-4-amine (53.68 mg, 0.55 mmol) and DIPEA (0.066 mL, 0.38 mmol) in MeCN (2 mL) was added dropwise. After 1 hours an equal volume of water was added and the mixture stirred for 2 h at room temperature. The product was recovered by filtration and washed with water. After drying, a white solid compound **2e** was recovered. (95 mg, 73% yield.)

¹H NMR (300 MHz, DMSO-*d*₆) δ 12.56 (s, 1H), 9.85 (s, 1H), 8.47 (d, *J* = 8.3 Hz, 1H), 7.86 (t, *J* = 7.4 Hz, 1H), 7.69 (d, *J* = 7.8 Hz, 1H), 7.61 (t, *J* = 7.7 Hz, 1H), 2.17 (s, 3H), 2.08 (s, 1H).

### 2) 3e

Compound **2e** (70 mg, 0.27 mmol) was dissolved in *t*-butanol (2 mL), and a solution of 4-aminobenzyl cyanide (53 mg, 0.40 mmol) in *t*-butanol (1 mL) was added to the mixture. 37% HCl in water (0.010 mL) was added and the reaction was heated at 110°C while stirring. After 1.5 h, a yellow solid compound **3e** was recovered by filtration washing with ethyl acetate. (47 mg, 49%).

¹H NMR (300 MHz, DMSO-d₆) δ 12.54 (s, 1H), 9.36 (d, *J* = 20.3 Hz, 2H), 8.32 (d, *J* = 8.2 Hz, 1H), 7.81 (d, *J* = 8.3 Hz, 2H), 7.68 (t, *J* = 7.9 Hz, 1H), 7.49 (d, *J* = 8.3 Hz, 1H), 7.29 (t, *J* = 7.6 Hz, 1H), 7.18 (d, *J* = 8.3 Hz, 2H), 3.94 (s, 2H), 2.18 (s, 3H), 1.06 (t, *J* = 7.0 Hz, 1H). ¹³C NMR (101 MHz, DMSO-d₆) δ 159.88, 156.42, 140.60, 133.69, 128.57, 128.51, 124.68, 123.74, 122.74, 119.99 (d, *J* = 6.9 Hz), 119.79, 111.90, 49.08, 22.20 (d, *J* = 7.1 Hz). MS (ESI) m/z calcd for C₂₀H₁₇N₇ [M⁺], 355.4 ; found, 356.3 [M⁺ + H⁺].

### 2-(4-((4-((1G-pyrazol-4-yl)amino)quinazolin-2-yl)amino)phenyl)acetonitrile (3f)

### 1) 2-chloro-N-(1H-pyrazol-4-yl)quinazolin-4-amine (2f)

To a stirring solution of 2,4-dichloro-quniazoline (100 mg, 0.50 mmol) and DIPEA (0.066 mL, 0.38 mmol) in MeCN (2 mL) in a round bottom glass flask at room temperature, a solution of 1*H*-pyrazol-4-amine (46 mg, 0.55 mmol) and DIPEA (0.066 mL, 0.38 mmol) in MeCN (2 mL) was added dropwise. After 1 hours an equal volume of water was added and the mixture stirred for 2 h at room temperature. The product was recovered by filtration and washed with water. After drying, a white solid compound **2f** was recovered. (102 mg, 83% yield.)

¹H NMR (300 MHz, DMSO-d₆) δ 12.82 (s, 1H), 10.52 (s, 1H), 8.47 (d, *J* = 8.2 Hz, 1H), 8.02 (s, 2H), 7.92 **-** 7.78 (m, 1H), 7.70 (d, *J* = 7.8 Hz, 1H), 7.64 (t, *J* = 7.3 Hz, 1H).

### 2) 3f

Compound **2f** (70 mg, 0.29 mmol) was dissolved in *t*-butanol (2 mL), and a solution of 4-aminobenzyl cyanide (56 mg, 0.43 mmol) in *t*-butanol (1 mL) was added to the mixture. 37% HCl in water (0.010 mL) was added and the reaction was heated at 110°C while stirring. After 1 h, a yellow solid compound **3f** was recovered by filtration washing with ethyl acetate. (110 mg, 113% yield.)

¹H NMR (300 MHz, DMSO-d₆) δ 12.68 (s, 1H), 11.38 (s, 1H), 10.64 (s, 1H), 8.63 (d, *J* = 8.2 Hz, 1H), 8.03 (s, 2H), 7.86 (t, *J* = 7.7 Hz, 1H), 7.66 (d, *J* = 8.3 Hz, 1H), 7.59 **-** 7.49 (m, 3H), 7.47 (d, *J* = 8.3 Hz, 2H), 4.14 (s, 2H). ¹³C NMR (101 MHz, DMSO-d₆) δ 156.96, 152.68, 139.41, 136.19, 135.61, 129.53, 125.37, 125.20, 124.91, 120.41, 119.68, 118.07, 111.18, 67.42, 31.77, 22.54. MS (ESI) m/z calcd for C₁₉H₁₅N₇ [M⁺], 341.4 ; found, 342.3 [M⁺ + H⁺].

### 2-(4-((4-((2-(isopropylsulfonyl)phenyl)amino)quinazolin-2-yl)amino)phenyl)acetonitrile (3g)

### 1) 2-chloro-N-(2-(isopropylsulfonyl)phenyl)quinazolin-4-amine (2g)

To a dried flask, sodium hydride (30 mg, 1.26 mmol) was dissolved in anhydrous DMF (1 mL) and 2-(isopropylsulfonyl)aniline (100 mg, 0.50 mmol) was added at 0°C while stirring. After 30 min, 2, 4-dichloroquinazoline (110 mg, 0.55 mmol) in DMF (2 mL) was added to the reaction mixture and heated at room temperature for 2.5 h. The reaction mixture was dissolved in ethyl acetate and washed with ammonium chloride 3 times. The combined organic layers were dried over Na₂SO₄ and the solvent was removed under vacuum. The crude mixture was purified using column chromatography (EtOAc: hexane). The solvent was evaporated, the solid filtered with hexane to afford compound as a light green solid compound **2g.** (135 mg, 74% yield.)

¹H NMR (300 MHz, Chloroform-*d*) δ 10.70 (s, 1H), 8.98 (dd, *J* = 8.5, 1.1 Hz, 1H), 8.07 (d, *J* = 8.3 Hz, 1H), 8.00 **-** 7.83 (m, 3H), 7.87 **-** 7.75 (m, 1H), 7.73 **-** 7.62 (m, 1H), 7.41 **-** 7.29 (m, 1H), 3.26 (p, *J* = 6.8 Hz, 1H), 1.33 (d, *J* = 6.8 Hz, 6H).

### 2) 3g

Compound **2g** (100 mg, 0.28 mmol) was dissolved in *t*-butanol (2 mL), and a solution of 4-aminobenzyl cyanide (55 mg, 0.42 mmol) in *t*-butanol (1 mL) was added to the mixture. 37% HCl in water (0.015 mL) was added and the reaction was heated at 110°C while stirring. After 2 h, a yellow solid compound **3g** was recovered by filtration washing with ethyl acetate. (122 mg, 97% yield.)

¹H NMR (400 MHz, DMSO-d₆) δ 11.37 (s, 1H), 10.49 (s, 1H), 8.50 (d, *J* = 8.2 Hz, 1H), 8.02 (d, *J* = 7.9 Hz, 1H), 8.00 **-** 7.83 (m, 3H), 7.78 **-** 7.65 (m, 2H), 7.61 (t, *J* = 7.7 Hz, 1H), 7.37 **-** 7.22 (m, 2H), 7.20 **-** 7.08 (m, 2H), 3.97 (s, 2H), 3.47 **-** 3.39 (m, 1H), 1.02 (d, *J* = 6.7 Hz, 6H). ¹³C NMR (101 MHz, DMSO-d₆) δ 161.95, 151.53, 136.80, 136.68, 136.54, 135.65, 131.93, 131.71, 129.00, 128.79, 127.75, 125.81, 125.24, 122.02, 119.66, 118.15, 110.69, 54.59, 22.23, 15.13. MS (ESI) m/z calcd for C₂₅H₂₃N₅O₂S [M⁺], 457.6 ; found, 458.5 [M⁺ + H⁺].

### 4-((2-((4-(cyanomethyl)phenyl)amino)quinazolin-4-yl)amino)-M,M-dimethylbenzenesulfonamide (3h)

### 1) 4-((2-chloroquinazolin-4-yl)amino)-N,N-dimethylbenzenesulfonamide (2h)

To a dried flask, sodium hydride (44 mg, 5.53 mmol) was dissolved in anhydrous DMF (5 mL) and *N,N*-Dimethyl 4-aminobenzenesulfonamide (221 mg, 1.11 mmol) was added at 5°C while stirring. After 5 min, 2,4-dichloroquinazoline (200 mg, 1.01 mmol) in DMF (2 mL) was added dropwise to the reaction mixture and heated at room temperature for 2.5 h. The reaction mixture was dissolved in ethyl acetate and washed with ammonium chloride 3 times. The combined organic layers were dried over Na₂SO₄ and the solvent was removed under vacuum. The crude mixture was purified using column chromatography (25% EtOAc: hexane) to obtain compound **2h.** (160 mg, 44% yield.)

¹H NMR (300 MHz, DMSO-d₆) δ 10.45 (s, 1H), 8.64 (d, *J* = 8.3 Hz, 1H), 8.23-8.11 (m, 2H), 8.01-7.89 (m, 1H), 7.88-7.66 (m, 4H), 2.65 (s, 6H). MS (ESI) m/z calcd C₁₆H₁₅ClN₄O₂S [M⁺], 362.1 ; found, 363.3 [M⁺ + H⁺].

### 2) 3h

Compound **2h** (88 mg, 0.24 mmol) was dissolved in *t*-butanol (3 mL), and a solution of 4-aminobenzyl cyanide (48 mg, 0.36 mmol) in *t*-butanol (0.5 mL) was added to the mixture. 37% HCl in water (0.015 mL) was added and the reaction was heated at 110°C while stirring. After 16 h, the solvent was evaporated, dissolved in EtOAc and washed with water. The combined organic layter was dried over Na₂SO₄ and purified by column chromatography (2% DCM/MeOH) to affrod compound **3h.** (52 mg, 47% yield.)

¹H NMR (300 MHz, DMSO-d₆) δ 9.93 (s, 1H), 9.39 (s, 1H), 8.45 (d, *J* = 8.2 Hz, 1H), 8.34 (d, *J* = 8.8 Hz, 2H), 7.95 (d, *J* = 8.4 Hz, 2H), 7.82-7.67 (m, 3H), 7.62-7.52 (m, 1H), 7.41-7.29 (m, 1H), 7.27 (d, *J* = 8.5 Hz, 2H), 3.97 (s, 2H), 2.65 (s, 6H). MS (ESI) m/z calcd C₂₄H₂₂N₆O₂S [M⁺], 458.2 ; found, 459.4 [M⁺ + H⁺].

### 2-(4-((4-((6-chloropyridazin-3-yl)amino)quinazolin-2-yl)amino)phenyl)acetonitrile (3i)

### 1) 2-chloro-N-(6-chloropyridazin-3-yl)quinazolin-4-amine (2i)

To a solution of 3-amino-6-chloropyridazine (70 mg, 0.54 mmol) in MeCN (5 mL), sodium *tert*-butoxide (68 mg, 0.70 mmol) was added at 0°C under nitrogen gas. After a few minutes, 2, 4-dichloroquinazoline (118 mg, 0.59 mmol) was added. The reaction mixture was stirred at room temperature for 3 h. The solvent was evaporated, the crude was dissolved in DCM and washed with water. The aqueous layer was extracted with DCM 2 times. The combined organic layer was dried over Na₂SO₄.The crude was purified with column chromatography. (60% Hex/EA) to afford a yellow solid product compound **2i.** (70 mg, 44% yield.)

¹H NMR (300 MHz, DMSO-d₆) δ 11.62 (s, 1H), 8.76 (d, *J* = 8.3 Hz, 1H), 8.54 (d, *J* = 9.4 Hz, 1H), 8.03 (d, *J* = 9.4 Hz, 1H), 7.97 (t, *J* = 7.6 Hz, 1H), 7.81 (d, *J* = 8.3 Hz, 1H), 7.70 (t, *J* = 7.6 Hz, 1H).

### 2) 3i

Compound **2i** (60 mg, 0.21 mmol) was dissolved in *t*-butanol (2 mL), and a solution of 4-aminobenzyl cyanide (41 mg, 0.31 mmol) in *t*-butanol (1 mL) was added to the mixture. 37% HCl in water (0.010 mL) was added and the reaction was heated at 110°C while stirring. After overnight, a yellow solid compound **3i** was recovered by filtration washing with ethyl acetate. (66 mg, 83% yield.)

¹H NMR (300 MHz, DMSO-d₆) δ 12.01 (s, 1H), 10.62 (s, 1H), 8.73 (d, *J* = 8.2 Hz, 1H), 8.43 (s, 1H), 7.94 (t, *J* = 8.1 Hz, 2H), 7.72 (d, *J* = 8.3 Hz, 1H), 7.62 **-** 7.48 (m, 3H), 7.40 (d, *J* = 8.2 Hz, 2H), 4.07 (s, 2H). ¹³C NMR (101 MHz, DMSO-d₆) δ 160.52, 155.25, 153.64, 152.45, 136.75, 136.34, 130.09, 129.29, 128.89, 127.36, 125.58, 125.44, 124.07, 119.76, 111.14, 22.46. MS (ESI) m/z calcd for C₂₀H₄ClN₇ [M⁺], 387.8 ; found, 388.4 [M⁺ + H⁺].

### 2-(4-((4-((4-oxo-4,5-dihydrothiazol-2-yl)amino)quinazolin-2-yl)amino)phenyl)acetonitrile (3j)

### 1) 2-((2-chloroquinazolin-4-yl)amino)thiazol-4(5H)-one (2j)

To a dried flask, sodium hydride (221 mg, 5.53 mmol) was dissolved in anhydrous DMF (15 mL) and 2-amino-1,3-thiazolin-4-one (641.88 mg, 5.53 mmol) was added at 5°C while stirring. After 30 min, 2,4-dichloroquinazoline (1.0 g, 5.02 mmol) in DMF (2 mL) was added dropwise to the reaction mixture and heated at room temperature for 2.5 h. The reaction mixture was dissolved in ethyl acetate and washed with ammonium chloride 3 times. The combined organic layers were dried over Na₂SO₄ and the solvent was removed under vacuum. The crude mixture was purified using column chromatography (25% EtOAc: hexane) to obtain compound **2j.** (555 mg, 40% yield.)

¹H NMR (300 MHz, DMSO-d₆) δ 12.62 (s, 1H), 8.39 (dd, *J* = 8.3, 1.4 Hz, 1H), 8.01 (ddd, *J* = 8.5, 6.9, 1.5 Hz, 1H), 7.93(m, 1H), 7.73 (ddd, *J* = 8.2, 6.9, 1.2 Hz, 1H), 4.07 (s, 2H). MS (ESI) m/z calcd C₁₁H₇ClN₄OS [M⁺], 278.0 ; found, 279.2 [M⁺ + H⁺].

### 2) 3j

Compound **2j** (70 mg, 0.25 mmol) was dissolved in *t*-butanol (3 mL), and a solution of 4-aminobenzyl cyanide (50 mg, 0.38 mmol) in *t*-butanol (0.5 mL) was added to the mixture. 37% HCl in water (0.012 mL) was added and the reaction was heated at 110°C while stirring. After 16 h, the solvent was evaporated, dissolved in EtOAc and washed with water. The combined organic layter was dried over Na₂SO₄ and purified by column chromatography (50% Hex/EtOAc) to afford compound **3j.** (76 mg, 84% yield.)

¹H NMR (400 MHz, DMSO-d₆) δ 12.35 (s, 1H), 9.41 (s, 1H), 8.24-8.13 (m, 1H), 8.07-7.95 (m, 2H), 7.82-7.71 (m, 1H), 7.68-7.56 (m, 1H), 7.40-7.24 (m, 3H), 4.05 (s, 2H), 3.98 (s, 2H). MS (ESI) m/z calcd C₁₉H₁₄N₆OS [M⁺], 374.1 ; found, 375.3 [M⁺ + H⁺].

### 2-(4-((4-((2-fluorophenyl)amino)quinazolin-2-yl)amino)phenyl)acetonitrile (3k)

### 1) 2-chloro-N-(2-fluorophenyl)quinazolin-4-amine (2k)

To a solution of 2,4-dichloroquinazoline (138 mg, 0.69 mmol) and 2-fluoroaniline (0.061 mL, 0.63 mmol) in THF (5 mL), sodium *tert*-butoxide (121 mg, 1.26 mmol) was added. The reaction mixture was stirred at room temperature for 2 h. The solvent was evaporated, the crude was dissolved in ethyl acetate and washed with water. The aqueous layer was extracted with ethyl acetate 2 times. The combined organic layer was dried over Na₂SO₄. The crude was purified by filtration to afford a white solid product compound **2k.** (78 mg, 45% yield.)

¹H NMR (400 MHz, DMSO) δ 10.31 (s, 1H), 8.50 (d, *J* = 8.2 Hz, 1H), 7.92 (t, *J* = 7.8 Hz, 1H), 7.74 (d, *J* = 8.4 Hz, 1H), 7.67 (t, *J* = 7.8 Hz, 1H), 7.56 (t, *J* = 7.8 Hz, 1H), 7.47 **-** 7.34 (m, 2H), 7.34 **-** 7.25 (m, 1H).

### 2) 3k

A mixture of 2-chloro-*N*-(2-fluorophenyl)quinazolin-4-amine (50 mg, 0.18 mmol) and 2-(4-aminophenyl)acetonitrile (36 mg, 0.27 mmol) in ethanol (2 mL) was stirred at 120°C in a sealed vial for 4 h. The reaction mixture was cooled and solvent was evaporated. The crude was purified by filtration washed with MeOH to afford a beige solid compound **3k.** (34 mg, 51% yield.)

¹H NMR (400 MHz, DMSO) δ 12.98 (s, 1H), 11.29 (s, 1H), 10.52 (s, 1H), 8.58 (d, *J* = 8.2 Hz, 1H), 7.94 (t, *J* = 7.9 Hz, 1H), 7.66 (d, *J* = 8.4 Hz, 1H), 7.58 (t, *J* = 7.9 Hz, 2H), 7.53 **-** 7.41 (m, 2H), 7.41 **-** 7.30 (m, 3H), 7.19 (d, *J* = 8.0 Hz, 2H), 3.98 (s, 2H).

### 2-(4-((4-((5-cyclopropyl-1,3,4-thiadiazol-2-yl)amino)quinazolin-2-yl)amino)phenyl)acetonitrile (3l)

### 1) N-(2-chloroquinazolin-4-yl)-5-cyclopropyl-1,3,4-thiadiazol-2-amine (2l)

To a solution of 2,4-dichloroquinazoline (100 mg, 0.50 mmol) and 5-cyclopropyl-1,3,4-thiadiazol-2-amine (78 mg, 0.55 mmol) in 1,4-dioxane (4 mL), cesium carbonate (327 mg, 1.01 mmol) was added. The reaction mixture was stirred at room temperature for 2 h. The solvent was evaporated, the crude was dissolved in ethyl acetate and washed with water. The aqueous layer was extracted with ethyl acetate 2 times. The combined organic layer was dried over Na₂SO₄. The crude was purified by filtration to afford a white solid product compound **2l.** (84 mg, 55% yield.)

¹H NMR (300 MHz, DMSO) δ 8.58 (s, 1H), 7.93 (ddd, *J* = 8.3, 6.9, 1.4 Hz, 1H), 7.79 (d, *J* = 8.3 Hz, 1H), 7.66 (td, *J* = 8.3, 1.3 Hz, 1H), 2.43 (td, *J* = 8.3, 4.2 Hz, 1H), 1.23 **-** 1.11 (m, 2H), 1.10 **-** 0.99 (m, 2H).

### 2) 3l

A mixture of *N*-(2-chloroquinazolin-4-yl)-5-cyclopropyl-1,3,4-thiadiazol-2-amine (50 mg, 0.17 mmol) and 2-(4-aminophenyl)acetonitrile (33 mg, 0.25 mmol) in ethanol (2 mL) was stirred at 120°C in a sealed vial for 2 h. The reaction was cooled and solvent was evaporated. The crude was purified by filtration washed with methanol to afford a yellow solid product compound **3l.** (47 mg, 71% yield.)

¹H NMR (400 MHz, DMSO) δ 10.47 (s, 1H), 8.37 (d, *J* = 8.1 Hz, 1H), 7.86 (t, *J* = 7.8 Hz, 1H), 7.66 (d, *J* = 8.3 Hz, 1H), 7.61 **-** 7.39 (m, 5H), 4.15 (s, 2H), 2.29 **-** 2.18 (m, 1H), 1.25 **-** 1.13 (m, 2H), 0.78 (s, 2H).

### sdqs-butyl (3-((2-((4-(cyanomethyl)phenyl)amino)quinazolin-4-yl) amino)phenyl)carbamate (3m)

### 1) tert-butyl (3-((2-chloroquinazolin-4-yl)amino)phenyl)carbamate (2m)

To a stirring solution of 2,4-dichloroquniazoline (105 mg, 0.53 mmol) and *tert*-butyl (3-aminophenyl)carbamate (100 mg, 0.48 mmol) in MeCN (5 mL) in a round bottom glass flask at room temperature, DIPEA (0.126 mL, 0.72 mmol) was added dropwise. The reaction mixture was heated at 45 °C and stirred. After 2 days an equal volume of water was added and 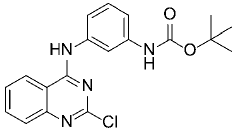 extracted with EtOAc. The combined organic layer was washed with brine and dried over anhydrous Na₂SO₄. After filtration, the crude was purified by column chromatography (30% Hex/EA) to afford a white solid product compound **2m.** (151 mg, 85% yield.)

¹H NMR (300 MHz, DMSO) δ 10.22 (s, 1H), 9.49 (s, 1H), 8.60 (d, *J* = 8.4 Hz, 1H), 8.00 **-** 7.77 (m, 2H), 7.72 (d, *J* = 8.2 Hz, 1H), 7.65 (t, *J* = 7.6 Hz, 1H), 7.48 (d, *J* = 7.0 Hz, 1H), 7.30 (t, *J* = 8.0 Hz, 1H), 7.22 (d, *J* = 8.4 Hz, 1H), 1.50 (s, 9H).

### 2) 3m

A mixture of *tert*-butyl (3-((2-chloroquinazolin-4-yl)amino)phenyl)carbamate (100 mg, 0.27 mmol) and 2-(4-aminophenyl)acetonitrile (53 mg, 0.40 mmol) in ethanol (3 mL) was stirred at 120°C in a sealed vial for 4 h. The reaction was cooled and solvent was evaporated. The crude was purified by filtration washed with EtOAc to afford product compound **3m.** (131 mg, 104% yield.)

¹H NMR (400 MHz, DMSO) δ 11.15 (s, 1H), 10.57 (s, 1H), 9.58 (s, 1H), 8.62 (d, *J* = 8.2 Hz, 1H), 7.91 (t, *J* = 7.8 Hz, 1H), 7.86 (s, 1H), 7.63 (d, *J* = 8.4 Hz, 1H), 7.56 (d, *J* = 7.6 Hz, 1H), 7.54 **-** 7.47 (m, 2H), 7.36 **-** 7.20 (m, 5H), 4.01 (s, 2H), 1.48 (s, 9H).

### 2-(4-((4-((3-aminophenyl)amino)quinazolin-2-yl)amino)phenyl)acetonitrile (3n)

Compound 3m (80 mg, 0.17 mmol) was dissolved in DCM (2 mL). TFA (0.5 mL) was dropwised to the solution and stirred at room temperature for 12 h. The solvent was evaporated and DCM was added repeat 2 times. The solvent was evaporated and diethyl ether was added repeat several times. The solvent was evaporated, the crude was dissolved in EtOAc and washed with saturated NaHCO₃ solution 2 times. The combined organic layer was dried over Na₂SO₄. The crude was purified by prep TLC (DCM 92: MeOH 7: NH₄OH 1) to afford light orange solid product compound **3n.** (30 mg, 48% yield.)

¹H NMR (300 MHz, DMSO) δ 9.42 (s, 1H), 9.14 (s, 1H), 8.38 (dd, *J* = 8.3, 1.4 Hz, 1H), 7.91 (d, *J* = 8.5 Hz, 2H), 7.74 **-** 7.59 (m, 1H), 7.48 (dd, *J* = 8.4, 1.2 Hz, 1H), 7.31 **-** 7.17 (m, 3H), 7.13 (s, 1H), 7.09 **-** 6.98 (m, 2H), 6.46 **-** 6.37 (m, 1H), 5.12 (s, 2H), 3.94 (s, 2H).

### 2-(4-((4-((1G-pyrazol-3-yl)amino)quinazolin-2-yl)amino)phenyl)acetonitrile (3o)

A mixture of 2-chloro-*N*-(1*H*-pyrazol-3-yl)quinazolin-4-amine (80 mg, 0.33 mmol) and 2-(4-aminophenyl)acetonitrile (65 mg, 0.49 mmol) in ethanol (4 mL) was stirred at 120°C in a sealed vial for 3 h. The reaction was cooled and solvent was evaporated. The crude was purified by filtration washed with EtOAc to afford a beige solid compound **3o.** (53 mg, 48% yield.)

¹H NMR (300 MHz, DMSO) δ 13.05 (s, 1H), 11.60 (s, 1H), 10.67 (s, 1H), 8.68 (d, *J* = 8.3 Hz, 1H), 7.88 (d, *J* = 7.7 Hz, 1H), 7.73 (d, *J* = 2.4 Hz, 1H), 7.65 (d, *J* = 8.3 Hz, 1H), 7.58 (d, *J* = 8.2 Hz, 2H), 7.51 (t, *J* = 7.7 Hz, 1H), 7.40 (d, *J* = 8.2 Hz, 2H), 6.56 (s, 1H), 4.08 (s, 2H). ¹³C NMR (101 MHz, DMSO) δ 158.93, 152.12, 145.54, 139.86, 136.23, 136.20, 130.17, 129.35, 128.94, 125.72, 124.72, 124.63, 119.83, 118.10, 110.57, 100.09, 22.41. MS (ESI) m/z calcd for C₁₉H₁₅N₇ [M⁺], 341.1 ; found, 342.1 [M⁺ + H⁺].

### 2-(4-((4-((5-methyl-1G-pyrazol-3-yl)amino)quinazolin-2-yl)amino)phenyl)acetonitrile (3p)

2-chloro-*N*-(5-methyl-1*H*-pyrazol-3-yl)quinazolin-4-amine (28 mg, 0.11 mmol) and 2-(4-aminophenyl)acetonitrile (21 mg, 0.16 mmol) were dissolved in *tert*-butanol (2 mL). 37% HCl in water (0.010 mL) was added and the reaction was heated at 110°C while stirring. After 5 h, the solvent was evaporated and product was recovered by filtration washed with EtOAc to afford light orange solid compound **3p.** (35 mg, 91%).

¹H NMR (400 MHz, DMSO) δ 11.52 (s, 1H), 10.54 (s, 1H), 8.66 (d, *J* = 8.3 Hz, 1H), 7.87 (t, *J* = 7.7 Hz, 1H), 7.64 (d, *J* = 8.3 Hz, 1H), 7.57 (d, *J* = 8.1 Hz, 2H), 7.50 (t, *J* = 7.7 Hz, 1H), 7.42 (d, *J* = 8.1 Hz, 2H), 6.30 (s, 1H), 4.09 (s, 2H), 2.23 (s, 3H). ¹³C NMR (101 MHz, DMSO) δ 158.62, 152.29, 145.88, 139.63, 136.39, 136.07, 129.84, 129.23, 128.96, 125.46, 125.08, 124.66, 119.73, 118.04, 110.80, 99.42, 22.42, 22.38, 11.17. MS (ESI) m/z calcd for C₂₀H₁₇N₇ [M⁺], 355.2 ; found, 356.2 [M⁺ + H⁺].

### 2-(4-((4-((5-bromo-1G-pyrazol-3-yl)amino)quinazolin-2-yl)amino)phenyl)acetonitrile (3q)

A mixture of *N*-(5-bromo-1*H*-pyrazol-3-yl)-2-chloroquinazolin-4-amine (32 mg, 0.10 mmol) and 2-(4-aminophenyl)acetonitrile (20 mg, 0.15 mmol) in ethanol (3 mL) was stirred at 120°C in a sealed vial for 2 h. The reaction was cooled and solvent was evaporated. The crude was purified by filtration washed with methanol to afford an ivory solid compound **3q.** (34 mg, 81% yield.)

¹H NMR (300 MHz, DMSO) δ 11.90 (s, 1H), 10.77 (s, 1H), 8.68 (d, *J* = 8.3 Hz, 1H), 7.89 (t, *J* = 7.8 Hz, 1H), 7.75 - 7.65 (m, 1H), 7.59 (d, *J* = 8.1 Hz, 2H), 7.52 (t, *J* = 7.7 Hz, 1H), 7.45 (d, *J* = 8.2 Hz, 2H), 6.56 (s, 1H), 4.11 (s, 2H). ¹³C NMR (101 MHz, DMSO) δ 158.47, 152.72, 136.29, 136.18, 129.51, 129.15, 125.50, 124.90, 124.62, 119.61, 119.01, 110.77, 22.54. MS (ESI) m/z calcd for C₁₉H₁₄BrN₇ [M⁺], 419.1 ; found, 420.1 [M⁺ + H⁺].

### 2-(4-((4-((5-methoxy-1G-pyrazol-3-yl)amino)quinazolin-2-yl)amino)phenyl)acetonitrile (3r)

A mixture of 2-chloro-*N*-(5-methoxy-1*H*-pyrazol-3-yl)quinazolin-4-amine (36 mg, 0.13 mmol) and 2-(4-aminophenyl)acetonitrile (26 mg, 0.20 mmol) in ethanol (3 mL) was stirred at 120°C in a sealed vial for 2 h. The reaction was cooled and solvent was evaporated. The crude was purified by column chromatography (5% DCM/MeOH) to afford an ivory solid compound **3r.** (39 mg, 81% yield.)

¹H NMR (400 MHz, DMSO) δ 12.35 (s, 1H), 10.60 (s, 1H), 9.85 (s, 1H), 8.30 (dd, *J* = 8.4, 1.4 Hz, 1H), 7.95 (d, *J* = 8.6 Hz, 2H), 7.74 - 7.66 (m, 1H), 7.58 (dd, *J* = 8.4, 1.2 Hz, 1H), 7.38 - 7.23 (m, 3H), 5.55 (d, *J* = 2.3 Hz, 1H), 3.98 (s, 2H), 3.82 (s, 3H). ¹³C NMR (101 MHz, DMSO) δ 162.52, 156.95, 155.92, 151.70, 140.89, 133.88, 128.95, 126.55, 123.76, 123.32, 123.05, 120.05, 118.92, 111.60, 78.00, 55.75, 22.28. MS (ESI) m/z calcd for C₂₀H₁₇N₇O [M⁺], 371.2 ; found, 372.1 [M⁺ + H⁺].

### 2-(4-((4-((1G-indazol-3-yl)amino)quinazolin-2-yl)amino)phenyl)acetonitrile (3s)

A mixture of 2-chloro-*N*-(1*H*-indazol-3-yl)quinazolin-4-amine (50 mg, 0.17 mmol) and 2-(4-aminophenyl)acetonitrile (34 mg, 0.25 mmol) in ethanol (3 mL) was stirred at 120°C in a sealed vial for 2 h. The reaction was cooled and solvent was evaporated. The crude was purified by filtration washed with EtOAc to afford yellow solid compound **3s.** (27 mg, 41% yield.)

¹H NMR (300 MHz, DMSO) δ 13.26 (s, 1H), 11.75 (s, 1H), 10.58 (s, 1H), 8.68 (d, *J* = 8.2 Hz, 1H), 7.96 (d, *J* = 7.6 Hz, 1H), 7.72 (d, *J* = 8.2 Hz, 1H), 7.69 - 7.54 (m, 3H), 7.51 - 7.39 (m, 1H), 7.20 - 7.02 (m, 3H), 6.83 (d, *J* = 8.2 Hz, 2H), 3.88 (s, 2H). ¹³C NMR (101 MHz, DMSO) δ 161.79, 151.61, 141.69, 140.31, 138.81, 136.60, 136.21, 128.42, 127.44, 127.25, 125.76, 125.15, 122.23, 121.16, 120.98, 119.53, 118.28, 118.04, 111.18, 110.63, 22.19. MS (ESI) m/z calcd for C₂₃H₁₇N₇ [M⁺], 391.2 ; found, 392.2 [M⁺ + H⁺].

### 2-(4-((4-((2-(benzyloxy)phenyl)amino)quinazolin-2-yl)amino)phenyl)acetonitrile (3t)

### 1) N-(2-(benzyloxy)phenyl)-2-chloroquinazolin-4-amine (2t)

To a stirring solution of 2,4-dichloro-quniazoline (300 mg, 1.51 mmol) and 2-(benzyloxy)aniline (330 mg, 1.66 mmol) was dissolved in MeCN (10 mL). DIPEA (0.395 mL, 2.26 mmol) was added in a round bottom glass flask at room temperature. After overnight, the solvent was evaporated. The crude was dissolved in EtOAc and washed with water. The combined organic layer was dried over Na₂SO₄. The crude was purified by column chromatography (10% Hex/EA) to afford light brown solid product compound **2t** (120 mg, 22% yield.)

¹H NMR (400 MHz, DMSO) δ 10.04 (s, 1H), 8.46 (dd, *J* = 8.4, 1.3 Hz, 1H), 7.87 (td, *J* = 8.3, 1.3 Hz, 1H), 7.71 (dd, *J* = 8.3, 1.2 Hz, 1H), 7.62 (td, *J* = 8.3, 1.3 Hz, 1H), 7.47 (dd, *J* = 7.7, 1.7 Hz, 1H), 7.36 - 7.21 (m, 6H), 7.18 (dd, *J* = 8.4, 1.3 Hz, 1H), 7.06 (td, *J* = 7.6, 1.3 Hz, 1H), 5.18 (s, 2H).

### 2) 3t

A mixture of *N*-(2-(benzyloxy)phenyl)-2-chloroquinazolin-4-amine (70 mg, 0.19 mmol) and 2-(4-aminophenyl)acetonitrile (38 mg, 0.29 mmol) in ethanol (3 mL) was stirred at 120°C in a sealed vial for 2 h. The reaction was cooled and solvent was evaporated. The crude was purified by filtration washed with EtOAc to afford an ivory solid compound **3t.** (82 mg, 98% yield.)

¹H NMR (300 MHz, DMSO) δ 11.08 (s, 1H), 10.49 (s, 1H), 8.55 (d, *J* = 8.2 Hz, 1H), 7.92 (t, *J* = 7.7 Hz, 1H), 7.64 (d, *J* = 8.2 Hz, 1H), 7.57 (t, *J* = 7.7 Hz, 1H), 7.53 - 7.40 (m, 2H), 7.39 - 7.28 (m, 3H), 7.28 - 7.22 (m, 2H), 7.22 - 7.07 (m, 6H), 5.13 (s, 2H), 3.98 (s, 2H).

### 2-(4-((4-((2-hydroxyphenyl)amino)quinazolin-2-yl)amino)phenyl)acetonitrile (3u)

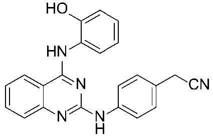

2-(4-((4-((2-(benzyloxy)phenyl)amino)quinazolin-2-yl)amino)phenyl)acetonitrile (50 mg, 0.11 mmol) and 10% Pd/C (10 mg) were dissolved in methanol (2 mL). The mixture was stirred under H₂ gas for 3 h. The crude was filtered through celite and the filtrate was evaporated. The crude was purified by column chromatography (3% DCM/MeOH) and filtration washed with petroleum ether to afford a light grey solid compound **3u.** (27 mg, 68% yield.)

¹H NMR (300 MHz, DMSO) δ 9.63 (s, 1H), 9.22 (s, 1H), 9.15 (s, 1H), 8.25 (d, *J* = 8.2 Hz, 1H), 7.75 (d, *J* = 8.3 Hz, 2H), 7.70 - 7.57 (m, 2H), 7.46 (dd, *J* = 8.4, 1.2 Hz, 1H), 7.30 - 7.21 (m, 1H), 7.19 - 7.02 (m, 3H), 6.99 (dd, *J* = 8.1, 1.5 Hz, 1H), 6.90 (td, *J* = 7.5, 1.5 Hz, 1H), 3.90 (s, 2H).

The compound represented by Formula 1-2 according to the present invention can be prepared according to the Reaction Scheme 2, but is not limited thereto. 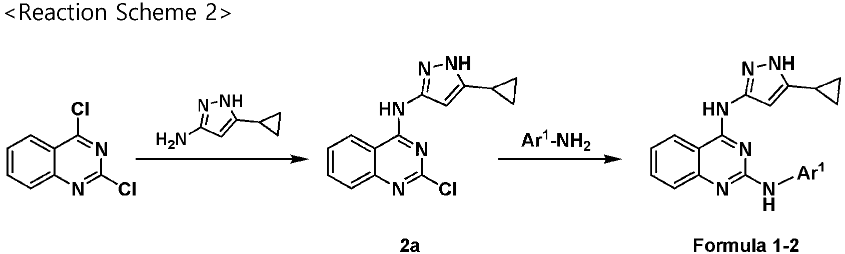 wherein, Ar¹ is the same as defined above.

### M⁴-(5-cyclopropyl-1G-pyrazol-3-yl)-M²-phenylquinazoline-2,4-diamine (4a)

Compound **2a** (70 mg, 0.25 mmol) was dissolved in *t*-butanol (3 mL), and a solution of aniline (34 mg, 0.37 mmol) in *t*-butanol (0.5 mL) was added to the mixture. 37% HCl in water (0.012 mL) was added and the reaction was heated at 110°C while stirring. After 6 h, the solvent was evaporated and the crude was dissolved in DCM. The reaction mixture was purified by column chromatography (4% DCM/MeOH) to afford compound **4a.** (139 mg, 98% yield.)

¹H NMR (300 MHz, DMSO-d₆) δ 11.51 (s, 1H), 10.56 (s, 1H), 8.68 (d, *J* = 8.2 Hz, 1H), 7.90-7.85 (m, 1H), 7.63 (d, *J* = 8.2 Hz, 1H), 7.57-7.40 (m, 5H), 7.37-7.24 (m, 2H), 6.17 (s, 1H), 1.89-1.83 (m, 1H), 1.01-0.88 (m, 2H), 0.66-0.53 (m, 2H). MS (ESI) m/z calcd C₂₀H₁₈N₆ [M⁺] ,342.2; found, 343.4 [M⁺ + H⁺].

### M²-(4-bromophenyl)-M⁴-(5-cyclopropyl-1G-pyrazol-3-yl)quinazoline-2,4-diamine (4b)

A mixture of compound **2a** (300 mg, 1.05 mmol) and 4-bromoaniline (271 mg, 1.58 mmol) in ethanol (5 mL) was stirred at 120°C in a sealed vial for 3 h. The reaction was cooled and evaporated in vacuo. The residue was purified by filtration washed with MeOH to afford compound **4b** as a white solid. (423 mg, 98% yield.)

¹H NMR (300 MHz, DMSO-d₆) δ 11.50 (s, 1H), 10.54 (s, 1H), 8.66 (d, *J* = 8.3 Hz, 1H), 7.87 (t, *J* = 7.7 Hz, 1H), 7.71 **-** 7.58 (m, 3H), 7.58 **-** 7.42 (m, 3H), 6.13 (s, 1H), 1.88 (td, *J* = 8.5, 5.1 Hz, 1H), 1.02 **-** 0.91 (m, 2H), 0.66 **-** 0.53 (m, 2H). ¹³C NMR (101 MHz, DMSO-d₆) δ 158.45, 152.30, 146.96, 145.78, 136.50, 136.03, 132.30, 126.28, 125.45, 125.10, 118.37, 118.14, 110.86, 95.68, 8.39, 7.30. MS (ESI) m/z calcd for C₂₀H₁₇BrN₆ [M⁺], 421.3; found, 421.2 [M⁺ + H⁺].

### M²-(4-bromo-2-fluorophenyl)-M⁴-(5-cyclopropyl-1G-pyrazol-3-yl)quinazoline-2,4-diamine (4c)

A mixture of compound **2a** (300 mg, 1.05 mmol) and 4-bromo-2-fluoroaniline (299 mg, 1.58 mmol) in EtOH (5 mL) was stirred at 120°C in a sealed vial for 3 h. The reaction was cooled and evaporated in vacuo. The residue was purified by filtration washed with MeOH to afford compound **4c** as a white solid. (359 mg, 78% yield.)

¹H NMR (300 MHz, DMSO-d₆) δ 11.61 (s, 1H), 10.29 (s, 1H), 8.69 (d, *J* = 8.3 Hz, 1H), 7.89 (t, *J* = 7.7 Hz, 1H), 7.80 (dd, *J* = 9.9, 2.1 Hz, 1H), 7.75 **-** 7.60 (m, 2H), 7.59 **-** 7.46 (m, 2H), 5.94 (s, 1H), 1.83 (td, *J* = 8.5, 4.4 Hz, 1H), 1.01 **-** 0.92 (m, 2H), 0.63 **-** 0.49 (m, 2H). ¹³C NMR (101 MHz, DMSO-d₆) δ 158.11, 157.94, 155.43, 152.67, 146.74, 145.92, 136.11, 130.11, 128.49, 128.46, 125.70, 125.17, 124.41, 120.16, 119.93, 118.11, 110.82, 95.28, 8.39, 7.30. MS (ESI) m/z calcd for C₂₀H₁₆BrFN₆ [M⁺], 439.3 ; found, 439.3 [M⁺ + H⁺].

### 4-((4-((5-cyclopropyl-1G-pyrazol-3-yl)amino)quinazolin-2-yl)amino)benzonitrile (4d)

Compound **2a** (70 mg, 0.25 mmol) was dissolved in *t*-butanol (3 mL), and a solution of 4-aminobenzyl cyanide (43 mg, 0.37 mmol) in *t*-butanol (0.5 mL) was added to the mixture. 37% HCl in water (0.012 mL) was added and the reaction was heated at 110°C while stirring. After 6 h, a compound **4d** was recovered by filtration washing with methanol. (76 mg, 84% yield.)

¹H NMR (300 MHz, DMSO-d₆) δ 11.57 (s, 1H), 11.04 (s, 1H), 8.67 (d, *J* = 8.2 Hz, 1H), 7.94-7.80 (m, 5H), 7.67 (dd, *J* = 8.4, 1.1 Hz, 1H), 7.56-7.47 (m, 1H), 6.15 (s, 1H), 1.98-1.91 (m, 1H), 1.05-0.92 (m, 2H), 0.73-0.58 (m, 2H). MS (ESI) m/z calcd C₂₁H₁₇N₇ [M⁺], 367.2 ; found, 368.3 [M⁺ + H⁺].

### M²-(4-((1G-tetrazol-5-yl)methyl)phenyl)-M⁴-(5-cyclopropyl-1G-pyrazol-3-yl)quinazoline-2,4-diamine (4e)

Compound **2a** (1.50 g, 5.25 mmol) was dissolved in *tert*-butanol 15 mL, and a solution of 4-aminobenzonitrile (1.04 g, 7.87 mmol) in *tert*-butanol (5 mL) was added to the mixture. 37% HCl in water (0.100 mL) was added and the reaction was heated at 110°C while stirring. After 6 h, the solvent was evaporated. The product was recovered by filtration washed with MeOH to afford compound **3a** as a white solid. A mixture of compound **3a** (50 mg, 0.131 mmol), NaN₃ (59.6 mg, 0.92 mmol) and NH₄Cl (49.1 mg, 0.92 mmol) in DMF (0.437 mL) was stirred at 120°C for 2 days. The reaction mixture was extracted with EtOAc and washed with water. The organic layer was dried over Na₂SO₄, filtered and concentrated. The residue was triturated with 5% DCM/MeOH to afford compound **4e** as a yellow solid. (2.7 mg, 4.85% yield.)

### M⁴-(5-cyclopropyl-1G-pyrazol-3-yl)-M²-(4-((methylsulfonyl)methyl)phenyl)quinazoline-2,4-diamine (4f)

A mixture of compound **2a** (71.4 mg, 0.25 mmol), 4-((methylsulfonyl)methyl)aniline (48.6 mg, 0.26 mmol) and 4 M HCl in Dioxane (0.188 mL, 0.75 mmol) in IPA (0.833 mL) was irradiated with microwave reactor at 150°C for 0.5 h. The reaction mixture was triturated with IPA. The filter cake was neutralized with saturated NaHCO₃ solution and extracted with EtOAc and water. The organic layer was dried over Na₂SO₄, filtered and concentrated. The residue was triturated with diethyl ether to afford compound **4f** as a yellow solid. (17 mg, 15% yield.)

### 4-((4-((5-cyclopropyl-1G-pyrazol-3-yl)amino)quinazolin-2-yl)amino)benzenesulfonamide (4g)

The mixture of compound **2a** (71.4 mg, 0.25 mmol), 4-aminobenzenesulfonamide (51.7 mg, 0.30 mmol) and 4 M HCl in Dioxane (0.188 mL, 0.75 mmol) was irradiated with microwave reactor at 130°C for 0.5 h. The reaction mixture was triturated with diethyl ether. The filter cake was neutralized with saturated NaHCO₃ solution and extracted with EtOAc and water. The organic layer was dried over Na₂SO₄, filtered and concentrated. The residue was purified by column chromatography (10% (7 N Ammonia in MeOH) in EtOAc:Hex=1:2) to afford compound **4g** as a yellow solid. (26 mg, 24% yield.)

¹H NMR (400 MHz, DMSO-d₆) δ 12.51 (s, 1H), 10.39 (s, 1H), 8.42 (s, 1H), 8.09 (d, *J* = 8.6 Hz, 2H), 7.75 **-** 7.73 (m, 2H), 7.57 (s, 1H), 7.30 (s, 1H), 7.17 (s, 1H), 1.99 **-** 1.88 (m, 1H), 0.98 **-** 0.89 (m, 2H), 0.82 **-** 0.69 (m, 2H)

### 4-((4-((5-cyclopropyl-1G-pyrazol-3-yl)amino)quinazolin-2-yl)amino)-M-methylbenzenesulfonamide (4h)

A mixture of compound **2a** (71.4 mg, 0.25 mmol), 4-amino-N-methylbenzenesulfonamide (69.8 mg, 0.38 mmol) and 4 M HCl in Dioxane (0.188 mL, 0.75 mmol) in IPA (0.833 mL) was irradiated with microwave reactor at 150°C for 1 h. The reaction mixture was triturated with diethyl ether. The filter cake was neutralized with saturated NaHCO₃ solution and extracted with EtOAc and water. The organic layer was dried over Na₂SO₄, filtered and concentrated. The residue was purified by column chromatography (2~10% (7 N Ammonia in MeOH) in EtOAc:Hex=1:1) to afford compound **4h** as a yellow solid. (79 mg, 73%)

### 4-((4-((5-cyclopropyl-1G-pyrazol-3-yl)amino)quinazolin-2-yl)amino)-M-(2-hydroxyethyl)benzenesulfonamide (4i)

A mixture of compound **2a** (71.4 mg, 0.25 mmol), 4-amino-*N*-(2-hydroxyethyl)benzenesulfonamide (70.3 mg, 0.33 mmol) and 4 M HCl in Dioxane (0.188 mL, 0.75 mmol) in IPA (0.833 mL) was irradiated with microwave reactor at 150°C for 0.5 h. The reaction mixture was triturated with diethyl ether. The filter cake was neutralized with saturated NaHCO₃ solution and extracted with EtOAc and water. The organic layer was dried over Na₂SO₄, filtered and concentrated. The residue was purified by column chromatography (5~15% (7 N Ammonia in MeOH) in EtOAc:Hex=1:1) and triturated with diethyl ether to afford compound **4i** as a white solid. (62 mg, 53% yield.)

### 4-((4-((5-cyclopropyl-1G-pyrazol-3-yl)amino)quinazolin-2-yl)amino)-M,M-dimethylbenzenesulfonamide (4j)

Compound **2a** (90 mg, 0.32 mmol) was dissolved in *t*-butanol (4.5 mL), and a solution of *N,N-*4-aminobezenesulfonamide (95 mg, 0.47 mmol) in *t*-butanol (0.75 mL) was added to the mixture. 37% HCl in water (0.015 mL) was added and the reaction was heated at 110°C while stirring. After 6 h, the solvent was evaporated, compound **4j** was obtained by recrystallization (EtOAc:Hex 4:1) (139 mg, 98% yield.)

### M²-(4-chloro-3-(trifluoromethyl)phenyl)-M⁴-(5-cyclopropyl-1G-pyrazol-3-yl)quinazoline-2,4-diamine (4k)

A mixture of compound **2a** (71.4 mg, 0.25 mmol), 4-chloro-3-(trifluoromethyl)aniline (51.3 mg, 0.26 mmol) and 4 M HCl in Dioxane (0.188 mL, 0.75 mmol) in IPA (0.833 mL) was irradiated with microwave reactor at 150°C for 0.5 h. The reaction mixture was filtered and washed with IPA. The filter cake was triturated with DCM. The solid was dissolved in methanol and triturated with diethyl ether to afford compound **4k** as an ivory solid. (69 mg, 62% yield.)

¹H NMR (400 MHz, DMSO-d₆) δ 11.44 (s, 1H), 10.59 (s, 1H), 8.64 (d, *J* = 7.5 Hz, 1H), 8.10 (s, 1H), 7.96 (d, *J* = 6.3 Hz, 1H), 7.90 (t, *J* = 7.4 Hz, 1H), 7.77 (d, *J* = 8.6 Hz, 1H), 7.67 (d, *J* = 8.2 Hz, 1H), 7.49 (t, *J* = 7.2 Hz, 1H), 6.08 (s, 1H), 1.85 (td, *J* = 8.5, 4.5 Hz, 1H), 0.94 **-** 0.93 (m, 2H), 0.57 (s, 2H).

### M²-(5-(benzo[c]thiazol-2-yl)-2-methylphenyl)-M⁴-(5-cyclopropyl-1G-pyrazol-3-yl)quinazoline-2,4-diamine (4l)

A mixture of compound **2a** (100 mg, 0.35 mmol) and 5-(benzo[d]thiazol-2-yl)-2-methylaniline (126 mg, 0.53 mmol) in ethanol (2 mL) was stirred at 120°C in a sealed vial for 2 h. The reaction was cooled and solvent was evaporated. The crude was purified by filtration washed with MeOH to afford compound **4l** as a light yellow solid. (116 mg, 68% yield.)

¹H NMR (300 MHz, DMSO-d₆) δ 13.28 (s, 1H), 12.42 (s, 1H), 11.54 (s, 1H), 10.22 (s, 1H), 8.71 (d, *J* = 8.3 Hz, 1H), 8.24 (s, 1H), 8.16 (d, *J* = 7.9 Hz, 1H), 8.09 (d, *J* = 7.9 Hz, 1H), 8.04 (d, *J* = 8.0 Hz, 1H), 7.88 (t, *J* = 7.8 Hz, 1H), 7.64 (t, *J* = 8.0 Hz, 2H), 7.59 **-** 7.41 (m, 3H), 5.85 (s, 1H), 2.37 (s, 3H), 1.46 (s, 1H), 0.63 (s, 2H), 0.25 (s, 2H). ¹³C NMR (101 MHz, DMSO) δ 167.03, 157.81, 153.99, 153.17, 146.26, 139.72, 138.52, 135.94, 134.91, 132.25, 132.02, 127.16, 126.40, 126.03, 125.34, 125.19, 123.26, 122.83, 117.78, 110.89, 95.31, 18.46, 7.95, 6.96. MS (ESI) m/z calcd for C₂₈H₂₃N₇S [M⁺], 489.6 ; found, 490.3 [M⁺ + H⁺].

### M²-(4-(benzo[c]thiazol-2-yl)phenyl)-M⁴-(5-cyclopropyl-1G-pyrazol-3-yl)quinazoline-2,4-diamine (4m)

A mixture of compound **2a** (100 mg, 0.35 mmol) and 4-(benzo[*d*]thiazol-2-yl)aniline (119 mg, 0.53 mmol) in ethanol (2 mL) was stirred at 120°C in a sealed vial for 2 h. The reaction was cooled and solvent was evaporated. The crude was purified by filtration washed with MeOH to afford compound **4m** as a yellow solid. (158 mg, 95%)

¹H NMR (300 MHz, DMSO-d₆) δ 11.54 (s, 1H), 10.83 (s, 1H), 8.67 (d, *J* = 8.3 Hz, 1H), 8.24 **-** 8.11 (m, 3H), 8.07 (d, *J* = 8.0 Hz, 1H), 7.90 (t, *J* = 7.8 Hz, 1H), 7.80 (d, *J* = 8.3 Hz, 2H), 7.67 (d, *J* = 8.3 Hz, 1H), 7.60 **-** 7.38 (m, 3H), 6.24 (s, 1H), 1.87 (td, *J* = 8.5, 4.5 Hz, 1H), 0.96 **-** 0.78 (m, 2H), 0.69 **-** 0.48 (m, 2H). MS (ESI) m/z calcd for C₂₇H₂₁N₇S [M⁺], 475.6 ; found, 476.3 [M⁺ + H⁺].

### M²,M⁴-bis(5-cyclopropyl-1G-pyrazol-3-yl)quinazoline-2,4-diamine (4n)

Compound 2a (100 mg, 0.35 mmol) and 5-cyclopropyl-1*H*-pyrazol-3-amine (52 mg, 0.42 mmol) were dissolved in *tert*-butanol (2 mL). 37% HCl in water (0.010 mL) was added and the reaction mixture was heated at 110°C while stirring. After overnight, the product was recovered by column chromatography (5% DCM/MeOH) and filtration washed with diethyl ether to afford a light yellow solid compound **4n.** (49 mg, 37% yield.)

¹H NMR (300 MHz, DMSO) δ 12.36 (s, 1H), 10.74 (s, 2H), 8.70 **-** 8.32 (m, 1H), 7.84 **-** 7.69 (m, 1H), 7.67 **-** 7.25 (m, 2H), 6.97 (s, 1H), 5.98 (s, 1H), 2.06 **-** 1.84 (m, 2H), 1.06 **-** 0.86 (m, 4H), 0.75 (td, *J* = 6.3, 2.7 Hz, 4H).

### Methyl 4-((4-((5-cyclopropyl-1G-pyrazol-3-yl)amino)quinazolin-2-yl)amino)benzoate (4o)

Compound **2a** (200 mg, 0.70 mmol) and metyl-4-aminobenzoate (127 mg, 0.84 mmol) were dissolved in *tert*-butanol (3 mL). 37% HCl in water (0.050 mL) was added and the reaction mixture was heated at 110°C while stirring. After 6 h, the product was recovered by filtration washed with EtOAc to afford a white solid compound **4o.** (296 mg, 106% yield.)

¹H NMR (300 MHz, DMSO) δ 11.58 (s, 1H), 10.97 (s, 1H), 8.68 (d, *J* = 7.3 Hz, 1H), 8.05 **-** 7.96 (m, 2H), 7.94 **-** 7.85 (m, 1H), 7.75 (d, *J* = 8.4 Hz, 2H), 7.65 (d, *J* = 7.3 Hz, 1H), 7.52 (d, *J* = 7.3 Hz, 1H), 6.15 (s, 1H), 3.87 (s, 3H), 1.90 (tt, *J* = 8.4, 5.1 Hz, 1H), 0.99 **-** 0.89 (m, 2H), 0.66 **-** 0.53 (m, 2H). MS (ESI) m/z calcd for C₂₂H₂₀N₆O₂ [M⁺], 400.2 ; found, 401.4 [M⁺ + H⁺].

### sdqs-butyl 4-((4-((5-cyclopropyl-1G-pyrazol-3-yl)amino)quinazolin-2-yl)amino)benzoate (4p)

Compound 2a (200 mg, 0.70 mmol) and *tert*-butyl-4-aminobenzoate (162 mg, 0.84 mmol) were dissolved in *tert*-butanol (3 mL). 37% HCl in water (0.020 mL) was added and the reaction mixture was heated at 110°C while stirring. After 5 h, the product was recovered by filtration washed with EtOAc to afford a white solid compound **4p.** (325 mg, 105% yield.)

¹H NMR (300 MHz, DMSO) δ 11.62 (s, 1H), 10.95 (s, 1H), 8.69 (d, *J* = 8.3 Hz, 1H), 8.01 - 7.83 (m, 3H), 7.75 - 7.59 (m, 3H), 7.52 (t, *J* = 7.7 Hz, 1H), 6.14 (s, 1H), 1.89 (t, *J* = 4.3 Hz, 1H), 1.57 (s, 9H), 1.00 - 0.87 (m, 2H), 0.64 - 0.52 (m, 2H). ¹³C NMR (101 MHz, DMSO) δ 164.85, 158.92, 152.02, 147.17, 145.54, 141.43, 136.20, 130.43, 127.85, 125.64, 125.21, 122.47, 118.19, 110.89, 95.84, 81.00, 28.28, 9.22, 8.46, 7.25.

MS (ESI) m/z calcd for C₂₅H₂₆N₆O₂ [M⁺], 442.2 ; found, 443.5 [M⁺ + H⁺].

### sdqs-butyl 2-(4-((4-((5-cyclopropyl-1G-pyrazol-3-yl)amino)quinazolin-2-yl) amino)phenyl)acetate (4q)

Compound 2a (130 mg, 0.46 mmol) and *tert*-butyl-2-(4-aminophenyl)acetate (113 mg, 0.55 mmol) were dissolved in *tert*-butanol (4 mL). 37% HCl in water (0.010 mL) was added and the reaction mixture was heated at 110°C while stirring. After 5 h, the product was recovered by filtration washed with EtOAc to afford a white solid compound **4q.** (232 mg, 112% yield.)

¹H NMR (300 MHz, DMSO) δ 12.65 (s, 1H), δ 11.52 (s, 1H), 10.57 (s, 1H), 8.68 (d, *J* = 8.3 Hz, 1H), 7.87 (t, *J* = 7.5 Hz, 1H), 7.68 **-** 7.56 (m, 1H), 7.54 **-** 7.41 (m, 3H), 7.37 **-** 7.29 (m, 2H), 6.21 (s, 1H), 3.62 (s, 2H), 1.88 (tt, *J* = 8.4, 5.1 Hz, 1H), 1.42 (s, 9H), 1.00 **-** 0.88 (m, 2H), 0.66 **-** 0.49 (m, 2H).

### 4-((4-((5-cyclopropyl-1G-pyrazol-3-yl)amino)quinazolin-2-yl)amino)benzoic acid (4r)

Compound 4p (150 mg, 0.34 mmol) was dissolved in DCM (3 mL). TFA (1 mL) was dropwised, the reaction mixture was stirred at room temperature for 2 h. The solvent was evaporated and DCM was added repeat 2 times. The solvent was evaporated and diethyl ether was added repeat several times. The solvent was evaporated and the solid was filtered washed with diethyl ether to afford compound **4r** as a white solid. (156 mg, 119% yield.)

¹H NMR (400 MHz, DMSO) δ 11.35 (s, 1H), 10.89 (s, 1H), 8.62 (d, *J* = 8.3 Hz, 1H), 7.98 (d, *J* = 8.3 Hz, 2H), 7.88 (t, *J* = 7.9 Hz, 1H), 7.75 (d, *J* = 8.3 Hz, 2H), 7.62 (d, *J* = 8.3 Hz, 1H), 7.49 (d, *J* = 7.9 Hz, 1H), 6.19 (s, 1H), 1.89 (tt, *J* = 8.5, 5.0 Hz, 1H), 1.01 - 0.85 (m, 2H), 0.67 - 0.54 (m, 2H). ¹³C NMR (101 MHz, DMSO) δ 166.85, 160.07, 159.73, 159.39, 158.53, 152.01, 146.78, 145.18, 141.57, 135.50, 130.32, 126.47, 124.91, 124.45, 121.63, 117.92, 114.98, 110.54, 95.49, 7.85, 6.90. MS (ESI) m/z calcd for C₂₁H₁₈N₆O₂ [M⁺], 386.2 ; found, 387.4 [M⁺ + H⁺].

### 2-(4-((4-((5-cyclopropyl-1G-pyrazol-3-yl)amino)quinazolin-2-yl)amino)phenyl)acetic acid (4s)

Compound 4q (150 mg, 0.33 mmol) was dissolved in DCM (3 mL). TFA (1 mL) was dropwised, the reaction mixture was stirred at room temperature for 2 h. The solvent was evaporated and DCM was added repeat 2 times. The solvent was evaporated and diethyl ether was added repeat several times. The solvent was evaporated and the solid was filtered washed with diethyl ether to afford compound **4s** as a white solid. (150 mg, 115% yield.)

¹H NMR (300 MHz, DMSO) δ 11.49 (s, 1H), 10.49 (s, 1H), 8.66 (d, *J* = 8.3 Hz, 1H), 7.86 (t, *J* = 7.8 Hz, 1H), 7.63 (d, *J* = 8.3 Hz, 1H), 7.53 - 7.42 (m, 3H), 7.35 (d, *J* = 8.2 Hz, 2H), 6.22 (s, 1H), 3.63 (s, 2H), 1.85 (td, *J* = 8.5, 4.3 Hz, 1H), 1.01 - 0.88 (m, 2H), 0.59 (s, 2H). ¹³C NMR (101 MHz, DMSO) δ 173.02, 160.42, 160.09, 158.52, 152.48, 146.90, 145.96, 135.92, 135.70, 132.78, 130.89, 130.44, 125.18, 124.98, 123.85, 118.63, 115.69, 110.78, 95.92, 40.70, 8.33, 7.32. MS (ESI) m/z calcd for C₂₂H₂₀N₆O₂ [M⁺], 400.2 ; found, 401.4 [M⁺ + H⁺].

### 4-((4-((5-cyclopropyl-1G-pyrazol-3-yl)amino)quinazolin-2-yl)amino)benzamide (4t)

To a solution of 4r (100 mg, 0.26 mmol) in 1,4-dioxane (3 mL) and then added DIPEA (0.136 mL, 0.78 mmol) and HATU (197 mg, 0.52 mmol). The reaction mixture was stirred at room temperature for 20 min. To the mixture, Ammonium chloride (138 mg, 2.59 mmol) was added and stirred at room temperature for 2 days. EtOAc was added to the mixture and washed with water 2 times. The organic layer was washed with brine several times. The combined organic layer was dried over Na₂SO₄, filtered and concentrated. The residue was purified by column chromatography (DCM 92: MeOH 7: NH₄OH 1) to afford compound **4t** as an ivory solid. (20 mg, 20% yield.)

¹H NMR (300 MHz, DMSO) δ 12.53 (d, *J* = 180.9 Hz, 1H), 10.39 (d, *J* = 138.2 Hz, 1H), 9.72 (d, *J* = 187.0 Hz, 1H), 8.61 - 8.22 (m, 1H), 7.99 (d, *J* = 8.5 Hz, 2H), 7.93 - 7.75 (m, 3H), 7.75 - 7.45 (m, 2H), 6.68 (s, 1H), 2.08 - 1.80 (m, 1H), 0.93 (s, 2H), 0.75 (s, 2H). MS (ESI) m/z calcd for C₂₁H₁₉N₇O [M+], 385.4 ; found, 386.4 [M⁺ + H⁺]

### 2-(4-((4-((5-cyclopropyl-1G-pyrazol-3-yl)amino)quinazolin-2-yl)amino)phenyl)acetamide (4u)

To a solution of **4s** (70 mg, 0.18 mmol) in 1,4-dioxane (2 mL) and then added DIPEA (0.092 mL, 0.52 mmol) and HATU (134 mg, 0.35 mmol). The reaction mixture was stirred at room temperature for 20 min. To the mixture, Ammonium chloride (94 mg, 1.75 mmol) was added and stirred at room temperature for 2 days. EtOAc was added to the mixture and washed with water 2 times. The organic layer was washed with brine several times. The combined organic layer was dried over Na₂SO₄, filtered and concentrated. The residue was purified by column chromatography (DCM 92: MeOH 7: NH₄OH 1) to afford compound **4u** as an ivory solid. (9 mg, 12% yield.)

¹H NMR (300 MHz, DMSO) δ 12.50 (d, *J* = 190.2 Hz, 1H), 10.30 (d, *J* = 144.4 Hz, 1H), 9.40 (d, *J* = 201.9 Hz, 1H), 8.51 **-** 8.20 (m, 1H), 7.92 **-** 7.73 (m, 2H), 7.65 (s, 1H), 7.55 **-** 7.34 (m, 2H), 6.85 (s, 1H), 6.68 (s, 1H), 3.33 (s, 2H), 2.06 **-** 1.80 (m, 1H), 0.93 (s, 2H), 0.75 (s, 2H).

### M²-(4-aminophenyl)-M⁴-(5-cyclopropyl-1G-pyrazol-3-yl)quinazoline-2,4-diamine (4v)

A mixture of compound **2a** (100 mg, 0.35 mmol) and benzene-1,4-diamine (57 mg, 0.53 mmol) in ethanol (3 mL) was stirred at 120°C in a sealed vial for 2 h. The reaction was cooled and solvent was evaporated. The crude was purified by filtration washed with 5% DCM/MeOH and diethyl ether to afford compound **4v** as a dark green solid. (124 mg, 99% yield.)

¹H NMR (300 MHz, DMSO) δ 12.54 (s, 1H), 11.29 (s, 1H), 10.07 (s, 1H), 8.62 (d, *J* = 8.3 Hz, 1H), 7.80 (t, *J* = 7.7 Hz, 1H), 7.60 (d, *J* = 8.3 Hz, 1H), 7.41 (t, *J* = 7.7 Hz, 1H), 7.21 (s, 2H), 6.84 - 6.64 (m, 2H), 6.25 (s, 1H), 1.97 - 1.78 (m, 1H), 1.06 - 0.88 (m, 2H), 0.79 - 0.50 (m, 2H). ¹³C NMR (101 MHz, DMSO) δ 157.78, 153.24, 135.55, 126.14, 124.86, 116.00, 110.73, 95.50, 8.39, 8.32, 7.49. MS (ESI) m/z calcd for C₂₀H₁₉N₇ [M⁺], 357.2 ; found, 358.5 [M⁺ + H⁺].

### sdqs-butyl (2-(4-((4-((5-cyclopropyl-1G-pyrazol-3-yl)amino)quinazolin-2-yl) amino)benzamido)ethyl)carbamate (4w)

Compound **4r** (150 mg, 0.39 mmol) and PyBOP (404 mg, 0.78 mmol) was dissolved in DMF (3 mL). *tert*-butyl (2-aminoethyl)carbamate (0.123 mL, 0.78 mmol) and TEA (0.108 mL, 0.78 mmol) were dropwised. The reaction mixture was stirred at room temperature for 24 h. After TLC checking, the reaction mixture was quenched with water and extracted with EA. The organic layer was washed with saturated NaHCO₃ and brine several times. The combined organic layer was dried over Na₂SO₄ and evaporated under vacuum. The crude mixture was purified by column chromatography (5% DCM/MeOH) and filtered with diethyl ether to afford a white solid product compound **4w.** (95 mg, 47% yield.)

¹H NMR (300 MHz, DMSO) δ 12.57 (d, *J* = 150.3 Hz, 1H), 10.59 (s, 1H), 9.83 (d, *J* = 101.7 Hz, 1H), 8.49 (s, 1H), 8.32 (s, 1H), 8.00 (d, *J* = 8.4 Hz, 1H), 7.94 (s, 1H), 7.84 (d, *J* = 8.2 Hz, 2H), 7.78 **-** 7.64 (m, 2H), 7.64 **-** 7.49 (m, 2H), 7.43 (t, *J* = 7.6 Hz, 1H), 7.34 (t, *J* = 6.6 Hz, 1H), 6.93 (t, *J* = 5.4 Hz, 1H), 6.53 (s, 1H), 3.30 **-** 3.23 (m, 2H), 3.18 **-** 3.05 (m, 2H), 1.91 (tt, *J* = 8.4, 5.0 Hz, 1H), 1.39 (s, 9H), 0.98 **-** 0.86 (m, 2H), 0.77 **-** 0.66 (m, 2H). MS (ESI) m/z calcd for C₂₈H₃₂N₈O₃ [M⁺], 528.3 ; found, 529.7 [M⁺ + H⁺].

### sdqs-butyl (3-(4-((4-((5-cyclopropyl-1G-pyrazol-3-yl)amino)quinazolin-2-yl) amino)benzamido)propyl)carbamate (4x)

Compound **4r** (150 mg, 0.39 mmol) and PyBOP (404 mg, 0.78 mmol) was dissolved in DMF (3 mL). *tert*-butyl (3-aminopropyl)carbamate (0.136 mL, 0.78 mmol) and TEA (0.108 mL, 0.78 mmol) were dropwised. The reaction mixture was stirred at room temperature for 24 h. After TLC checking, the reaction mixture was quenched with water and extracted with EA. The organic layer was washed with saturated NaHCO₃ and brine several times. The combined organic layer was dried over Na₂SO₄ and evaporated under vacuum. The crude mixture was purified by column chromatography (5% DCM/MeOH) and filtered with diethyl ether to afford an ivory solid product. (96 mg, 46% yield.)

¹H NMR (300 MHz, DMSO) δ 12.52 (d, *J* = 180.8 Hz, 1H), 10.37 (d, *J* = 154.5 Hz, 1H), 9.38 (d, *J* = 191.9 Hz, 1H), 8.42 (s, 1H), 8.26 (s, 1H), 8.00 (d, *J* = 8.4 Hz, 2H), 7.93 **-** 7.75 (m, 2H), 7.69 (s, 1H), 7.55 (s, 1H), 7.30 (s, 1H), 6.84 (t, *J* = 5.2 Hz, 1H), 6.58 (d, *J* = 67.6 Hz, 1H), 5.84 (s, 1H), 3.26 (q, *J* = 6.3 Hz, 2H), 2.99 (q, *J* = 6.5 Hz, 2H), 2.02 **-** 1.84 (m, 1H), 1.77 **-** 1.56 (m, 2H), 1.39 (s, 9H), 1.09 - 0.85 (m, 2H), 0.87 **-** 0.56 (m, 2H). MS (ESI) m/z calcd for C₂₉H₃₄N₈O₃ [M⁺], 542.3 ; found, 543.7 [M⁺ + H⁺].

### sdqs-butyl (4-(4-((4-((5-cyclopropyl-1G-pyrazol-3-yl)amino)quinazolin-2-yl) amino)benzamido)butyl)carbamate (4y)

Compound **4r** (150 mg, 0.39 mmol) and PyBOP (404 mg, 0.78 mmol) was dissolved in DMF (3 mL), *tert*-butyl (4-aminobutyl)carbamate (0.149 mL, 0.78 mmol) and TEA (0.108 mL, 0.78 mmol) were dropwised. The reaction mixture was stirred at room temperature for 24 h. After TLC checking, the reaction mixture was quenched with water and extracted with EA. The organic layer was washed with saturated NaHCO₃ and brine several times. The combined organic layer was dried over Na₂SO₄ and evaporated under vacuum. The crude mixture was purified by column chromatography (5% DCM/MeOH) and filtered with diethyl ether to afford a light pink solid product. (105 mg, 49% yield.)

¹H NMR (300 MHz, DMSO) δ 12.51 (d, *J* = 180.8 Hz, 1H), 10.36 (d, *J* = 143.4 Hz, 1H), 9.38 (d, *J* = 194.3 Hz, 1H), 8.42 (s, 1H), 8.30 **-** 8.21 (m, 1H), 8.00 (d, *J* = 8.7 Hz, 2H), 7.82 (d, *J* = 8.5 Hz, 2H), 7.76 **-** 7.62 (m, 1H), 7.55 (s, 1H), 7.30 (s, 1H), 6.82 (t, *J* = 5.6 Hz, 1H), 6.58 (d, *J* = 68.2 Hz, 1H), 5.85 (s, 1H), 3.24 (q, *J* = 6.3 Hz, 2H), 2.95 (q, *J* = 6.3 Hz, 2H), 1.93 (tt, *J* = 8.8, 5.2 Hz, 1H), 1.56 **-** 1.32 (m, 13H), 1.03 **-** 0.85 (m, 2H), 0.85 **-** 0.63 (m, 2H). MS (ESI) m/z calcd for C₃₀H₃₆N₈O₃ [M⁺], 556.3 ; found, 557.8 [M⁺ + H⁺].

### sdqs-butyl (5-(4-((4-((5-cyclopropyl-1G-pyrazol-3-yl)amino)quinazolin-2-yl) amino)benzamido)pentyl)carbamate (4z)

Compound **4r** (150 mg, 0.39 mmol) and PyBOP (404 mg, 0.78 mmol) were dissolved in DMF (3 mL). *tert*-butyl (5-aminopentyl)carbamate (0.162 mL, 0.78 mmol) and TEA (0.108 mL, 0.78 mmol) were dropwised. The reaction mixture was stirred at room temperature for 24 h. After TLC checking, the reaction mixture was quenched with water and extracted with EA. The organic layer was washed with saturated NaHCO₃ and brine several times. The combined organic layer was dried over Na₂SO₄ and evaporated under vacuum. The crude mixture was purified by column chromatography (5% DCM/MeOH) and filtered with diethyl ether to afford a light yellow solid product. (121 mg, 55% yield.)

¹H NMR (300 MHz, DMSO) δ 12.52 (d, *J* = 181.9 Hz, 1H), 10.36 (d, *J* = 143.0 Hz, 1H), 9.37 (d, *J* = 190.8 Hz, 1H), 8.42 (s, 1H), 8.29 **-** 8.16 (m, 1H), 8.00 (d, *J* = 8.8 Hz, 2H), 7.82 (d, *J* = 8.5 Hz, 2H), 7.74 **-** 7.62 (m, 1H), 7.55 (s, 1H), 7.29 (s, 1H), 6.79 (t, *J* = 5.7 Hz, 1H), 6.56 (d, *J* = 75.3 Hz, 1H), 5.84 (s, 1H), 3.25 (q, *J* = 6.6 Hz, 2H), 2.92 (q, *J* = 6.6 Hz, 2H), 2.01 **-** 1.85 (m, 1H), 1.60 **-** 1.46 (m, 2H), 1.46 **-** 1.33 (m, 11H), 1.33 **-** 1.21 (m, 2H), 1.02 **-** 0.85 (m, 2H), 0.85 **-** 0.63 (m, 2H). MS (ESI) m/z calcd for C₃₁H₃₈N₈O₃ [M⁺], 570.3 ; found, 571.8 [M⁺ + H⁺].

### M-(2-aminoethyl)-4-((4-((5-cyclopropyl-1G-pyrazol-3-yl)amino)quinazolin-2-yl) amino)benzamide.trifluoroacetic acid (4aa)

Compound **4w** (50 mg, 0.10 mmol) was dissolved in DCM (2 mL), and TFA (0.5 mL) was dropwised at 0°C. The reaction mixture was stirred for 1 h at room temperature. The solvent was evaporated and DCM was added repeat 2 times. The solvent was evaporated and ethyl ether was added repeat several times. The product was obtained as a white solid. (62 mg, quant.)

¹H NMR (400 MHz, DMSO) δ 8.57 (s, 2H), 7.92 (d, *J* = 8.2 Hz, 2H), 7.84 (s, 5H), 7.60 (d, *J* = 8.3 Hz, 1H), 7.50 **-** 7.37 (m, 1H), 6.24 (s, 1H), 3.55 **-** 3.51 (m, 2H), 3.01 (q, *J* = 6.1 Hz, 2H), 1.89 (tt, *J* = 9.0, 5.1 Hz, 1H), 0.97 **-** 0.88 (m, 2H), 0.72 **-** 0.56 (m, 2H). MS (ESI) m/z calcd for C₂₃H₂₄N₈O [M⁺], 428.2 ; found, 429.7 [M⁺ + H⁺].

### M-(3-aminopropyl)-4-((4-((5-cyclopropyl-1G-pyrazol-3-yl)amino)quinazolin-2-yl) amino)benzamide.trifluoroacetic acid (4ab)

Compound **4x** (50 mg, 0.09 mmol) was dissolved in DCM (2 mL), and TFA (0.5 mL) was dropwised at 0°C. The reaction mixture was stirred for 1 h at room temperature. The solvent was evaporated and DCM was added repeat 2 times. The solvent was evaporated and ethyl ether was added repeat several times. The product was obtained as a white solid. (63 mg, quant.)

¹H NMR (400 MHz, DMSO) δ 8.58 (s, 2H), 7.91 (d, *J* = 8.3 Hz, 2H), 7.86 **-** 7.68 (m, 6H), 7.59 (d, *J* = 8.3 Hz, 1H), 7.45 (d, *J* = 6.5 Hz, 1H), 6.21 (s, 1H), 3.36 (q, *J* = 6.4 Hz, 2H), 2.87 (q, *J* = 6.4 Hz, 2H), 1.93 **-** 1.85 (m, 1H), 1.85 **-** 1.77 (m, 2H), 0.96 **-** 0.87 (m, 2H), 0.67 **-** 0.53 (m, 2H). MS (ESI) m/z calcd for C₂₄H₂₆N₈O [M⁺], 442.2 ; found, 443.7 [M⁺ + H⁺].

### M-(4-aminobutyl)-4-((4-((5-cyclopropyl-1G-pyrazol-3-yl)amino)quinazolin-2-yl) amino)benzamide.trifluoroacetic acid (4ac)

Compound **4y** (50 mg, 0.09 mmol) was dissolved in DCM (2 mL), and TFA (0.5 mL) was dropwised at 0°C. The reaction mixture was stirred for 1 h at room temperature. The solvent was evaporated and DCM was added repeat 2 times. The solvent was evaporated and ethyl ether was added repeat several times. The product was obtained as a white solid. (65 mg, quant.)

¹H NMR (400 MHz, DMSO) δ 8.59 (d, *J* = 8.1 Hz, 1H), 8.54 **-** 8.41 (m, 1H), 7.91 (d, *J* = 8.3 Hz, 2H), 7.84 (t, *J* = 8.0 Hz, 1H), 7.75 (s, 4H), 7.59 (d, *J* = 8.3 Hz, 1H), 7.46 (t, *J* = 7.7 Hz, 1H), 6.21 (s, 1H), 3.31 (q, *J* = 6.3 Hz, 2H), 2.84 (q, *J* = 6.3 Hz, 2H), 1.88 (tt, *J* = 8.4, 5.1 Hz, 1H), 1.67 **-** 1.51 (m, 4H), 0.99 **-** 0.85 (m, 2H), 0.69 **-** 0.55 (m, 2H). MS (ESI) m/z calcd for C₂₅H₂₈N₈O [M⁺], 456.2 ; found, 457.8 [M⁺ + H⁺].

### M⁴-(5-cyclopropyl-1G-pyrazol-3-yl)-M²-(4-methoxybenzyl)quinazoline-2,4-diamine (4ad)

The mixture of compound 2a (71.4 mg, 0.25 mmol), 4-methoxybenzylamine (0.147 mL, 0.750 mmol) was irradiated with microwave reactor at 150°C for 1.0 h. The reaction mixture was triturated with Diethyl ether. The filter cake was neutralized with saturated NaHCO₃ solution and extracted with EtOAc and water. The organic layer was dried over Na₂SO₄, filtered and concentrated. The residue was triturated with DCM and Diethyl ether to afford compound 4q as a white solid. (46 mg, 48% yield.)

¹H NMR (400 MHz, DMSO- d₆) δ 12.20 (d, *J* = 251.2 Hz, 1H), 10.12 (d, *J* = 189.7 Hz, 1H), 8.26 (d, *J* = 51.1 Hz, 1H), 7.51 **-** 7.28 (m, 5H), 7.09 (d, *J* = 42.3 Hz, 2H), 6.99 **-** 6.81 (m, 2H), 4.51 (d, *J* = 6.0 Hz, 2H), 3.71 (s, 3H), 1.92 **-** 1.79 (m, 1H), 0.96 **-** 0.83 (m, 2H), 0.80 **-** 0.48 (m, 2H)

### 4-((4-((5-cyclopropyl-1G-pyrazol-3-yl)amino)quinazolin-2-yl)amino)-M-methylbenzamide (4ae)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, methylamine solution (2M in THF, 0.647 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (69 mg, 67%)

¹H NMR (400 MHz, DMSO) δ 12.51 (d, *J* = 240.1 Hz, 1H), 10.36 (d, *J* = 207.1 Hz, 1H), 9.36 (d, *J* = 260.1 Hz, 1H), 8.42 (s, 1H), 8.24 (d, *J* = 5.3 Hz, 1H), 8.00 (d, *J* = 8.4 Hz, 2H), 7.90 - 7.75 (m, 2H), 7.69 (s, 1H), 7.56 (s, 1H), 7.29 (s, 1H), 6.68 (s, 1H), 2.79 (d, *J* = 4.4 Hz, 3H), 2.00 - 1.88 (m, 1H), 1.02 - 0.85 (m, 2H), 0.85 - 0.64 (m, 2H). ¹³C NMR (101 MHz, DMSO) δ 167.92, 152.67, 145.23, 134.64, 134.58, 129.29, 128.03, 127.33, 124.72, 123.97, 119.09, 27.71, 16.68, 9.32. MS (ESI) m/z calcd for C₂₂H₂₁N₇O [M⁺], 399.2 ; found, 400.5 [M⁺ + H⁺].

### 4-((4-((5-cyclopropyl-1G-pyrazol-3-yl)amino)quinazolin-2-yl)amino)-M-ethylbenzamide (4af)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, ethylamine (0.085 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (56 mg, 53%)

¹H NMR (400 MHz, DMSO) δ 12.50 (d, *J* = 241.7 Hz, 1H), 10.36 (d, *J* = 194.2 Hz, 1H), 9.37 (d, *J* = 257.6 Hz, 1H), 8.41 (s, 1H), 8.27 (d, *J* = 5.8 Hz, 1H), 8.00 (d, *J* = 8.8 Hz, 2H), 7.82 (d, *J* = 7.9 Hz, 2H), 7.69 (s, 1H), 7.55 (s, 1H), 7.29 (s, 1H), 6.68 (s, 1H), 3.32 **-** 3.25 (m, 2H), 1.99 **-** 1.87 (m, 1H), 1.14 (t, *J* = 7.2 Hz, 3H), 1.02 **-** 0.85 (m, 2H), 0.85 **-** 0.65 (m, 2H). ¹³C NMR (101 MHz, DMSO) δ 166.14, 151.64, 144.16, 133.47, 128.29, 127.13, 126.35, 123.63, 118.11, 112.04, 34.39, 15.64, 15.46, 8.27. MS (ESI) m/z calcd for C₂₃H₂₃N₇O [M⁺], 413.2 ; found, 414.5 [M⁺ + H⁺].

### sdqs-butyl 6-((4-((5-cyclopropyl-1G-pyrazol-3-yl)amino)quinazolin-2-yl)amino)-3-azabicyclo[3.1.0]hexane-3-carboxylate (4ag)

Compound **2a** (70 mg, 0.25 mmol) was dissolved in t-butanol (3 mL), and a solution of tert-butyl 6-amino-3-azabicyclo[3.1.0]hexane-3-carboxylate (73 mg, 0.37 mmol) in t-butanol (0.5 mL) was added to the mixture. DIPEA (48 mg, 0.37 mmol) was added and the reaction was heated at 110°C while stirring. After 4 days, the solvent was evaporated, purified by column chromatography (4% DCM/MeOH) to afford compound **4cg**. (20 mg, 18% yield.)

MS (ESI) m/z calcd C₂₄H₂₉N₇O₂ [M⁺],447.2 found, 448.6 [M⁺ + H⁺].

### 4-((4-((5-cyclopropyl-1G-pyrazol-3-yl)amino)quinazolin-2-yl)amino)-M-(cyclopropylmethyl)benzamide (4ah)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, cyclopropylmethanamine (0.112 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with 3% DCM/MeOH to afford an ivory solid. (58 mg, 51%)

¹H NMR (400 MHz, DMSO) δ 12.52 (d, *J* = 242.1 Hz, 1H), 10.37 (d, *J* = 194.0 Hz, 1H), 9.70 (d, *J* = 252.9 Hz, 1H), 8.54 **-** 8.25 (m, 2H), 8.00 (d, *J* = 8.5 Hz, 2H), 7.89 **-** 7.76 (m, 2H), 7.69 (s, 1H), 7.55 (s, 1H), 7.29 (s, 1H), 6.26 (d, *J* = 343.1 Hz, 1H), 3.15 (t, *J* = 6.2 Hz, 2H), 1.97 **-** 1.86 (m, 1H), 1.10 **-** 1.00 (m, 1H), 1.00 **-** 0.86 (m, 2H), 0.76 (s, 2H), 0.49 **-** 0.38 (m, 2H), 0.29 **-** 0.18 (m, 2H). ¹³C NMR (101 MHz, DMSO) δ 166.30, 151.64, 144.20, 133.58, 128.38, 127.09, 126.34, 123.62, 122.98, 117.94, 112.04, 43.86, 11.64, 8.26, 3.77. MS (ESI) m/z calcd for C₂₅H₂₅N₇O [M⁺], 439.2 ; found, 440.5 [M⁺ + H⁺].

### 4-((4-((5-cyclopropyl-1G-pyrazol-3-yl)amino)quinazolin-2-yl)amino)-M-(2-methylbutyl)benzamide (4ai)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, 2-methylbutan-1-amine (0.153 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (69 mg, 58%)

¹H NMR (400 MHz, DMSO) δ 12.53 (d, *J* = 238.8 Hz, 1H), 10.38 (d, *J* = 193.6 Hz, 1H), 9.39 (d, *J* = 249.2 Hz, 1H), 8.42 (s, 1H), 8.25 (t, *J* = 5.9 Hz, 1H), 8.00 (d, *J* = 8.5 Hz, 2H), 7.83 (d, *J* = 8.4 Hz, 2H), 7.74 **-** 7.63 (m, 1H), 7.61 **-** 7.47 (m, 1H), 7.29 (s, 1H), 6.27 (d, *J* = 334.4 Hz, 1H), 3.20 (dt, *J* = 12.6, 6.1 Hz, 1H), 3.07 (dt, *J* = 12.6, 6.1 Hz, 1H), 1.93 (tt, *J* = 8.6, 5.0 Hz, 1H), 1.65 (ddd, *J* = 14.1, 9.6, 5.9 Hz, 1H), 1.43 (tt, *J* = 12.5, 7.5 Hz, 1H), 1.17 **-** 1.06 (m, 1H), 1.02 **-** 0.85 (m, 8H), 0.80 **-** 0.64 (m, 2H). ¹³C NMR (101 MHz, DMSO) δ 166.49, 151.66, 144.15, 133.58, 128.36, 127.25, 126.30, 123.67, 122.96, 117.93, 112.04, 45.40, 35.05, 27.13, 17.67, 11.70, 8.26. MS (ESI) m/z calcd for C₂₆H₂₉N₇O [M⁺], 455.2 ; found, 456.6 [M⁺ + H⁺].

### 4-((4-((5-cyclopropyl-1G-pyrazol-3-yl)amino)quinazolin-2-yl)amino)-M,M-dimethylbenzamide (4aj)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, dimethylamine (0.087 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (55 mg, 52%)

¹H NMR (400 MHz, DMSO) δ 12.53 (d, *J* = 241.7 Hz, 1H), 10.35 (d, *J* = 192.0 Hz, 1H), 9.66 (d, *J* = 248.9 Hz, 1H), 8.41 (s, 1H), 7.99 (d, *J* = 8.3 Hz, 2H), 7.76 **-** 7.61 (m, 1H), 7.54 (s, 1H), 7.47 **-** 7.34 (m, 2H), 7.28 (s, 1H), 6.26 (d, *J* = 332.8 Hz, 1H), 3.00 (s, 6H), 1.93 (tt, *J* = 8.8, 5.1 Hz, 1H), 0.99 **-** 0.86 (m, 2H), 0.83 **-** 0.65 (m, 2H). ¹³C NMR (101 MHz, DMSO) δ 170.73, 151.71, 142.81, 133.59, 128.54, 126.26, 123.61, 122.88, 118.00, 111.99, 55.38, 40.60, 40.44, 8.27. MS (ESI) m/z calcd for C₂₃H₂₃N₇O [M⁺], 413.2 ; found, 414.5 [M⁺ + H⁺].

### (4-((4-((5-cyclopropyl-1G-pyrazol-3-yl)amino)quinazolin-2-yl) amino)phenyl)(morpholino)methanone (4ak)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, morpholine (0.112 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (62 mg, 53%)

¹H NMR (300 MHz, DMSO) δ 12.52 (d, *J* = 175.8 Hz, 1H), 10.35 (d, *J* = 142.2 Hz, 1H), 9.38 (d, *J* = 184.6 Hz, 1H), 8.41 (s, 1H), 8.01 (d, *J* = 8.6 Hz, 2H), 7.68 (t, *J* = 7.7 Hz, 1H), 7.62 **-** 7.49 (m, 1H), 7.39 (d, *J* = 8.2 Hz, 2H), 7.29 (s, 1H), 6.26 (d, *J* = 240.2 Hz, 1H), 3.69 **-** 3.46 (m, 8H), 1.92 (dq, *J* = 8.7, 4.4 Hz, 1H), 1.01 **-** 0.84 (m, 2H), 0.84 **-** 0.65 (m, 2H). ¹³C NMR (101 MHz, DMSO) δ 169.82, 151.67, 143.10, 133.59, 128.69, 127.60, 126.27, 123.61, 122.93, 118.11, 111.99, 66.67, 40.63, 40.42, 40.21, 8.29. MS (ESI) m/z calcd for C₂₅H₂₅N₇O₂ [M⁺], 455.2 ; found, 456.5 [M⁺ + H⁺].

### 4-((4-((5-cyclopropyl-1G-pyrazol-3-yl)amino)quinazolin-2-yl)amino)-M-phenylbenzamide (4al)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, aniline (0.118 mL, 1.29 mmol) was added and stirred at room temperature for overnight. To the reaction mixture, EtOAc was added. The organic layer was washed with water and brine several times. The combined organic layer was dried over anhydrous Na₂SO₄, filtered and purified by filtration washed with diethyl ether to afford an ivory solid. (79 mg, 66%)

¹H NMR (300 MHz, DMSO) δ 12.53 (d, *J* = 177.2 Hz, 1H), 10.55 (d, *J* = 114.5 Hz, 1H), 10.07 (s, 1H), 9.52 (s, 1H), 8.43 (s, 1H), 8.10 (d, *J* = 8.8 Hz, 2H), 7.97 (d, *J* = 8.5 Hz, 2H), 7.85 **-** 7.76 (m, 2H), 7.71 (t, *J* = 7.6 Hz, 1H), 7.57 (d, *J* = 8.1 Hz, 1H), 7.41 **-** 7.25 (m, 3H), 7.14 **-** 7.04 (m, 1H), 6.28 (d, *J* = 248.0 Hz, 1H), 1.94 (ddd, *J* = 13.4, 7.1, 4.4 Hz, 1H), 1.02 **-** 0.87 (m, 2H), 0.83 **-** 0.65 (m, 2H). ¹³C NMR (101 MHz, DMSO) δ 165.61, 151.60, 144.79, 139.98, 133.64, 129.04, 127.04, 126.37, 123.76, 123.10, 120.71, 117.96, 112.10, 15.65, 8.30. MS (ESI) m/z calcd for C₂₇H₂₃N₇O [M⁺], 461.2 ; found, 462.5 [M⁺ + H⁺].

### 4-((4-((5-cyclopropyl-1G-pyrazol-3-yl)amino)quinazolin-2-yl)amino)-M-(4-fluorophenyl)benzamide (4am)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, 4-fluoroaniline (0.050 mL, 0.49 mmol) was added and stirred at room temperature for overnight. To the reaction mixture, water was added. The solid was filtered and washed with water and diethyl ether to afford a white solid. (81 mg, 63%)

¹H NMR (400 MHz, DMSO) δ 12.52 (d, *J* = 242.6 Hz, 1H), 10.40 (d, *J* = 187.1 Hz, 1H), 10.12 (s, 1H), 9.48 (s, 1H), 8.43 (s, 1H), 8.09 (d, *J* = 8.8 Hz, 2H), 8.02 **-** 7.86 (m, 2H), 7.86 **-** 7.77 (m, 2H), 7.70 (s, 1H), 7.64 **-** 7.49 (m, 1H), 7.30 (s, 1H), 7.25 **-** 7.15 (m, 2H), 6.28 (d, *J* = 337.7 Hz, 1H), 1.97 **-** 1.87 (m, 1H), 1.02 **-** 0.85 (m, 2H), 0.85 **-** 0.64 (m, 2H). MS (ESI) m/z calcd for C₂₇H₂₂FN₇O [M⁺], 479.2 ; found, 480.5 [M⁺ + H⁺].

### 4-((4-((5-cyclopropyl-1G-pyrazol-3-yl)amino)quinazolin-2-yl)amino)-M-phenethylbenzamide (4an)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, phenylethylamine (0.065mL, 0.52 mmol) was added and stirred at room temperature for overnight. To the reaction mixture, EtOAc was added. The organic layer was washed with water and brine several times. The combined organic layer was dried over anhydrous Na₂SO₄, filtered and purified by column chromatography (5% DCM/MeOH) to afford a pale pink solid. (49 mg, 39%)

¹H NMR (400 MHz, DMSO) δ 12.53 (d, *J* = 245.5 Hz, 1H), 10.37 (d, *J* = 202.2 Hz, 1H), 9.70 (d, *J* = 262.3 Hz, 1H), 8.52 **-** 8.30 (m, 2H), 8.00 (d, *J* = 8.4 Hz, 2H), 7.87 **-** 7.50 (m, 4H), 7.38 **-** 7.18 (m, 6H), 6.27 (d, *J* = 345.3 Hz, 1H), 3.49 (dd, *J* = 13.9, 6.0 Hz, 2H), 2.86 (t, *J* = 7.5 Hz, 2H), 2.01 **-** 1.85 (m, 1H), 1.02 **-** 0.84 (m, 2H), 0.84 **-** 0.64 (m, 2H). MS (ESI) m/z calcd for C₂₉H₂₇N₇O [M⁺], 489.2 ; found, 490.5 [M⁺ + H⁺].

### 4-((4-((5-cyclopropyl-1G-pyrazol-3-yl)amino)quinazolin-2-yl)amino)-M-(4-fluorobenzyl)benzamide (4ao)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, (4-fluorophenyl)methanamine (0.059mL, 0.52 mmol) was added and stirred at room temperature for overnight. To the reaction mixture, EtOAc was added. The organic layer was washed with water and brine several times. The combined organic layer was dried over anhydrous Na₂SO₄, filtered and purified by column chromatography (5% DCM/MeOH) to afford a pale pink solid. (75 mg, 59%)

¹H NMR (400 MHz, DMSO) δ 12.52 (d, *J* = 244.1 Hz, 1H), 10.38 (d, *J* = 201.6 Hz, 1H), 9.72 (d, *J* = 258.9 Hz, 1H), 8.88 (s, 1H), 8.54 **-** 8.28 (m, 1H), 8.02 (d, *J* = 8.5 Hz, 2H), 7.95 **-** 7.79 (m, 2H), 7.77 **-** 7.48 (m, 2H), 7.42 **-** 7.21 (m, 3H), 7.21 **-** 7.10 (m, 2H), 6.26 (d, *J* = 344.8 Hz, 1H), 4.47 (d, *J* = 5.9 Hz, 2H), 2.00 **-** 1.86 (m, 1H), 1.02 **-** 0.84 (m, 2H), 0.84 **-** 0.64 (m, 2H). MS (ESI) m/z calcd for C₂₈H₂₄FN₇O [M⁺], 493.2 ; found, 494.6 [M⁺ + H⁺].

### 4-((4-((5-cyclopropyl-1G-pyrazol-3-yl)amino)quinazolin-2-yl)amino)-M-(2,4-difluorobenzyl)benzamide (4ap)

To a solution of compound **4r** (100 mg, 0.26 mmol) in DMF (2 mL) and then added DIPEA (0.181 mL, 1.04 mmol), EDC.HCl (99 mg, 0.52 mmol) and HOBt (70 mg, 0.52 mmol). The reaction mixture was stirred at room temperature for 20 min. To the mixture, (2,4-difluorophenyl)methanamine (0.062mL, 0.52 mmol) was added and stirred at room temperature for overnight. To the reaction mixture, EtOAc was added. The organic layer was washed with water and brine several times. The combined organic layer was dried over anhydrous Na₂SO₄, filtered and purified by column chromatography (5% DCM/MeOH) to afford a pale pink solid. (75 mg, 56%)

¹H NMR (400 MHz, DMSO) δ 12.52 (d, *J* = 242.7 Hz, 1H), 10.38 (d, *J* = 196.4 Hz, 1H), 9.73 (d, *J* = 254.4 Hz, 1H), 8.85 (s, 1H), 8.53 **-** 8.28 (m, 1H), 8.02 (d, *J* = 8.6 Hz, 2H), 7.87 (s, 2H), 7.69 (s, 1H), 7.63 **-** 7.48 (m, 1H), 7.43 (td, *J* = 8.6, 6.6 Hz, 1H), 7.36 **-** 7.18 (m, 2H), 7.07 (td, *J* = 8.6, 2.6 Hz, 1H), 6.26 (d, *J* = 341.9 Hz, 2H), 4.49 (d, *J* = 5.7 Hz, 2H), 2.00 **-** 1.84 (m, 1H), 1.02 **-** 0.84 (m, 2H), 0.84 **-** 0.62 (m, 2H). MS (ESI) m/z calcd for C₂₈H₂₃F₂N₇O [M⁺], 511.2 ; found, 512.6 [M⁺ + H⁺].

### M⁴-(5-cyclopropyl-1G-pyrazol-3-yl)-M²-(1G-pyrrolo[2,3-b]pyridin-5-yl)quinazoline-2,4-diamine (4bg)

Compound **2a** (100 mg, 0.35 mmol), 1*H*-pyrrolo[2,3-*b*]pyridin-5-amine (70 mg, 0.53 mmol) and amine (1.5 eq, 0.53 mmol) in ethanol (2 mL) was added 0-1 µL of concentrated HCl. The reaction mixture was stirred at 120°C in a sealed vial for 2-24 h. The reaction was cooled and solvent was evaporated. The crude was purified by filtration washed with MeOH or EA to afford compound **4bg** as a grey solid. (125 mg, 94% yield.)

¹H NMR (300 MHz, DMSO-d₆) δ 12.52 (s, 1H), 11.89 (s, 1H), 11.47 (s, 1H), 10.32 (s, 1H), 8.68 (d, *J* = 8.3 Hz, 1H), 8.30 (s, 1H), 8.08 (s, 1H), 7.84 (t, *J* = 7.2 Hz, 1H), 7.64 (d, *J* = 8.3 Hz, 1H), 7.61 **-** 7.54 (m, 1H), 7.46 (t, *J* = 7.7 Hz, 1H), 6.54 (s, 1H), 5.58 (s, 1H), 1.54 (s, 1H), 0.75 (s, 2H), 0.11 (s, 2H). ¹³C NMR (101 MHz, DMSO-d₆) δ 157.83, 153.68, 147.44, 146.52, 141.16, 139.80, 135.81, 128.02, 126.00, 125.18, 120.05, 117.85, 110.79, 100.70, 95.17, 95.15, 8.15, 7.05. MS (ESI) m/z calcd for C₂₁H₁₈N₈ [M⁺], 382.4 ; found, 383.3 [M⁺ + H⁺].

### M⁴-(5-cyclopropyl-1G-pyrazol-3-yl)-M²-(imidazo[1,2-a]pyridazin-3-yl)quinazoline-2,4-diamine (4bh)

Compound **2a** (100 mg, 0.35 mmol) and imidazo[1,2-*b*]pyridazin-3-amine (70 mg, 0.53 mmol) reacted using the same method as **4bg** and purified by prep TLC to afford compound **4bh** as a green solid. (38 mg, 29% yield.)

¹H NMR (300 MHz, DMSO- d₆) δ 12.80 (s, 1H), 10.92 (s, 1H), 9.95 (d, *J* = 9.5 Hz, 1H), 9.16 (dd, *J* = 4.6, 1.5 Hz, 1H), 8.72 (d, *J* = 8.3 Hz, 1H), 8.06 **-** 7.86 (m, 4H), 7.71 **-** 7.61 (m, 1H), 6.67 (s, 2H), 6.23 (s, 1H), 2.04 (td, *J* = 8.6, 4.4 Hz, 1H), 1.09 **-** 0.96 (m, 2H), 0.87 **-** 0.73 (m, 2H). ¹³C NMR (101 MHz, DMSO) δ 159.33, 150.78, 149.85, 148.11, 146.80, 146.53, 136.62, 134.85, 132.02, 127.72, 127.13, 125.95, 124.83, 124.40, 114.29, 99.82, 95.56, 8.64, 7.42. MS (ESI) m/z calcd for C₂₀H₁₇N₉ [M⁺], 383.4 ; found, 384.3 [M⁺ + H⁺].

### 5-((4-((5-cyclopropyl-1G-pyrazol-3-yl)amino)quinazolin-2-yl)amino)indolin-2-one (4bi)

Compound **2a** (100 mg, 0.35 mmol) and 5-aminoindolin-2-one (78 mg, 0.53 mmol) reacted using the same method as **4bg**, to afford compound **4bi** as a dark green solid. (66 mg, 48% yield.

¹H NMR (400 MHz, DMSO-d₆) δ 12.57 (s, 1H), 11.40 (s, 1H), 10.57 (s, 1H), 10.28 (s, 1H), 8.64 (d, *J* = 8.3 Hz, 1H), 7.83 (t, *J* = 7.8 Hz, 1H), 7.61 (d, *J* = 8.3 Hz, 1H), 7.54 **-** 7.16 (m, 3H), 6.91 (d, *J* = 8.2 Hz, 1H), 6.01 (s, 1H), 3.53 (s, 2H), 1.81 (s, 1H), 0.90 (d, *J* = 8.2 Hz, 2H), 0.51 (s, 2H). ¹³C NMR (101 MHz, DMSO) δ 176.80, 158.00, 153.14, 146.41, 142.48, 135.76, 130.41, 126.97, 125.01, 122.23, 122.16, 118.09, 110.73, 109.85, 95.66, 36.50, 8.19, 7.23. MS (ESI) m/z calcd for C₂₂H₁₉N₇O [M⁺], 397.4 ; found, 398.3 [M⁺ + H⁺].

### N⁴-(5-cyclopropyl-1H-pyrazol-3-yl)quinazoline-2,4-diamine (4ca)

A mixture of compound **2a** (200 mg, 0.7 mmol) and 2,4-Dimethoxybenzylamine (0.315 mL, 2.10 mmol) was irradiated with microwave reactor at 150°C for 1.5 h. The resulting mixture was concentrated under reduced pressure and diluted with EtOAc. The organic layer was washed with water and dried over Na₂SO₄, filtered and concentrated. The crude was purified by column chromatography. The residue was triturated with DCM and diethyl ether to afford a pale yellow solid. (221 mg). A solution of *N*⁴-(5-cyclopropyl-1*H*-pyrazol-3-yl)-*N*²-(2,4-dimethoxybenzyl)quinazoline-2,4-diamine (70 mg, 0.168 mmol) in 25% TFA in DCE was stirred at room temperature for 4 h. The reaction mixture was diluted with EtOAc and washed with saturated NaHCO₃ solution. The organic layer was washed with water and dried over Na₂SO₄, filtered and concentrated. The residue was triturated with diethyl ether to afford compound **4ca** as a pale yellow solid. (23 mg, 51%)

### N⁴-(5-cyclopropyl-1H-pyrazol-3-yl)-N²-methylquinazoline-2,4-diamine (4cb)

The mixture of compound **2a** (71.4 mg, 0.25 mmol) and methylamine hydrochloride (50.6 mg, 0.75 mmol) in IPA (0.3 M) was irradiated with microwave reactor at 150°C for 0.5 h. The reaction mixture was triturated with diethyl ether. The filter cake was neutralized with saturated NaHCO₃ solution and extracted with EtOAc and water. The organic layer was dried over Na₂SO₄, filtered and concentrated. The residue was purified by column chromatography (5% (7 N Ammonia in MeOH) in EtOAc:Hex=2:1) and triturated with diethyl ether to afford compound **4cb** as a pale yellow solid. (3 mg, 5% yield.)

¹H NMR (400 MHz, DMSO-d₆) δ 12.39 (d, *J* = 217.0 Hz, 1H), 10.08 (d, *J* = 143.9 Hz, 1H), 8.28 (s, 1H), 7.61 **-** 7.49 (m, 1H), 7.40 **-** 7.24 (m, 2H), 7.20 **-** 6.98 (m, 1H), 6.70 (s, 1H), 2.86 (d, *J* = 4.6 Hz, 3H), 1.92 **-** 1.80 (m, 1H), 0.98 **-** 0.85 (m, 2H), 0.79 **-** 0.63 (m, 2H) MS (ESI) m/z calcd for C₁₅H₁₆N₆ [M⁺], 280.1 ; found, 281.1 [M⁺ + H⁺].

### N²-benzyl-N⁴-(5-cyclopropyl-1H-pyrazol-3-yl)quinazoline-2,4-diamine (4cc)

The mixture of compound **2a** (71.4 mg, 0.25 mmol) and benzylamine (246 µL, 2.25 mmol) was irradiated with microwave reactor at 150°C for 1.5 h. The reaction mixture was triturated with Diethyl ether. The filtrate was neutralized with saturated NaHCO₃ solution and extracted with EtOAc and water. The organic residue was dried over Na₂SO₄, filtered and concentrated. The residue was purified by column chromatography to afford the desired product (3% (7N Ammonia in MeOH) in EtOAc:Hex=1:2-1:1). The product was triturated with DCM and Diethyl ether to afford compound **4cc** as a pale yellow solid. (54 mg, 61% yield.)

¹H NMR (400 MHz, DMSO- d₆) δ 12.42 (d, *J* = 257.5 Hz, 1H), 10.13 (d, *J* = 193.8 Hz, 1H), 8.27 (d, *J* = 44.5 Hz, 1H), 7.51 **-** 7.04 (m, 10H), 4.59 (d, *J* = 6.0 Hz, 2H), 1.91 **-** 1.76 (m, 1H), 0.96 **-** 0.80 (m, 2H), 0.77 **-** 0.61 (m, 2H). MS (ESI) m/z calcd for C₂₁H₂₀N₆ [M⁺], 356.2 ; found, 357.1 [M⁺ + H⁺].

### N⁴-(5-cyclopropyl-1H-pyrazol-3-yl)-N²-(4-methoxybenzyl)quinazoline-2,4-diamine (4cd)

The mixture of compound **2a** (71.4 mg, 0.25 mmol), 4-methoxybenzylamine (0.147 mL, 0.750 mmol) was irradiated with microwave reactor at 150°C for 1.0 h. The reaction mixture was triturated with Diethyl ether. The filter cake was neutralized with saturated NaHCO₃ solution and extracted with EtOAc and water. The organic layer was dried over Na₂SO₄, filtered and concentrated. The residue was triturated with DCM and Diethyl ether to afford compound **4cd** as a white solid. (46 mg, 48% yield.)

¹H NMR (400 MHz, DMSO- d₆) δ 12.20 (d, *J* = 251.2 Hz, 1H), 10.12 (d, *J* = 189.7 Hz, 1H), 8.26 (d, *J* = 51.1 Hz, 1H), 7.51 **-** 7.28 (m, 5H), 7.09 (d, *J* = 42.3 Hz, 2H), 6.99 **-** 6.81 (m, 2H), 4.51 (d, *J* = 6.0 Hz, 2H), 3.71 (s, 3H), 1.92 **-** 1.79 (m, 1H), 0.96 **-** 0.83 (m, 2H), 0.80 **-** 0.48 (m, 2H) MS (ESI) m/z calcd for C₂₂H₂₂N₆O [M⁺], 386.2 ; found, 387.1 [M⁺ + H⁺].

### N⁴-(5-cyclopropyl-1H-pyrazol-3-yl)-N²-phenethylquinazoline-2,4-diamine (4ce)

The mixture of compound **2a** (71.4 mg, 0.25 mmol) and 2-phenylethan-1-amine (0.303 mL, 2.40 mmol) in IPA (0.3 M) was irradiated with microwave reactor at 150°C for 1.5 h. The reaction mixture was triturated with Diethyl ether. The filter cake was neutralized with saturated NaHCO₃ solution and extracted with EtOAc and water. The organic layer was dried over Na₂SO₄, filtered and concentrated. The residue was triturated with DCM and Diethyl ether to afford compound **4ce** as a white solid. (41 mg, 44% yield.)

### 2-((4-((5-cyclopropyl-1G-pyrazol-3-yl)amino)quinazolin-2-yl)amino)ethan-1-ol hydrochloride (4cf)

A mixture of compound **2a** (71.4 mg, 0.25 mmol) and 2-aminoethan-1-ol (45.8 mg, 0.75 mmol) in IPA (0.833 mL) was irradiated with microwave reactor at 150°C for 0.5 h. The reaction mixture was triturated with Diethyl ether. The filter cake was neutralized with saturated NaHCO₃ solution and extracted with EtOAc and water. The organic residue was dried over Na₂SO₄, filtered and concentrated. The residue was dissolved in MeOH, concentrated, and then triturated with Diethyl ether. The residue was dissolved in 20% MeOH in DCM, concentrated, and triturated with n-Hexane. The half of obtained solid was dissolved in DCM, 4 M HCl in Dioxane (64.4 µL, 0.26 mmol) dropwise at 0°C. The mixture was warmed to r.t. and stirred for 0.5 h. The reaction mixture was concentrated and triturated with Diethyl ether to afford compound **4cf** as a white solid. (16 mg, 19% yield.)

MS (ESI) m/z calcd for C₁₆H₁₈N₆O [M⁺], 310.2 ; found, 311.1 [M⁺ + H⁺].

### tert-butyl 6-((4-((5-cyclopropyl-1H-pyrazol-3-yl)amino)quinazolin-2-yl)amino)-3-azabicyclo[3.1.0]hexane-3-carboxylate (4cg)

Compound **2a** (70 mg, 0.25 mmol) was dissolved in t-butanol (3 mL), and a solution of tert-butyl 6-amino-3-azabicyclo[3.1.0]hexane-3-carboxylate (73 mg, 0.37 mmol) in t-butanol (0.5 mL) was added to the mixture. DIPEA (48 mg, 0.37 mmol) was added and the reaction was heated at 110°C while stirring. After 4 days, the solvent was evaporated, purified by column chromatography (4% DCM/MeOH) to afford compound **4cg**. (20 mg, 18% yield.)

MS (ESI) m/z calcd C₂₄H₂₉N₇O₂ [M⁺],447.2 found, 448.6 [M⁺ + H⁺].

### 4-(((4-((5-cyclopropyl-1G-pyrazol-3-yl)amino)quinazolin-2-yl)amino)methyl)benzonitrile (4ch)

Compound 2a (150 mg, 0.53 mmol) and 4-(Aminomethyl)benzonitrile (83 mg, 0.63 mmol) were dissolved in tert-butanol (2 mL). 37% HCl in water (0.010 mL) was added and the reaction was heated at 110°C while stirring. After 2 days, the white solid was recovered by column chromatography (3% DCM/MeOH) to afford compound **4ch**. (20 mg, 10% yield.)

¹H NMR (300 MHz, DMSO) δ 12.44 (d, *J* = 181.1 Hz, 1H), 10.20 (d, *J* = 136.1 Hz, 1H), 8.46 **-** 8.17 (m, 1H), 7.79 (d, *J* = 7.8 Hz, 2H), 7.71 **-** 7.40 (m, 3H), 7.40 **-** 7.21 (m, 1H), 7.21 **-** 6.99 (m, 1H), 6.73 (s, 1H), 4.68 (s, 2H), 1.86 (s, 1H), 1.04 **-** 0.27 (m, 4H). MS (ESI) m/z calcd for C₂₂H₁₉N₇ [M⁺], 381.2 ; found, 382.4 [M⁺ + H⁺].

### N⁴-(5-cyclopropyl-1H-pyrazol-3-yl)-N²-(1H-indol-4-yl)quinazoline-2,4-diamine (4da)

Compound **2a** (100 mg, 0.35 mmol) and 1*H*-indol-4-amine (69 mg, 0.53 mmol) reacted to afford compound **4da** as a charcoal solid. (55 mg, 42% yield.)

¹H NMR (300 MHz, DMSO- d₆) δ 12.56 (s, 1H), 11.44 (d, *J* = 7.7 Hz, 2H), 10.56 (s, 1H), 8.68 (d, *J* = 8.3 Hz, 1H), 7.85 (t, *J* = 7.8 Hz, 1H), 7.58 (d, *J* = 8.3 Hz, 1H), 7.52 **-** 7.37 (m, 3H), 7.37 **-** 7.27 (m, 1H), 7.17 (t, *J* = 7.8 Hz, 1H), 6.59 (s, 1H), 6.10 (s, 1H), 1.82 (s, 1H), 0.98 **-** 0.84 (m, 2H), 0.56 (s, 2H). ¹³C NMR (101 MHz, DMSO-d₆) δ 158.25, 152.62, 146.50, 139.67, 137.47, 135.92, 126.07, 125.22, 125.14, 123.24, 121.60, 117.71, 110.74, 110.44, 99.45, 95.74, 8.39, 7.28. MS (ESI) m/z calcd for C₂₂H₁₉N₇ [M⁺], 381.4 ; found, 382.3 [M⁺ + H⁺].

### N⁴-(5-cyclopropyl-1H-pyrazol-3-yl)-N²-(1H-indol-5-yl)quinazoline-2,4-diamine (4db)

Compound **2a** (100 mg, 0.35 mmol) and 1*H*-indol-5-amine (69 mg, 0.53 mmol) reacted to afford compound **4db** as a white solid. (109 mg, 82% yield.)

¹H NMR (300 MHz, DMSO- d₆) δ 12.51 (s, 1H), 11.40 (d, *J* = 16.1 Hz, 2H), 10.30 (s, 1H), 8.67 (d, *J* = 8.3 Hz, 1H), 7.89 **-** 7.75 (m, 1H), 7.71 **-** 7.57 (m, 2H), 7.52 (d, *J* = 8.5 Hz, 1H), 7.48 **-** 7.38 (m, 2H), 7.16 (dd, *J* = 8.6, 2.1 Hz, 1H), 6.50 (s, 1H), 5.78 (s, 1H), 1.57 (s, 1H), 0.76 (s, 2H), 0.14 (s, 2H). ¹³C NMR (101 MHz, DMSO-d₆) δ 157.81, 153.25, 146.45, 139.72, 135.80, 128.35, 127.10, 125.02, 119.66, 117.50, 112.38, 110.67, 101.91, 95.55, 8.13, 7.07. MS (ESI) m/z calcd for C₂₂H₁₉N₇ [M⁺], 381.4 ; found, 382.3 [M⁺ + H⁺].

### N⁴-(5-cyclopropyl-1H-pyrazol-3-yl)-N²-(1H-indol-6-yl)quinazoline-2,4-diamine (4dc)

Compound **2a** (100 mg, 0.35 mmol) and 1*H*-indol-6-amine (69 mg, 0.53 mmol) reacted to afford compound **4dc** as a dark brown solid. (90 mg, 68% yield.)

¹H NMR (300 MHz, DMSO- d₆) δ 12.49 (s, 1H), 11.34 (d, *J* = 28.9 Hz, 2H), 10.43 (s, 1H), 8.66 (d, *J* = 8.4 Hz, 1H), 7.82 (t, *J* = 7.8 Hz, 1H), 7.73 **-** 7.51 (m, 3H), 7.51 **-** 7.34 (m, 2H), 7.12 (dd, *J* = 8.4, 1.9 Hz, 1H), 6.50 (s, 1H), 6.11 (s, 1H), 1.69 (s, 1H), 0.79 (s, 2H), 0.38 (s, 2H). ¹³C NMR (101 MHz, DMSO-d₆) δ 157.70, 153.26, 147.03, 136.34, 135.60, 126.70, 124.97, 120.86, 117.14, 110.85, 108.49, 101.68, 95.24, 8.16, 7.31. MS (ESI) m/z calcd for C₂₂H₁₉N₇ [M⁺], 381.4 ; found, 382.3 [M⁺ + H⁺].

### N⁴-(5-cyclopropyl-1H-pyrazol-3-yl)-N²-(1H-indazol-5-yl)quinazoline-2,4-diamine (4dd)

Compound **2a** (100 mg, 0.35 mmol) and 1H-indazol-5-amine (70 mg, 0.53 mmol) reacted to afford compound **4dd** as a green solid. (104 mg, 78% yield.)

¹H NMR (300 MHz, DMSO-d₆) δ 13.31 (s, 1H), 12.55 (s, 1H), 11.47 (s, 1H), 10.50 (s, 1H), 8.67 (d, *J* = 8.3 Hz, 1H), 8.14 (s, 1H), 7.91 (s, 1H), 7.85 (t, *J* = 7.8 Hz, 1H), 7.64 (t, *J* = 8.7 Hz, 2H), 7.52 **-** 7.37 (m, 2H), 5.83 (s, 1H), 1.63 (s, 1H), 0.76 (s, 2H), 0.20 (s, 2H). ¹³C NMR (101 MHz, DMSO-d₆) δ 158.03, 153.21, 146.35, 139.74, 138.86, 135.84, 134.13, 125.12, 123.44, 111.28, 110.72, 95.56, 8.08, 7.03. MS (ESI) m/z calcd for C₂₁H₁₈N₈ [M⁺], 382.4 ; found, 383.3 [M⁺ + H⁺].

### N⁴-(5-cyclopropyl-1H-pyrazol-3-yl)-N²-(1H-indazol-6-yl)quinazoline-2,4-diamine (4de)

Compound **2a** (100 mg, 0.35 mmol) and 1H-indazol-6-amine (70 mg, 0.53 mmol) reacted to afford compound **4de** as an ivory solid. (133 mg, 99% yield.)

¹H NMR (300 MHz, DMSO-d₆) δ 13.20 (s, 1H), 12.58 (s, 1H), 11.51 (s, 1H), 10.70 (s, 1H), 8.69 (d, *J* = 8.3 Hz, 1H), 8.13 (s, 1H), 7.93 **-** 7.80 (m, 2H), 7.76 (s, 1H), 7.65 (dd, *J* = 8.4, 1.1 Hz, 1H), 7.48 (s, 1H), 7.24 (dd, *J* = 8.6, 1.7 Hz, 1H), 6.15 (s, 1H), 1.69 (s, 1H), 0.93 **-** 0.57 (m, 2H), 0.34 (s, 2H). ¹³C NMR (101 MHz, DMSO-d₆) δ 158.00, 152.80, 140.57, 135.85, 133.94, 125.23, 125.09, 121.51, 118.50, 110.85, 95.42, 8.06, 7.10. MS (ESI) m/z calcd for C₂₁H₁₈N₈ [M⁺], 382.4 ; found, 383.3 [M⁺ + H⁺].

### N²-(1H-benzo[d]imidazol-5-yl)-N⁴-(5-cyclopropyl-1H-pyrazol-3-yl)quinazoline-2,4-diamine (4df)

Compound **2a** (100 mg, 0.35 mmol) and 1*H*-benzo[*d*]imidazol-5-amine (70 mg, 0.53 mmol) reacted to afford compound **4df** as a white solid. (66 mg, 49% yield.)

¹H NMR (300 MHz, DMSO-d₆) δ 12.51 (s, 1H), 11.08 (s, 1H), 10.25 (s, 1H), 8.75 (s, 1H), 8.60 (d, *J* = 8.3 Hz, 1H), 8.09 (s, 1H), 7.79 (t, *J* = 7.7 Hz, 1H), 7.73 (d, *J* = 8.7 Hz, 1H), 7.60 (d, *J* = 8.3 Hz, 1H), 7.51 (d, *J* = 8.7 Hz, 1H), 7.40 (t, *J* = 7.7 Hz, 1H), 6.18 (s, 1H), 1.77 (s, 1H), 0.97 - 0.68 (m, 2H), 0.48 (s, 2H). ¹³C NMR (101 MHz, DMSO-d₆) δ 157.65, 154.35, 142.06, 134.99, 124.58, 124.32, 120.10, 115.70, 111.30, 8.13. MS (ESI) m/z calcd for C₂₁H₁₈N₈ [M⁺], 382.4 ; found, 383.3 [M⁺ + H⁺].

### N⁴-(5-cyclopropyl-1H-pyrazol-3-yl)-N²-(1H-pyrrolo[2,3-b]pyridin-5-yl)quinazoline-2,4-diamine (4dg)

Compound **2a** (100 mg, 0.35 mmol) and 1*H*-pyrrolo[2,3-*b*]pyridin-5-amine (70 mg, 0.53 mmol) reacted to afford compound **4dg** as a grey solid. (125 mg, 94% yield.)

¹H NMR (300 MHz, DMSO-d₆) δ 12.52 (s, 1H), 11.89 (s, 1H), 11.47 (s, 1H), 10.32 (s, 1H), 8.68 (d, *J* = 8.3 Hz, 1H), 8.30 (s, 1H), 8.08 (s, 1H), 7.84 (t, *J* = 7.2 Hz, 1H), 7.64 (d, *J* = 8.3 Hz, 1H), 7.61 **-** 7.54 (m, 1H), 7.46 (t, *J* = 7.7 Hz, 1H), 6.54 (s, 1H), 5.58 (s, 1H), 1.54 (s, 1H), 0.75 (s, 2H), 0.11 (s, 2H). ¹³C NMR (101 MHz, DMSO-d₆) δ 157.83, 153.68, 147.44, 146.52, 141.16, 139.80, 135.81, 128.02, 126.00, 125.18, 120.05, 117.85, 110.79, 100.70, 95.17, 95.15, 8.15, 7.05. MS (ESI) m/z calcd for C₂₁H₁₈N₈ [M⁺], 382.4 ; found, 383.3 [M⁺ + H⁺].

### N⁴-(5-cyclopropyl-1H-pyrazol-3-yl)-N²-(imidazo[1,2-b]pyridazin-3-yl)quinazoline-2,4-diamine (4dh)

Compound **2a** (100 mg, 0.35 mmol) and imidazo[1,2-*b*]pyridazin-3-amine (70 mg, 0.53 mmol) reacted and purified by prep TLC to afford compound **4dh** as a green solid. (38 mg, 29% yield.)

¹H NMR (300 MHz, DMSO- d₆) δ 12.80 (s, 1H), 10.92 (s, 1H), 9.95 (d, *J* = 9.5 Hz, 1H), 9.16 (dd, *J* = 4.6, 1.5 Hz, 1H), 8.72 (d, *J* = 8.3 Hz, 1H), 8.06 **-** 7.86 (m, 4H), 7.71 **-** 7.61 (m, 1H), 6.67 (s, 2H), 6.23 (s, 1H), 2.04 (td, *J* = 8.6, 4.4 Hz, 1H), 1.09 **-** 0.96 (m, 2H), 0.87 **-** 0.73 (m, 2H). ¹³C NMR (101 MHz, DMSO) δ 159.33, 150.78, 149.85, 148.11, 146.80, 146.53, 136.62, 134.85, 132.02, 127.72, 127.13, 125.95, 124.83, 124.40, 114.29, 99.82, 95.56, 8.64, 7.42. MS (ESI) m/z calcd for C₂₀H₁₇N₉ [M⁺], 383.4 ; found, 384.3 [M⁺ + H⁺].

### 5-((4-((5-cyclopropyl-1G-pyrazol-3-yl)amino)quinazolin-2-yl)amino)indolin-2-one (4di)

Compound **2a** (100 mg, 0.35 mmol) and 5-aminoindolin-2-one (78 mg, 0.53 mmol) reacted to afford compound **4di** as a dark green solid. (66 mg, 48% yield.)

¹H NMR (400 MHz, DMSO-d₆) δ 12.57 (s, 1H), 11.40 (s, 1H), 10.57 (s, 1H), 10.28 (s, 1H), 8.64 (d, *J* = 8.3 Hz, 1H), 7.83 (t, *J* = 7.8 Hz, 1H), 7.61 (d, *J* = 8.3 Hz, 1H), 7.54 **-** 7.16 (m, 3H), 6.91 (d, *J* = 8.2 Hz, 1H), 6.01 (s, 1H), 3.53 (s, 2H), 1.81 (s, 1H), 0.90 (d, *J* = 8.2 Hz, 2H), 0.51 (s, 2H). ¹³C NMR (101 MHz, DMSO) δ 176.80, 158.00, 153.14, 146.41, 142.48, 135.76, 130.41, 126.97, 125.01, 122.23, 122.16, 118.09, 110.73, 109.85, 95.66, 36.50, 8.19, 7.23. MS (ESI) m/z calcd for C₂₂H₁₉N₇O [M⁺], 397.4 ; found, 398.3 [M⁺ + H⁺].

### M-(5-cyclopropyl-1G-pyrazol-3-yl)-2-(1,4-dioxa-8-azaspiro[4.5]decan-8-yl)quinazolin-4-amine (6a)

Compound **2a** (70 mg, 0.25 mmol) was dissolved in t-butanol (3 mL), and a solution of amine (1.5 eq, 0.37 mmol) in t-butanol (0.5 mL) was added to the mixture. DIPEA (1.5 eq, 0.37 mmol) and 1,4-dioxa-8-azaspiro[4,5]decane (53 mg, 0.37 mmol) was added and the reaction was heated at 110°C while stirring for 30 h-4 days. The solvent was evaporated, purified by column chromatography (DCM/MeOH) to afford compound **6a**. (77 mg, 80% yield.)

¹H NMR (300 MHz, DMSO-d₆) δ 12.20 (s, 1H), 10.00 (s, 1H), 8.34 (d, *J* = 8.1 Hz, 1H), 7.61-7.48 (m, 1H), 7.32 (d, *J* = 8.3 Hz, 1H), 7.15-7.03 (m, 1H), 6.33 (s, 1H), 3.94 (s, 5H), 3.94-3.84 (m, 7H), 2.00-1.84 (m, 1H), 1.73-1.58 (m, 4H), 1.03-0.90 (m, 2H), 0.73-0.62 (m, 2H). MS (ESI) m/z calcd C₂₁H₂₄N₆O₂ [M⁺], 392.2 ; found, 393.4 [M⁺ + H⁺].

### sdqs-butyl 9-(4-((5-cyclopropyl-1G-pyrazol-3-yl)amino)quinazolin-2-yl)-3,9-diazaspiro[5.5]undecane-3-carboxylate (6b)

Compound **2a** (70 mg, 0.25 mmol) and tert-butyl 3,9-diazaspiro[5.5]undecane-3-carboxylate (93 mg, 0.37 mmol) reacted to afford compound **6b**. (33 mg, 27% yield.)

¹H NMR (300 MHz, DMSO-d₆) δ 12.16 (s, 1H), 9.95 (s, 1H), 8.32 (d, *J* = 8.2 Hz, 1H), 7.59-7.48 (m, 1H), 7.36-7.26 (m, 1H), 7.07 (t, *J* = 7.5 Hz, 1H), 6.35 (s, 1H), 3.87-3.73 (m, 4H), 1.98-1.86 (m, 1H), 1.56-1.33 (m, 17H), 1.02-0.91 (m, 2H), 0.73-0.63 (m, 2H). MS (ESI) m/z calcd C₂₈H₃₇N₇O₂ [M⁺], 503.3 ; found, 504.6 [M⁺ + H⁺].

### sdqs-butyl 7-(4-((5-cyclopropyl-1G-pyrazol-3-yl)amino)quinazolin-2-yl)-2,7-diazaspiro[3.5]nonane-2-carboxylate (6c)

Compound **2a** (70 mg, 0.25 mmol) and tert-butyl 2,7-diazaspiro[3,5]nonane-2-carboxylate (83 mg, 0.37 mmol) reacted to afford compound **6c**. (75 mg, 64% yield.)

¹H NMR (300 MHz, DMSO-d₆) δ 12.19 (s, 1H), 9.97 (s, 1H), 8.32 (d, *J* = 8.2 Hz, 1H), 7.60-7.48 (m, 1H), 7.31 (d, *J* = 8.3 Hz, 1H), 7.14-7.02 (m, 1H), 6.33 (s, 1H), 3.76 (s, 4H), 3.62 (s, 4H), 2.00-1.85 (m, 1H), 1.70 (t, *J* = 5.4 Hz, 4H), 1.40 (s, 9H), 1.04-0.91 (m, 2H), 0.75-0.63 (m, 2H). MS (ESI) m/z calcd C₂₆H₃₃N₇O₂ [M⁺],475.3 found, 476.6 [M⁺ + H⁺].

### sdqs-butyl 2-(4-((5-cyclopropyl-1G-pyrazol-3-yl)amino)quinazolin-2-yl)-2,7-diazaspiro[3.5]nonane-7-carboxylate (6d)

Compound **2a** (70 mg, 0.25 mmol) and tert-butyl 2,7-diazaspiro[3,5]nonane-7-carboxylate (83 mg, 0.37 mmol) reacted to afford compound 6c. (92 mg, 79% yield.)

¹H NMR (300 MHz, DMSO-d₆) δ 12.11 (s, 1H), 10.08 (s, 1H), 8.37 (d, *J* = 7.6 Hz, 1H), 7.61-7.48 (m, 1H), 7.32 (d, *J* = 8.3 Hz, 1H), 7.10 (t, *J* = 7.5 Hz, 1H), 6.58 (s, 1H), 3.82 (s, 4H), 2.01-1.85 (m, 1H), 1.71 (t, *J* = 5.5 Hz, 4H), 1.41 (s, 9H), 1.01-0.88 (m, 2H), 0.78-0.66 (m, 2H). MS (ESI) m/z calcd C₂₆H₃₃N₇O₂ [M⁺], 475.3 ; found, 476.5 [M⁺ + H⁺].

The compound represented by Formula 3-5 according to the present invention can be prepared according to the Reaction Scheme 3 but is not limited thereto. 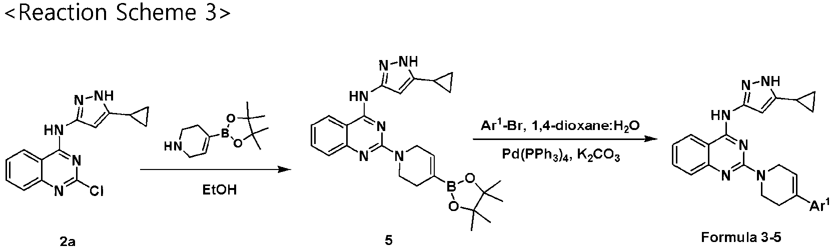 wherein: Ar¹ is the same as defined above.

### M-(5-cyclopropyl-1G-pyrazol-3-yl)-2-(4-(4,4,5,5-tetramethyl-1,3,2-dioxaborolan-2-yl)-3,6-dihydropyridin-1(2G)-yl)quinazolin-4-amine (5)

A mixture of compound **2a** (1.0 g, 3.50 mmol) and 4-(4,4,5,5-tetramethyl 1,3,2-dioxaborolan-2-yl)-1,2,3,6-tetrahydropyridine (1.70 g, 5.25 mmol) in ethanol (10 mL) was stirred at 120°C in a sealed vial for 4 h. The reaction was cooled and evaporated in vacuum. The solid was filtered with 3% DCM/MeOH to afford compound **5** as a white solid. (1.25 g, 78%)

### M-(5-cyclopropyl-1G-pyrazol-3-yl)-2-(4-phenyl-3,6-dihydropyridin-1(2G)-yl)quinazolin-4-amine (6e)

Compound **5** (100 mg, 0.22 mmol) and bromobenzene (51 mg, 0.33 mmol) were dissolved in 1,4-dioxane : H₂O : 5:1 solution. K₂CO₃ (2.5 eq), Pd(PPh₃)₄ (10 mol%) was added and stirred with microwave reactor at 110°C for 1 h. The reaction mixture was filtered through celite, extracted with EtOAc and washed with water. The organic layer was washed with brine, dried over anhydrous Na₂SO₄, filtered and concentrated. The crude was purified by column chromatography (DCM/MeOH) to afford compound **6e** as an orange solid. (35 mg, 40% yield.)

¹H NMR (300 MHz, DMSO- d₆) δ 12.20 (s, 1H), 10.02 (s, 1H), 8.36 (d, *J* = 8.2 Hz, 1H), 7.56 (ddd, *J* = 8.3, 6.8, 1.3 Hz, 1H), 7.53 **-** 7.46 (m, 2H), 7.36 (ddd, *J* = 7.8, 4.2, 2.6 Hz, 3H), 7.30 **-** 7.22 (m, 1H), 7.11 (t, *J* = 7.5 Hz, 1H), 6.46 (s, 1H), 6.32 (s, 1H), 4.44 (d, *J* = 3.2 Hz, 2H), 4.06 (t, *J* = 5.6 Hz, 2H), 2.59 (s, 2H), 2.05 **-** 1.87 (m, 1H), 1.04 **-** 0.88 (m, 2H), 0.78 **-** 0.65 (m, 2H).

### M-(5-cyclopropyl-1G-pyrazol-3-yl)-2-(4-(4-(trifluoromethyl)phenyl)-3,6-dihydropyridin-1(2G)-yl)quinazolin-4-amine (6f)

Compound **5** (100 mg, 0.22 mmol) and 4-bromobenzotrifluoride (74 mg, 0.33 mmol) reacted using the same method as **6e** to afford compound **6f** as an orange red solid. (55 mg, 53% yield.)

¹H NMR (300 MHz, DMSO- d₆) δ 12.21 (s, 1H), 10.04 (s, 1H), 8.37 (d, *J* = 8.2 Hz, 1H), 7.71 (s, 4H), 7.57 (t, *J* = 7.6 Hz, 1H), 7.37 (d, *J* = 8.2 Hz, 1H), 7.11 (d, *J* = 7.6 Hz, 1H), 6.51 (s, 1H), 6.46 (s, 1H), 4.47 (d, *J* = 3.2 Hz, 2H), 4.08 (t, *J* = 5.6 Hz, 2H), 2.63 (s, 2H), 1.96 (tt, *J* = 8.6, 5.0 Hz, 1H), 1.05 **-** 0.90 (m, 2H), 0.80 **-** 0.67 (m, 2H).

### M-(5-cyclopropyl-1G-pyrazol-3-yl)-2-(4-(3-(trifluoromethyl)phenyl)-3,6-dihydropyridin-1(2G)-yl)quinazolin-4-amine (6g)

Compound **5** (200 mg, 0.44 mmol) and 3-bromobenzotrifluoride (147 mg, 0.65 mmol) reacted using the same method as **6e** to afford compound **6g** as an orange solid. (106 mg, 51% yield.) ¹H NMR (500 MHz, DMSO- d₆) δ 12.21 (s, 1H), 10.04 (s, 1H), 8.38 (d, *J* = 8.2 Hz, 1H), 7.84 **-** 7.78 (m, 2H), 7.66 **-** 7.59 (m, 2H), 7.59 **-** 7.54 (m, 1H), 7.37 (d, *J* = 8.2 Hz, 1H), 7.11 (t, *J* = 7.6 Hz, 1H), 6.52 **-** 6.45 (m, 2H), 4.50 **-** 4.42 (m, 2H), 4.08 (t, *J* = 5.6 Hz, 2H), 2.69 **-** 2.59 (m, 2H), 2.01 **-** 1.92 (m, 1H), 1.03 **-** 0.95 (m, 2H), 0.79 **-** 0.70 (m, 2H).

### 2-(4-benzyl-3,6-dihydropyridin-1(2G)-yl)-M-(5-cyclopropyl-1G-pyrazol-3-yl)quinazolin-4-amine (6h)

Compound **5** (100 mg, 0.22 mmol) and benzyl bromide (56 mg, 0.33 mmol) reacted using the same method as **6e** to afford compound **6h** as an orange solid. (30 mg, 32% yield.)

¹H NMR (300 MHz, DMSO- d₆) δ 12.18 (s, 1H), 9.99 (s, 1H), 8.33 (d, *J* = 8.2 Hz, 1H), 7.60 **-** 7.47 (m, 1H), 7.38 **-** 7.25 (m, 3H), 7.25 **-** 7.14 (m, 3H), 7.08 (t, *J* = 7.3 Hz, 1H), 6.38 (s, 1H), 5.59 (s, 1H), 4.24 (s, 2H), 3.88 (t, *J* = 5.7 Hz, 2H), 3.34 (s, 2H), 2.03 (s, 2H), 1.92 (tt, *J* = 8.8, 5.0 Hz, 1H), 1.30 **-** 1.19 (m, 1H), 1.02 **-** 0.90 (m, 2H), 0.76 **-** 0.62 (m, 2H).

### M-(5-cyclopropyl-1G-pyrazol-3-yl)-2-(4-(naphthalen-1-yl)-3,6-dihydropyridin-1(2G)-yl)quinazolin-4-amine (6i)

Compound **5** (150 mg, 0.33 mmol) and 1-bromonaphthalene (102 mg, 0.49 mmol) reacted using the same method as **6e** to afford compound **6i** purified by prep TLC (3% DCM/MeOH). (19 mg, 12% yield.)

¹H NMR (400 MHz, DMSO- d₆) δ 12.19 (s, 1H), 10.05 (s, 1H), 8.38 (d, J = 8.4 Hz, 1H), 8.05 **-** 7.91 (m, 2H), 7.86 (d, *J* = 8.2 Hz, 1H), 7.58 (t, *J* = 7.8 Hz, 1H), 7.55 **-** 7.45 (m, 3H), 7.37 (t, *J* = 8.7 Hz, 2H), 7.13 (t, *J* = 7.6 Hz, 1H), 6.45 (s, 1H), 5.91 (s, 1H), 4.51 (d, *J* = 3.7 Hz, 2H), 4.19 (t, *J* = 5.4 Hz, 2H), 2.57 (s, 2H), 1.97 **-** 1.85 (m, 1H), 0.96 **-** 0.87 (m, 2H), 0.75 **-** 0.63 (m, 2H).

### M-(5-cyclopropyl-1G-pyrazol-3-yl)-2-(4-(2,4-dimethylphenyl)-3,6-dihydropyridin-1(2G)-yl)quinazolin-4-amine (6j)

Compound **5** (150 mg, 0.33 mmol) and 1-bromo-2,4-dimethylbenzene (91 mg, 0.49 mmol) reacted using the same method as **6e** to afford compound **6j** purified by prep TLC (3% DCM/MeOH).

¹H NMR (500 MHz, DMSO- d₆) δ 12.22 (s, 1H), 10.07 (s, 1H), 8.37 (d, *J* = 8.2 Hz, 1H), 7.57 (t, *J* = 7.6 Hz, 1H), 7.37 (d, *J* = 8.2 Hz, 1H), 7.12 (t, *J* = 7.6 Hz, 1H), 7.04 **-** 6.98 (m, 2H), 6.96 (d, *J* = 7.8 Hz, 1H), 6.43 (s, 1H), 5.68 (s, 1H), 4.39 (s, 2H), 4.06 (t, *J* = 5.6 Hz, 2H), 2.38 (s, 2H), 2.25 (d, J = 11.8 Hz, 6H), 1.93 (tt, *J* = 8.4, 5.0 Hz, 1H), 1.01 **-** 0.89 (m, 2H), 0.73 **-** 0.66 (m, 2H).

### M-(5-cyclopropyl-1G-pyrazol-3-yl)-2-(4-(2-fluorophenyl)-3,6-dihydropyridin-1(2G)-yl)quinazolin-4-amine (6k)

Compound **5** (150 mg, 0.33 mmol) and 1-bromo-2-fluorobenzene (86 mg, 0.49 mmol) reacted using the same method as **6e** to afford compound **6k** as a white solid. (66 mg, 47% yield.)

¹H NMR (300 MHz, DMSO- d₆) δ 12.21 (s, 1H), 10.03 (s, 1H), 8.38 (d, *J* = 8.3 Hz, 1H), 7.57 (t, *J* = 7.6 Hz, 1H), 7.46 **-** 7.27 (m, 3H), 7.26 **-** 7.15 (m, 2H), 7.11 (t, *J* = 7.6 Hz, 1H), 6.46 (s, 1H), 6.14 (t, *J* = 3.3 Hz, 1H), 4.50 **-** 4.38 (m, 2H), 4.05 (t, *J* = 5.5 Hz, 2H), 2.56 (s, 2H), 1.95 (tt, *J* = 8.5, 5.0 Hz, 1H), 1.03 **-** 0.91 (m, 2H), 0.76 **-** 0.65 (m, 2H).

### 2-(4-(4-chloro-2-methylphenyl)-3,6-dihydropyridin-1(2G)-yl)-M-(5-cyclopropyl-1G-pyrazol-3-yl)quinazolin-4-amine (6l)

Compound **5** (150 mg, 0.33 mmol) and 2-bromo-5-chlorotoluene (101 mg, 0.49 mmol) reacted using the same method as **6e** to afford compound **6l** as a pink solid. (79 mg, 53% yield.)

¹H NMR (300 MHz, DMSO- d₆) δ 12.20 (s, 1H), 10.02 (s, 1H), 8.37 (d, *J* = 8.2 Hz, 1H), 7.56 (t, *J* = 7.6 Hz, 1H), 7.35 (d, *J* = 8.2 Hz, 1H), 7.29 (d, *J* = 2.2 Hz, 1H), 7.21 (dd, *J* = 8.2, 2.2 Hz, 1H), 7.17 **-** 7.05 (m, 2H), 6.44 (s, 1H), 5.74 (t, *J* = 1.6 Hz, 1H), 4.39 (d, *J* = 3.1 Hz, 2H), 4.06 (t, *J* = 5.4 Hz, 2H), 2.39 (s, 2H), 2.27 (s, 3H), 1.93 (tt, *J* = 8.5, 5.0 Hz, 1H), 1.03 **-** 0.89 (m, 2H), 0.75 **-** 0.64 (m, 2H).

### M-(5-cyclopropyl-1G-pyrazol-3-yl)-2-(4-(2-ethylphenyl)-3,6-dihydropyridin-1(2G)-yl)quinazolin-4-amine (6m)

Compound **5** (150 mg, 0.33 mmol) and 1-bromo-2-ethylbenzene (91 mg, 0.49 mmol) reacted using the same method as **6e** to afford compound **6m** as a light orange solid. (41 mg, 28% yield.)

¹H NMR (500 MHz, DMSO- d₆) δ 12.21 (s, 1H), 10.05 (s, 1H), 8.37 (d, *J* = 8.3 Hz, 1H), 7.57 (t, *J* = 7.6 Hz, 1H), 7.37 (d, *J* = 8.3 Hz, 1H), 7.27 **-** 7.19 (m, 2H), 7.19 **-** 7.07 (m, 3H), 6.43 (s, 1H), 5.70 (s, 1H), 4.39 (d, *J* = 3.2 Hz, 2H), 4.07 (t, *J* = 5.6 Hz, 2H), 2.61 (q, *J* = 7.5 Hz, 2H), 2.40 (s, 2H), 1.93 (tt, *J* = 8.9, 5.2 Hz, 1H), 1.14 (t, *J* = 7.5 Hz, 3H), 0.98 **-** 0.90 (m, 2H), 0.73 **-** 0.65 (m, 2H).

### 2-(4-(1-(4-((5-cyclopropyl-1G-pyrazol-3-yl)amino)quinazolin-2-yl)-1,2,3,6-tetrahydropyridin-4-yl)-2-fluorophenyl)acetonitrile (6n)

Compound **5** (150 mg, 0.33 mmol) and 4-bromo-2-fluorobenzyl cyanide (105 mg, 0.49 mmol) reacted using the same method as **6e** to afford compound **6n** as a light pink solid. (74 mg, 49% yield.)

¹H NMR (300 MHz, DMSO- d₆) δ 12.21 (s, 1H), 10.03 (s, 1H), 8.37 (d, *J* = 8.3 Hz, 1H), 7.56 (t, *J* = 7.6 Hz, 1H), 7.48 **-** 7.43 (m, 1H), 7.42 **-** 7.32 (m, 3H), 7.11 (t, *J* = 7.6 Hz, 1H), 6.52 **-** 6.40 (m, 2H), 4.45 (d, *J* = 3.3 Hz, 2H), 4.12 **-** 3.98 (m, 4H), 2.58 (s, 2H), 1.96 (tt, *J* = 8.6, 5.1 Hz, 1H), 1.05 **-** 0.92 (m, 2H), 0.80 **-** 0.70 (m, 2H).

### 5-chloro-2-(1-(4-((5-cyclopropyl-1G-pyrazol-3-yl)amino)quinazolin-2-yl)-1,2,3,6-tetrahydropyridin-4-yl)benzonitrile (6o)

Compound 5 (150 mg, 0.33 mmol) and 2-bromo-5-chlorobenzonitrile (106 mg, 0.49 mmol) reacted using the same method as **6e** to afford compound **6o** as an ivory solid. (86 mg, 56% yield.)

¹H NMR (300 MHz, DMSO- d₆) δ 12.21 (s, 1H), 10.05 (s, 1H), 8.38 (d, *J* = 8.3 Hz, 1H), 8.04 (d, *J* = 2.3 Hz, 1H), 7.77 (dd, *J* = 8.5, 2.3 Hz, 1H), 7.63 - 7.52 (m, 2H), 7.37 (d, *J* = 8.3 Hz, 1H), 7.11 (t, *J* = 7.5 Hz, 1H), 6.44 (d, *J =* 2.2 Hz, 1H), 6.24 - 6.17 (m, 1H), 4.46 (d, *J* = 3.5 Hz, 2H), 4.07 (t, *J* = 5.5 Hz, 2H), 2.59 (s, 2H), 1.94 (tt, *J* = 8.6, 5.1 Hz, 1H), 1.02 - 0.91 (m, 2H), 0.75 - 0.66 (m, 2H).

The compound represented by Formula 3-6 according to the present invention can be prepared according to the Reaction Scheme 4 but is not limited thereto. 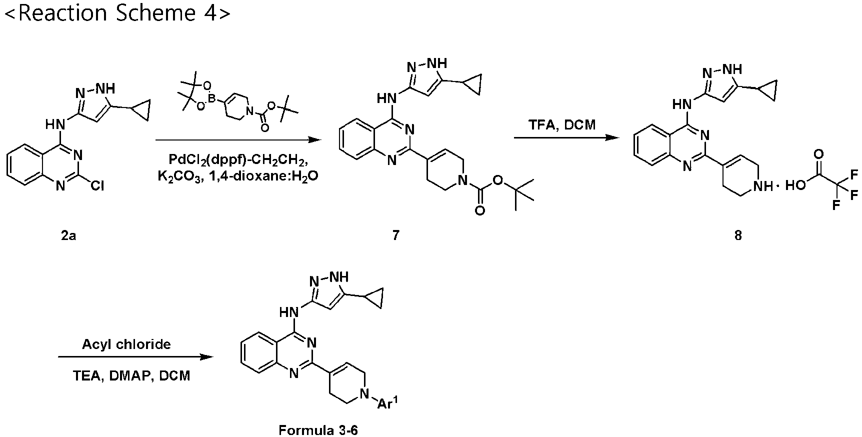 wherein: Ar¹ is the same as defined above.

### sdqs-butyl 4-(4-((5-cyclopropyl-1G-pyrazol-3-yl)amino)quinazolin-2-yl)-3,6-dihydropyridine-1(2G)-carboxylate (7)

To a mixture of compound **2a** (500 mg, 1.75 mmol), tert-butyl 4-(4,4,5,5-tetramethyl-1,3,2-dioxaborolan-2-yl)-3,6-dihydropyridine-1(2*H*)-carboxylate (812 mg, 2.63 mmol), potassium carbonate (484 mg, 3.50 mmol), and PdCl₂(dppf)-CH₂Cl₂ adduct (286 mg, 0.35 mmol) under nitrogen in a vial was added 1,4-dioxane (7.5 mL) and water (1.5 mL). The mixture was then heated in a sealed tube at 100°C for overnight. The crude was filtered through celite, extracted with EtOAc and washed with water. The organic layer was dried over anhydrous Na₂SO₄, filtered, concentrated. The crude was purified by column chromatography to afford compound **7** as a light yellow solid. (382 mg, 51% yield.)

¹H NMR (300 MHz, DMSO- d₆) δ 12.21 (s, 1H), 10.25 (s, 1H), 8.59 (d, *J* = 8.3 Hz, 1H), 7.85 **-** 7.64 (m, 2H), 7.49 (t, *J* = 7.5 Hz, 1H), 7.14 (s, 1H), 6.58 (s, 1H), 4.13 (s, 2H), 3.56 (t, *J* = 5.4 Hz, 2H), 2.69 (s, 2H), 2.03 **-** 1.92 (m, 1H), 1.45 (s, 9H), 1.04 **-** 0.92 (m, 2H), 0.78 **-** 0.69 (m, 2H).

### M-(5-cyclopropyl-1G-pyrazol-3-yl)-2-(1,2,3,6-tetrahydropyridin-4-yl)quinazolin-4-amine trifluoroacetic acid (8)

Compound **7** (380 mg, 0.88 mmol) was dissolved in DCM (6 mL), and 50% TFA in DCM (4 mL) was added at 0°C. The reaction mixture was stirred for 1 h at room temperature. The solvent was evaporated and DCM was added repeat 2 times. The solvent was evaporated and diethyl ether was added repeat several times. The solvent was evaporated and the solid was filtered washed with diethyl ether to afford compound **8** as a yellow solid. (489 mg, 125% yield.)

¹H NMR (300 MHz, DMSO) δ 10.86 (s, 1H), 9.01 (s, 2H), 8.67 (d, J = 8.3 Hz, 1H), 7.95 **-** 7.78 (m, 2H), 7.67 **-** 7.56 (m, 1H), 7.14 (s, 1H), 6.51 (s, 1H), 3.95 (s, 2H), 3.38 (d, J = 5.8 Hz, 2H), 2.88 (s, 2H), 1.98 (tt, J = 8.4, 5.1 Hz, 1H), 1.05 **-** 0.94 (m, 2H), 0.80 **-** 0.71 (m, 2H).

### (4-(4-((5-cyclopropyl-1G-pyrazol-3-yl)amino)quinazolin-2-yl)-3,6-dihydropyridin-1(2G)-yl)(phenyl)methanone (9a)

Compound **8** (50 mg, 0.11 mmol) was dissolved in DCM (1 mL), TEA (0.031 mL, 0.22 mmol) and DMAP(1 mg, 0.008 mmol) was added at 0°C under nitrogen atmosphere. Benzoyl chloride (0.014 mL, 0.12 mmol) was added to the mixture over 5 min. The mixture was stirred at room temperature for 2 h. The reaction mixture was quenched with 10% aqueous NaOH and extracted with DCM. The organic layer was dried over Na₂SO₄. The solvent was removed and purified by column chromatography (5% DCM/MeOH) to afford compound **9a** as an ivory solid. (23 mg, 46% yield.)

¹H NMR (300 MHz, DMSO-d₆) δ 12.22 (s, 1H), 10.28 (s, 1H), 8.58 (d, J = 8.3 Hz, 1H), 7.85 **-** 7.64 (m, 2H), 7.49 (d, J = 4.4 Hz, 6H), 7.14 (d, J = 55.9 Hz, 1H), 6.56 (s, 1H), 4.30 (d, J = 58.5 Hz, 2H), 3.98 **-** 3.46 (m, 2H), 2.79 (s, 2H), 1.96 (s, 1H), 0.97 (s, 2H), 0.73 (s, 2H).

### 1-(4-(4-((5-cyclopropyl-1G-pyrazol-3-yl)amino)quinazolin-2-yl)-3,6-dihydropyridin-1(2G)-yl)-2-methylpropan-1-one (9b)

Compound **8** (70 mg, 0.16 mmol) was dissolved in DCM (2 mL), TEA (0.043 mL, 0.31 mmol) and DMAP(1 mg, 0.011 mmol) was added at 0°C under nitrogen atmosphere. Isobutyryl chloride (0.018 mL, 0.17 mmol) was added to the mixture over 5 min. The mixture was stirred at r.t. for 2 h. The reaction mixture was quenched with 10% aqueous NaOH and extracted with DCM. The organic layer was dried over Na2SO4. The solvent was removed and purified by column chromatography (3% DCM/MeOH) to afford compound **9b** as a light yellow solid.

### (11 mg, 17% yield.)

¹H NMR (400 MHz, DMSO-d₆) δ 12.19 (s, 1H), 10.31 (d, J = 32.9 Hz, 1H), 8.58 (d, J = 8.3 Hz, 1H), 7.79 (t, J = 7.7 Hz, 1H), 7.73 (d, J = 8.3 Hz, 1H), 7.50 (t, J = 7.6 Hz, 1H), 7.17 (s, 1H), 6.56 (s, 1H), 4.30 (d, J = 54.4 Hz, 2H), 3.78 - 3.61 (m, 2H), 3.10 - 2.85 (m, 1H), 2.78 (s, 1H), 2.67 (s, 1H), 2.03 - 1.91 (m, 1H), 1.10 - 1.01 (m, 6H), 1.01 - 0.92 (m, 2H), 0.72 (d, J = 25.5 Hz, 2H).

### sdqs-butyl (4-(4-((5-cyclopropyl-1G-pyrazol-3-yl)amino)quinazolin-2-yl)benzyl)(methyl)carbamate (9c)

A mixture of compound **2a** (100 mg, 0.35 mmol), (4-(((tert-butoxycarbonyl)(methyl)amino)methyl)phenyl)boronic acid (111 mg, 0.42 mmol), Pd(PPh₃)₄ (40 mg, 0.035 mmol) and K₂CO₃ (97 mg, 0.700 mmol) in Dioxane (3 mL)/Water (0.5 mL)(6:1) was stirred at 100°C for overnight. The resulting mixture was diluted with EtOAc and washed twice with water. The organic layer was dried over Na2SO4 and concentrated under reduced pressure. The residue was purified by prep TLC (DCM 92:MeOH 7:NH₄OH 1) to afford the compound **9c** as a pale yellow solid. (46 mg, 28%)

¹H NMR (300 MHz, DMSO) δ 12.28 (s, 1H), 10.41 (s, 1H), 8.63 (d, *J* = 8.3 Hz, 1H), 8.44 (d, *J* = 8.0 Hz, 2H), 7.83 (d, *J* = 4.0 Hz, 2H), 7.54 (dt, *J* = 8.3, 4.1 Hz, 1H), 7.38 (d, *J* = 8.0 Hz, 2H), 6.67 (s, 1H), 4.47 (s, 2H), 2.82 (s, 3H), 2.00 (dq, *J* = 8.7, 4.4 Hz, 1H), 1.43 (s, 9H), 1.05 **-** 0.97 (m, 2H), 0.81 **-** 0.75 (m, 2H).

### N-(5-cyclopropyl-1H-pyrazol-3-yl)-2-(4-((methylamino)methyl)phenyl)quinazolin-4-amine hydrogen chloride (9d)

To a solution of compound **9c** (30 mg, 0.064 mmol) in DCM (1 mL) was added 10 eq of 4 M HCl in Dioxane (0.159 mL, 0.64 mmol) at 0°C and stirred for 4 h. The resulting mixture was filtered and washed with DCM. The filter cake was dissolved in MeOH (0.5 mL) and triturated with diethyl ether to afford a light yellow solid. (16 mg, 63%)

¹H NMR (300 MHz, DMSO) δ 12.00 (s, 1H), 9.52 (s, 2H), 8.86 (d, *J* = 8.4 Hz, 1H), 8.51 (d, *J* = 8.4 Hz, 2H), 8.40 (d, *J* = 8.4 Hz, 1H), 8.08 (t, *J* = 7.8 Hz, 1H), 7.85 (d, *J* = 8.2 Hz, 2H), 7.83 - 7.76 (m, 1H), 6.61 (s, 1H), 4.27 (t, *J* = 5.9 Hz, 2H), 2.59 (t, *J* = 5.3 Hz, 3H), 2.04 (tt, *J* = 8.4, 5.1 Hz, 1H), 1.09 - 1.00 (m, 2H), 0.84 - 0.74 (m, 2H). MS (ESI) m/z calcd for C₂₂H₂₂N₆ [M⁺], 370.2 ; found, 371.5 [M⁺ + H⁺].

### sdqs-butyl 4-(4-((5-cyclopropyl-1G-pyrazol-3-yl)amino)quinazolin-2-yl)piperazine-1-carboxylate (10)

Compound **2a** (500 mg, 1.75 mmol) was dissolved in t-butanol (21 mL), and a solution of *tert-*butyl piperazine-1-carboxylate (489 mg, 2.63 mmol) in t-butanol (3.5 mL) was added to the mixture. DIPEA (339 mg, 2.63 mmol) was added and the reaction was heated at 110°C while stirring. After 4 days, the solvent was evaporated and the crude was dissolved in DCM. The reaction mixture was purified by column chromatography (4% DCM/MeOH) to afford compound **10**. (420 mg, 55% yield.)

¹H NMR (300 MHz, DMSO-d₆) δ 12.18 (s, 1H), 10.03 (s, 1H), 8.35 (d, *J* = 8.2 Hz, 1H), 7.59-7.54 (m, 1H), 7.36-7.33 (m, 1H), 7.15-7.09 (m, 1H), 6.34 (s, 1H), 3.79-3.76 (m, 4H), 3.49-3.36 (m, 4H), 2.02-1.88 (m, 1H), 1.44 (s, 9H), 1.02-0.92 (m, 2H), 0.76-0.65 (m, 2H). MS (ESI) m/z calcd C₂₃H₂₉N₇O₂ [M⁺], 435.2 ; found, 436.5 [M⁺ + H⁺].

The compound represented by Formula 3-7 according to the present invention can be prepared according to the Reaction Scheme 5-1 but is not limited thereto. 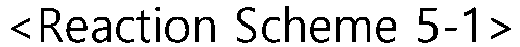 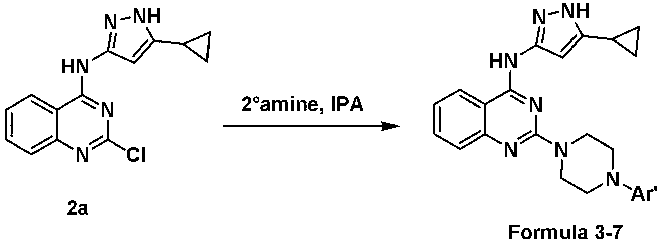 wherein: Ar' is the same as defined above.

### M-(5-cyclopropyl-1G-pyrazol-3-yl)-2-(4-methylpiperazin-1-yl)quinazolin-4-amine (12a)

A mixture of compound **2a** (71 mg, 0.25 mmol) and 1-methylpiperazine (0.083 mL, 0.75 mmol) in IPA (0.833 mL) was irradiated with microwave reactor at 150°C for 30 min. The reaction mixture was triturated with Diethyl ether. The filter cake was neutralized with saturated NaHCO₃ solution and extracted with EtOAc and water. The organic residue was dried over Na₂SO₄, filtered and concentrated. The residue was triturated with 10% DCM in Diethyl ether and dried. The product was dissolved in 10% MeOH in DCM, concentrated and triturated with n-Hexane to afford compound **12a** as a white solid. (18 mg, 21% yield.)

MS (ESI) m/z calcd C₁₉H₂₃N₇ [M⁺],349.2 found, 350.1 [M⁺ + H⁺].

### 2-(4-(4-((5-cyclopropyl-1G-pyrazol-3-yl)amino)quinazolin-2-yl)piperazin-1-yl)ethan-1-ol (12b)

A mixture of compound **2a** (71 mg, 0.25 mmol) and 2-(piperazin-1-yl)ethan-1-ol (0.046 mL, 0.38 mmol) in IPA (0.833 mL) was irradiated with microwave reactor at 150°C for 30 min. The reaction mixture was concentrated, neutralized with saturated NaHCO₃ solution and extracted with EtOAc and water. The organic residue was dried over Na₂SO₄, filtered and concentrated. The residue was purified by column chromatography (3% (7 N Ammonia in MeOH) in EtOAc:Hex=1:1) to afford compound **12b** as a white solid. (23 mg, 24% yield.)

The compound represented by Formula 3-7 according to the present invention can be prepared according to the reaction scheme 5-2 but is not limited thereto. 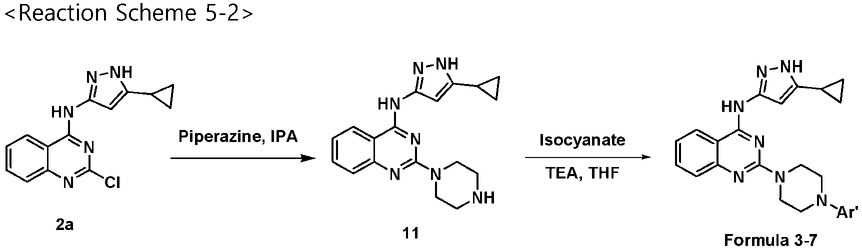 wherein: Ar' is the same as defined above.

### M-(5-cyclopropyl-1G-pyrazol-3-yl)-2-(piperazin-1-yl)quinazolin-4-amine (11)

A mixture of compound **2a** (200 mg, 0.7 mmol) and piperazine (181 mg, 2.10 mmol) in IPA (2.33 mL) was irradiated with microwave reactor at 150°C for 30 min. The resulting mixture was concentrated under reduced pressure, diluted with EtOAc and neutralized with saturated NaHCO₃ solution. The organic residue was dried over Na₂SO₄, filtered and concentrated. The residue was purified by column chromatography to afford the compound **11** as an ivory solid. (8.9 mg, 3% yield.)

MS (ESI) m/z calcd C₁₈H₂₁N₇ [M⁺],335.2 found, 336.1 [M⁺ + H⁺].

### 4-(4-((5-cyclopropyl-1G-pyrazol-3-yl)amino)quinazolin-2-yl)-M-ethylpiperazine-1-carboxamide (12c)

To a mixture of compound **11** (20 mg, 0.06 mmol) and TEA (8.31 µL, 0.06 mmol) in THF (0.2 mL) was added ethyl isocyanate (3.54 µL, 0.045 mmol) at 0°C and warmed to r.t. The reaction mixture was stirred at r.t for 1 h. Ethyl isocyanate (1.65 µL, 0.015 mmol) was added to the mixture at 0°C and stirred for 15 min. The resulting mixture was diluted with EtOAc and washed with water. The organic residue was dried over Na₂SO₄, filtered and concentrated. The residue was purified by column chromatography to afford compound **12c** as an off-white solid. (8 mg, 33% yield.)

### 4-(4-((5-cyclopropyl-1G-pyrazol-3-yl)amino)quinazolin-2-yl)-M-(4-fluorophenyl)piperazine-1-carboxamide (12d)

To a mixture of compound **11** (20 mg, 0.06 mmol) and TEA (8.31 µL, 0.06 mmol) in THF (0.2 mL) was added 4-fluorophenyl isocyanate (6.70 µL, 0.06 mmol) at 0°C and stirred for 15 min. The resulting mixture was diluted with EtOAc and washed with water. The organic residue was dried over Na₂SO₄, filtered and concentrated. The residue was purified by column chromatography to afford compound **12d** as an off-white solid. (11 mg, 39% yield.)

### sdqs-butyl 5-(4-((5-cyclopropyl-1G-pyrazol-3-yl)amino)quinazolin-2-yl)-2,5-diazabicyclo[2.2.1]heptane-2-carboxylate (13)

Compound **2a** (200 mg, 0.70 mmol) was dissolved in t-butanol (9 mL), and a solution of *tert-*butyl 2,5-diazabicyclo[2.2.1]heptane-2-carboxylate (208 mg, 1.05 mmol) in t-butanol (2 mL) was added to the mixture. DIPEA (136 mg, 1.05 mmol) was added and the reaction was heated at 110°C while stirring. After 30 hours, the solvent was evaporated and the crude was dissolved in DCM. The reaction mixture was purified by column chromatography (4% DCM/MeOH) to afford compound **13**. (230 mg, 73% yield.)

¹H NMR (300 MHz, DMSO-d₆) δ 11.51 (s, 1H), 10.56 (s, 1H), 8.72-8.63 (m, 1H), 7.87 (ddd, *J* = 8.4, 7.1, 1.2 Hz, 1H), 7.63 (dd, *J* = 8.4, 1.1 Hz, 1H), 7.57-7.40 (m, 5H), 7.37-7.24 (m, 2H), 6.17 (s, 1H), 1.86 (ddd, *J* = 8.5, 5.1, 3.4 Hz, 1H), 1.01-0.88 (m, 2H), 0.66-0.53 (m, 2H). MS (ESI) m/z calcd C₂₄H₂₉N₇O₂ [M⁺], 447.2 ; found, 448.6 [M⁺ + H⁺].

### 2-(2,5-diazabicyclo[2.2.1]heptan-2-yl)-M-(5-cyclopropyl-1G-pyrazol-3-yl)quinazolin-4-amine Trifluoroacetic acid (14)

Compound **13** (138 mg, 0.31 mmol) was dissolved in DCM (3 mL), and 50% TFA in DCM (2 mL) was added at 0°C. The reaction mixture was stirred for 2 h at room temperature. The solvent was evaporated and DCM was added repeat 2 times. The solvent was evaporated and diethyl ether was added repeat several times. The solvent was evaporated and the solid was filtered washed with diethyl ether to afford compound **14** as a white solid. (230 mg, quant.)

¹H NMR (300 MHz, DMSO-d₆) δ 11.54 (s, 1H), 9.53 (s, 1H), 8.92 (s, 1H), 8.66 (d, *J* = 8.3 Hz, 1H), 7.97**-**7.84 (m, 1H), 7.71 (s, 1H), 7.50 (t, *J* = 7.7 Hz, 1H), 6.42 (s, 1H), 5.17 (d, *J* = 45.8 Hz, 1H), 4.66 (s, 1H), 3.88 (s, 2H), 3.47 (d, *J* = 38.9 Hz, 2H), 2.26 (d, *J* = 11.2 Hz, 1H), 2.14**-**1.92 (m, 2H), 1.07**-**0.94 (m, 2H), 0.83**-**0.69 (m, 2H). MS (ESI) m/z calcd C₁₉H₂₁N₇ [M⁺], 347.2 ; found, 348.5 [M⁺ + H⁺].

The compound represented by Formula 3-8 according to the present invention can be prepared according to the Reaction Scheme 6-1 but is not limited thereto. 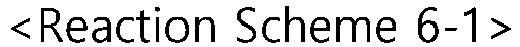 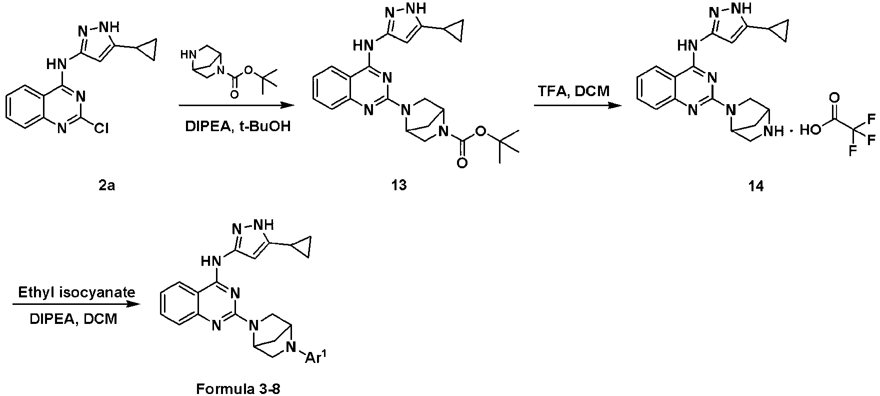 wherein: Ar¹ is the same as defined above.

### 5-(4-((5-cyclopropyl-1G-pyrazol-3-yl)amino)quinazolin-2-yl)-M-ethyl-2,5-diazabicyclo[2.2.1]heptane-2-carboxamide (15a)

Compound **14** (170 mg, 0.44 mmol) was dissolved in DCM (6 mL), DIPEA (0.116 mL, 0.66 mmol) was added. The reaction mixture was stirred at r.t for 5 min. Ethyl isocyanate (39 µL, 0.49 mmol) was added, stirred at r.t for 30 min. The residue was diluted with DCM, washed with saturated ammonium chloride solution. The organic layer was dried over Na₂SO₄, filtered, concentrated. The crude was purified by prep TLC (8% DCM/MeOH) to afford compound **15a.** (27 mg, 15% yield.)

The compound represented by Formula 3-8 according to the present invention can be prepared according to the Reaction Scheme 6-1 but is not limited thereto. 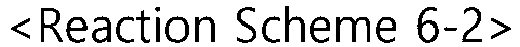 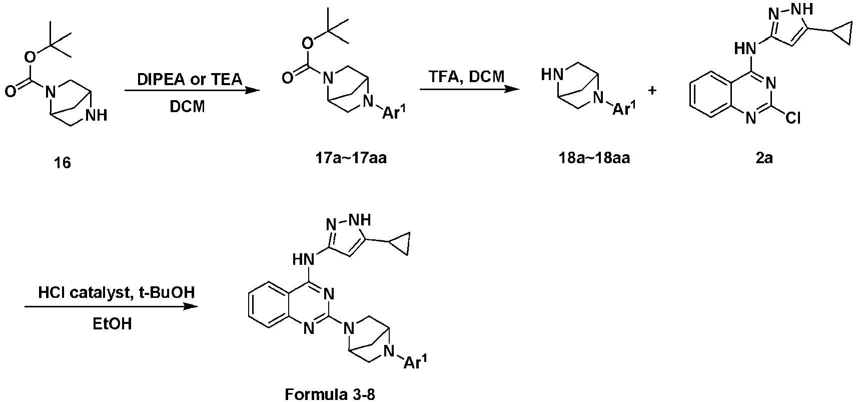 wherein: Ar¹ is the same as defined above.

### 5-(4-((5-cyclopropyl-1G-pyrazol-3-yl)amino)quinazolin-2-yl)-M-isopropyl-2,5-diazabicyclo[2.2.1]heptane-2-carboxamide(15b)

### 1) tert-butyl 5-(isopropylcarbamoyl)-2,5-diazabicyclo[2.2.1]heptane-2-carboxylate (17a)

Compound 16 (300 mg, 1.51 mmol) was dissolved in DCM (15 mL) and TEA (0.63 mL, 4.54 mmol) was added at 0°C. The reaction mixture was stirred for 10 min. To a stirring solution, isopropyl isocyanate (0.16 mL, 1.67 mmol) was added. The mixture was stirred at room temperature, under a nitrogen atmosphere for overnight. The reaction mixture was quenched with water. The organic layer was washed with 10% NH₄Cl solution and water. The combined organic layer was dried over Na₂SO₄ and evaporated under vacuum. The crude mixture was purified by filtration and washed with hexane to afford compound 17a as a white solid compound. (343 mg, 80% yield.)

¹H NMR (300 MHz, Chloroform-*d*) δ 4.67 - 4.37 (m, 2H), 3.97 (p, *J* = 6.5 Hz, 1H), 3.42 (d, *J =* 9.9 Hz, 1H), 3.37 - 3.18 (m, 3H), 1.80 (s, 2H), 1.45 (s, 9H), 1.15 (dd, *J* = 6.5, 2.9 Hz, 6H).

### 2) N-isopropyl-2,5-diazabicyclo[2.2.1]heptane-2-carboxamide (18a)

Compound **17a** (417 mg, 1.47 mmol) was dissolved in DCM (4 mL), and 33% TFA in DCM (6 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18a**. (168 mg, 62%).

### 3) 15b

Compound **2a** (70 mg, 0.25 mmol) and compound **18a** (54 mg, 0.29 mmol) was dissolved in ethanol (3 mL). The reaction mixture was heated at 110°C for 2 days. The solvent was evaporated and purified with column chromatography (6% DCM/MeOH) to afford compound **15b** as a white solid compound. (38 mg, 36% yield.)

¹H NMR (400 MHz, DMSO-d₆) δ 12.17 (s, 1H), 10.07 (s, 1H), 8.36 (d, *J* = 8.3 Hz, 1H), 7.55 (t, *J* = 7.5 Hz, 1H), 7.33 (d, *J* = 8.3 Hz, 1H), 7.09 (t, *J* = 7.5 Hz, 1H), 6.60 **-** 6.31 (m, 1H), 5.94 (d, *J* = 7.8 Hz, 1H), 5.02 **-** 4.73 (m, 1H), 4.59 (s, 1H), 3.81 **-** 3.36 (m, 4H), 2.01 **-** 1.78 (m, 3H), 1.10 **-** 0.89 (m, 8H), 0.76 **-** 0.64 (m, 2H). ¹³C NMR (101 MHz, DMSO) δ 157.66, 156.39, 152.48, 133.20, 125.27, 123.77, 121.25, 110.97, 94.44, 57.59, 56.45, 56.29, 55.23, 53.93, 53.61, 41.90, 37.12, 23.56, 23.45, 23.37, 8.43, 7.98, 7.56. MS (ESI) m/z calcd for C₂₃H₂₈N₈O [M⁺], 432.5 ; found, 433.5 [M⁺ + H⁺].

### 5-(4-((5-cyclopropyl-1G-pyrazol-3-yl)amino)quinazolin-2-yl)-M-phenyl-2,5-diazabicyclo[2.2.1]heptane-2-carboxamide(15c)

### 1) tert-butyl 5-(phenylcarbamoyl)-2,5-diazabicyclo[2.2.1]heptane-2-carboxylate (17b)

Compound **16** (300 mg, 1.51 mmol) was dissolved in DCM (15 mL) and TEA (0.63 mL, 4.54 mmol) was added at 0°C. The reaction mixture was stirred for 10 min. To a stirring solution, phenyl isocyanate (0.18 mL, 1.67 mmol) was added. The mixture was stirred at room temperature, under a nitrogen atmosphere for 1 h. The reaction mixture was quenched with water. The organic layer was washed with 10% NH₄Cl solution and water. The combined organic layer was dried over Na₂SO₄ and evaporated under vacuum. The crude mixture was purified using column chromatography (66% Hex/EA) to afford compound **17b** as a white solid compound. (455 mg, 95% yield.)

¹H NMR (300 MHz, DMSO-d₆) δ 8.29 (s, 1H), 7.50 (d, *J* = 8.4 Hz, 2H), 7.23 (t, *J* = 7.9 Hz, 2H), 6.94 (t, *J* = 7.3 Hz, 1H), 4.64 (s, 1H), 4.40 (d, *J* = 12.0 Hz, 1H), 3.53 **-** 3.38 (m, 2H), 3.32 **-** 3.19 (m, 2H), 1.92 **-** 1.76 (m, 2H), 1.41 (d, *J* = 8.5 Hz, 9H).

### 2) N-phenyl-2,5-diazabicyclo[2.2.1]heptane-2-carboxamide (18b)

Compound **17b** (509 mg, 1.60 mmol) was dissolved in DCM (4 mL), and 33% TFA in DCM (6 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18b**. (132 mg, 38% yield.)

### 3) 15c

Compound **2a** (70 mg, 0.25 mmol) and compound 18b (64 mg, 0.29 mmol) were dissolved in ethanol (3 mL). The reaction mixture was heated at 110°C for 2 days. The solvent was evaporated and purified with column chromatography (3% DCM/MeOH) to afford compound **15c** as a white solid compound. (27 mg, 24% yield.)

¹H NMR (300 MHz, DMSO-d₆) δ 12.16 (s, 1H), 10.08 (s, 1H), 8.48 **-** 8.14 (m, 2H), 7.65 **-** 7.40 (m, 3H), 7.33 (s, 1H), 7.26 **-** 6.99 (m, 3H), 6.90 (t, *J* = 7.3 Hz, 1H), 6.49 (s, 1H), 4.87 (d, *J* = 51.7 Hz, 2H), 3.86 **-** 3.42 (m, 4H), 2.12 **-** 1.76 (m, 3H), 1.08 **-** 0.84 (m, 2H), 0.84 **-** 0.57 (m, 2H). ¹³C NMR (101 MHz, DMSO) δ 157.77, 154.37, 152.51, 140.74, 133.19, 128.73, 125.36, 123.80, 122.14, 121.29, 119.87, 111.03, 94.53, 57.63, 56.87, 55.43, 54.03, 53.75, 37.08, 8.45, 7.97, 7.58. MS (ESI) m/z calcd for C₂₆H₂₆N₈O [M⁺], 466.6 ; found, 467.6 [M⁺ + H⁺].

### 1-(5-(4-((5-cyclopropyl-1G-pyrazol-3-yl)amino)quinazolin-2-yl)-2,5-diazabicyclo[2.2.1]heptan-2-yl)-2-methylpropan-1-one (15d)

### 1) tert-butyl 5-isobutyryl-2,5-diazabicyclo[2.2.1]heptane-2-carboxylate(17c)

Compound **16** (300 mg, 1.51 mmol) was dissolved in DCM (15 mL) and TEA (0.32 mL, 2.27 mmol) was added at 0°C. To a stirring solution, isobutyryl chloride (0.17 mL, 1.66 mmol) was added dropwise. The mixture was stirred at room temperature, under a nitrogen atmosphere for overnight. The reaction mixture was quenched with saturated NaHCO₃ and extracted with DCM. The organic layer was washed with saturated NH₄Cl solution and brine. The combined organic layer was dried over Na₂SO₄ and evaporated under vacuo to afford compound **17c** as a pink solid compound. (426 mg, 105% yield.)

¹H NMR (300 MHz, DMSO-d₆) δ 4.64 (d, *J* = 8.6 Hz, 1H), 4.44 **-** 4.30 (m, 1H), 3.64 **-** 3.39 (m, 1H), 3.30 **-** 3.02 (m, 3H), 2.75 (p, *J* = 6.7 Hz, 1H), 1.94 **-** 1.70 (m, 2H), 1.40 (d, *J* = 6.2 Hz, 9H), 0.98 (ddd, *J* = 15.3, 7.7, 5.7 Hz, 6H).

### 2) 1-(2,5-diazabicyclo[2.2.1]heptan-2-yl)-2-methylpropan-1-one(18c)

Compound **17c** (419 mg, 1.56 mmol) was dissolved in DCM (4 mL), and 33% TFA in DCM (6 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18c**. (168 mg, 64% yield.)

### 3) 15d

Compound **2a** (80 mg, 0.28 mmol) and compound 18c (57 mg, 0.34 mmol) was dissolved in ethanol (3 mL). The reaction mixture was heated at 110°C for 2 days. The solvent was evaporated and purified with column chromatography (DCM:MeOH:H₂O = 79:9:1) to afford compound **15d** as a yellow solid. (90 mg, 77% yield.)

¹H NMR (300 MHz, DMSO-d₆) δ 12.21 (s, 1H), 10.10 (s, 1H), 8.37 (d, *J* = 8.2 Hz, 1H), 7.55 (t, *J* = 7.6 Hz, 1H), 7.33 (d, *J* = 8.4 Hz, 1H), 7.09 (t, *J* = 7.5 Hz, 1H), 6.44 (s, 1H), 5.10 **-** 4.73 (m, 2H), 3.76 **-** 3.38 (m, 4H), 2.91 **-** 2.74 (m, 1H), 2.07 **-** 1.84 (m, 3H), 1.09 **-** 0.92 (m, 6H), 0.85 (d, *J* = 6.6 Hz, 2H), 0.76 **-** 0.63 (m, 2H). ¹³C NMR (101 MHz, DMSO) δ 174.54, 174.43, 157.85, 157.65, 152.62, 133.14, 129.37, 128.67, 125.43, 123.78, 121.25, 97.45, 57.91, 56.37, 55.63, 36.26, 31.59, 31.20, 20.14, 19.57, 19.39, 19.19, 11.50. MS (ESI) m/z calcd for C₂₃H₂₇N₇O [M⁺], 417.5 ; found, 418.5 [M⁺ + H⁺].

### (5-(4-((5-cyclopropyl-1G-pyrazol-3-yl)amino)quinazolin-2-yl)-2,5-diazabicyclo[2.2.1]heptan-2-yl)(phenyl)methanone (15e)

### 1) tert-butyl 5-benzoyl-2,5-diazabicyclo[2.2.1]heptane-2-carboxylate (17d)

Compound **16** (300 mg, 1.51 mmol) was dissolved in DCM (15 mL), TEA (0.42 mL, 3.03 mmol) and DMAP (13 mg, 0.11 mmol) was added at 0°C under nitrogen atmosphere. To a stirring solution, benzoyl chloride (0.19 mL, 1.66 mmol) was added dropwise. The mixture was stirred at room temperature, under a nitrogen atmosphere for 2 h. The reaction mixture was quenched with 10% aqueous NaOH and extracted with DCM. The combined organic layer was dried over Na₂SO₄ and evaporated under vacuum. The crude was purified using column chromatography (3% DCM/MeOH) to afford compound **17d** as a yellow oil compound. (483 mg, 106% yield.)

¹H NMR (300 MHz, Chloroform-*d*) δ 7.55 **-** 7.33 (m, 5H), 4.84 (d, *J* = 97.4 Hz, 1H), 4.45 (d, *J* = 41.2 Hz, 1H), 3.72 **-** 3.30 (m, 4H), 1.96 **-** 1.76 (m, 2H), 1.46 (d, *J* = 16.8 Hz, 9H).

### 2) (2,5-diazabicyclo[2.2.1]heptan-2-yl)(phenyl)methanone (18d)

Compound **17d** (478 mg, 1.58 mmol) was dissolved in DCM (4 mL), and 33% TFA in DCM (6 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18d**. (232 mg, 73% yield.)

### 3) 15e

Compound **2a** (40 mg, 0.14 mmol) and compound **18d** (34 mg, 0.17 mmol) was dissolved in ethanol (3 mL). The reaction mixture was heated at 110°C for overnight. The solvent was evaporated and purified with column chromatography (5% DCM/MeOH) and prep TLC (5% DCM/MeOH) to afford compound **15e** as a light yellow solid. (45 mg, 71% yield.)

¹H NMR (300 MHz, DMSO-d₆) δ 12.17 (s, 1H), 10.14 (s, 1H), 8.37 (t, *J* = 10.0 Hz, 1H), 7.67 **-** 7.43 (m, 5H), 7.44 **-** 7.27 (m, 2H), 7.11 (q, *J* = 9.6, 8.6 Hz, 1H), 6.39 (d, *J* = 51.5 Hz, 1H), 5.14 **-** 4.35 (m, 2H), 3.88 **-** 3.55 (m, 3H), 3.48 (d, *J* = 11.2 Hz, 1H), 2.19 **-** 1.84 (m, 3H), 1.35 **-** 1.11 (m, 1H), 0.98 (d, *J* = 8.5 Hz, 2H), 0.72 (s, 2H). ¹³C NMR (101 MHz, DMSO) δ 169.25, 168.57, 136.27, 133.26, 130.73, 130.43, 128.95, 128.73, 127.74, 125.27, 123.82, 121.43, 110.96, 94.36, 60.39, 57.08, 55.90, 55.38, 54.65, 53.50, 37.93, 36.26, 8.51, 8.46, 7.56. MS (ESI) m/z calcd for C₂₆H₂₅N₇O [M⁺], 451.5 ; found, 452.5 [M⁺ + H⁺].

### M-(5-cyclopropyl-1G-pyrazol-3-yl)-2-(5-isobutyl-2,5-diazabicyclo[2.2.1]heptan-2-yl)quinazolin-4-amine (15f)

### 1) tert-butyl 5-isobutyl-2,5-diazabicyclo[2.2.1]heptane-2-carboxylate (17e)

Compound **16** (300 mg, 1.51 mmol) was dissolved in DMF (10 mL) and potassium carbonate (627 mg, 4.54 mmol) was added. To a stirring solution, 2-methyl propyl bromide (0.181 mL, 1.66 mmol) in DMF (2 mL) was added dropwise. The mixture was stirred at room temperature to 80°C, under a nitrogen atmosphere for 2 days. The reaction mixture was quenched with water and extracted with ethyl acetate. The organic layer was washed with brine. The combined organic layer was dried over Na₂SO₄ and evaporated under vacuum. The crude was purified using column chromatography to afford compound **17e**. (136 mg, 35% yield.)

¹H NMR (300 MHz, DMSO-d₆) δ 4.12 (d, *J* = 7.5 Hz, 1H), 3.34 **-** 3.23 (m, 2H), 3.11 **-** 2.97 (m, 1H), 2.79 (d, *J* = 9.4 Hz, 1H), 2.44 **-** 2.33 (m, 1H), 2.24 (qd, *J* = 11.7, 7.1 Hz, 2H), 1.70 (d, *J* = 9.4 Hz, 1H), 1.64 **-** 1.49 (m, 2H), 1.39 (s, 9H), 0.85 (d, *J* = 6.7 Hz, 6H).

### 2) 2-isobutyl-2,5-diazabicyclo[2.2.1]heptane (18e)

Compound **17e** (460 mg, 1.81 mmol) was dissolved in DCM (4 mL), and 33% TFA in DCM (6 mL) was added at 0°C. The reaction mixture was stirred for 1 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18e** as a brown oil compound. (216 mg, 77% yield.)

### 3) 15f

Compound **2a** (80 mg, 0.28 mmol) and compound **18e** (52 mg, 0.34 mmol) was dissolved in ethanol (3 mL). The reaction mixture was heated at 110°C for 2 days. The solvent was evaporated and purified with column chromatography (DCM:MeOH:H₂O = 79:9:1) to afford compound **15f** as a brown solid. (48 mg, 42% yield.)

¹H NMR (400 MHz, DMSO-d₆) δ 12.17 (s, 1H), 10.08 (s, 1H), 8.36 (d, *J* = 8.3 Hz, 1H), 7.55 (t, *J* = 7.6 Hz, 1H), 7.33 (d, *J* = 8.3 Hz, 1H), 7.09 (t, *J* = 7.8 Hz, 1H), 6.47 (d, *J* = 61.4 Hz, 1H), 4.76 (s, 1H), 3.92 **-** 3.38 (m, 3H), 2.99 (s, 2H), 2.42 **-** 2.10 (m, 2H), 2.03 **-** 1.68 (m, 3H), 1.71 **-** 1.44 (m, 1H), 1.03 **-** 0.63 (m, 10H). ¹³C NMR (101 MHz, DMSO) δ 157.56, 157.37, 152.42, 133.18, 125.20, 123.78, 121.21, 110.95, 94.32, 62.46, 60.48, 50.80, 31.43, 29.48, 22.54, 21.05, 20.98, 14.44, 8.45, 7.56. MS (ESI) m/z calcd for C₂₃H₂₉N₇ [M⁺], 403.5 ; found, 404.5 [M⁺ + H⁺].

### 2-(5-benzyl-2,5-diazabicyclo[2.2.1]heptan-2-yl)-M-(5-cyclopropyl-1G-pyrazol-3-yl)quinazolin-4-amine (15g)

### 1) tert-butyl 5-benzyl-2,5-diazabicyclo[2.2.1]heptane-2-carboxylate (17f)

Compound **16** (300 mg, 1.51 mmol) was dissolved in DMF (10 mL), potassium iodide (251 mg, 1.51 mmol) and potassium carbonate (627 mg, 4.54 mmol) was added. To a stirring solution, benzyl chloride (0.19 mL, 1.66 mmol) in DMF (2 mL) was added dropwise. The mixture was stirred at 60°C, under a nitrogen atmosphere for 3.5 h. The reaction mixture was quenched with water and extracted with ethyl acetate. The organic layer was washed with brine. The combined organic layer was dried over Na₂SO₄ and evaporated under vacuum. The crude was purified using column chromatography to afford compound **17f**. (408 mg, 94% yield.)

¹H NMR (400 MHz, DMSO-d₆) δ 7.40 **-** 7.27 (m, 4H), 7.27 **-** 7.17 (m, 1H), 4.16 (d, *J* = 14.1 Hz, 1H), 3.67 (s, 2H), 3.44 **-** 3.35 (m, 2H), 3.16 **-** 3.02 (m, 1H), 2.76 (t, *J* = 8.8 Hz, 1H), 2.48 **-** 2.39 (m, 1H), 1.79 (d, *J* = 9.7 Hz, 1H), 1.68 **-** 1.56 (m, 1H), 1.40 (d, *J* = 4.9 Hz, 9H).

### 2) 2-benzyl-2,5-diazabicyclo[2.2.1]heptane (18f)

Compound **17f** (770 mg, 2.67 mmol) was dissolved in DCM (4 mL), and 33% TFA in DCM (6 mL) was added at 0°C. The reaction mixture was stirred for 1 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **18f**. (382 mg, 76% yield.)

### 3) 15g

Compound **2a** (100 mg, 0.35 mmol) and compound **18f** (79 mg, 0.42 mmol) was dissolved in ethanol (3 mL). The reaction mixture was heated at 110°C for 4 days. The solvent was evaporated and purified with column chromatography (DCM:MeOH:H₂O = 79:9:1) to afford compound **15g** as a white solid. (52 mg, 34%)

¹H NMR (300 MHz, DMSO-d₆) δ 12.15 (s, 1H), 10.03 (s, 1H), 8.35 (d, *J* = 8.2 Hz, 1H), 7.53 (t, *J* = 7.7 Hz, 1H), 7.38 - 7.12 (m, 6H), 7.07 (t, *J* = 7.5 Hz, 1H), 6.49 (d, *J* = 68.4 Hz, 1H), 4.76 (s, 1H), 3.85 - 3.62 (m, 3H), 3.57 (s, 1H), 3.43 (s, 1H), 2.91 (d, *J* = 9.2 Hz, 1H), 2.63 - 2.54 (m, 1H), 2.03 - 1.86 (m, 2H), 1.86 - 1.76 (m, 1H), 0.95 (d, *J* = 8.4 Hz, 2H), 0.68 (s, 2H). ¹³C NMR (101 MHz, DMSO) δ 157.67, 152.82, 140.20, 137.82, 133.07, 129.37, 128.67, 128.65, 128.63, 127.16, 125.78, 125.34, 123.73, 120.93, 61.37, 59.69, 58.20, 51.56, 21.51, 8.42, 8.40. MS (ESI) m/z calcd for C₂₆H₂₇N₇ [M⁺], 437.6 ; found, 438.5 [M⁺ + H⁺].

### Synthesis example of 18g to 18aa

Compound **16** (1eq) was dissolved in DCM and DIPEA (3 eq) was added. To a stirring mixture, sulfonyl chloride (1.5 eq) in DCM was added dropwise at 0°C. The mixture was stirred at room temperature, under a nitrogen atmosphere for 1 h - overnight. The solvent was removed and purified by column chromatography to afford compound **17g-17aa**.

Compound **17g-17aa** was dissolved in DCM, TFA (TFA/DCM 20% v/v) was added. After TLC checking, the solvent was evaporated and DCM was added repeat 2 times. The solvent was evaporated and diethyl ether was added repeat several times. The solvent was concentrated, TFA salt was dissolved in MeOH and saturated NaHCO₃ solution was added. All solvent was evaporated, the crude was extracted with 2% DCM/MeOH. After filtration, the organic layer was dried over anhydrous Na₂SO₄ and evaporated under vacuum to afford compound **18g-18aa**.

### M-(5-cyclopropyl-1G-pyrazol-3-yl)-2-(5-(isopropylsulfonyl)-2,5-diazabicyclo[2.2.1]heptan-2-yl)quinazolin-4-amine (15h)

### 1) tert-butyl 5-(isopropylsulfonyl)-2,5-diazabicyclo[2.2.1]heptane-2-carboxylate (17g)

Compound **16** (100 mg, 0.50 mmol) and isopropylsulfonyl chloride (0.085 mL, 0.76 mmol) reacted to afford compound **17g**. (111 mg, 73% yield.**)**

¹H NMR (300 MHz, Chloroform-*d*) δ 4.57 (s, 1H), 4.45 (s, 1H), 3.62 **-** 3.25 (m, 4H), 3.17 (p, *J* = 6.8 Hz, 1H), 1.94 **-** 1.81 (m, 2H), 1.65 (s, 1H), 1.49 (s, 9H), 1.43 (d, *J* = 6.9 Hz, 3H), 1.38 (dd, *J* = 6.8, 2.8 Hz, 6H).

### 2-(isopropylsulfonyl)-2,5-diazabicyclo[2.2.1]heptane (18g)

Compound **17g** (103 mg, 0.34 mmol) reacted to afford compound **18g**. (TFA salt, 111 mg, 108% yield.)

¹H NMR (400 MHz, DMSO-d₆) δ 9.08 (d, J = 78.0 Hz, 2H), 4.44 (s, 2H), 3.56 **-** 3.41 (m, 2H), 3.37 (p, J = 6.8 Hz, 1H), 3.33 **-** 3.18 (m, 2H), 2.01 **-** 1.81 (m, 2H), 1.24 (d, J = 6.7 Hz, 6H).

### 3) 15h

A solution of compound **2a** (69 mg, 0.24 mmol) in *t*-butanol (1 mL), and a solution of compound **18g** (115 mg, 0.36 mmol) in t-butanol (1 mL) was added. DIPEA (0.063 mL, 0.36 mmol) was added and the reaction was heated at 110°C while stirring. After 18 h, the solvent was evaporated. The reaction mixture was dissolved in ethyl acetate and washed with water. The organic layer was dried over Na₂SO₄ and evaporated under vacuum. The crude mixture was purified by column chromatography (4% DCM/MeOH) to afford compound **15h** as a yellow solid. (58 mg, 53% yield.)

¹H NMR (300 MHz, DMSO-d₆) δ 12.19 (s, 1H), 10.12 (s, 1H), 8.38 (d, *J* = 8.2 Hz, 1H), 7.56 (t, *J* = 7.4 Hz, 1H), 7.35 (d, *J* = 8.3 Hz, 1H), 7.11 (t, *J* = 7.6 Hz, 1H), 6.44 (s, 1H), 4.97 (s, 1H), 4.48 (s, 1H), 3.86 **-** 3.44 (m, 3H), 3.38 (s, 1H), 3.34 **-** 3.25 (m, 2H), 3.01 (s, 1H), 2.12 **-** 1.99 (m, 1H), 2.01 **-** 1.83 (m, 2H), 1.57 (s, 1H), 1.32 (q, *J* = 7.3 Hz, 1H), 1.22 (dd, *J* = 6.8, 2.3 Hz, 7H), 1.02 **-** 0.89 (m, 4H), 0.81 **-** 0.60 (m, 2H). ¹³C NMR (101 MHz, DMSO-d₆) δ 157.71, 133.25, 125.32, 123.81, 121.42, 111.02, 60.56, 56.07, 53.41, 52.27, 37.93, 19.90, 16.77, 16.64, 14.01, 8.44. MS (ESI) m/z calcd for C₂₂H₂₇N₇O₂S [M⁺], 453.6 ; found, 454.5 [M⁺ + H⁺].

### M-(5-cyclopropyl-1G-pyrazol-3-yl)-2-(5-(cyclopropylsulfonyl)-2,5-diazabicyclo[2.2.1]heptan-2-yl)quinazolin-4-amine (15i)

### 1) tert-butyl 5-(cyclopropylsulfonyl)-2,5-diazabicyclo[2.2.1]heptane-2-carboxylate (17h)

Compound **16** (70 mg, 0.35 mmol) and cyclopropanesulfonyl chloride (0.054 mL, 0.53 mmol) reacted to afford compound **17h**. (91 mg, 85% yield.)

¹H NMR (300 MHz, Chloroform-*d*) δ 4.67 **-** 4.39 (m, 2H), 3.66 **-** 3.26 (m, 4H), 2.34 (tt, J = 7.9, 4.9 Hz, 1H), 1.89 (s, 2H), 1.46 (s, 9H), 1.30 **-** 1.14 (m, 2H), 1.06 **-** 0.93 (m, 2H).

### 2) 2-(cyclopropylsulfonyl)-2,5-diazabicyclo[2.2.1]heptane (18h)

Compound **17h** (90 mg, 0.30 mmol) reacted to afford compound **18h**. (23 mg, 38% yield.)

¹H NMR (300 MHz, Chloroform-*d*) δ 4.44 (d, *J* = 23.3 Hz, 2H), 3.69 **-** 3.39 (m, 3H), 2.40 (ddd, *J* = 8.0, 5.0, 3.0 Hz, 1H), 2.05 (s, 2H), 1.19 **-** 1.06 (m, 2H), 1.10 **-** 0.93 (m, 2H).

### 3) 15i

A solution of compound **2a** (45 mg, 0.16 mmol) in *t*-butanol (1 mL), and a solution of compound **18h** (48 mg, 0.24 mmol) in t-butanol (1 mL) was added. 37% HCl in water (0.010 mL) was added and the reaction was heated at 110°C while stirring. After overnight, a white solid was recovered by filtration washing with ethyl acetate to afford compound **15i**. (50 mg, 70%)

¹H NMR (300 MHz, DMSO-d₆) δ 12.94 (s, 2H), 11.45 (s, 1H), 8.64 (d, *J* = 8.3 Hz, 1H), 7.93 (d, *J* = 14.2 Hz, 1H), 7.86 (t, *J* = 7.7 Hz, 1H), 7.46 (t, *J* = 7.7 Hz, 1H), 6.43 (d, *J* = 30.3 Hz, 1H), 5.57 (s, 1H), 5.10 (s, 1H), 4.63 (d, *J* = 16.7 Hz, 1H), 3.80 (s, 2H), 3.73 **-** 3.48 (m, 3H), 2.87 (t, *J* = 6.3 Hz, 1H), 2.13 (s, 2H), 2.05 **-** 1.87 (m, 1H), 1.08 **-** 0.90 (m, 6H), 0.74 (d, *J* = 5.4 Hz, 2H). ¹³C NMR (101 MHz, DMSO-d₆) δ 157.32, 140.32, 135.63, 124.86, 117.84, 95.64, 59.27, 58.89, 57.26, 54.89, 31.75, 27.53, 27.39, 8.59, 7.39, 5.05, 4.97. MS (ESI) m/z calcd for C₂₂H₂₅N₇O₂S [M⁺], 451.6 ; found, 452.4 [M⁺ + H⁺].

### M-(5-cyclopropyl-1G-pyrazol-3-yl)-2-(5-(propylsulfonyl)-2,5-diazabicyclo[2.2.1]heptan-2-yl)quinazolin-4-amine (15j)

### 1) tert-butyl 5-(propylsulfonyl)-2,5-diazabicyclo[2.2.1]heptane-2-carboxylate (17i)

Compound **16** (100 mg, 0.50 mmol) and 1-propanesulfonyl chloride (0.085 mL, 0.76 mmol) reacted to afford compound **17i**. (149 mg, 97% yield.)

¹H NMR (300 MHz, Chloroform-*d*) δ 4.69 **-** 4.50 (m, 1H), 4.47 (s, 1H), 3.58 **-** 3.27 (m, 4H), 3.03 **-** 2.88 (m, 2H), 1.95 **-** 1.79 (m, 4H), 1.48 (s, 9H), 1.08 (t, *J* = 7.5 Hz, 3H).

### 2) 2-(propylsulfonyl)-2,5-diazabicyclo[2.2.1]heptane (18i)

Compound **17i** (145 mg, 0.48 mmol) reacted to afford compound **18i**. (TFA salt, 167 mg, 110% yield.)

¹H NMR (400 MHz, DMSO-d₆) δ 9.16 (d, J = 106.1 Hz, 2H), 4.46 (s, 2H), 3.56 **-** 3.34 (m, 2H), 3.32 **-** 3.09 (m, 4H), 1.90 (dd, J = 34.5, 11.7 Hz, 2H), 1.69 (h, J = 7.5 Hz, 2H), 0.99 (t, J = 7.4 Hz, 3H).

### 3) 15j

A solution of compound **2a** (89 mg, 0.31 mmol) in *t*-butanol (1 mL), and a solution of compound **18i** (149 mg, 0.47 mmol) in t-butanol (1 mL) was added. DIPEA (0.082 mL, 0.47 mmol) was added and the reaction was heated at 110°C while stirring. After 18 h, the solvent was evaporated. The reaction mixture was dissolved in ethyl acetate and washed with water. The organic layer was dried over Na₂SO₄ and evaporated under vacuum. The crude mixture was purified by column chromatography (5% DCM/MeOH) to afford compound **15j** as a white solid. (61 mg, 43% yield.)

¹H NMR (300 MHz, DMSO-d₆) δ 12.17 (s, 1H), 10.10 (s, 1H), 8.38 (d, *J* = 8.3 Hz, 1H), 7.56 (t, *J* = 7.6 Hz, 1H), 7.35 (d, *J* = 8.3 Hz, 1H), 7.11 (t, *J* = 7.6 Hz, 1H), 6.46 (s, 1H), 4.96 (s, 1H), 4.49 (s, 1H), 3.78 **-** 3.41 (m, 4H), 3.31 (s, 2H), 3.17 **-** 3.00 (m, 2H), 2.06 **-** 1.85 (m, 3H), 1.67 (h, *J* = 7.4 Hz, 2H), 1.05 **-** 0.88 (m, 5H), 0.77 **-** 0.65 (m, 2H). ¹³C NMR (101 MHz, DMSO-d₆) δ 157.72, 133.21, 125.39, 123.80, 121.39, 111.05, 59.92, 55.94, 52.33, 37.49, 17.20, 13.17, 8.44. MS (ESI) m/z calcd for C₂₂H₂₇N₇O₂S [M⁺], 453.6 ; found, 454.5 [M⁺ + H⁺].

### 2-(5-(cyclohexylsulfonyl)-2,5-diazabicyclo[2.2.1]heptan-2-yl)-M-(5-cyclopropyl-1G-pyrazol-3-yl)quinazolin-4-amine (15k)

### 1) tert-butyl 5-(cyclohexylsulfonyl)-2,5-diazabicyclo[2.2.1]heptane-2-carboxylate (17j)

Compound **16** (100 mg, 0.50 mmol) and cyclohexanesulfonyl chloride (0.11 mL, 0.76 mmol) reacted to afford compound **17j**. (101 mg, 58% yield.)

¹H NMR (400 MHz, Chloroform-*d*) δ 4.69 **-** 4.37 (m, 2H), 3.71 **-** 3.20 (m, 4H), 2.94 **-** 2.76 (m, 1H), 2.25 **-** 2.09 (m, 2H), 1.97 **-** 1.83 (m, 3H), 1.74 (d, J = 11.3 Hz, 1H), 1.60 (s, 2H), 1.49 (s, 9H), 1.27 (q, J = 11.1, 9.6 Hz, 3H).

### 2) 2-(cyclopropylsulfonyl)-2,5-diazabicyclo[2.2.1]heptane (18j)

Compound **17i** (98 mg, 0.29 mmol) reacted to afford compound **18j**. (63 mg, 90% yield.)

### 3) 15k

A solution of compound **2a** (50 mg, 0.18 mmol) in *t*-butanol (1 mL), and a solution of compound **18j** (64 mg, 0.26 mmol) in t-butanol (1 mL) was added. 37% HCl in water (0.005 mL) was added and the reaction was heated at 110°C while stirring. After overnight, a white solid was recovered by filtration washing with ethyl acetate to afford compound **15k**. (75 mg, 87% yield.)

¹H NMR (400 MHz, DMSO-d₆) δ 12.99 **-** 12.29 (m, 2H), 11.44 (s, 1H), 8.64 (d, *J* = 8.3 Hz, 1H), 8.04 **-** 7.74 (m, 2H), 7.46 (t, *J* = 7.7 Hz, 1H), 6.41 (d, *J* = 32.8 Hz, 1H), 5.32 (d, *J* = 172.8 Hz, 1H), 4.61 (d, *J* = 19.5 Hz, 1H), 3.89 **-** 3.68 (m, 2H), 3.68 **-** 3.46 (m, 2H), 3.23 **-** 3.03 (m, 1H), 2.18 **-** 1.93 (m, 4H), 1.78 (d, *J* = 12.2 Hz, 2H), 1.62 (d, *J* = 12.7 Hz, 1H), 1.47 **-** 1.20 (m, 4H), 1.19 **-** 1.06 (m, 1H), 1.00 (d, *J* = 8.4 Hz, 2H), 0.82 **-** 0.60 (m, 2H). ¹³C NMR (101 MHz, DMSO-d₆) δ 157.34, 135.57, 125.08, 124.87, 117.89, 110.18, 95.51, 61.01, 59.44, 59.17, 57.68, 57.36, 55.11, 26.50, 26.34, 25.24, 24.94, 24.92, 8.61, 7.40. MS (ESI) m/z calcd for C₂₅H₃₁N₇O₂S [M+], 494.6 ; found, 502.6 [M+ + H+].

### 2-(5-(benzylsulfonyl)-2,5-diazabicyclo[2.2.1]heptan-2-yl)-M-(5-cyclopropyl-1G-pyrazol-3-yl)quinazolin-4-amine (15l)

### 1) tert-butyl 5-(benzylsulfonyl)-2,5-diazabicyclo[2.2.1]heptane-2-carboxylate (17k)

Compound **16** (100 mg, 0.50 mmol) and phenylmethanesulfonyl chloride (144 mg, 0.76 mmol) reacted to afford compound **17k**. (138 mg, 78% yield.)

¹H NMR (300 MHz, Chloroform-d) δ 7.41 (s, 5H), 4.46 (d, J = 40.4 Hz, 1H), 4.26 (s, 2H), 4.17 (s, 1H), 3.39 **-** 3.26 (m, 2H), 3.26 **-** 3.03 (m, 2H), 1.80 **-** 1.66 (m, 2H), 1.48 (s, 9H).

### 2) 2-(benzylsulfonyl)-2,5-diazabicyclo[2.2.1]heptane (18k)

Compound **17k** (134 mg, 0.41 mmol) reacted to afford compound **18k**. (TFA salt, 128 mg, 85% yield.)

¹H NMR (400 MHz, DMSO-d₆) δ 9.16 (s, 1H), 7.61 **-** 7.30 (m, 5H), 4.60 **-** 4.44 (m, 2H), 4.43 (s, 1H), 4.34 (s, 1H), 3.41 (d, J = 2.4 Hz, 2H), 3.20 (s, 2H), 1.92 (d, 1H), 1.83 (d, 1H).

### 3) 15l

A solution of compound **2a** (65 mg, 0.23 mmol) in *t*-butanol (1 mL), and a solution of compound 18k (125 mg, 0.34 mmol) in t-butanol (1 mL) was added. 37% HCl in water (0.005 mL) was added and the reaction was heated at 110°C while stirring. After overnight, a white solid was recovered by filtration washing with ethyl acetate to afford compound **15l**. (31 mg, 27% yield.)

¹H NMR (300 MHz, DMSO-d₆) δ 12.19 (s, 1H), 10.10 (s, 1H), 8.39 (d, *J* = 7.7 Hz, 1H), 7.62 **-** 7.51 (m, 2H), 7.43 **-** 7.26 (m, 6H), 7.12 (t, *J* = 7.5 Hz, 2H), 5.77 (s, 1H), 4.48 (d, *J* = 2.5 Hz, 1H), 4.36 (s, 1H), 2.46 **-** 2.25 (m, 15H), 2.03 **-** 1.82 (m, 3H), 1.45 **-** 1.10 (m, 36H), 1.03 **-** 0.92 (m, 2H), 0.88 (t, *J* = 7.1 Hz, 27H), 0.75 **-** 0.57 (m, 4H).

### M-(5-cyclopropyl-1G-pyrazol-3-yl)-2-(5-(phenylsulfonyl)-2,5-diazabicyclo[2.2.1]heptan-2-yl)quinazolin-4-amine (15m)

### 1) tert-butyl 5-(phenylsulfonyl)-2,5-diazabicyclo[2.2.1]heptane-2-carboxylate (17l)

Compound **16** (200 mg, 1.00 mmol) and benzenesulfonyl chloride (0.19 mL, 1.51 mmol) reacted to afford compound **17l.** (332 mg, 97% yield.)

¹H NMR (300 MHz, Chloroform-*d*) δ 7.91 **-** 7.79 (m, 2H), 7.68 **-** 7.47 (m, 3H), 4.39 (d, J = 36.1 Hz, 2H), 3.43 **-** 3.34 (m, 2H), 3.33 **-** 3.13 (m, 2H), 1.68 (t, J = 10.5 Hz, 1H), 1.41 (d, J = 8.8 Hz, 9H), 1.32 **-** 1.20 (m, 1H).

### 2) 2-(phenylsulfonyl)-2,5-diazabicyclo[2.2.1]heptane (18l)

Compound **17l** (326 mg, 0.96 mmol) reacted to afford compound **18l**. (TFA salt, 350 mg, 103% yield.)

¹H NMR (300 MHz, Chloroform-*d*) δ 10.14 (s, 1H), 9.83 (s, 1H), 7.85 (d, J = 7.4 Hz, 2H), 7.70 **-** 7.62 (m, 1H), 7.61 **-** 7.51 (m, 2H), 4.55 (s, 1H), 4.28 (s, 1H), 3.68 (d, J = 11.3 Hz, 1H), 3.37 (dd, J = 8.9, 3.2 Hz, 2H), 3.25 (d, J = 11.1 Hz, 1H), 1.93 (d, J = 11.4 Hz, 1H), 1.53 (d, J = 11.5 Hz, 1H).

### 3) 15m

A solution of compound **2a** (103 mg, 0.36 mmol) in t-butanol (1 mL), and a solution of compound **18**l (190 mg, 0.54 mmol) in t-butanol (1 mL) was added. 37% HCl in water (0.010 mL) was added and the reaction was heated at 110°C while stirring. After overnight, a white solid was recovered by filtration washing with ethyl acetate to afford compound **15m**. (65 mg, 37% yield.)

¹H NMR (300 MHz, DMSO-d₆) δ 12.14 (s, 1H), 10.06 (s, 1H), 8.36 (d, *J* = 8.3 Hz, 1H), 7.92 **-** 7.75 (m, 2H), 7.64 (t, *J* = 7.2 Hz, 1H), 7.55 (t, *J* = 7.5 Hz, 3H), 7.31 (d, *J* = 8.4 Hz, 1H), 7.11 (t, *J* = 7.5 Hz, 1H), 6.39 (s, 1H), 4.84 (s, 1H), 4.59 (s, 1H), 3.55 (s, 2H), 3.38 (d, *J* = 9.2 Hz, 1H), 2.00 **-** 1.90 (m, 1H), 1.81 (d, *J* = 9.8 Hz, 1H), 1.24 (s, 1H), 1.22 **-** 1.11 (s, 2H), 1.03 **-** 0.90 (m, 2H), 0.75 **-** 0.65 (m, 2H). ¹³C NMR (101 MHz, DMSO-d₆) δ 157.63, 138.26, 133.54, 133.31, 129.92, 127.54, 123.83, 110.98, 94.70, 60.54, 55.27, 54.93, 52.13, 36.35, 14.00, 8.42, 7.58. MS (ESI) m/z calcd for C₂₅H₂₅N₇O₂S [M⁺], 487.6 ; found, 488.4 [M⁺ + H⁺].

### M-(5-cyclopropyl-1G-pyrazol-3-yl)-2-(5-((4-fluorophenyl)sulfonyl)-2,5-diazabicyclo[2.2.1]heptan-2-yl)quinazolin-4-amine (15n)

### 1) tert-butyl 5-((4-fluorophenyl)sulfonyl)-2,5-diazabicyclo[2.2.1]heptane-2-carboxylate (17m)

Compound **16** (150 mg, 0.76 mmol) and 4-fluorobenzenesulfonyl chloride (0.15 mL, 1.14 mmol) reacted to afford compound **17m.** (254 mg, 94% yield.)

¹H NMR (400 MHz, DMSO-d₆) δ 8.01 **-** 7.88 (m, 2H), 7.48 (q, J = 8.1 Hz, 2H), 4.46 (s, 1H), 4.27 (d, J = 20.2 Hz, 1H), 3.27 **-** 3.06 (m, 4H), 1.70 (d, J = 10.2 Hz, 1H), 1.34 (d, J = 23.7 Hz, 9H), 1.25 **-** 1.05 (m, 1H).

### 2) 2-((4-fluorophenyl)sulfonyl)-2,5-diazabicyclo[2.2.1]heptane (18m)

Compound **17m** (246 mg, 0.69 mmol) reacted to afford compound **18m**. (TFA salt, 298 mg, 117% yield.)

¹H NMR (300 MHz, DMSO-d₆) δ 9.14 (d, J = 64.8 Hz, 2H), 8.01 **-** 7.90 (m, 2H), 7.58 **-** 7.45 (m, 2H), 4.59 (s, 1H), 4.34 (s, 1H), 3.48 (d, J = 11.0 Hz, 1H), 3.31 **-** 3.12 (m, 3H), 1.66 (d, J = 11.4 Hz, 1H), 1.13 (d, J = 11.3 Hz, 1H).

### 3) 15n

A solution of compound **2a** (103 mg, 0.36 mmol) in t-butanol (1 mL), and a solution of compound **18m** (200 mg, 0.54 mmol) in t-butanol (1 mL) was added. 37% HCl in water (0.010 mL) was added and the reaction was heated at 110°C while stirring. After overnight, a white solid was recovered by filtration washing with ethyl acetate to afford compound **15n**. (33 mg, 18% yield**.)**

¹H NMR (300 MHz, DMSO-d₆) δ 12.13 (s, 1H), 10.09 (s, 1H), 8.37 (d, *J* = 8.3 Hz, 1H), 8.01 **-** 7.83 (m, 2H), 7.56 (t, *J* = 7.6 Hz, 1H), 7.34 (q, *J* = 8.7 Hz, 3H), 7.12 (t, *J* = 7.6 Hz, 1H), 6.39 (s, 1H), 4.84 (s, 1H), 4.60 (s, 1H), 3.61 **-** 3.47 (m, 2H), 3.40 (s, 1H), 2.02 **-** 1.89 (m, 1H), 1.85 (d, *J* = 9.8 Hz, 1H), 1.24 (s, 3H), 1.01 **-** 0.90 (m, 2H), 0.74 **-** 0.65 (m, 2H). ¹³C NMR (101 MHz, DMSO-d₆) δ 166.23, 163.72, 157.65, 134.79, 133.18, 130.69, 130.60, 125.41, 123.77, 121.47, 117.18, 116.95, 70.26, 60.56, 55.02, 8.40, 7.57. MS (ESI) m/z calcd for C₂₅H₂₄FN₇O₂S [M⁺], 505.3; found, 506.5 [M⁺ + H⁺].

### M-(5-cyclopropyl-1G-pyrazol-3-yl)-2-(5-((2,4-difluorophenyl)sulfonyl)-2,5-diazabicyclo[2.2.1]heptan-2-yl)quinazolin-4-amine (15o)

### 1) tert-butyl 5-((2,4-difluorophenyl)sulfonyl)-2,5-diazabicyclo[2.2.1]heptane-2-carboxylate (17n)

Compound **16** (100 mg, 0.50 mmol) and 2,4-difluorobenzenesulfonyl chloride (0.10 mL, 0.78 mmol) reacted to afford compound **17n.** (178 mg, 94% yield.)

¹H NMR (300 MHz, Chloroform-*d*) δ 8.01 **-** 7.86 (m, 1H), 7.09 **-** 6.88 (m, 2H), 4.65 **-** 4.38 (m, 2H), 3.62 **-** 3.42 (m, 2H), 3.41 **-** 3.20 (m, 2H), 1.85 (t, J = 11.8 Hz, 1H), 1.67 (s, 1H), 1.47 (d, J = 3.8 Hz, 9H).

### 22-((2,4-difluorophenyl)sulfonyl)-2,5-diazabicyclo[2.2.1]heptane (18n)

Compound **17n** (246 mg, 0.69 mmol) reacted to afford compound **18n**. (TFA salt, 181 mg, 101% yield.)

¹H NMR (400 MHz, DMSO-d₆) δ 9.13 (s, 2H), 7.92 (dd, J = 8.5, 6.2 Hz, 1H), 7.72 **-** 7.58 (m, 1H), 7.35 (td, J = 8.5, 2.5 Hz, 1H), 7.29 **-** 7.09 (m, 2H), 4.60 (s, 1H), 4.39 (s, 1H), 3.53 (d, J = 10.7 Hz, 1H), 3.24 (s, 2H), 2.30 (s, 1H), 1.79 (d, J = 11.4 Hz, 1H), 1.58 (d, J = 11.4 Hz, 1H).

### 3) 15o

A solution of compound **2a** (100 mg, 0.35 mmol) in DMF (5 mL), and TEA (1.05 mL, 0.15 mmol) added to the mixture for a few minutes. Compound **18n** (149 mg, 0.39 mmol) was added and the reaction was heated at 50°C to 80°C. After 24 h, the solvent was evaporated. The reaction mixture was dissolved in ethyl acetate and washed with water and brine. The aqueous layer was extracted with ethyl acetate several times. The combined organic layer was dried over Na₂SO₄ and evaporated under vacuum. The crude mixture was purified by prep TLC (5% DCM/MeOH) to afford compound **15o**. (80 mg, 44% yield.)

¹H NMR (300 MHz, DMSO-d₆) δ 12.17 (s, 1H), 10.09 (s, 1H), 8.38 (d, *J* = 8.3 Hz, 1H), 7.90 (td, *J* = 8.6, 6.3 Hz, 1H), 7.61 **-** 7.51 (m, 1H), 7.44 **-** 7.18 (m, 3H), 7.12 (t, *J* = 7.4 Hz, 1H), 6.39 (s, 1H), 4.90 (s, 1H), 4.61 (s, 1H), 3.63 **-** 3.37 (m, 4H), 3.17 (d, *J* = 5.2 Hz, 1H), 2.05 **-** 1.86 (m, 3H), 1.67 (s, 1H), 0.96 (d, *J* = 7.9 Hz, 2H), 0.73 **-** 0.64 (m, 2H). ¹³C NMR (101 MHz, DMSO-d₆) δ 157.67, 152.47, 133.16, 133.04, 125.51, 123.80, 121.47, 113.10, 112.88, 111.04, 106.69, 60.71, 55.05, 54.70, 54.68, 49.07, 8.39, 7.39. MS (ESI) m/z calcd for C₂₅H₂₃F₂N₇O₂S [M⁺], 523.6 ; found, 524.5 [M⁺ + H⁺].

### M-(5-cyclopropyl-1G-pyrazol-3-yl)-2-(5-((2,6-difluorophenyl)sulfonyl)-2,5-diazabicyclo[2.2.1]heptan-2-yl)quinazolin-4-amine (15p)

### 1) tert-butyl 5-((2,6-difluorophenyl)sulfonyl)-2,5-diazabicyclo[2.2.1]heptane-2-carboxylate (17o)

Compound **16** (100 mg, 0.50 mmol) and 2,6-difluorobenzenesulfonyl chloride (0.10 mL, 0.76 mmol) reacted to afford compound **17o.** (143 mg, 76% yield.)

¹H NMR (300 MHz, Chloroform-*d*) δ 7.52 (tt, J = 8.5, 5.9 Hz, 1H), 7.03 (t, J = 9.0 Hz, 2H), 4.66 (s, 1H), 4.50 (d, J = 38.2 Hz, 1H), 3.68 **-** 3.35 (m, 3H), 3.34 **-** 3.27 (m, 1H), 1.91 **-** 1.76 (m, 1H), 1.69 **-** 1.60 (m, 1H), 1.43 (s, 9H), 1.25 (s, 1H).

### 2) 2-((2,6-difluorophenyl)sulfonyl)-2,5-diazabicyclo[2.2.1]heptane (18o)

Compound **17o** (136 mg, 0.36 mmol) reacted to afford compound **18n**. (81 mg, 82% yield.)

¹H NMR (300 MHz, Chloroform-*d*) δ 7.51 (tt, J = 8.4, 5.9 Hz, 1H), 7.02 (t, J = 8.8 Hz, 2H), 4.60 (s, 1H), 3.93 (s, 1H), 3.52 **-** 3.33 (m, 2H), 3.29 **-** 3.00 (m, 2H), 2.38 (s, 5H), 1.82 (d, J = 10.4 Hz, 1H), 1.59 (d, J = 10.6 Hz, 1H), 1.22 (s, 1H).

### 3) 15p

A solution of compound **2a** (60 mg, 0.21 mmol) in *t*-butanol (1 mL), and a solution of compound **18o** (86 mg, 0.32 mmol) in *t*-butanol (1 mL) was added. 37% HCl in water (0.010 mL) was added and the reaction was heated at 110°C while stirring. After overnight, a white solid compound **15p** was recovered by filtration washing with ethyl acetate. (84 mg, 76% yield.)

¹H NMR (300 MHz, DMSO-d₆) δ 11.45 (s, 1H), 8.63 (d, *J* = 8.3 Hz, 1H), 8.11 **-** 7.65 (m, 3H), 7.54 **-** 7.21 (m, 3H), 6.37 (d, *J* = 11.8 Hz, 1H), 4.83 (d, *J* = 11.6 Hz, 1H), 3.92 **-** 3.75 (m, 2H), 3.75 **-** 3.60 (m, 2H), 3.56 **-** 3.46 (m, 2H), 3.25 (s, 1H), 2.13 **-** 2.04 (m, 1H), 1.99 (tt, *J* = 8.4, 5.0 Hz, 1H), 1.87 **-** 1.63 (m, 1H), 1.07 **-** 0.92 (m, 2H), 0.80 **-** 0.66 (m, 2H). ¹³C NMR (101 MHz, DMSO-d₆) δ 160.59, 158.02, 136.62, 135.62, 125.17, 124.87, 117.88, 114.43, 114.20, 95.65, 58.94, 57.88, 57.04, 52.26, 50.55, 35.52, 8.60, 7.38. MS (ESI) m/z calcd for C₂₅H₂₃F₂N₇O₂S [M⁺], 523.6 ; found, 524.5 [M⁺ + H⁺].

### 2-(5-((3-chlorophenyl)sulfonyl)-2,5-diazabicyclo[2.2.1]heptan-2-yl)-M-(5-cyclopropyl-1G-pyrazol-3-yl)quinazolin-4-amine (15q)

### 1) tert-butyl 5-((3-chlorophenyl)sulfonyl)-2,5-diazabicyclo[2.2.1]heptane-2-carboxylate (17p)

Compound **16** (150 mg, 0.76 mmol) and 3-chlorobenzenesulfonyl chloride (0.16 mL, 1.14 mmol) reacted to afford compound **17p.** (263 mg, 93% yield.)

¹H NMR (300 MHz, DMSO-d₆) δ 7.93 **-** 7.86 (m, 1H), 7.88 **-** 7.77 (m, 2H), 7.74 **-** 7.60 (m, 1H), 4.52 (d, J = 2.3 Hz, 1H), 4.29 (d, J = 14.3 Hz, 1H), 3.30 **-** 3.06 (m, 4H), 1.72 (d, J = 9.6 Hz, 1H), 1.35 (d, J = 18.4 Hz, 9H), 1.18 (dd, J = 31.0, 10.3 Hz, 1H).

### 2) 2-((3-chlorophenyl)sulfonyl)-2,5-diazabicyclo[2.2.1]heptane (18p)

Compound **17p** (262 mg, 0.70 mmol) reacted to afford compound **18p**. (205 mg, 75% yield.)

¹H NMR (300 MHz, DMSO-d₆) δ 8.92 (s, 2H), 8.01 **-** 7.79 (m, 3H), 7.78 **-** 7.61 (m, 1H), 4.65 (s, 1H), 4.32 (s, 1H), 3.48 (d, J = 11.0 Hz, 1H), 3.31 **-** 3.13 (m, 3H), 1.67 (d, J = 11.5 Hz, 1H), 1.18 (d, J = 11.3 Hz, 1H).

### 3) 15q

A solution of compound **2a** (103 mg, 0.36 mmol) in t-butanol (1 mL), and a solution of compound **18p** (200 mg, 0.54 mmol) in t-butanol (1 mL) was added. 37% HCl in water (0.010 mL) was added and the reaction was heated at 110°C while stirring. After overnight, a white solid compound **15q** was recovered by filtration washing with ethyl acetate. (42 mg, 22% yield.)

¹H NMR (300 MHz, DMSO-d₆) δ 12.14 (s, 1H), 10.07 (s, 1H), 8.37 (d, *J* = 8.2 Hz, 1H), 7.88 **-** 7.72 (m, 2H), 7.66 (d, *J* = 8.1 Hz, 1H), 7.60 **-** 7.49 (m, 2H), 7.31 (d, *J* = 8.4 Hz, 1H), 7.11 (t, *J* = 7.6 Hz, 1H), 6.38 (s, 1H), 4.84 (s, 1H), 4.65 (s, 1H), 3.62 **-** 3.45 (m, 2H), 3.41 **-** 3.36 (m, 1H), 2.01 **-** 1.91 (m, 1H), 1.87 (s, 1H), 1.33 **-** 1.11 (m, 2H), 1.02 **-** 0.89 (m, 2H), 0.77 **-** 0.65 (m, 2H). ¹³C NMR (101 MHz, DMSO-d₆) δ 157.69, 152.45, 140.34, 134.65, 133.48, 133.11, 131.87, 127.02, 126.21, 125.53, 123.77, 121.43, 111.05, 60.67, 55.00, 8.41. MS (ESI) m/z calcd for C₂₅H₂₄ClN₇O₂S [M⁺], 522.0 ; found, 524.4 [M⁺ + H⁺].

### M-(5-cyclopropyl-1G-pyrazol-3-yl)-2-(5-((3-methoxyphenyl)sulfonyl)-2,5-diazabicyclo[2.2.1]heptan-2-yl)quinazolin-4-amine (15r)

### 1) tert-butyl 5-((3-methoxyphenyl)sulfonyl)-2,5-diazabicyclo[2.2.1]heptane-2-carboxylate (17q)

Compound **16** (100 mg, 0.50 mmol) and 3-methoxybenzenesulfonyl chloride (0.11 mL, 0.76 mmol) reacted to afford compound **17q.** (164 mg, 89% yield.)

¹H NMR (300 MHz, Chloroform-*d*) δ 7.51 **-** 7.40 (m, 2H), 7.38 **-** 7.33 (m, 1H), 7.19 **-** 7.09 (m, 1H), 4.41 (d, J = 33.7 Hz, 2H), 3.88 (s, 3H), 3.55 **-** 3.35 (m, 2H), 3.35 **-** 3.17 (m, 2H), 1.77 **-** 1.64 (m, 1H), 1.44 (d, J = 8.8 Hz, 9H), 1.35 **-** 1.24 (m, 1H).

### 2) 2-((3-methoxyphenyl)sulfonyl)-2,5-diazabicyclo[2.2.1]heptane (18q)

Compound **17q** (158 mg, 0.43 mmol) reacted to afford compound **18q**. (98 mg, 86% yield.)

¹H NMR (400 MHz, Chloroform-*d*) δ 7.54 **-** 7.40 (m, 2H), 7.36 (s, 1H), 7.20 **-** 7.07 (m, 1H), 4.43 (s, 1H), 3.89 (s, 3H), 3.84 (s, 1H), 3.43 **-** 3.25 (m, 2H), 3.25 **-** 2.96 (m, 2H), 2.69 (s, 3H), 1.67 (d, J = 10.3 Hz, 1H), 1.29 (d, J = 10.2 Hz, 1H).

### 3) 15r

A solution of compound **2a** (70 mg, 0.25 mmol) in *t*-butanol (1 mL), and a solution of compound **18q** (99 mg, 0.37 mmol) in t-butanol (1 mL) was added. 37% HCl in water (0.010 mL) was added and the reaction was heated at 110°C while stirring. After overnight, a white solid was recovered by filtration washing with ethyl acetate to afford compound **15r**. (126 mg, 99% yield.)

¹H NMR (300 MHz, DMSO-d₆) δ 11.44 (s, 1H), 8.63 (d, *J* = 8.3 Hz, 1H), 8.06 (dd, *J* = 35.5, 8.4 Hz, 1H), 7.84 (t, *J* = 7.8 Hz, 1H), 7.55 **-** 7.38 (m, 3H), 7.38 **-** 7.29 (m, 1H), 7.20 (d, *J* = 7.8 Hz, 1H), 6.35 (d, *J* = 4.9 Hz, 1H), 4.76 (d, *J* = 11.7 Hz, 1H), 3.84 **-** 3.67 (m, 5H), 3.67 **-** 3.53 (m, 2H), 3.29 **-** 3.14 (m, 1H), 2.07 **-** 1.86 (m, 2H), 1.31 (d, *J* = 10.1 Hz, 1H), 1.07 **-** 0.90 (m, 2H), 0.80 **-** 0.68 (m, 2H). ¹³C NMR (101 MHz, DMSO-d₆) δ 160.15, 139.21, 135.51, 131.32, 124.83, 117.94, 112.57, 112.38, 110.12, 95.68, 59.57, 57.61, 56.66, 56.22, 56.10, 55.29, 36.65, 8.60, 7.41. MS (ESI) m/z calcd for C₂₆H₂₇N₇O₃S [M⁺], 517.6 ; found, 519.5 [M⁺ + H⁺].

### 1-(4-((5-(4-((5-cyclopropyl-1G-pyrazol-3-yl)amino)quinazolin-2-yl)-2,5-diazabicyclo[2.2.1]heptan-2-yl)sulfonyl)phenyl)ethan-1-one (15s)

### 1) tert-butyl 5-((4-acetylphenyl)sulfonyl)-2,5-diazabicyclo[2.2.1]heptane-2-carboxylate (17r)

Compound **16** (100 mg, 0.50 mmol) and 4-acetylbenzenesulfonyl chloride (165 mg, 0.76 mmol) reacted to afford compound **17r.** (167 mg, 87% yield.)

¹H NMR (400 MHz, Chloroform-*d*) δ 8.09 (d, J = 8.1 Hz, 2H), 7.93 (d, J = 8.1 Hz, 2H), 4.53 **-** 4.29 (m, 2H), 3.45 (dd, J = 35.5, 9.8 Hz, 2H), 3.33 **-** 3.13 (m, 2H), 2.65 (s, 3H), 1.78 **-** 1.66 (m, 1H), 1.40 (d, J = 15.3 Hz, 9H), 1.34 **-** 1.24 (m, 1H).

### 2) 1-(4-((2,5-diazabicyclo[2.2.1]heptan-2-yl)sulfonyl)phenyl)ethan-1-one (18r)

Compound **17r** (162 mg, 0.43 mmol) reacted to afford compound **18r**. (102 mg, 85% yield.)

### 3) 15s

A solution of compound **2a** (65 mg, 0.23 mmol) in *t*-butanol (1 mL), and a solution of compound **18r** (96 mg, 0.34 mmol) in t-butanol (1 mL) was added. 37% HCl in water (0.005 mL) was added and the reaction was heated at 110°C while stirring. After overnight, a white solid was recovered by filtration washing with ethyl acetate. The solid was dissolved in DCM, washed with water. The organic layer was dried over Na₂SO₄ and evaporated under vacuum to afford compound **15s** as a white solid. (102 mg, 85%)

¹H NMR (300 MHz, DMSO-d₆) δ 12.17 (s, 1H), 10.08 (s, 1H), 8.37 (d, *J* = 8.2 Hz, 1H), 8.06 **-** 7.89 (m, 4H), 7.55 (t, *J* = 7.6 Hz, 1H), 7.32 (s, 1H), 7.12 (t, *J* = 7.6 Hz, 1H), 6.35 (s, 1H), 4.83 (s, 1H), 4.63 (s, 1H), 3.58 **-** 3.36 (m, 4H), 2.52 (s, 3H), 2.02 **-** 1.84 (m, 2H), 1.49 **-** 1.31 (m, 1H), 0.97 (d, *J* = 8.2 Hz, 2H), 0.71 (dd, *J* = 5.1, 2.1 Hz, 2H). ¹³C NMR (101 MHz, DMSO-d₆) δ 197.36, 157.55, 142.19, 140.09, 133.23, 129.51, 127.86, 123.79, 121.55, 110.95, 60.62, 54.96, 54.75, 27.27, 8.40, 7.56. MS (ESI) m/z calcd for C₂₇H₂₇N₇O₃S [M⁺], 529.6 ; found, 530.6 [M⁺ + H⁺].

### M-(5-cyclopropyl-1G-pyrazol-3-yl)-2-(5-(/ -tolylsulfonyl)-2,5-diazabicyclo[2.2.1]heptan-2-yl)quinazolin-4-amine (15t)

### 1) tert-butyl 5-(m-tolylsulfonyl)-2,5-diazabicyclo[2.2.1]heptane-2-carboxylate (17s)

Compound **16** (100 mg, 0.50 mmol) and 3-methylbenzenesulfonyl chloride (0.11 mL, 0.76 mmol) reacted to afford compound **17s.** (188 mg, 106% yield.)

¹H NMR (400 MHz, Chloroform-*d*) δ 7.71 **-** 7.58 (m, 2H), 7.49 **-** 7.35 (m, 2H), 4.39 (d, J = 45.2 Hz, 2H), 3.54 **-** 3.33 (m, 2H), 3.33 **-** 3.13 (m, 2H), 2.43 (s, 3H), 1.67 (d, J = 12.5 Hz, 1H), 1.41 (d, J = 13.0 Hz, 9H), 1.27 (d, J = 12.5 Hz, 2H).

### 2) 2-(m-tolylsulfonyl)-2,5-diazabicyclo[2.2.1]heptane (18s)

Compound **17s** (185 mg, 0.53 mmol) reacted to afford compound **18r**. (114 mg, 86% yield.)

### 3) 15t

A solution of compound **2a** (85 mg, 0.30 mmol) in *t*-butanol (1 mL), and a solution of compound **18r** (113 mg, 0.45 mmol) in *t*-butanol (1 mL) was added. 37% HCl in water (0.005 mL) was added and the reaction was heated at 110°C while stirring. After overnight, a white solid was recovered by filtration washing with ethyl acetate to afford compound **15t** as a white solid. (140 mg, 94% yield.)

¹H NMR (300 MHz, DMSO-d₆) δ 12.59 (s, 1H), 11.44 (s, 1H), 8.63 (d, *J* = 8.3 Hz, 1H), 8.01 **-** 7.75 (m, 2H), 7.68 (d, *J* = 9.0 Hz, 2H), 7.59 **-** 7.35 (m, 3H), 6.36 (s, 1H), 4.72 (d, *J* = 13.7 Hz, 1H), 3.86 **-** 3.45 (m, 4H), 2.32 (s, 3H), 2.05 **-** 1.82 (m, 2H), 1.36 (d, *J* = 10.3 Hz, 1H), 1.08 **-** 0.91 (m, 2H), 0.81 **-** 0.64 (m, 2H). ¹³C NMR (101 MHz, DMSO-d₆) δ 139.95, 137.98, 135.57, 134.38, 129.88, 127.75, 124.83, 124.75, 117.92, 110.14, 95.71, 67.40, 59.40, 56.55, 31.78, 21.16, 8.59, 7.39. MS (ESI) m/z calcd for C₂₆H₂₇N₇O₂S [M⁺], 501.6 ; found, 502.6 [M⁺ + H⁺].

### M-(5-cyclopropyl-1G-pyrazol-3-yl)-2-(5-((3-(trifluoromethyl)phenyl)sulfonyl)-2,5-diazabicyclo[2.2.1]heptan-2-yl)quinazolin-4-amine (15u)

### 1) tert-butyl 5-((3-(trifluoromethyl)phenyl)sulfonyl)-2,5-diazabicyclo[2.2.1]heptane-2-carboxylate (17t)

Compound **16** (100 mg, 0.50 mmol) and 3-(trifluoromethyl)benzenesulfonyl chloride (0.121 mL, 0.76 mmol) reacted to afford compound **17t.** (154 mg, 75% yield.)

¹H NMR (400 MHz, Chloroform-*d*) δ 8.11 (s, 1H), 8.04 (d, J = 7.8 Hz, 1H), 7.87 (d, J = 7.8 Hz, 1H), 7.70 (t, J = 7.8 Hz, 1H), 4.44 (d, J = 46.4 Hz, 2H), 3.52 **-** 3.33 (m, 2H), 3.33 **-** 3.16 (m, 2H), 1.83 **-** 1.67 (m, 1H), 1.41 (d, J = 14.6 Hz, 9H), 1.32 **-** 1.16 (m, 1H).

### 2) 2-((3-(trifluoromethyl)phenyl)sulfonyl)-2,5-diazabicyclo[2.2.1]heptane (18t)

Compound **17t** (152 mg, 0.37 mmol) reacted to afford compound **18t**. (111 mg, 98% yield.)

### 3) 15u

A solution of compound **2a** (85 mg, 0.30 mmol) in *t*-butanol (1 mL), and a solution of compound **18t** (109 mg, 0.36 mmol) in *t*-butanol (1 mL) was added. 37% HCl in water (0.005 mL) was added and the reaction was heated at 110°C while stirring. After overnight, a white solid was recovered by filtration washing with ethyl acetate to afford compound **15u**. (164 mg, 99% yield.)

¹H NMR (300 MHz, DMSO-d₆) δ 12.59 (s, 1H), 11.44 (s, 1H), 8.63 (d, *J* = 8.3 Hz, 1H), 8.25 (d, *J* = 7.9 Hz, 1H), 8.16 (s, 1H), 8.06 (d, *J* = 7.9 Hz, 1H), 7.96 **-** 7.75 (m, 3H), 7.52 **-** 7.39 (m, 1H), 6.36 (s, 1H), 5.11 **-** 4.75 (m, 2H), 3.81 **-** 3.41 (m, 4H), 2.05 **-** 1.90 (m, 2H), 1.49 **-** 1.31 (m, 1H), 1.00 (s, 2H), 0.78 **-** 0.67 (m, 2H). ¹³C NMR (101 MHz, DMSO) δ 157.41, 157.23, 139.67, 135.53, 131.72, 130.84, 130.47, 125.10, 124.83, 124.07, 122.39, 117.93, 110.14, 95.67, 67.40, 60.26, 59.60, 59.41, 58.86, 56.77, 55.37, 36.85, 36.20, 31.77, 8.57, 7.39. MS (ESI) m/z calcd for C₂₆H₂₃F₃N₇O₂S [M⁺], 555.6 ; found, 556.6 [M⁺ + H⁺].

### 2-((5-(4-((5-cyclopropyl-1G-pyrazol-3-yl)amino)quinazolin-2-yl)-2,5-diazabicyclo[2.2.1]heptan-2-yl)sulfonyl)benzonitrile (15v)

### 1) tert-butyl 5-((2-cyanophenyl)sulfonyl)-2,5-diazabicyclo[2.2.1]heptane-2-carboxylate (17u)

Compound **16** (200 mg, 1.01 mmol) and 2-cyanobenzenesulfonyl chloride (305 mg, 1.51 mmol) reacted to afford compound **17u** as a white solid**.** (348 mg, 95% yield.)

¹H NMR (300 MHz, Chloroform-*d*) δ 8.10 (dd, J = 7.5, 1.6 Hz, 1H), 7.88 (dd, J = 7.2, 1.6 Hz, 1H), 7.81 **-** 7.63 (m, 2H), 4.71 (d, J = 31.7 Hz, 1H), 4.51 (d, J = 31.7 Hz, 1H), 3.65 **-** 3.22 (m, 4H), 1.95 **-** 1.84 (m, 1H), 1.75 (d, J = 10.3 Hz, 1H), 1.44 (s, 9H).

### 2) 2-((2,5-diazabicyclo[2.2.1]heptan-2-yl)sulfonyl)benzonitrile (18u)

Compound **17u** (343 mg, 0.94 mmol) reacted to afford compound **18u**. (94 mg, 38% yield.)

### 3) 15v

A solution of compound **2a** (53 mg, 0.19 mmol) in *t*-butanol (1 mL), and a solution of compound **18u** (109 mg, 0.36 mmol) in t-butanol (1 mL) was added. 37% HCl in water (0.005 mL) was added and the reaction was heated at 110°C while stirring. After overnight, a white solid was recovered by filtration washing with ethyl acetate to afford compound **15v**. (81 mg, 85% yield.)

¹H NMR (300 MHz, DMSO-d₆) δ 12.60 (s, 1H), 11.45 (s, 1H), 8.64 (d, *J* = 8.3 Hz, 1H), 8.27 **-** 8.05 (m, 2H), 8.01 **-** 7.75 (m, 4H), 7.56 **-** 7.39 (m, 1H), 6.46 **-** 6.27 (m, 1H), 4.83 (s, 1H), 3.91 **-** 3.60 (m, 3H), 3.60 **-** 3.31 (m, 2H), 2.12 (d, *J* = 10.3 Hz, 1H), 1.98 (tt, *J* = 8.4, 5.1 Hz, 1H), 1.82 (d, *J* = 10.3 Hz, 1H), 1.07 **-** 0.89 (m, 2H), 0.84 **-** 0.62 (m, 2H). ¹³C NMR (101 MHz, DMSO-d₆) δ 157.32, 141.12, 136.77, 135.59, 134.62, 134.23, 130.33, 124.87, 116.84, 110.18, 109.61, 95.62, 60.00, 58.97, 56.79, 55.20, 8.62, 7.39. MS (ESI) m/z calcd for C₂₆H₂₄N₈O₂S [M⁺], 512.6 ; found, 513.5 [M⁺ + H⁺].

### M-(5-cyclopropyl-1G-pyrazol-3-yl)-2-(5-((3-fluoro-4-methylphenyl)sulfonyl)-2,5-diazabicyclo[2.2.1]heptan-2-yl)quinazolin-4-amine (15w)

### 1) tert-butyl 5-((3-fluoro-4-methylphenyl)sulfonyl)-2,5-diazabicyclo[2.2.1]heptane-2-carboxylate (17v)

Compound **16** (200 mg, 1.01 mmol) and 3-fluoro-4-methylbenzenesulfonyl chloride (316 mg, 1.51 mmol) reacted to afford compound **17v** as a light yellow solid**.** (344 mg, 92% yield.)

¹H NMR (300 MHz, Chloroform-*d*) δ 7.56 **-** 7.42 (m, 2H), 7.35 (t, J = 7.5 Hz, 1H), 4.52 **-** 4.27 (m, 2H), 3.56 **-** 3.32 (m, 2H), 3.32 **-** 3.12 (m, 2H), 2.36 (d, J = 2.1 Hz, 3H), 1.79 **-** 1.65 (m, 1H), 1.41 (d, J = 9.2 Hz, 9H), 1.32 (d, J = 10.2 Hz, 1H).

### 2) 2-((3-fluoro-4-methylphenyl)sulfonyl)-2,5-diazabicyclo[2.2.1]heptane (18v)

Compound **17v** (342 mg, 0.93 mmol) reacted to afford compound **18v**. (60 mg, 24% yield.)

### 3) 15w

A solution of compound **2a** (52 mg, 0.18 mmol) in *t*-butanol (1 mL), and a solution of compound **18v** (59 mg, 0.22 mmol) in *t*-butanol (1 mL) was added. 37% HCl in water (0.005 mL) was added and the reaction was heated at 110°C while stirring. After overnight, a white solid was recovered by filtration washing with ethyl acetate to afford compound **15w**. (96 mg, 101% yield.)

¹H NMR (300 MHz, DMSO-d₆) δ 12.58 (s, 1H), 11.45 (s, 1H), 8.64 (d, *J* = 8.3 Hz, 1H), 7.86 (s, 2H), 7.72 **-** 7.58 (m, 3H), 7.56 **-** 7.38 (m, 2H), 6.35 (s, 1H), 4.76 (d, *J* = 15.3 Hz, 1H), 3.79 **-** 3.41 (m, 5H), 3.30 **-** 3.14 (m, 1H), 2.24 (s, 3H), 2.06 **-** 1.90 (m, 2H), 1.52 **-** 1.34 (m, 1H), 1.07 **-** 0.94 (m, 2H), 0.79 **-** 0.67 (m, 2H). ¹³C NMR (101 MHz, DMSO) δ 162.04, 159.57, 157.04, 150.38, 140.40, 135.49, 133.32, 131.45, 131.29, 131.12, 125.11, 124.81, 123.86, 123.63, 117.92, 114.71, 114.45, 114.14, 110.29, 110.08, 95.82, 95.52, 67.40, 60.07, 59.46, 58.76, 58.51, 57.62, 56.78, 56.45, 55.34, 54.55, 51.93, 50.87, 36.81, 36.16, 34.84, 31.77, 14.83, 14.80, 14.71, 8.57, 7.42. MS (ESI) m/z calcd for C₂₆H₂₆N₇O₂S [M⁺], 519.6 ; found, 520.5 [M⁺ + H⁺].

### M-(5-cyclopropyl-1G-pyrazol-3-yl)-2-(5-((4-propylphenyl)sulfonyl)-2,5-diazabicyclo[2.2.1]heptan-2-yl)quinazolin-4-amine (15x)

### 1) tert-butyl 5-((4-propylphenyl)sulfonyl)-2,5-diazabicyclo[2.2.1]heptane-2-carboxylate (17w)

Compound **16** (200 mg, 1.01 mmol) and 4-propylbenzenesulfonyl chloride (0.27 mL, 1.51 mmol) reacted to afford compound **17w** as a light yellow solid**.** (363 mg, 95% yield.)

¹H NMR (300 MHz, Chloroform-*d*) δ 7.76 (d, J = 8.1 Hz, 2H), 7.35 (d, J = 8.1 Hz, 2H), 4.40 (d, J = 33.4 Hz, 2H), 3.56 **-** 3.34 (m, 2H), 3.34 **-** 3.14 (m, 2H), 2.68 (t, J = 7.5 Hz, 2H), 1.77 **-** 1.62 (m, 3H), 1.43 (d, J = 5.8 Hz, 9H), 1.31 **-** 1.19 (m, 1H), 0.97 (t, J = 7.3 Hz, 3H).

### 2) 2-((4-propylphenyl)sulfonyl)-2,5-diazabicyclo[2.2.1]heptane (18w)

Compound **17w** (361 mg, 0.95 mmol) reacted to afford compound **18w**. (128 mg, 48% yield.)

### 3) 15x

A solution of compound **2a** (105 mg, 0.37 mmol) in *t*-butanol (2 mL), and a solution of compound **18w** (124 mg, 0.44 mmol) in t-butanol (1 mL) was added. 37% HCl in water (0.015 mL) was added and the reaction was heated at 110°C while stirring. After overnight, a white solid was recovered by filtration washing with ethyl acetate to afford compound **15x**. (180 mg, 92% yield.)

¹H NMR (300 MHz, DMSO-d₆) δ 12.53 (d, *J* = 39.5 Hz, 1H), 11.49 **-** 11.34 (m, 1H), 8.63 (d, *J* = 8.3 Hz, 1H), 8.07 **-** 7.73 (m, 4H), 7.53 **-** 7.32 (m, 3H), 6.36 (s, 1H), 4.71 (d, *J* = 13.5 Hz, 1H), 3.79 **-** 3.42 (m, 4H), 3.34 **-** 3.16 (m, 1H), 2.58 (t, *J* = 7.5 Hz, 2H), 2.01 (tt, *J* = 8.5, 5.1 Hz, 1H), 1.93 (d, *J* = 10.2 Hz, 1H), 1.55 (h, *J* = 7.7 Hz, 2H), 1.33 (d, *J* = 10.2 Hz, 1H), 1.09 **-** 0.93 (m, 2H), 0.85 (t, *J* = 7.3 Hz, 3H), 0.79 **-** 0.68 (m, 2H). ¹³C NMR (101 MHz, DMSO-d₆) δ 157.08, 148.65, 135.52, 135.36, 129.90, 127.70, 124.83, 117.91, 110.10, 95.67, 60.05, 59.50, 58.77, 56.43, 55.14, 37.28, 36.67, 36.08, 23.97, 13.95, 8.59, 7.42. MS (ESI) m/z calcd for C₂₈H₃₁N₇O₂S [M⁺], 529.6 ; found, 530.6 [M⁺ + H⁺].

### M-(5-cyclopropyl-1G-pyrazol-3-yl)-2-(5-(naphthalen-2-ylsulfonyl)-2,5-diazabicyclo[2.2.1]heptan-2-yl)quinazolin-4-amine (15y)

### 1) tert-butyl 5-(naphthalen-2-ylsulfonyl)-2,5-diazabicyclo[2.2.1]heptane-2-carboxylate (17x)

Compound **16** (100 mg, 0.50 mmol) and naphthalene-2-sulfonyl chloride (171 mg, 0.76 mmol) reacted to afford compound **17x** as a white solid**.** (169 mg, 87% yield.)

¹H NMR (400 MHz, Chloroform-*d*) δ 8.47 **-** 8.32 (m, 1H), 7.95 (dd, J = 23.9, 8.3 Hz, 3H), 7.83 (d, J = 8.5 Hz, 1H), 7.72 **-** 7.54 (m, 2H), 4.53 (s, 1H), 4.37 (d, J = 44.7 Hz, 1H), 3.55 **-** 3.35 (m, 2H), 3.35 **-** 3.12 (m, 2H), 1.71 **-** 1.61 (m, 1H), 1.46 **-** 1.18 (m, 10H).

### 2) 2-(naphthalen-2-ylsulfonyl)-2,5-diazabicyclo[2.2.1]heptane (18x)

Compound **17x** (150 mg, 0.44 mmol) reacted to afford compound **18x**. (119 mg, 97% yield.)

### 3) 15y

A solution of compound **2a** (75 mg, 0.26 mmol) in *t*-butanol (1 mL), and a solution of compound **18x** (114 mg, 0.39 mmol) in t-butanol (1 mL) was added. 37% HCl in water (0.005 mL) was added and the reaction was heated at 110°C while stirring. After overnight, a white solid compound **15y** was recovered by filtration washing with ethyl acetate. (68 mg, 48% yield.)

¹H NMR (300 MHz, DMSO-d₆) δ 12.14 (s, 1H), 10.02 (s, 1H), 8.51 (s, 1H), 8.33 (d, *J* = 8.2 Hz, 1H), 8.19 **-** 8.03 (m, 2H), 8.00 (d, *J* = 8.0 Hz, 1H), 7.87 (dd, *J* = 8.7, 1.9 Hz, 1H), 7.75 **-** 7.58 (m, 2H), 7.53 (d, *J* = 7.6 Hz, 1H), 7.27 (s, 1H), 7.10 (t, *J* = 7.6 Hz, 1H), 6.37 (s, 1H), 4.81 (s, 1H), 4.68 (s, 1H), 3.67 **-** 3.36 (m, 4H), 1.92 (td, *J* = 8.7, 4.4 Hz, 1H), 1.81 (d, *J* = 9.9 Hz, 1H), 1.19 (s, 1H), 1.03 **-** 0.88 (m, 2H), 0.74 **-** 0.63 (m, 2H). ¹³C NMR (101 MHz, DMSO-d₆) δ 157.54, 135.34, 134.81, 132.23, 130.06, 129.69, 129.40, 128.84, 128.27, 128.07, 123.90, 122.93, 110.88, 60.55, 55.51, 54.97, 36.53, 8.44, 8.41, 7.55. MS (ESI) m/z calcd for C₂₉H₂₇N₇O₂S [M⁺], 537.6 ; found, 538.6 [M⁺ + H⁺].

### M-(5-cyclopropyl-1G-pyrazol-3-yl)-2-(5-(quinolin-8-ylsulfonyl)-2,5-diazabicyclo[2.2.1]heptan-2-yl)quinazolin-4-amine (15z)

### 1) tert-butyl 5-(quinolin-8-ylsulfonyl)-2,5-diazabicyclo[2.2.1]heptane-2-carboxylate (17y)

Compound **16** (100 mg, 0.50 mmol) and quinoline-8-sulfonyl chloride (172 mg, 0.76 mmol) reacted to afford compound **17y** as a white solid. (107 mg, 55% yield.)

¹H NMR (300 MHz, Chloroform-*d*) δ 9.07 (s, 1H), 8.49 (d, J = 7.2 Hz, 1H), 8.25 (d, J = 8.1 Hz, 1H), 8.04 (d, J = 8.1 Hz, 1H), 7.62 (d, J = 8.1 Hz, 1H), 7.58 **-** 7.47 (m, 1H), 5.04 (d, J = 20.1 Hz, 1H), 4.42 (d, J = 30.1 Hz, 1H), 3.79 (t, J = 9.4 Hz, 1H), 3.69 **-** 3.16 (m, 3H), 1.81 **-** 1.67 (m, 1H), 1.41 (d, J = 10.3 Hz, 9H), 1.27 **-** 1.22 (m, 1H).

### 2) 8-((2,5-diazabicyclo[2.2.1]heptan-2-yl)sulfonyl)quinoline (18y)

Compound **17y** (85 mg, 0.22 mmol) reacted to afford compound **18y**. (92 mg, 145% yield.)

### 3) 15z

A solution of compound **2a** (75 mg, 0.26 mmol) in *t*-butanol (1 mL), and a solution of compound **18y** (91 mg, 0.32 mmol) in t-butanol (1 mL) was added. 37% HCl in water (0.005 mL) was added and the reaction was heated at 110°C while stirring. After overnight, a white solid was recovered by filtration washing with ethyl acetate. The solid was dissolved in DCM, washed with water. The organic layer was dried over Na₂SO₄ and evaporated under vacuum, The crude was purified by prep TLC (5% DCM/MeOH) to afford compound 15z. (82 mg, 58% yield.)

0 **-** 7.93 (m, 1H), 7.82 **-** 7.63 (m, 1H), 7.63 **-** 7.45 (m, 1H), 7.45 **-** 7.31 (m, 1H), 7.21 **-** 6.99 (m, 2H), 6.41 (s, 1H), 4.78 (d, *J* = 21.1 Hz, 2H), 4.58 **-** 4.20 (m, 1H), 3.62 (d, *J* = 8.7 Hz, 1H), 3.30 **-** 3.00 (m, 2H), 2.03 **-** 1.77 (m, 2H), 1.77 **-** 1.63 (m, 1H), 1.03 **-** 0.87 (m, 2H), 0.64 (d, *J* = 38.9 Hz, 2H). ¹³C NMR (101 MHz, DMSO-d₆) δ 152.50, 150.79, 143.43, 136.95, 134.32, 133.07, 133.01, 132.71, 129.19, 125.44, 123.67, 122.27, 121.23, 111.02, 60.11, 56.97, 55.12, 37.92, 8.34, 7.51. MS (ESI) m/z calcd for C₂₈H₂₆N₈O₂S [M⁺], 538.6 ; found, 539.5 [M⁺ + H⁺].

### 2-(5-([1,1'-biphenyl]-4-ylsulfonyl)-2,5-diazabicyclo[2.2.1]heptan-2-yl)-M-(5-cyclopropyl-1G-pyrazol-3-yl)quinazolin-4-amine (15aa)

### 1) tert-butyl 5-([1,1'-biphenyl]-4-ylsulfonyl)-2,5-diazabicyclo[2.2.1]heptane-2-carboxylate (17z)

Compound **16** (100 mg, 0.50 mmol) and [1,1'-biphenyl]-4-sulfonyl chloride (191 mg, 0.76 mmol) reacted to afford compound **17z**. (155 mg, 74% yield.)

¹H NMR (300 MHz, Chloroform-*d*) δ 8.00 **-** 7.86 (m, 2H), 7.77 (d, J = 8.1 Hz, 2H), 7.71 **-** 7.56 (m, 2H), 7.57 **-** 7.38 (m, 3H), 4.45 (d, J = 40.8 Hz, 2H), 3.66 **-** 3.37 (m, 2H), 3.38 **-** 3.19 (m, 2H), 1.78 **-** 1.66 (m, 1H), 1.43 (d, J = 14.3 Hz, 8H), 1.40 **-** 1.24 (m, 2H).

### 2) 2-([1,1'-biphenyl]-4-ylsulfonyl)-2,5-diazabicyclo[2.2.1]heptane (18z)

Compound **17z** (150 mg, 0.36 mmol) reacted to afford compound **18z**. (101 mg, 89% yield.)

### 3) 15aa

A solution of compound **2a** (60 mg, 0.21 mmol) in *t*-butanol (1 mL), and a solution of compound **18z** (64 mg, 0.26 mmol) in t-butanol (1 mL) was added. 37% HCl in water (0.005 mL) was added and the reaction was heated at 110°C while stirring. After overnight, the reaction mixture was quenched with water and extracted with ethyl acetate. The combined organic layer was dried over Na₂SO₄ and evaporated under vacuo to afford compound **15aa** as a yellow solid. (119 mg, 101% yield.)

¹H NMR (300 MHz, DMSO-d₆) δ 12.18 (s, 1H), 10.21 (s, 1H), 8.38 (d, *J* = 8.3 Hz, 1H), 7.93 (d, *J* = 8.5 Hz, 2H), 7.86 (d, *J* = 8.5 Hz, 2H), 7.71 **-** 7.63 (m, 2H), 7.63 **-** 7.27 (m, 5H), 7.21 **-** 7.06 (m, 1H), 6.40 (s, 1H), 4.78 (d, *J* = 71.8 Hz, 2H), 3.68 **-** 3.53 (m, 2H), 3.50 **-** 3.36 (m, 2H), 1.98 **-** 1.82 (m, 2H), 1.24 (s, 1H), 1.03 **-** 0.90 (m, 2H), 0.76 **-** 0.66 (m, 2H). ¹³C NMR (101 MHz, DMSO-d₆) δ 157.60, 144.98, 138.73, 137.11, 133.48, 129.55, 129.04, 128.28, 128.05, 127.54, 123.92, 110.89, 94.75, 70.26, 60.51, 60.23, 55.38, 54.96, 21.53, 21.23, 14.56, 8.43, 7.59. MS (ESI) m/z calcd for C₃₁H₂₉N₇O₂S [M⁺], 563.68 ; found, 564.6 [M⁺ + H⁺].

### M-(5-cyclopropyl-1G-pyrazol-3-yl)-2-(5-(thiophen-2-ylsulfonyl)-2,5-diazabicyclo[2.2.1]heptan-2-yl)quinazolin-4-amine (15ab)

### 1) tert-butyl 5-(thiophen-2-ylsulfonyl)-2,5-diazabicyclo[2.2.1]heptane-2-carboxylate (17aa)

Compound **16** (100 mg, 0.50 mmol) and thiophene-2-sulfonyl chloride (138 mg, 0.76 mmol) reacted to afford compound **17aa**. (165 mg, 95% yield.)

¹H NMR (300 MHz, Chloroform-*d*) δ 7.72 **-** 7.53 (m, 2H), 7.16 (dd, J = 5.0, 3.8 Hz, 1H), 4.57 **-** 4.29 (m, 2H), 3.51 (dd, J = 26.5, 10.0 Hz, 2H), 3.38 **-** 3.16 (m, 2H), 1.80 **-** 1.65 (m, 1H), 1.45 (d, J = 6.2 Hz, 9H), 1.36 **-** 1.18 (m, 2H).

### 2) 2-(thiophen-2-ylsulfonyl)-2,5-diazabicyclo[2.2.1]heptane (18aa)

Compound **17aa** (150 mg, 0.44 mmol) reacted to afford compound **18aa**. (107 mg, 101% yield.)

### 3) 15ab

A solution of compound **2a** (60 mg, 0.21 mmol) in *t*-butanol (1 mL), and a solution of compound **18aa** (64 mg, 0.26 mmol) in t-butanol (1 mL) was added. 37% HCl in water (0.005 mL) was added and the reaction was heated at 110°C while stirring. After overnight, a white solid compound **15ab** was recovered by filtration washing with ethyl acetate. (84 mg, 61% yield.)

¹H NMR (400 MHz, DMSO-d₆) δ 12.13 (s, 1H), 10.09 (s, 1H), 8.37 (d, *J* = 8.3 Hz, 1H), 8.01 **-** 7.85 (m, 1H), 7.77 **-** 7.67 (m, 1H), 7.57 (t, *J* = 7.7 Hz, 1H), 7.34 (d, *J* = 8.3 Hz, 1H), 7.23 **-** 7.06 (m, 2H), 6.40 (s, 1H), 4.88 (s, 1H), 4.56 (s, 1H), 3.63 **-** 3.48 (m, 2H), 3.47 **-** 3.35 (m, 2H), 1.98 **-** 1.89 (m, 1H), 1.84 (d, *J* = 10.0 Hz, 1H), 1.28 **-** 1.21 (m, 1H), 1.04 **-** 0.91 (m, 2H), 0.75 **-** 0.62 (m, 2H). ¹³C NMR (101 MHz, DMSO-d₆) δ 170.82, 157.69, 138.03, 134.01, 133.25, 133.08, 128.75, 123.80, 121.55, 111.02, 94.66, 60.85, 60.23, 55.22, 54.85, 36.14, 21.23, 14.56, 8.43, 8.40, 7.57. MS (ESI) m/z calcd for C₂₃H₂₃N₇O₂S₂ [M⁺], 493.6 ; found, 494.5 [M⁺ + H⁺].

### sdqs,butyl 5-(4-((5-cyclopropyl-1G-pyrazol-3-yl)amino)quinazolin-2-yl)-2,5-diazabicyclo[2.2.2]octane-2-carboxylate (19)

Compound **2a** (200 mg, 0.70 mmol) was dissolved in *tert*-butanol 4 mL, and a solution of compound **21** (178 mg, 0.84 mmol) in tert-butanol (2 mL) was added to the mixture. 37% HCl in water (0.020 mL) was added and the reaction was heated at 110°C while stirring. After overnight, the product was recovered by filtration washed with EA. The solid dissolved in MeOH/DCM and purified by column chromatography to afford compound **19**. (195 mg, 60% yield.)

¹H NMR (300 MHz, DMSO) δ 12.66 (s, 2H), 11.33 (s, 1H), 8.61 (d, *J* = 8.2 Hz, 1H), 8.08 (d, *J* = 8.3 Hz, 1H), 7.83 (t, *J* = 7.6 Hz, 1H), 7.45 (t, *J* = 7.8 Hz, 1H), 6.39 (d, *J* = 78.5 Hz, 1H), 5.32 **-** 4.80 (m, 1H), 4.43 **-** 4.21 (m, 1H), 3.97 **-** 3.76 (m, 2H), 3.71 **-** 3.44 (m, 2H), 2.11 **-** 1.82 (m, 5H), 1.43 (d, *J* = 5.4 Hz, 9H), 1.07 **-** 0.91 (m, 2H), 0.80 **-** 0.64 (m, 2H). ¹³C NMR (101 MHz, DMSO) δ 157.40, 154.05, 151.87, 140.78, 135.45, 125.08, 124.77, 118.29, 110.32, 95.15, 79.50, 52.96, 52.77, 49.43, 48.90, 46.98, 46.39, 45.20, 44.07, 43.58, 28.57, 24.92, 24.08, 8.72, 7.30.

### 2-(2,5-diazabicyclo[2.2.2]octan-2-yl)-M-(5-cyclopropyl-1G-pyrazol-3-yl)quinazolin-4-amine trifluoroacetic acid (20)

Compound **19** (192 mg, 0.42 mmol) was dissolved in DCM (4 mL), and 33% TFA in DCM (6 mL) was added at 0°C. The reaction mixture was stirred for 1.5 h at room temperature. The solvent was evaporated and DCM was added repeat 2 times. The solvent was evaporated and ethyl ether was added repeat several times. The solvent was evaporated under vacuum and filtered to afford compound **20** as a light grey solid. (265 mg, quant.)

¹H NMR (400 MHz, DMSO-d₆) δ 12.30 (s, 1H), 11.50 (d, *J* = 26.1 Hz, 1H), 9.46 (d, *J* = 59.9 Hz, 2H), 8.64 (d, *J* = 8.6 Hz, 1H), 7.95 **-** 7.69 (m, 2H), 7.50 (s, 1H), 6.37 (d, *J* = 78.5 Hz, 1H), 4.85 (s, 1H), 4.18 **-** 3.97 (m, 2H), 3.88 (d, *J* = 12.3 Hz, 1H), 3.67 **-** 3.43 (m, 2H), 2.13 (s, 2H), 2.05 **-** 1.85 (m, 3H), 1.05 **-** 0.91 (m, 2H), 0.74 (s, 2H). ¹³C NMR (101 MHz, DMSO) δ 157.49, 151.79, 151.26, 147.04, 140.23, 135.91, 125.51, 124.93, 121.11, 118.19, 117.97, 115.26, 112.34, 110.41, 95.32, 47.55, 45.31, 43.47, 42.48, 20.55, 19.78, 8.64, 7.33.

### sdqs-butyl 3-(4-((5-cyclopropyl-1G-pyrazol-3-yl)amino)quinazolin-2-yl)-3,8-diazabicyclo[3.2.1]octane-8-carboxylate (19')

A mixture of compound **2a** (100 mg, 0.35 mmol) and *tert*-butyl 3,8-diazabicyclo[3.2.1]octane-8-carboxylate (111 mg, 0.53 mmol) in ethanol (3 mL) was stirred at 120°C in a sealed vial for 2 h. The reaction was cooled and solvent was evaporated. The crude was purified by filtration washed with EtOAc to afford compound **19'** as a white solid. (101 mg, 63%)

¹H NMR (300 MHz, DMSO) δ 12.60 (s, 2H), 11.40 (s, 1H), 8.61 (d, *J* = 8.2 Hz, 1H), 8.02 (d, *J* = 8.4 Hz, 1H), 7.85 (t, *J* = 7.7 Hz, 1H), 7.47 (t, *J* = 7.7 Hz, 1H), 6.34 (s, 1H), 4.39 (d, *J* = 12.7 Hz, 2H), 4.30 (s, 2H), 3.30 (s, 2H), 2.05 **-** 1.97 (m, 1H), 1.91 (s, 2H), 1.73 (s, 2H), 1.46 (s, 9H), 1.07 **-** 0.94 (m, 2H), 0.76 **-** 0.66 (m, 2H). ¹³C NMR (101 MHz, DMSO) δ 157.41, 154.02, 153.29, 135.53, 125.31, 124.72, 118.50, 110.34, 95.70, 79.93, 60.23, 55.41, 28.53, 28.43, 21.24, 14.56, 8.65, 7.35. MS (ESI) m/z calcd for C₂₅H₃₁N₇O₂ [M⁺], 461.3 ; found, 462.6 [M⁺ + H⁺].

### 2-(3,8-diazabicyclo[3.2.1]octan-3-yl)-M-(5-cyclopropyl-1G-pyrazol-3-yl)quinazolin-4-amine trifluoroacetic acid (20')

Compound **19'** (70 mg, 0.15 mmol) was dissolved in DCM (2 mL), and TFA (0.5 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum and filtered to afford compound **20'** as a white solid. (94 mg, quant.)

¹H NMR (400 MHz, DMSO) δ 11.40 (s, 1H), 9.47 (d, *J* = 19.3 Hz, 2H), 8.62 (d, *J* = 8.3 Hz, 1H), 7.88 (t, *J* = 7.9 Hz, 1H), 7.77 (d, *J* = 8.3 Hz, 1H), 7.50 (t, *J* = 7.9 Hz, 1H), 6.32 (s, 1H), 4.44 (d, *J* = 13.8 Hz, 2H), 4.29 (s, 2H), 3.60 (d, *J* = 13.8 Hz, 2H), 2.06 **-** 1.81 (m, 5H), 1.05 **-** 0.94 (m, 2H), 0.80 **-** 0.65 (m, 2H). ¹³C NMR (101 MHz, DMSO) δ 159.48, 159.14, 158.80, 158.46, 157.74, 153.90, 147.04, 145.89, 140.62, 135.88, 125.65, 124.79, 121.10, 118.51, 118.18, 115.25, 112.33, 110.53, 95.75, 53.60, 48.45, 25.12, 8.67, 7.37. MS (ESI) m/z calcd for C₂₀H₂₃N₇ [M⁺], 361.2 ; found, 362.5 [M⁺ + H⁺].

The compound represented by Formula 3-9 according to the present invention can be prepared according to the Reaction Scheme but is not limited thereto. 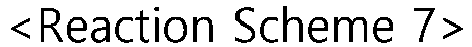 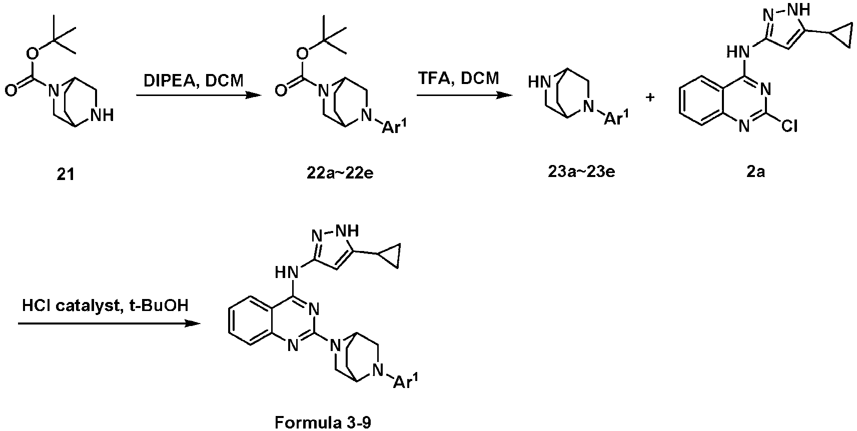 wherein: Ar¹ is the same as defined above.

### M (5-cyclopropyl-1G-pyrazol-3-yl)-2-(5-(isopropylsulfonyl)-2,5-diazabicyclo[2.2.2] octan-2-yl)quinazolin-4-amine (24a)

### 1) tert-butyl 5-(isopropylsulfonyl)-2,5-diazabicyclo[2.2.2]octane-2-carboxylate (22a)

Compound **21** (200 mg, 0.94 mmol) was dissolved in DCM 8 mL. DIPEA (0.49 mL, 2.83 mmol) was added and isopropylsulfonyl chloride (0.16 mL, 1.41 mmol) in DCM was added dropwise. The mixture was stirred at room temperature for overnight. The reaction mixture was washed with water and dried over anhydrous Na₂SO₄. The solvent was evaporated and the crude was purified using column chromatography (40% Hex/EA) to afford compound 22a as a white solid. (164 mg, 55% yield.)

### ¹H NMR (400 MHz, Chloroform-d) δ 4.39 - 4.14 (m, 1H), 3.96 (d, J = 14.8 Hz, 1H), 3.75 - 3.68 (m, 1H), 3.66 - 3.57 (m, 1H), 3.56 - 3.47 (m, 1H), 3.47 - 3.37 (m, 1H), 3.15 (p, J = 6.7 Hz, 1H), 2.25 - 2.09 (m, 1H), 2.10 - 1.89 (m, 1H), 1.89 - 1.71 (m, 2H), 1.47 (s, 9H), 1.35 (d, J = 6.9, 3.1 Hz, 6H).

### 2) 2-(isopropylsulfonyl)-2,5-diazabicyclo[2.2.2]octane (23a)

Compound **22a** (160 mg, 0.50 mmol) was dissolved in DCM (1 mL), and 33% TFA in DCM (2 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **23a**. (95 mg, 86% yield.)

### 3) 24a

Compound **2a** (100 mg, 0.35 mmol) was dissolved in tert-butanol (2 mL), and a solution of **23a** (92 mg, 0.42 mmol) in tert-butanol (1 mL) was added to the mixture. 37% HCl in water (0.005 mL) was added and the reaction was heated at 110°C while stirring. After overnight, the product was recovered by filtration washed with EA. The crude solid was dissolved in DCM/MeOH, purified by prep TLC (3% DCM/MeOH) to afford compound **24a** as an ivory solid. (26 mg, 16% yield.)

¹H NMR (300 MHz, DMSO-d₆) δ 12.18 (s, 1H), 10.07 (d, *J* = 17.7 Hz, 1H), 8.36 (d, *J* = 8.2 Hz, 1H), 7.55 (t, *J* = 8.3 Hz, 1H), 7.42 **-** 7.23 (m, 1H), 7.09 (t, *J* = 7.5 Hz, 1H), 6.60 **-** 6.18 (m, 1H), 5.01 (d, *J* = 37.2 Hz, 1H), 4.03 (s, 1H), 3.96 **-** 3.81 (m, 1H), 3.73 (s, 1H), 3.67 **-** 3.51 (m, 2H), 3.44 **-** 3.36 (m, 1H), 2.14 **-** 1.78 (m, 5H), 1.24 (dd, *J* = 6.7, 3.0 Hz, 6H), 1.02 **-** 0.91 (m, 2H), 0.76 **-** 0.61 (m, 2H). MS (ESI) m/z calcd for C₂₃H₂₉N₇O₂S [M⁺], 467.6 ; found, 468.5 [M⁺ + H⁺].

### 2-(5-(cyclohexylsulfonyl)-2,5-diazabicyclo[2.2.2]octan-2-yl)-M-(5-cyclopropyl-1G-pyrazol-3-yl)quinazolin-4-amine (24b)

### 1) tert-butyl 5-((2,4-difluorophenyl)sulfonyl)-2,5-diazabicyclo[2.2.2]octane-2-carboxylate (22b)

Compound **21** (200 mg, 0.94 mmol) was dissolved in DCM (8 mL). DIPEA (0.49 mL, 2.83 mmol) was added and cyclohexanesulfonyl chloride (0.21 mL, 1.41 mmol) in DCM was added dropwise. The mixture was stirred at room temperature for overnight. The reaction mixture was washed with water and dried over anhydrous Na₂SO₄. The solvent was evaporated and the crude was purified using column chromatography (60% Hex/EA) to afford compound **22b** as a white solid. (186 mg, 55% yield.)

¹H NMR (400 MHz, Chloroform-*d*) δ 4.28 (d, *J* = 64.7 Hz, 1H), 3.96 (d, *J* = 14.4 Hz, 1H), 3.80 **-** 3.57 (m, 2H), 3.55 **-** 3.34 (m, 2H), 2.87 (t, *J* = 12.3 Hz, 1H), 2.15 (d, *J* = 12.8 Hz, 3H), 2.03 (q, *J* = 13.5 Hz, 1H), 1.96 **-** 1.86 (m, 2H), 1.86 **-** 1.76 (m, 2H), 1.76 **-** 1.68 (m, 1H), 1.59 **-** 1.41 (m, 10H), 1.26 (h, *J* = 12.5, 11.8 Hz, 3H).

### 2) 2-(cyclohexylsulfonyl)-2,5-diazabicyclo[2.2.2]octane (23b)

Compound **22b** (182 mg, 0.51 mmol) was dissolved in DCM (1 mL), and 33% TFA in DCM (2 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **23b**. (43 mg, 33% yield.)

### 3) 24b

Compound **2a** (40 mg, 0.14 mmol) was dissolved in tert-butanol (1 mL), and a solution of **23b** (43 mg, 0.17 mmol) in tert-butanol (1 mL) was added to the mixture. 37% HCl in water (0.002 mL) was added and the reaction was heated at 110°C while stirring. After overnight, the product was recovered by filtration washed with EA to afford compound **24b** as a white solid. (64 mg, 90% yield.)

¹H NMR (300 MHz, DMSO-d₆) δ 12.65 (s, 1H), 12.32 (s, 1H), 11.40 (s, 1H), 8.62 (d, *J* = 8.3 Hz, 1H), 8.03 (d, *J* = 8.4 Hz, 1H), 7.85 (t, *J* = 7.9 Hz, 1H), 7.47 (t, *J* = 7.5 Hz, 1H), 6.37 (d, *J* = 65.5 Hz, 1H), 5.08 (d, *J* = 77.2 Hz, 1H), 4.20 **-** 3.67 (m, 4H), 3.59 (t, *J* = 10.7 Hz, 1H), 3.21 **-** 3.07 (m, 1H), 2.21 **-** 1.85 (m, 7H), 1.79 (d, *J* = 10.7 Hz, 2H), 1.63 (d, *J* = 12.3 Hz, 1H), 1.48 **-** 1.05 (m, 5H), 1.05 **-** 0.92 (m, 2H), 0.80 **-** 0.62 (m, 2H). MS (ESI) m/z calcd for C₂₆H₃₃N₇O₂S [M⁺], 507.7 ; found, 508.5 [M⁺ + H⁺].

### M-(5-cyclopropyl-1G-pyrazol-3-yl)-2-(5-((2,4-difluorophenyl)sulfonyl)-2,5-diazabicyclo[2.2.2]octan-2-yl)quinazolin-4-amine (24c)

### 1) tert-butyl 5-((2,4-difluorophenyl)sulfonyl)-2,5-diazabicyclo[2.2.2]octane-2-carboxylate (22c)

Compound **21** (200 mg, 0.94 mmol) was dissolved in DCM (8 mL). DIPEA (0.49 mL, 2.83 mmol) was added and 2,4-difluorobenzenesulfonyl chloride (0.19 mL, 1.41 mmol) in DCM was added dropwise. The mixture was stirred at room temperature for 2 h. The reaction mixture was washed with water and dried over anhydrous Na₂SO₄. The solvent was evaporated and the crude was purified using column chromatography (30% Hex/EA) to afford compound **24c** as a white solid. (337 mg, 92% yield.)

¹H NMR (300 MHz, Chloroform-*d*) δ 8.00 **-** 7.86 (m, 1H), 7.09 **-** 6.91 (m, 2H), 4.41 **-** 4.09 (m, 2H), 3.73 **-** 3.43 (m, 3H), 3.43 **-** 3.31 (m, 1H), 2.15 **-** 1.90 (m, 2H), 1.90 **-** 1.66 (m, 2H), 1.45 (s, 9H).

### 2) 2-((2,4-difluorophenyl)sulfonyl)-2,5-diazabicyclo[2.2.2]octane (23c)

Compound **22c** (332 mg, 0.86 mmol) was dissolved in DCM (1 mL), and 33% TFA in DCM (2 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in small amount of MeOH, and saturated NaHCO₃ was added. The crude was extracted with EtOAc twice, the combined organic layer was dried over Na₂SO₄ and evaporated under vacuum to afford compound **23c**. (312 mg, quant.)

### 3) 24c

Compound **2a** (100 mg, 0.35 mmol) was dissolved in tert-butanol (1 mL), and a solution of **23c** (151 mg, 0.53 mmol) in *tert*-butanol (1 mL) was added to the mixture. 37% HCl in water (0.005 mL) was added and the reaction was heated at 110°C while stirring. After overnight, the product was recovered by filtration washed with EA to afford compound **24c** as a white solid. (174 mg, 92% yield.)

¹H NMR (300 MHz, DMSO-d₆) δ 12.65 (s, 1H), 12.31 (s, 1H), 11.40 (s, 1H), 8.62 (d, *J* = 8.1 Hz, 1H), 8.12 **-** 7.89 (m, 2H), 7.84 (t, *J* = 7.8 Hz, 1H), 7.67 **-** 7.38 (m, 2H), 7.39 **-** 7.22 (m, 1H), 6.29 (d, *J* = 25.2 Hz, 1H), 5.10 (d, *J* = 92.0 Hz, 1H), 4.31 (d, *J* = 14.5 Hz, 1H), 3.94 **-** 3.53 (m, 4H), 2.19 **-** 1.73 (m, 5H), 1.12 **-** 0.90 (m, 2H), 0.71 (s, 2H). MS (ESI) m/z calcd for C₂₆H₂₅F₂N₇O₂S [M⁺], 537.6 ; found, 538.3 [M⁺ + H⁺].

### M-(5-cyclopropyl-1G-pyrazol-3-yl)-2-(5-(thiophen-2-ylsulfonyl)-2,5-diazabicyclo[2.2.2]octan-2-yl)quinazolin-4-amine (24d)

### 1) tert-butyl 5-(thiophen-2-ylsulfonyl)-2,5-diazabicyclo[2.2.2]octane-2-carboxylate (22d)

Compound **21** (200 mg, 0.94 mmol) was dissolved in DCM (8 mL). DIPEA (0.49 mL, 2.83 mmol) was added thiophene-2-sulfonyl chloride (258 mg, 1.41 mmol) in DCM was added dropwise. The mixture was stirred at room temperature for 2 h. The reaction mixture was washed with water and dried over anhydrous Na₂SO₄. The solvent was evaporated and the crude was purified using column chromatography (30% Hex/EA) to afford compound **22d** as a white solid. (338 mg, 100% yield.)

¹H NMR (300 MHz, Chloroform-*d*) δ 7.66 **-** 7.55 (m, 2H), 7.17 **-** 7.09 (m, 1H), 4.38 **-** 4.05 (m, 2H), 3.72 **-** 3.34 (m, 3H), 3.34 **-** 3.21 (m, 1H), 2.05 **-** 1.87 (m, 2H), 1.79 **-** 1.64 (m, 2H), 1.44 (d, *J* = 6.7 Hz, 9H).

### 2) 2-(thiophen-2-ylsulfonyl)-2,5-diazabicyclo[2.2.2]octane (23d)

Compound **22d** (332 mg, 0.93 mmol) was dissolved in DCM (1 mL), and 33% TFA in DCM (2 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in small amount of MeOH, and saturated NaHCO₃ was added. The crude was extracted with EtOAc (x2), the combined organic layer was dried over Na₂SO₄ and evaporated under vacuum to afford compound **23d**. (192 mg, 80% yield.)

### 3) 24d

Compound **2a** (100 mg, 0.35 mmol) was dissolved in tert-butanol (1 mL), and a solution of **23d** (136 mg, 0.53 mmol) in tert-butanol (1 mL) was added to the mixture. 37% HCl in water (0.005 mL) was added and the reaction was heated at 110°C while stirring. After overnight, the product was recovered by filtration washed with EtOAc. The crude solid was dissolved in DCM and purified by column chromatography (3% DCM/MeOH) to afford compound **24d** as a white solid. (39 mg, 22% yield.)

¹H NMR (300 MHz, DMSO-d₆) δ 12.19 (s, 1H), 10.05 (s, 1H), 8.36 (d, *J* = 8.2 Hz, 1H), 7.95 (d, *J* = 5.0 Hz, 1H), 7.79 **-** 7.67 (m, 1H), 7.54 (t, *J* = 7.6 Hz, 1H), 7.30 (d, *J* = 8.5 Hz, 1H), 7.19 (s, 1H), 7.09 (t, *J* = 6.9 Hz, 1H), 6.31 (d, *J* = 19.4 Hz, 1H), 4.96 (d, *J* = 34.8 Hz, 1H), 4.15 (s, 1H), 3.72 **-** 3.47 (m, 3H), 3.47 **-** 3.35 (m, 1H), 2.00 **-** 1.65 (m, 5H), 1.00 (s, 2H), 0.70 (s, 2H). MS (ESI) m/z calcd for C₂₄H₂₅N₇O₂S₂ [M⁺], 507.6 ; found, 508.4 [M⁺ + H⁺].

### 2-((5-(4-((5-cyclopropyl-1G-pyrazol-3-yl)amino)quinazolin-2-yl)-2,5-diazabicyclo[2.2.2]octan-2-yl)sulfonyl)benzonitrile (24e)

### 1) tert-butyl 5-((2-cyanophenyl)sulfonyl)-2,5-diazabicyclo[2.2.2]octane-2-carboxylate (22e)

Compound **21** (200 mg, 0.94 mmol) was dissolved in DCM (8 mL). DIPEA (0.49 mL, 2.83 mmol) was added and 2-cyanobenzenesulfonyl chloride (285 mg, 1.41 mmol) in DCM was added dropwise. The mixture was stirred at room temperature for overnight. The reaction mixture was washed with water and dried over anhydrous Na₂SO₄. The solvent was evaporated and the crude was purified using column chromatography (60% Hex/EA) to afford compound **22e** as a white solid. (149 mg, 42% yield.)

¹H NMR (400 MHz, Chloroform-*d*) δ 8.10 (t, *J* = 7.1 Hz, 1H), 7.89 (d, *J* = 7.4 Hz, 1H), 7.79 **-** 7.64 (m, 2H), 4.39 **-** 4.16 (m, 2H), 3.76 **-** 3.58 (m, 2H), 3.58 **-** 3.44 (m, 1H), 3.40 (d, *J* = 11.8 Hz, 1H), 2.19 **-** 1.93 (m, 3H), 1.85 **-** 1.70 (m, 2H), 1.43 (s, 9H).

### 2) 2-((2,5-diazabicyclo[2.2.2]octan-2-yl)sulfonyl)benzonitrile (23e)

Compound **22e** (145 mg, 0.38 mmol) was dissolved in DCM (1 mL), and 33% TFA in DCM (2 mL) was added at 0°C. The reaction mixture was stirred for 2 h at room temperature. The solvent was evaporated and DCM was added repeat 2 times. The solvent was evaporated and ethyl ether was added repeat several times. The solvent was evaporated under vacuum. TFA salt was dissolved in MeOH, and saturated NaHCO₃ was added. All solvent was evaporated, and the salt was extracted with 2% DCM/MeOH. After filtration, the organic layer was dried over Na₂SO₄ and evaporated under vacuum to afford compound **23e**. (84 mg, 79% yield.)

### 3) 24e

Compound **2a** (60 mg, 0.21 mmol) was dissolved in tert-butanol (1 mL), and a solution of **23e** (82 mg, 0.29 mmol) in tert-butanol (1 mL) was added to the mixture. 37% HCl in water (0.005 mL) was added and the reaction was heated at 110°C while stirring. After overnight, the product was recovered by filtration washed with EA to afford compound **24e** as a white solid.

### (103 mg, 93% yield.)

¹H NMR (300 MHz, DMSO-d₆) δ 12.64 (s, 1H), 11.41 (s, 1H), 8.62 (d, *J* = 8.2 Hz, 1H), 8.24 **-** 8.06 (m, 2H), 8.05 **-** 7.73 (m, 4H), 7.54 **-** 7.37 (m, 1H), 6.31 (d, *J* = 34.3 Hz, 1H), 5.10 (d, *J* = 76.0 Hz, 1H), 4.36 (s, 1H), 3.93 **-** 3.56 (m, 4H), 2.22 **-** 1.80 (m, 5H), 1.07 **-** 0.90 (m, 2H), 0.81 **-** 0.60 (m, 2H). ¹³C NMR (101 MHz, DMSO) δ 157.41, 141.42, 140.39, 136.77, 135.62, 134.62, 134.20, 130.44, 125.30, 124.82, 118.14, 116.79, 110.35, 109.43, 95.53, 95.27, 52.27, 51.82, 49.70, 48.22, 47.70, 46.52, 25.26, 24.24, 23.62, 8.70, 7.39. MS (ESI) m/z calcd for C₂₇H₂₆N₈O₂S [M⁺], 526.6 ; found, 527.4 [M⁺ + H⁺].

### 4-((2-((4-(cyanomethyl)phenyl)amino)quinazolin-4-yl)amino)-M,M-dimethylbenzenesulfonamide (25a)

Compound **2h** (88 mg, 0.24 mmol) was dissolved in *tert*-butanol (3 mL). A solution of 2-(4-aminophenyl)acetonitrile (48.1 mg, 0.36 mmol) in tert-butanol (0.5 mL) was added to the mixture. Then, 37% HCl in water (0.015 mL) was added, and the reaction mixture was stirred at 110°C for 16 hours. The solvent was removed under reduced pressure. To the mixture, water was added and extracted with ethyl acetate. The organic layer was dried over anhydrous Na₂SO₄, then purified by column chromatography (2% MeOH/DCM) to afford compound **25a**.

### (52 mg, 47%)

¹H NMR (300 MHz, DMSO-d₆) δ 9.93 (s, 1H), 9.39 (s, 1H), 8.45 (d, *J* = 8.2 Hz, 1H), 8.34 (d, *J* = 8.8 Hz, 2H), 7.95 (d, *J* = 8.4 Hz, 2H), 7.82(m, 3H), 7.62-7.52 (m, 1H), 7.41-7.29 (m, 1H), 7.27 (d, *J* = 8.5 Hz, 2H), 3.97 (s, 2H), 2.65 (s, 6H). MS (ESI) m/z calcd C₂₄H₂₂N₆O₂S [M⁺] ,458.2 found, 459.4 [M⁺ + H⁺].

### 4-((2-((4-cyanophenyl)amino)quinazolin-4-yl)amino)-M,M-dimethylbenzenesulfonamide (25b)

Compound **2h** (70 mg, 0.19 mmol) was dissolved in tert-butanol (3 mL). A solution of 4-aminobenzonitrile (34 mg, 0.29 mmol) in tert-butanol (0.5 mL) was added to the mixture. Then, 37% HCl in water (0.012 mL) was added, and the reaction mixture was stirred at 110°C for 16 hours. The solvent was removed under reduced pressure. To the mixture, water was added and extracted with ethyl acetate. The organic layer was dried over anhydrous Na2SO4, then purified by column chromatography (20-33% EA/Hex) to afford compound **25b**. (21 mg, 25%)

¹H NMR (300 MHz, DMSO-d₆) δ 10.05 (s, 1H), 9.85 (s, 1H), 8.49 (d, *J* = 8.2 Hz, 1H), 8.31 (d, *J* = 8.7 Hz, 2H), 8.16 (d, *J* = 8.8 Hz, 2H), 7.85(m, 5H), 7.64 (d, *J* = 8.2 Hz, 1H), 7.49(m, 1H), 2.65 (s, 6H). MS (ESI) m/z calcd C₂₃H₂₀N₆O₂S [M⁺] ,444.1 found, 445.4 [M⁺ + H⁺].

### M,M-dimethyl-4-((2-((4-(trifluoromethyl)phenyl)amino)quinazolin-4-yl)amino)benzenesulfonamide (25c)

Compound **2h** (70 mg, 0.19 mmol) was dissolved in tert-butanol (3 mL). A solution of 4-(trifluoromethyl)aniline (47 mg, 0.29 mmol) in tert-butanol (0.5 mL) was added to the mixture. Then, 37% HCl in water (0.012 mL) was added, and the reaction mixture was stirred at 110°C for 16 hours. The solvent was removed under reduced pressure. To the mixture, water was added and extracted with ethyl acetate. The organic layer was dried over anhydrous Na₂SO₄, then purified by column chromatography (20-33% EA/Hex) to afford compound **25c**. (66 mg, 70%)

¹H NMR (300 MHz, DMSO-d₆) δ 10.02 (s, 1H), 9.74 (s, 1H), 8.48 (d, *J* = 8.2 Hz, 1H), 8.37(m, 2H), 8.15 (d, *J* = 8.5 Hz, 2H), 7.83(m, 3H), 7.68-7.57 (m, 3H), 7.47-7.35 (m, 1H), 2.65 (s, 6H). MS (ESI) m/z calcd C₂₃H₂₀F₃N₅O₂S [M⁺] ,487.1 found, 488.4 [M⁺ + H⁺].

### M,M-dimethyl-4-((2-(phenylamino)quinazolin-4-yl)amino)benzenesulfonamide (25d)

Compound **2h** (70 mg, 0.19 mmol) was dissolved in tert-butanol (3 mL). A solution of aniline (27 mg, 0.29 mmol) in tert-butanol (0.5 mL) was added to the mixture. Then, 37% HCl in water (0.012 mL) was added, and the reaction mixture was stirred at 110°C for 16 hours. The solvent was removed under reduced pressure. To the mixture, water was added and extracted with ethyl acetate. The organic layer was dried over anhydrous Na₂SO₄, then purified by column chromatography (20-33% EA/Hex) to afford compound **25d**. (12 mg, 15%)

¹H NMR (300 MHz, DMSO-d₆) δ 9.92 (s, 1H), 9.32 (s, 1H), 8.44 (d, *J* = 8.3 Hz, 1H), 8.35 (d, *J* = 8.8 Hz, 2H), 7.92 (d, *J* = 8.0 Hz, 2H), 7.80(m, 3H), 7.56 (d, *J* = 8.2 Hz, 1H), 7.40(m, 3H), 7.02-6.90 (m, 1H), 2.64 (s, 6H). MS (ESI) m/z calcd C₂₂H₂₁N₅O₂S [M⁺] ,419.1 found, 420.4 [M⁺ + H⁺].

### 2-(4-((4-((4-oxo-4,5-dihydrothiazol-2-yl)amino)quinazolin-2-yl)amino)phenyl)acetonitrile (26a)

Compound **2j** (70 mg, 0.25 mmol) was dissolved in *tert*-butanol (3 mL). A solution of 2-(4-aminophenyl)acetonitrile (50 mg, 0.38 mmol) in *tert*-butanol (0.5 mL) was added to the mixture. Then, 37% HCl in water (0.012 mL) was added, and the reaction mixture was stirred at 110°C for 16 hours. The solvent was removed under reduced pressure. To the mixture, water was added and extracted with ethyl acetate. The organic layer was dried over anhydrous Na₂SO₄, then purified by column chromatography (50% EA/Hex) to afford compound **26a** (76 mg, 84%)

¹H NMR (400 MHz, DMSO-d₆) δ 12.35 (s, 1H), 9.41 (s, 1H), 8.24(m, 1H), 8.07-7.95 (m, 2H), 7.82-7.71 (m, 1H), 7.68(m, 1H), 7.40-7.24 (m, 3H), 4.05 (s, 2H), 3.98 (s, 2H). MS (ESI) m/z calcd C₁₉H₁₄N₆OS [M⁺] ,374.1 found, 375.3 [M⁺ + H⁺].

### 2-((2-(phenylamino)quinazolin-4-yl)amino)thiazol-4(5H)-one (26b)

Compound **2j** (70 mg, 0.25 mmol) was dissolved in *tert*-butanol (3 mL). A solution of aniline (35 mg, 0.38 mmol) in *tert*-butanol (0.5 mL) was added to the mixture. Then, 37% HCl in water (0.012 mL) was added, and the reaction mixture was stirred at 110°C for 16 hours. The solvent was removed under reduced pressure. To the mixture, water was added and extracted with ethyl acetate. The organic layer was dried over anhydrous Na₂SO₄, then purified by column chromatography (33% EA/Hex) to afford compound **26b** (8 mg, 10%)

¹H NMR (300 MHz, DMSO-d₆) δ 12.34 (s, 1H), 9.32 (s, 1H), 8.24(m, 1H), 8.05-7.93 (m, 2H), 7.76 (ddd, *J* = 8.4, 6.9, 1.6 Hz, 1H), 7.60 (d, *J* = 8.3 Hz, 1H), 7.40(m, 3H), 7.05-6.92 (m, 1H), 4.04 (s, 2H). MS (ESI) m/z calcd C₁₇H₁₃N₅OS [M⁺] ,335.1 found, 336.3 [M⁺ + H⁺].

### M-isopropyl-5-(4-((5-methyl-1G-pyrazol-3-yl)amino)quinazolin-2-yl)-2,5-

### diazabicyclo[2.2.1]heptane-2-carboxamide (27a)

Compound **2p** (70 mg, 0.27 mmol) and compound **18a** (59 mg, 0.32 mmol) was dissolved in ethanol (3 mL). The reaction mixture was heated at 110°C for overnight. The solvent was evaporated and purified with column chromatography (6% DCM/MeOH) to afford compound **27a** as a white solid compound. (70 mg, 63%)

¹H NMR (300 MHz, DMSO-d₆) δ 12.19 (s, 1H), 10.24 (s, 1H), 8.42 (d, *J* = 8.3 Hz, 1H), 7.59 (t, *J* = 7.7 Hz, 1H), 7.38 (d, *J* = 8.4 Hz, 1H), 7.25 **-** 7.03 (m, 1H), 6.57 (d, *J* = 18.9 Hz, 1H), 5.95 (t, *J* = 8.5 Hz, 1H), 4.94 (s, 1H), 4.60 (s, 1H), 3.84 **-** 3.66 (m, 1H), 3.66 **-** 3.45 (m, 2H), 3.24 **-** 3.03 (m, 1H), 2.28 (s, 3H), 2.02 **-** 1.78 (m, 2H), 1.03 (q, *J* = 6.3, 5.6 Hz, 6H). ¹³C NMR (101 MHz, DMSO) δ 158.87, 158.56, 158.25, 157.48, 156.41, 133.55, 123.98, 119.23, 116.25, 110.83, 57.94, 56.98, 56.34, 56.29, 55.57, 55.38, 53.93, 53.57, 53.37, 42.20, 41.91, 37.14, 36.92, 23.55, 23.52, 23.44, 23.35, 18.47, 17.16, 12.82, 11.48. MS (ESI) m/z calcd for C₂₁H₂₆N₈O [M⁺], 406.5 ; found, 407.5 [M⁺ + H⁺].

### 5-(4-((5-methyl-1G-pyrazol-3-yl)amino)quinazolin-2-yl)-M-phenyl-2,5-diazabicyclo[2.2.1]heptane-2-carboxamide (27b)

Compound **2p** (60 mg, 0.23 mmol) and compound **18b** (55 mg, 0.25 mmol) was dissolved in ethanol (2 mL). The reaction mixture was heated at 110°C for 2 days. The solvent was evaporated and purified with column chromatography (2% DCM/MeOH) to afford compound **27b** as a white solid compound. (26 mg, 26%)

¹H NMR (400 MHz, DMSO-d₆) δ 12.11 (s, 1H), 10.05 (s, 1H), 8.38 (d, *J* = 8.3 Hz, 1H), 8.24 (s, 1H), 7.55 (t, *J* = 7.6 Hz, 1H), 7.47 (d, *J* = 7.3 Hz, 2H), 7.34 (s, 1H), 7.27 **-** 7.14 (m, 2H), 7.09 (t, *J* = 7.2 Hz, 1H), 6.90 (t, *J* = 7.3 Hz, 1H), 6.58 (s, 1H), 4.98 (s, 1H), 4.78 (s, 1H), 3.78 **-** 3.39 (m, 4H), 2.27 (s, 3H), 2.00 (s, 2H). ¹³C NMR (101 MHz, DMSO) δ 157.87, 154.36, 152.51, 140.73, 133.18, 128.74, 125.36, 123.79, 122.15, 121.28, 119.85, 110.95, 97.52, 57.54, 56.85, 55.51, 55.38, 54.11, 53.70, 37.06, 11.51. MS (ESI) m/z calcd for C₂₄H₂₄N₈O [M⁺], 440.5 ; found, 441.5 [M⁺ + H⁺].

### 2-methyl-1-(5-(4-((5-methyl-1G-pyrazol-3-yl)amino)quinazolin-2-yl)-2,5-diazabicyclo[2.2.1]heptan-2-yl)propan-1-one (27c)

Compound **2p** (80 mg, 0.31 mmol) and compound **18c** (57 mg, 0.34 mmol) was dissolved in ethanol (3 mL). The reaction mixture was heated at 110°C for 2 days. The solvent was evaporated and purified with column chromatography (DCM:MeOH:H₂O = 79:9:1) to afford compound **27c** as a white solid compound. (81 mg, 68%)

¹H NMR (300 MHz, DMSO-d₆) δ 12.12 (s, 1H), 10.06 (s, 1H), 8.38 (d, *J* = 8.2 Hz, 1H), 7.55 (t, *J* = 8.2 Hz, 1H), 7.34 (d, *J* = 8.4 Hz, 1H), 7.11 (t, *J* = 7.7 Hz, 1H), 6.56 (s, 1H), 5.11 **-** 4.67 (m, 2H), 3.79 **-** 3.38 (m, 4H), 2.89 **-** 2.74 (m, 1H), 2.28 (s, 3H), 2.08 **-** 1.82 (m, 2H), 1.10 **-** 0.91 (m, 4H), 0.84 (d, *J* = 6.1 Hz, 2H). ¹³C NMR (101 MHz, DMSO-d₆) δ 174.54, 174.43, 157.85, 157.65, 152.62, 133.14, 125.43, 123.78, 121.28, 97.47, 57.91, 56.37, 55.63, 55.13, 36.25, 31.59, 31.20, 20.14, 19.57, 19.39, 19.19, 11.51. MS (ESI) m/z calcd for C₂₁H₂₅N₇O [M⁺], 391.5 ; found, 392.5 [M⁺ + H⁺].

### (5-(4-((5-methyl-1G-pyrazol-3-yl)amino)quinazolin-2-yl)-2,5-diazabicyclo[2.2.1]heptan-2-yl)(phenyl)methanone (27d)

Compound **2p** (80 mg, 0.31 mmol) and compound **18d** (75 mg, 0.37 mmol) was dissolved in ethanol (3 mL). The reaction mixture was heated at 110°C for overnight. The solid was filtered and washed with ethyl acetate to afford compound **27d** as a white solid product. (60 mg, 46%)

¹H NMR (400 MHz, DMSO-d₆) δ 12.57 (s, 1H), 11.54 **-** 11.16 (m, 1H), 8.75 **-** 8.53 (m, 1H), 8.10 **-** 7.74 (m, 2H), 7.67 **-** 7.29 (m, 6H), 6.70 **-** 6.34 (m, 1H), 5.63 **-** 5.06 (m, 1H), 5.05 **-** 4.47 (m, 1H), 4.04 **-** 3.67 (m, 3H), 3.67 **-** 3.49 (m, 1H), 2.40 **-** 2.25 (m, 3H), 2.25 **-** 2.00 (m, 2H). ¹³C NMR (101 MHz, DMSO) δ 169.27, 168.60, 136.87, 136.21, 133.26, 130.78, 130.46, 129.73, 128.98, 128.73, 127.79, 127.58, 123.34, 122.24, 111.08, 60.38, 57.03, 56.88, 56.03, 55.38, 54.99, 37.99, 36.28. MS (ESI) m/z calcd for C₂₄H₂₃N₇O [M⁺], 425.5 ; found, 426.5 [M⁺ + H⁺].

### 2-(5-isobutyl-2,5-diazabicyclo[2.2.1]heptan-2-yl)-M-(5-methyl-1G-pyrazol-3-yl)quinazolin-4-amine (27e)

Compound **2p** (80 mg, 0.31 mmol) and compound **18e** (57 mg, 0.34 mmol) was dissolved in ethanol (3 mL). The reaction mixture was heated at 110°C for 4 days. The solvent was evaporated and purified with column chromatography (5% DCM/MeOH) to afford compound **27e** as a light brown solid compound. (79 mg, 68%)

¹H NMR (400 MHz, DMSO-d₆) δ 12.16 (s, 1H), 10.16 (s, 1H), 8.92 (d, *J* = 81.0 Hz, 1H), 8.41 (d, *J* = 7.4 Hz, 1H), 7.58 (t, *J* = 7.7 Hz, 1H), 7.37 (d, *J* = 8.4 Hz, 1H), 7.13 (t, *J* = 7.5 Hz, 1H), 6.57 (d, *J* = 52.6 Hz, 1H), 5.11 **-** 4.28 (m, 2H), 4.28 **-** 3.48 (m, 4H), 3.24 **-** 2.78 (m, 2H), 2.47 **-** 2.22 (m, 4H), 2.12 **-** 1.68 (m, 2H), 0.92 (s, 6H). ¹³C NMR (101 MHz, DMSO) δ 158.58, 158.27, 157.66, 157.10, 133.33, 125.03, 123.87, 121.59, 119.27, 116.28, 111.06, 111.04, 97.57, 60.82, 53.74, 42.05, 20.95, 20.85, 12.79, 11.51. MS (ESI) m/z calcd for C₂₁H₂₇N₇ [M⁺], 377.5 ; found, 378.5 [M⁺ + H⁺].

### 2-(5-benzyl-2,5-diazabicyclo[2.2.1]heptan-2-yl)-M-(5-methyl-1G-pyrazol-3-yl)quinazolin-4-amine (27f)

Compound **2p** (70 mg, 0.31 mmol) and compound **18f** (70 mg, 0.37 mmol) was dissolved in ethanol (3 mL). The reaction mixture was heated at 110°C for 3 days. The solvent was evaporated and purified with column chromatography (3% DCM/MeOH) to afford compound **27f** as a white solid compound. (66 mg, 52%)

¹H NMR (300 MHz, DMSO-d₆) δ 12.16 (s, 1H), 10.17 (s, 1H), 8.40 (d, *J* = 8.2 Hz, 1H), 7.58 (t, *J* = 7.7 Hz, 1H), 7.52 **-** 7.17 (m, 6H), 7.13 (t, *J* = 7.7 Hz, 1H), 6.77 **-** 6.34 (m, 1H), 4.86 (s, 1H), 4.14 **-** 3.42 (m, 5H), 3.24 **-** 3.05 (m, 1H), 2.95 (s, 1H), 2.26 (s, 3H), 2.11 **-** 1.68 (m, 2H). ¹³C NMR (101 MHz, DMSO) δ 158.58, 158.27, 157.52, 156.98, 133.35, 129.96, 129.64, 128.84, 127.95, 124.69, 123.87, 121.54, 110.93, 97.51, 59.82, 58.03, 53.78, 51.29, 42.08, 14.44, 12.81, 11.51. MS (ESI) m/z calcd for C₂₄H₂₅N₇ [M⁺], 411.5 ; found, 412.5 [M⁺ + H⁺].

### 5-(4-((1G-pyrazol-4-yl)amino)quinazolin-2-yl)-M-isopropyl-2,5-diazabicyclo[2.2.1]heptane-2-carboxamide (28a)

Compound **2f** (70 mg, 0.29 mmol) and compound **18a** (63 mg, 0.34 mmol) was dissolved in ethanol (3 mL). The reaction mixture was heated at 110°C for overnight. The solvent was evaporated and purified with column chromatography (9% DCM/MeOH) to afford compound **28a** as a white solid compound. (81 mg, 72%)

¹H NMR (300 MHz, DMSO-d₆) δ 12.80 (s, 1H), 10.48 (s, 1H), 8.34 (s, 1H), 8.24 **-** 7.81 (m, 2H), 7.81 **-** 7.59 (m, 1H), 7.62 **-** 7.43 (m, 1H), 7.32 (s, 1H), 6.17 **-** 5.90 (m, 1H), 5.04 (s, 1H), 4.76 **-** 4.56 (m, 1H), 3.85 **-** 3.52 (m, 3H), 3.52 **-** 3.39 (m, 1H), 2.07 **-** 1.88 (m, 2H), 1.03 (dd, *J* = 6.6, 5.0 Hz, 6H). ¹³C NMR (101 MHz, DMSO) δ 159.17, 158.86, 158.55, 123.80, 122.13, 121.72, 121.40, 119.15, 116.17, 110.77, 58.70, 57.46, 56.21, 55.70, 53.96, 53.36, 42.23, 41.98, 37.27, 37.02, 23.50, 23.31, 12.79. MS (ESI) m/z calcd for C₂₀H₂₄N₈O [M⁺], 392.5 ; found, 393.5 [M⁺ + H⁺].

### 5-(4-((1G-pyrazol-4-yl)amino)quinazolin-2-yl)-M-phenyl-2,5-diazabicyclo[2.2.1]heptane-2-carboxamide (28b)

Compound **2f** (60 mg, 0.24 mmol) and compound **18b** (58 mg, 0.27 mmol) were dissolved in ethanol (2 mL). The reaction mixture was heated at 110°C for overnight. The solvent was evaporated and purified with column chromatography (7% DCM/MeOH) to afford compound **28b** as a white solid compound. (33 mg, 32%)

¹H NMR (300 MHz, DMSO-d₆) δ 12.69 (s, 1H), 10.02 (s, 1H), 8.37 **-** 8.17 (m, 2H), 8.03 (s, 2H), 7.61 (t, *J* = 7.4 Hz, 1H), 7.54 **-** 7.33 (m, 3H), 7.29 **-** 7.06 (m, 3H), 6.96 **-** 6.81 (m, 1H), 5.05 (s, 1H), 4.80 (s, 1H), 3.92 **-** 3.45 (m, 4H), 2.03 (s, 2H). ¹³C NMR (101 MHz, DMSO) δ 156.95, 154.37, 140.74, 133.47, 128.74, 123.51, 122.15, 119.81, 110.96, 58.11, 57.05, 56.78, 55.93, 55.72, 53.85, 42.13, 37.13, 12.80. MS (ESI) m/z calcd for C₂₃H₂₂N₈O [M⁺], 426.5 ; found, 427.5 [M⁺ + H⁺].

### 1-(5-(4-((1G-pyrazol-4-yl)amino)quinazolin-2-yl)-2,5-diazabicyclo[2.2.1]heptan-2-yl)-2-methylpropan-1-one (28c)

Compound **2f** (70 mg, 0.29 mmol) and compound **18c** (63 mg, 0.34 mmol) was dissolved in ethanol (3 mL). The reaction mixture was heated at 110°C for overnight. The solvent was evaporated and purified with column chromatography (7% DCM/MeOH) to afford compound **28c** as a white solid compound. (87 mg, 81%)

¹H NMR (400 MHz, DMSO-d₆) δ 12.70 (s, 1H), 10.14 (s, 1H), 8.28 (d, *J* = 8.1 Hz, 1H), 8.21 **-** 7.72 (m, 2H), 7.62 (t, *J* = 8.2 Hz, 1H), 7.54 **-** 7.33 (m, 1H), 7.33 **-** 7.13 (m, 1H), 5.06 (d, *J* = 32.0 Hz, 1H), 4.83 (s, 1H), 3.89 **-** 3.36 (m, 4H), 2.91 **-** 2.76 (m, 1H), 2.11 **-** 1.88 (m, 2H), 1.09 **-** 0.92 (m, 4H), 0.87 (d, *J* = 6.7 Hz, 2H). ¹³C NMR (101 MHz, DMSO) δ 174.67, 174.44, 158.60, 158.29, 156.82, 133.61, 123.56, 110.97, 57.76, 56.66, 55.44, 55.38, 53.52, 49.07, 37.89, 36.27, 31.60, 31.26, 20.15, 19.59, 19.42, 19.16. MS (ESI) m/z calcd for C₂₀H₂₃N₇O [M⁺], 377.5 ; found, 378.5 [M⁺ + H⁺]

### (5-(4-((1G-pyrazol-4-yl)amino)quinazolin-2-yl)-2,5-diazabicyclo[2.2.1]heptan-2-yl)(phenyl)methanone (28d)

Compound **2f** (70 mg, 0.29 mmol) and compound **18d** (69 mg, 0.34 mmol) was dissolved in ethanol (3 mL). The reaction mixture was heated at 110°C for overnight. The solvent was evaporated and purified with column chromatography (7% DCM/MeOH) to afford compound **28d** as a white solid compound. (84 mg, 71%) ¹H NMR (400 MHz, DMSO-d₆) δ 12.65 (s, 1H), 9.93 (s, 1H), 8.24 (dd, *J* = 15.2, 8.2 Hz, 1H), 8.15 **-** 7.74 (m, 2H), 7.71 **-** 7.46 (m, 5H), 7.39 (p, *J* = 7.0 Hz, 2H), 7.19 (t, *J* = 8.1 Hz, 1H), 5.16 **-** 4.40 (m, 2H), 3.75 (q, *J* = 19.3 Hz, 3H), 3.50 (d, *J* = 11.0 Hz, 1H), 2.22 **-** 1.87 (m, 2H). ¹³C NMR (101 MHz, DMSO) δ 174.56, 174.44, 157.82, 157.66, 152.64, 133.14, 125.42, 123.80, 121.25, 57.93, 56.34, 55.65, 55.00, 53.19, 37.85, 36.25, 31.60, 31.21, 20.11, 19.57, 19.38, 19.20, 8.47, 8.42, 7.65. MS (ESI) m/z calcd for C₂₃H₂₁N₇O [M⁺], 411.5 ; found, 412.5 [M⁺ + H⁺].

### 2-(5-isobutyl-2,5-diazabicyclo[2.2.1]heptan-2-yl)-M-(1G-pyrazol-4-yl)quinazolin-4-amine (28e)

Compound **2f** (70 mg, 0.29 mmol) and compound **18e** (53 mg, 0.34 mmol) was dissolved in ethanol (3 mL). The reaction mixture was heated at 110°C for overnight. The solvent was evaporated and purified with column chromatography (11% DCM/MeOH) to afford compound **28e** as an ivory solid compound. (95 mg, 92%)

¹H NMR (300 MHz, DMSO-d₆) δ 12.71 (s, 1H), 10.05 (s, 1H), 9.42 (s, 1H), 8.34 (d, *J* = 8.5 Hz, 1H), 8.02 (s, 2H), 7.60 (t, *J* = 7.7 Hz, 1H), 7.52 **-** 7.31 (m, 1H), 7.20 (t, *J* = 7.5 Hz, 1H), 4.97 (s, 1H), 4.72 **-** 3.45 (m, 4H), 3.28 **-** 2.74 (m, 2H), 2.41 **-** 1.51 (m, 3H), 0.94 (s, 6H). ¹³C NMR (101 MHz, DMSO) δ 158.84, 158.53, 157.07, 152.98, 133.30, 123.78, 122.25, 121.75, 119.21, 116.23, 111.26, 60.83, 26.16, 21.01, 20.94. MS (ESI) m/z calcd for C₂₀H₂₅N₇ [M⁺], 363.5 ; found, 364.5 [M⁺ + H⁺].

### 2-(5-benzyl-2,5-diazabicyclo[2.2.1]heptan-2-yl)-M-(1G-pyrazol-4-yl)quinazolin-4-amine (28f)

Compound **2f** (70 mg, 0.29 mmol) and compound **18f** (64 mg, 0.34 mmol) was dissolved in ethanol (3 mL). The reaction mixture was heated at 110°C for 3 days. The solvent was evaporated and purified with column chromatography (7% DCM/MeOH) to afford compound **28f** as a white solid compound. (71 mg, 62%)

¹H NMR (300 MHz, DMSO-d₆) δ 12.70 (s, 1H), 10.03 (s, 1H), 8.30 (d, *J* = 8.2 Hz, 1H), 7.99 (s, 2H), 7.72 **-** 6.89 (m, 8H), 4.96 (s, 1H), 4.73 **-** 3.48 (m, 6H), 3.17 (d, *J* = 4.7 Hz, 1H), 2.04 (s, 2H). ¹³C NMR (101 MHz, DMSO) δ 158.63, 158.32, 156.91, 135.07, 133.53, 129.96, 129.63, 128.93, 123.57, 116.28, 111.09, 59.80, 53.83, 40.58, 40.37, 40.16, 39.95, 39.74, 39.53, 39.32, 31.43, 22.53, 14.44. MS (ESI) m/z calcd for C₂₃H₂₃N₇ [M⁺], 397.5 ; found, 398.5 [M⁺ + H⁺].

### Examples

### 1.1. HTRF Analysis

Recombinant GST-TAG-MASTL (Thermo Fisher Scientific, MA, USA) was used for HTRF-based kinase assays. The HTRF KinEASE assay kit (CisBio, MA, USA) was used according to the manufacturer's instructions.

### 1.2. In vitro kinase analysis

Two recombinant GST-tag-MASTL (Thermo Fisher Scientific) and His-tag-ENSA (Sino Biological Inc., Beijing, China) were used for in *vitro* kinase analysis. In *vitro* kinase analysis was performed as described in the literature [Front Oncol 2020, 10, 571601, 39-202021-10-20doi:10.3389/fonc.2020.571601.].

### 1.3. Cell viability analysis

MCF7 breast cancer cell lines and MCF10A normal breast cancer cell lines were treated with serial dilutions of the compounds of the present invention ranging from 1 µM to 15.63 nM for 72 hours. Cell viability was measured by the WST-8 assay (Cyto XTM cell viability assay kit; LPS solution, Daejeon, Korea) according to the manufacturer's instructions.

The results of the above analysis are shown in Table 1.

**[Table 1]**

| cpd | MASTL activity (%) | pENSA (%) | Cell viability (%) | |
|---|---|---|---|---|
| | HTRF (1 µM) | Immunofluor ecense (1 µM) | WST-8, MCF7 (1 µM) | WST-8, MCF10A (1 µM) |
| 3a | 20.8 | 5.1 | 0.05 | 1.6 |
| 3b | 123^{b} | 44.3^{b} | 55.5^{b} | - |
| 3c | 123 | 87.0 | 110^{d} | 115^{d} |
| 3d | 93.7 | 108 | 132^{d} | 101^{d} |
| 3e | 100^{b} | 102^{b} | 108^{b} | - |
| 3f | 92.5 | 107 | 117^{d} | 98.3^{d} |
| 3g | 127^{c} | 108 | 109^{d} | 99.7^{d} |
| 3h | 114 | 91.2 | 105 | 71.6 |
| 3i | 126 | 83.8 | 95.6 | 51.8 |
| 3j | 92.5 | 93.2 | 108 | 84.2 |
| 3k | 99.1 | 103 | 120 | 96.6 |
| 3l | 114 | 112 | 80.5 | 104 |
| 3m | 117 | 85 | 116 | 91.7 |
| 3n | 125 | 97.7 | 117 | 95.2 |
| 3o | 102 | 31.3 | 108 | - |
| 3p | 53.1 | 27.5 | 106 | - |
| 3q | 63.1 | 21.1 | 105 | - |
| 3r | 67.2 | 30.3 | 98.3 | - |
| 3s | 102 | 60.6 | 100 | - |
| 3t | 120 | 53.1 | 45.4 | - |
| 3u | 121 | 53.2 | 49.3 | - |
| 4a | 54.9^{a} | 3.7 | 8.1^{a} | 34^{a} |
| 4b | 43.2 | 82 | 47.7 | 68.2 |
| 4c | 47.1 | 69 | 50.8 | 83.3 |
| 4d | 32.6^{a} | 6.6 | 5.6^{a} | 3.2^{a} |
| 4e | 15.1^{b} | 90.7 | 112^{a} | 110^{a} |
| 4f | 29^{b} | 50.5 | 94.5^{a} | 33^{a} |
| 4g | 18.6^{b} | 10.4 | 85.3^{a} | 9.3^{a} |
| 4h | 17.6^{b} | 17.1 | 41.8^{a} | 4.7^{a} |
| 4i | 8.7b | 16 | 45.9^{a} | 5.8^{a} |
| 4j | 27^{a} | 5.2 | 22.8^{a} | 21.6^{a} |
| 4k | 59.2^{b} | 105 | 80.8^{a} | 115^{a} |
| 4l | 81.5 | 88.6 | 100 | 118 |
| 4m | 73.0 | 88.8 | 110 | 85.8 |
| 4n | 106 | 92.8 | 21.5 | 3.2 |
| 4o | 53.8 | 19.2 | 53.4 | 79.4 |
| 4p | 58 | 56.2 | 100 | 86.8 |
| 4q | 76.1 | 53.6 | 107 | 98.8 |
| 4r | 56.6 | 12 | 98.8 | 84.2 |
| 4s | 56.2 | 89.1 | 102 | 113 |
| 4t | 14.9 | 0.9 | 2.7 | 18.4 |
| 4u | 30.5 | 41.2 | 64.8 | 25.5 |
| 4v | 41.4 | 15.3 | 32.8 | 34.1 |
| 4w | 48.5 | 135 | 72.0 | 63.4 |
| 4x | 70.8 | 185 | 75.8 | 80.0 |
| 4y | 75.3 | 164 | 78.7 | 80.2 |
| 4z | 85.3 | 188 | 86.3 | 77.7 |
| 4aa | 26.8 | 142.5 | 87.6 | 111 |
| 4ab | 36.2 | 188 | 101 | 101 |
| 4ac | 58.3 | 149 | 104 | 102 |
| 4ad | 65.7 | 143 | 96.4 | 96.0 |
| 4ae | 41.7 | | 72.8 | 70.5 |
| 4af | 49.6 | | 66.6 | 66.5 |
| 4ag | 67.1 | | 56.4 | 74.5 |
| 4ca | 37.4^{b} | 81.6 | 43.0^{a} | 99.1^{a} |
| 4cb | 53.1^{b} | 85.6 | 87.5^{a} | 102^{a} |
| 4cc | 22.0^{b} | 2.4 | 65.5^{a} | 98.5^{a} |
| 4cd | 20.3^{b} | 16.5 | 83.8^{a} | 83.9^{a} |
| 4ce | 14.1^{b} | 19.8 | 72.8^{a} | 100^{a} |
| 4cf | 23.7^{b} | 17.4 | 87.0^{a} | 56.4^{a} |
| 4cg | 76.4 | 65.5 | 73.6 | 117 |
| 4ch | 43 | 24.5 | 73.6 | 32 |
| 4da | 58.5 | 9.2 | 30.1 | 18.9 |
| 4db | 28.9 | 19.2 | 3.2 | 8.9 |
| 4dc | 22.1 | 6.3 | 6.4 | 54.0 |
| 4dd | 17.9 | 16.4 | 12.9 | 3.7 |
| 4de | 31.8 | 48.1 | 44.2 | 24.0 |
| 4df | 31.8 | 54.5 | 78.4 | 4.8 |
| 4dg | 37.9 | 53.5 | 49.0 | 12.0 |
| 4dh | 88.7 | 85.6 | 116 | 111 |
| 4di | 38.8 | 51.1 | 55.4 | 12.1 |
| 6a | 15.2 | 10.1 | 13.2 | 20.9 |
| 6b | 43.5 | 98.2 | 116 | 122 |
| 6c | 32.9 | 90.5 | 109 | 129 |
| 6d | 48 | 96.4 | 88.6 | 104 |
| 6e | 43.3 | 97 | 90.9 | 91.4 |
| 6f | 55.8 | 110 | 91.9 | 102 |
| 6g | 41.4 | 113 | 95 | 104 |
| 6h | 46 | 93 | 89.7 | 59.8 |
| 6i | 74.6 | 97.2 | 113 | 122 |
| 6j | 80 | 97.4 | 45.6 | 33.1 |
| 6k | 40.6 | 82 | 91 | 90.8 |
| 6l | 76.1 | 101 | 47.8 | 15.2 |
| 6m | 51.8 | 97.1 | 65.7 | 115 |
| 6n | 30.8 | 79.1 | 110 | 116 |
| 6o | 61.2 | 92.6 | 48.4 | 24.9 |
| 7 | 31 | 68.2 | 84.2 | 54.9 |
| 8 | 57.2 | 53 | 64.3 | 80.8 |
| 9a | 45.5 | 50.5 | 63.8 | 49.7 |
| 9b | 21.7 | 34.6 | 52.5 | 12.3 |
| 9d | 20.4^{b} | 44.1 | 36.2^{a} | 11.1^{a} |
| 10 | 24.1 | 50.3 | 61.4 | 60.7 |
| 11 | 18.1^{b} | 66.4 | 104^{a} | 98.0^{a} |
| 12a | 26.3^{b} | 63.8 | 26.2^{a} | 82.3^{a} |
| 12b | 17.7^{b} | 41.7 | 69.4^{a} | 123^{a} |
| 12c | 45.7^{b} | 75.8 | 107^{a} | 61.3^{a} |
| 12d | 14.1b | 46.3 | 55.8^{a} | 63.7^{a} |
| 13 | 53.8^{a} | 90 | 62^{a} | 15.9^{a} |
| 14 | 25.5 | 9.2 | 21.8 | 63.4 |
| 15a | 61.1^{a} | 86.1 | 61^{a} | 37^{a} |
| 15b | 104 | 95.4 | 75.3 | 77.2 |
| 15c | 41.2 | 11.3 | 93.0 | 40.0 |
| 15d | 58.8 | 68.6 | 51.5 | 40.3 |
| 15e | 80.0 | 48.2 | 68.3 | 31.3 |
| 15f | 65.7 | 65.3 | 71.7 | 90.1 |
| 15g | 27.9 | 65.6 | 54.4 | 42.9 |
| 15h | 15.7 | 51.7 | 94.5^{d} | 45.0^{d} |
| 15i | 15.5^{b} | 0.2^{b} | 5.2^{b} | - |
| 15j | 31.6 | 65.2 | 104^{d} | 65.2^{d} |
| 15k | 11.3^{b} | 0.5^{b} | 12.7^{b} | - |
| 15l | 18.1^{b} | 1.9^{b} | 2.4^{b} | - |
| 15m | 28.7 | 82.6 | 111^{d} | 82.2^{d} |
| 15n | 32.4 | 77.5 | 108^{d} | 63.9^{d} |
| 15o | 20.3 | 90.3 | 119^{d} | 92.6^{d} |
| 15p | 180b | 2.6^{b} | 7.4^{b} | - |
| 15q | 49.2 | 62.5 | 106^{d} | 108^{d} |
| 15r | 22.7^{b} | 0.2^{b} | 19.0^{b} | - |
| 15s | 19.2^{b} | 0.2^{b} | 5.1^{b} | - |
| 15t | 15.5^{b} | 0^{b} | 35.5^{b} | - |
| 15u | 48.3 | 100 | 107 | 121 |
| 15v | 29.5 | 62.8 | 80.5 | 81.8 |
| 15w | 64.1 | 86.2 | 93.5 | 107 |
| 15x | 68.4 | 94.0 | 122 | 98.3 |
| 15y | 18.5^{b} | 35.5^{b} | 64.1^{b} | - |
| 15z | 105 | 102 | 110 | 111 |
| 15aa | 55.5^{d} | 61.2^{d} | 87.5^{d} | - |
| 15ab | 17.3^{b} | 0.4^{b} | 3.9^{b} | - |
| 19 | 39.9 | 84.7 | 41.2 | 71.3 |
| 19' (B) | 32.2 | 74.7 | 74.8 | 101 |
| 20 | 36.5 | 30.9 | 5.5 | 3.9 |
| 20' (B) | 6.6 | 21 | 32 | 34.9 |
| 24a | 29.9 | 14 | 4.8 | 15.7 |
| 24b | 24.8 | 36 | 90.7 | 26.5 |
| 24c | 16.6 | 24.3 | 68.3 | 10.8 |
| 24d | 32.9 | 2.6 | 6.4 | 4.9 |
| 24e | 10.8 | 4.1 | 2.2 | 2.8 |
| 2a | 73.4 | 105 | 106 | 93.3 |
| 25a | 114 | 91.2 | 105 | 71.6 |
| 25b | 91.6 | 102 | 86.4 | 58.1 |
| 25c | 108 | 96.5 | 86.4 | 90.0 |
| 25d | 107 | 80.6 | 90.8 | 106 |
| 26a | 92.5 | 93.2 | 108 | 84.2 |
| 26b | 112 | 84.8 | 95.8 | 98.8 |
| 27a | 121 | 85.9 | 95.6 | 90.7 |
| 27b | 84.3 | 38.4 | 83.2 | 36.1 |
| 27c | 102 | 90.5 | 103 | 46.7 |
| 27d | 121 | 60.6 | 94.9 | 48.3 |
| 27e | 67.4 | 90.1 | 88.3 | 74.8 |
| 27f | 78.4 | 77.4 | 109 | 113 |
| 28a | 113 | 89.6 | 99.0 | 93.92a |
| 28b | 133 | 44.2 | 91.2 | 37.0 |
| 28c | 117 | 80.0 | 105 | 85.3 |
| 28d | 119 | 66.9 | 93.1 | 49.7 |
| 28e | 103 | 95.8 | 97.3 | 105 |
| 28f | 118 | 85.1 | 110 | 101 |

In Table 1, the concentration of a = 1.25 µM, the concentration of b = 10 µM, the concentration of c = 3.3 µM, the concentration of d = 1.1 µM. Based on the results according to Table 1, the IC₅₀ value was calculated and shown in Table 2.

**[Table 2]**

| Phosphorylated ENSA (pENSA), which is activated by phosphorylation by MASTL, is MASTL | | | | |
|---|---|---|---|---|
| cpd | IC50(nM) | | | |
| | MASTL activity | pENSA | Cell viability (MCF7) | Cell viability (MCF10A) |
| 3a | 178 | 100 | 207 nM | 29 |
| 3o | 2.6µM | 280 | - | - |
| 3p | 330 | 239 | - | - |
| 3q | 640 | 930 | - | - |
| 3r | 816 | 497 | - | - |
| 3s | 4.4 µM | - | - | - |
| 3u | - | 678 | - | - |
| 4a | 987 | 501 | 649 | 881 |
| 4b | 1.4 µM | 1.1 µM | - | - |
| 4c | 1.0 | 850 | - | - |
| 4d | 625 | 211 | 176 | - |
| 4e | 164 | - | - | - |
| 4f | - | 899 | 2.3 µM | - |
| 4g | 189 | 344 | 2.7 µM | - |
| 4h | 843 | 408 | 758 | 57.2 |
| 4i | 1.2 µM | 378 | 1.1 µM | 132 |
| 4j | 527 | 420 | 713 | - |
| 4n | - | - | 287 | 116 |
| 4o | - | 680 | - | - |
| 4r | - | 988 | - | - |
| 4s | - | 89.1 | 102 | 113 |
| 4t | 198 | 341 | 324 | 226 |
| 4u | 392 | - | - | 718 |
| 4v | 1.5 µM | - | 390 | 438 |
| 4w | 1.3 µM | - | 1.8 µM | 908 nM |
| 4aa | 720 | - | 4.5 µM | 9.0 µM |
| 4ab | 1.1 µM | - | 12.4 µM | 5.8 µM |
| 4ca | 1.8 µM | - | 1.7 µM | 2.8 µM |
| 4cc | 298 | 363 | 1.1 µM | 2.8 µM |
| 4cd | 476 | 543 | 2.6 µM | 2.7 µM |
| 4ce | 692 | 491 | 1.5 µM | 3.7 µM |
| 4cf | 893 | 423 | 1.8 µM | 1.2 µM |
| 4ch | - | 943 | - | 569 |
| 4da | 309 | 1.8 µM | 702 | 683 |
| 4db | - | 2.1µM | 221 | 300 |
| 4dc | 480 | 2.5µM | 467 | 1.3µM |
| 4dd | 157 | 1.5µM | 317 | 107 |
| 6a | 340 | 532 | 499 | 392 |
| 6c | 796 | - | - | - |
| 6e | 569 | - | - | - |
| 6h | 995 | - | - | - |
| 6j | - | - | 674 | 566 |
| 6k | 406 | - | - | - |
| 6l | - | - | 484 | 332 |
| 6n | 121 | - | - | - |
| 60 | - | - | - | 293 |
| 7 | 315 | - | - | - |
| 9a | 203 | - | - | - |
| 9b | 136 | 832 | 1.0 µM | 316 |
| 9d | 119 | 577 | 539 | 413 |
| 10 | 569 | - | - | - |
| 11 | 1.0 µM | - | 3.2 µM | 4.1 µM |
| 12a | - | 1.1 µM | 697 | 2.4 µM |
| 12b | 807 | 852 | 1.5 µM | 2.6 |
| 12c | - | - | - | 1.4 µM |
| 12d | 832 | 571 | 1.5 µM | 1.5 µM |
| 13 | 1.0 µM | - | 932 | 316 |
| 14 | 593 | 645 | 647 | 1.7 µM |
| 15a | 1.1 µM | - | - | 820 |
| 15c | 265 | 576 | - | - |
| 15g | 225 | - | - | - |
| 15h | 147 | - | 1.7 µM | 833 |
| 15i | 310 | 2.1 µM | 2.0 µM | - |
| 15j | 284 | - | 3.2 µM | 1.3 µM |
| 15k | 152 | 3.5 µM | 4.1 µM | - |
| 15l | 226 | 3.7 µM | 2.7 µM | - |
| 15m | 490 | - | 4.2 µM | 1.9 µM |
| 15n | 327 | - | 3.5 µM | 1.3 pM |
| 150 | 170 | - | 3.7 µM | 2.4 µM |
| 15p | 331 | 4.4 µM | 4.1 µM | - |
| 15q | 545 | - | - | - |
| 15r | 492 | 5.5 µM | 4.3 µM | - |
| 15s | 354 | 2.2 µM | 2.9 µM | - |
| 15t | 563 | 6.2 µM | 7.9 µM | - |
| 15u | 269 | - | - | - |
| 15v | 52.7 | - | - | - |
| 15y | 928 | 6.9 µM | 8.2 µM | - |
| 15ab | 222 | 2.5 µM | 2.4 µM | - |
| 19 | 569 | - | 1.1 µM | 810 |
| 19'(B) | 282 | - | - | - |
| 20 | 194 | 66.5 | 150 | 131 |
| 20'(B) | 106 | 755 | 365 | 596 |
| 24a | 180 | 263 | 954 | 420 |
| 24b | 198 | 770 | - | 684 |
| 24d | 597 | 1.9 µM | 626 | 210 |
| 24e | 213 | 1.9 µM | 543 | 184 |

Phosphorylated ENSA (pENSA), which is activated by phosphorylation by MASTL, can be utilized as a biomarker as it is expected that its activity will be inhibited along with the inhibition of MASTL. In the cell viability assay, MCF7 refers to a breast cancer cell line, and MCF10A refers to a normal breast cancer cell line. As shown in Tables 1 and 2, it was confirmed that compounds 4d and 4j of the present invention selectively inhibit only breast cancer cells in the cell viability assay of breast cancer cells and normal cells. In the case of 4d, the IC₅₀ value in MCF7 breast cancer cells was 176 nM, confirming that it has an excellent inhibitory effect on breast cancer cells.

Consequently, it was confirmed that the compound of the present invention exhibits excellent MASTL inhibitory activity and effectively inhibits breast cancer cells; thus, it was confirmed that the compound of the present invention can be applied to the prevention, improvement, or treatment of cancer due to its MASTL inhibitory effect.

The foregoing description is merely illustrative of the present invention, and those skilled in the art to which the present invention pertains will be able to make various modifications within the scope of the essential characteristics of the present invention. Accordingly, the embodiments disclosed herein are intended to illustrate, not limit, the present invention, and the spirit and scope of the present invention are not limited by such embodiments. The scope of protection of the present invention shall be interpreted by the claims below, and all technology within an equivalent scope shall be interpreted as being included within the scope of rights of the present invention.

### [Industrial Applicability]

The compound of the present invention has a MASTL inhibitory effect, is useful as a medicine, and can be used for the prevention or treatment of cancer.

## Claims

1. A compound represented by Formula 1, a stereoisomer thereof, a hydrate thereof, a solvate thereof, or a salt thereof: 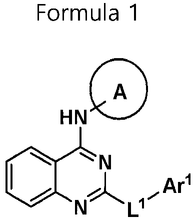 wherein:
L¹ is a single bond; -NH-; or a C₂-C₃₀ heterocyclic group containing at least one N;
Ar¹ is selected from the group consisting of hydrogen; a C₁-C₃₀ alkyl group; a C₁-C₃₀ hydroxyalkyl group; a C₆-C₃₀ aryl group; a C₇-C₃₀ arylalkyl group; a C₂-C₃₀ heterocyclic group containing at least one N; -S(=O)₂-R'; and -C(=O)-NH-R'; and said Ar¹ may be optionally substituted with one or more R¹s.
Ring A is a C₆-C₃₀ aryl group; a -(CH₂)n-aryl group; or a C₂-C₃₀ heterocyclic group containing at least one N; and the A ring can be optionally substituted with one or more R²s.
n is an integer of 1 to 10,
R' is a C₁-C₃₀ alkyl group; a C₃-C₃₀ cycloalkyl group; a C₇-C₃₀ arylalkyl group; a C₆-C₃₀ aryl group; or a C₂-C₃₀ heterocyclic group;
R¹ is independently selected from the group consisting of hydrogen; halogen; hydroxyl group; oxo group; -(CH₂)ₙ-cyano group; C₃-C₃₀ cycloalkyl group; C₁-C₃₀ alkyl group substituted or unsubstituted with halogen; C₁-C₃₀ alkoxy group; -L'- C₂-C₃₀ heterocyclic group; -L'-C(=O)-NH₂; -L'-C(=O)O-R^{a}; -L'-S(=O)₂-R^{a}; and -S(=O)₂-NR^{b}R^{c};
R² are independently selected from the group consisting of hydrogen; halogen; hydroxyl group; amino group; oxo group; C₃-C₃₀ cycloalkyl group; C₁-C₃₀ alkyl group; C₂-C₃₀ alkenyl group; C₁-C₃₀ alkoxy group; -NH-C(=O)O-R^{d}; -S(=O)₂-R^{d}; -S(=O)₂-NR^{e}R^{f}; and -O-C₇-C₃₀ arylalkyl group; or multiple adjacent R²s may bond to each other to form a ring.
L' is a single bond; or C₁-C₁₀ alkylene group;
R^{a}, R^{b}, R^{c}, R^{d}, R^{e} and R^{f} are independently hydrogen; a C₁-C₁₀ alkyl group; a C₃-C₃₀ cycloalkyl group; a C₆-C₁₂ aryl group substituted or unsubstituted with a halogen; a C₃-C₃₀ heterocyclic group containing at least one N; or a C₁-C₁₀ hydroxyalkyl group.

2. The compound, a stereoisomer thereof, a hydrate thereof, a solvate thereof, or a salt thereof according to claim 1, wherein Formula 1 is represented by Formula 1-1: 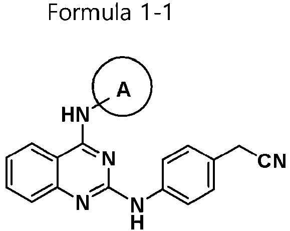 wherein:
Ring A is a substituent represented by any one of Formulas A-1 to A-5, 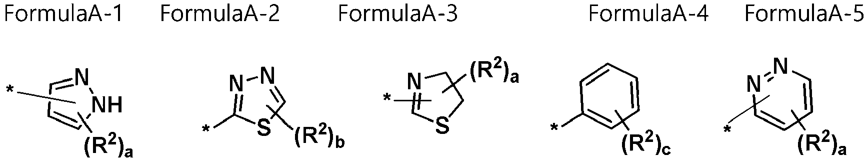
wherein:
* indicates a position to be bonded,
R² is the same as defined in claim 1,
a is an integer of 0 to 3, b is an integer of 0 to 2, and c is an integer of 0 to 5.

3. The compound, a stereoisomer thereof, a hydrate thereof, a solvate thereof, or a salt thereof according to claim 1, wherein Formula 1 is represented by Formula 2-1: 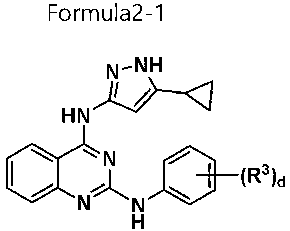 wherein:
R³ is the same as the definition of R¹ in claim 1,
provided that the case where R3 is entirely hydrogen is excluded,
d is an integer of 0 to 5:

4. The compound, a stereoisomer thereof, a hydrate thereof, a solvate thereof, or a salt thereof according to claim 1, wherein Formula 1 is represented by any one of the following formulas 2-2 to 2-6: 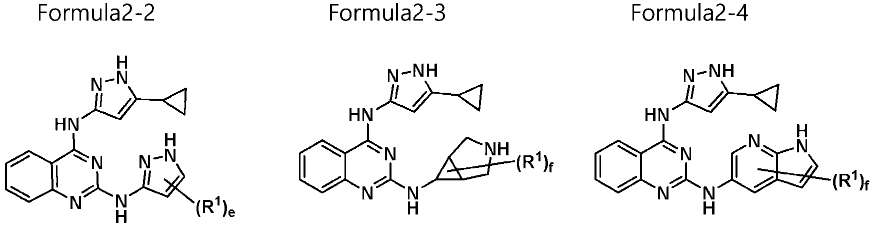 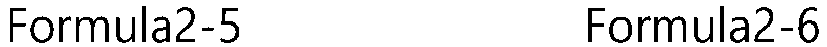 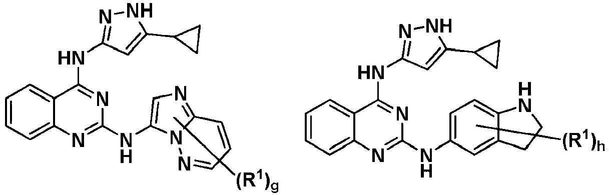 wherein:
R¹ is the same as defined in claim 1,
e is an integer of 0 to 3, f is an integer of 0 to 5, g is an integer of 0 to 4, and h is an integer of 0 to 6.

5. The compound, a stereoisomer thereof, a hydrate thereof, a solvate thereof, or a salt thereof according to claim 1, wherein Formula 1 is represented by any one of the following formulas 3-1 to 3-9: 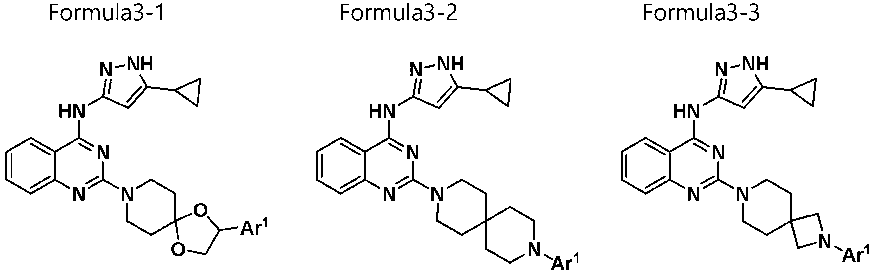 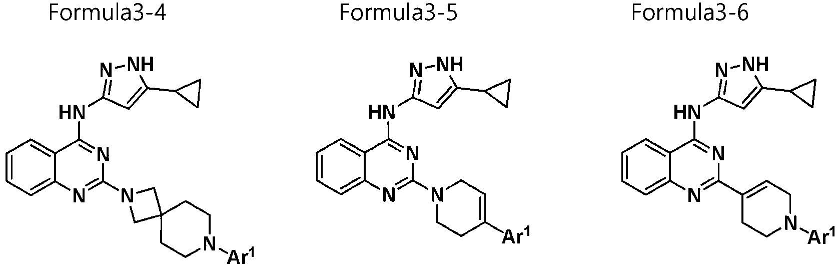 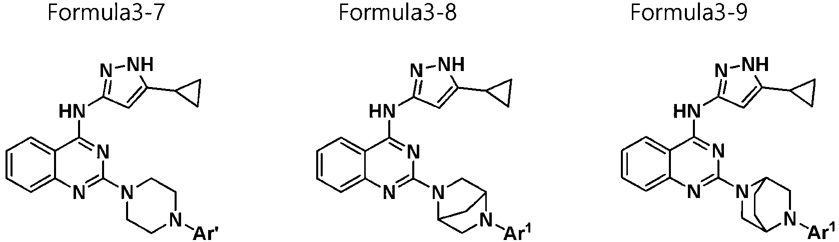 wherein:
Ar' is selected from the group consisting of a C₁-C₃₀ hydroxyalkyl group; a C₆-C₃₀ aryl group; a C₇-C₃₀ arylalkyl group; and a C₂-C₃₀ heterocyclic group containing at least one N; -S(=O)₂-R'; and -C(=O)-NH-R'; and the Ar' can be optionally substituted with one or more R¹s,
Ar¹, R¹, and R' are the same as defined in claim 1.

6. The compound, a stereoisomer thereof, a hydrate thereof, a solvate thereof, or a salt thereof according to claim 1, wherein Formula 1 is represented by any one of the following compounds:

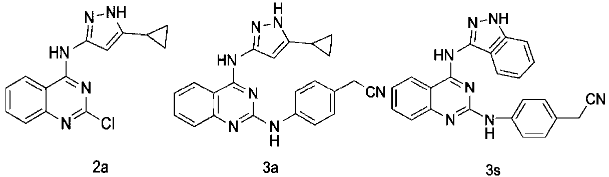

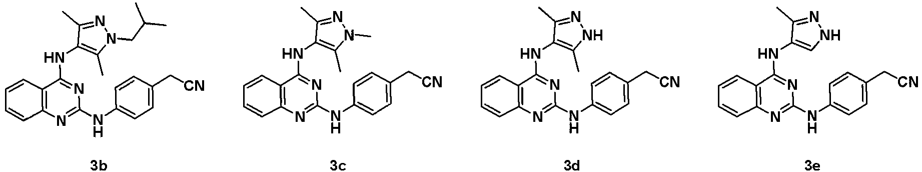

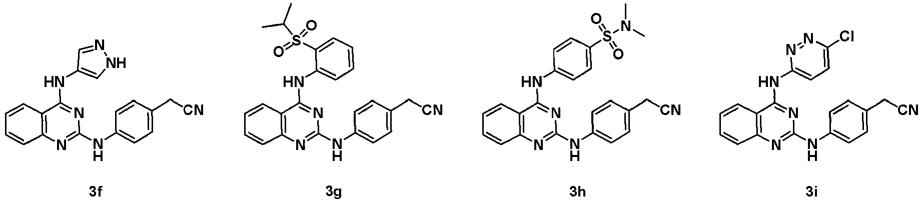

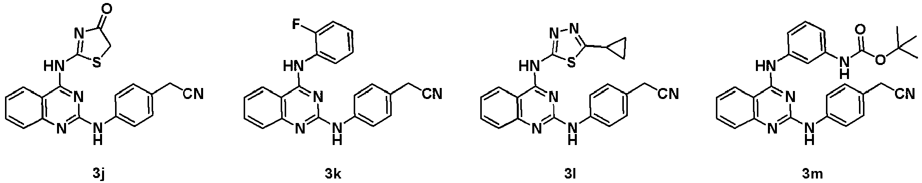

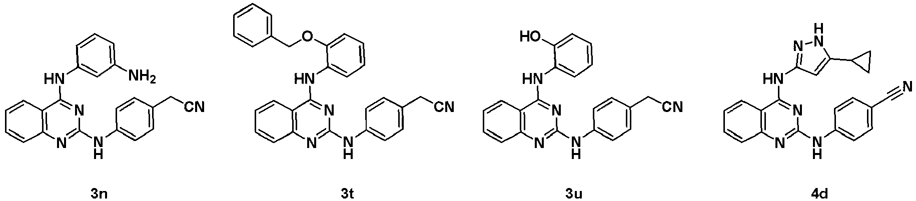

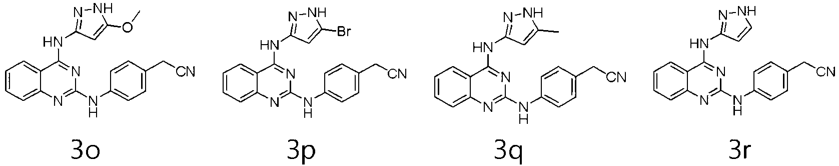

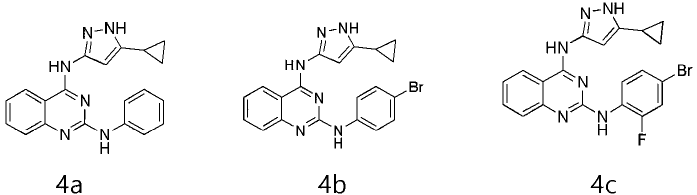

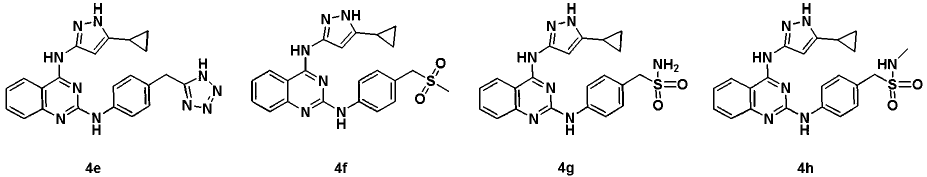

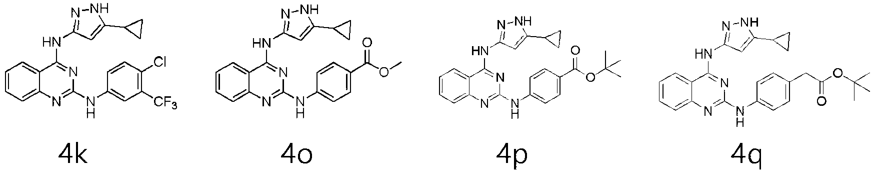

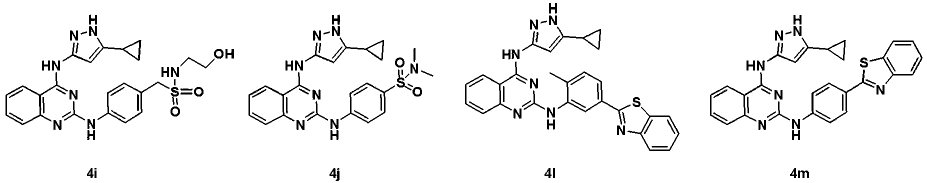

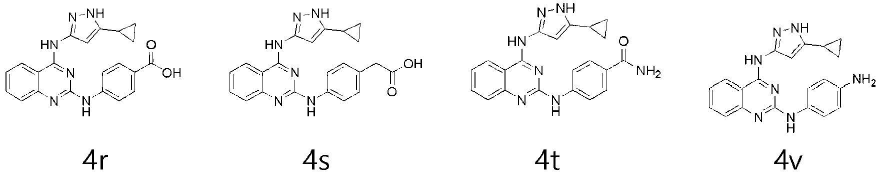

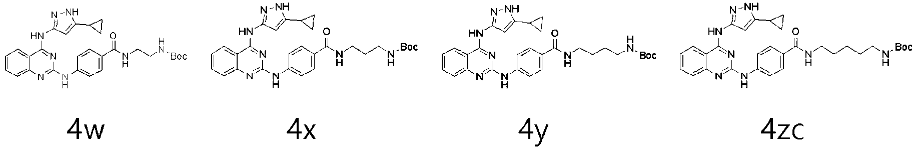

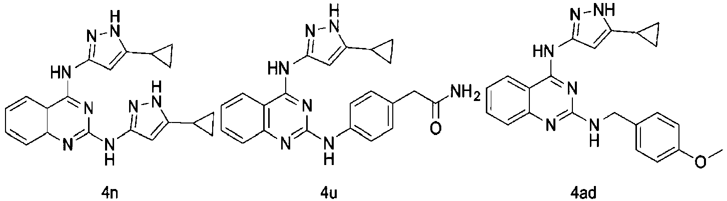

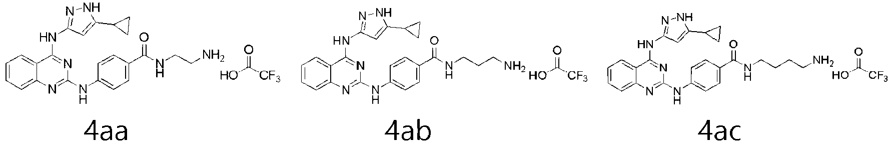

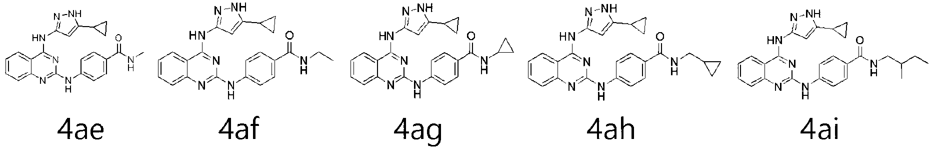

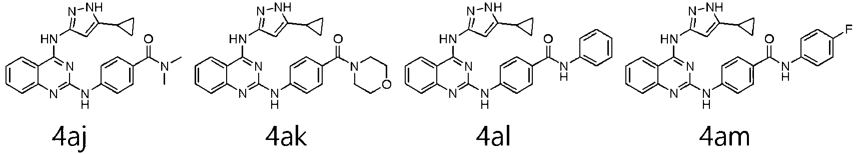

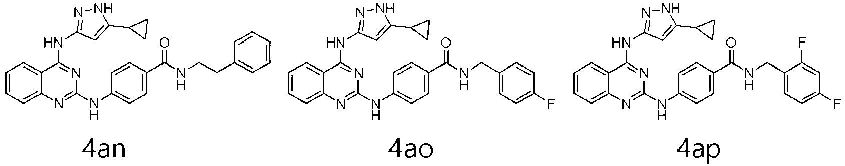

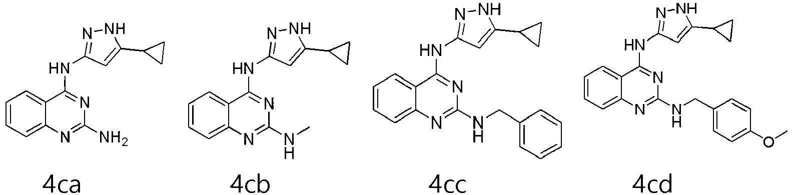

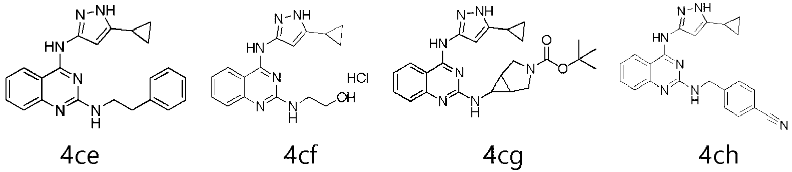

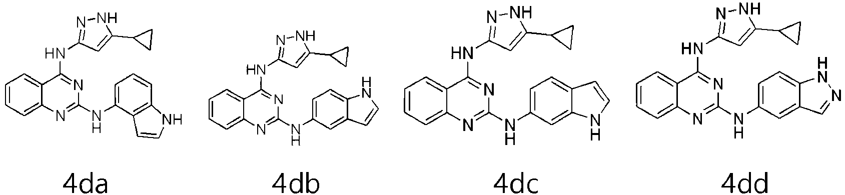

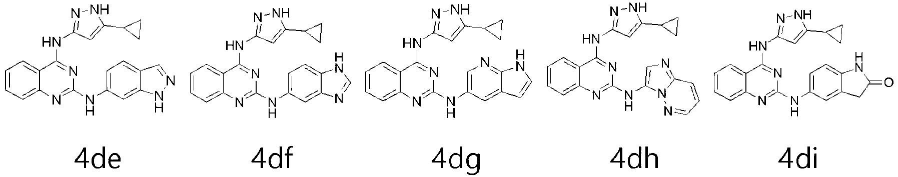

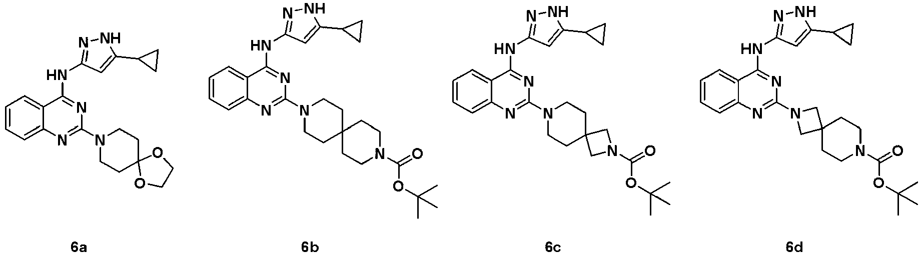

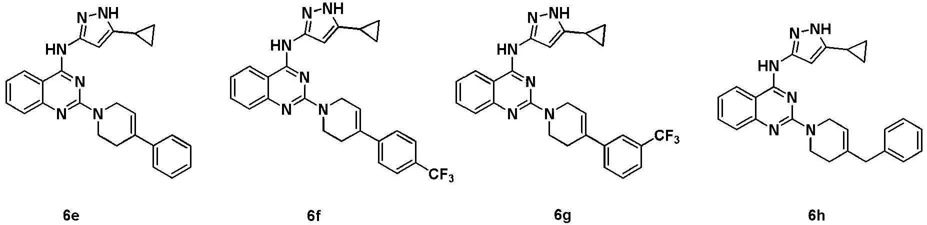

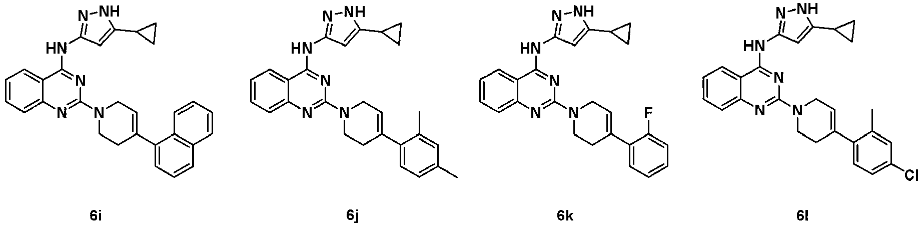

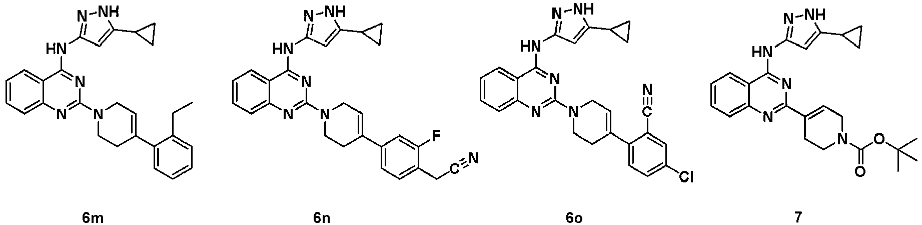

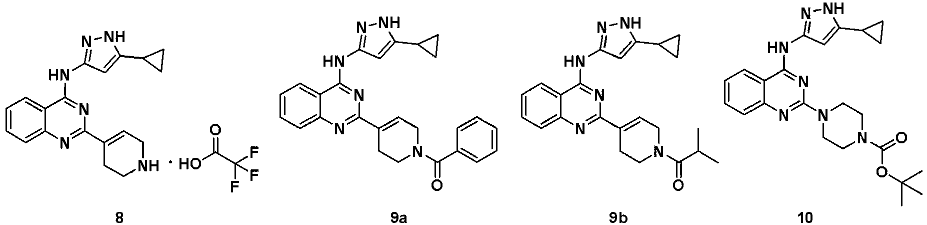

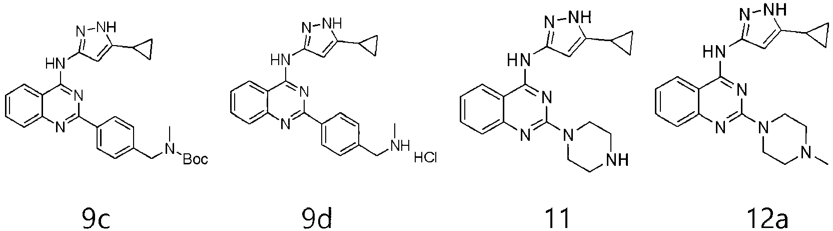

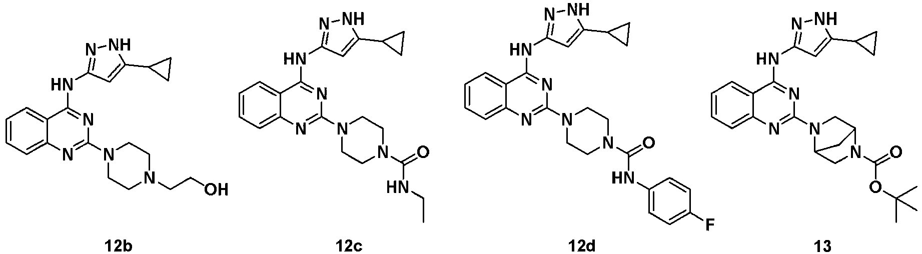

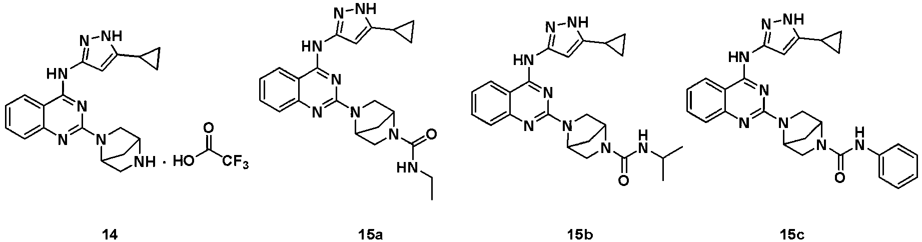

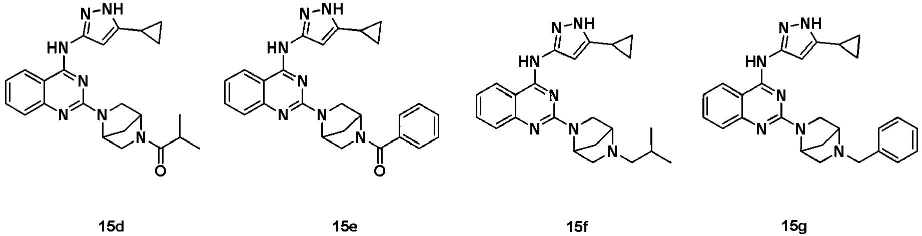

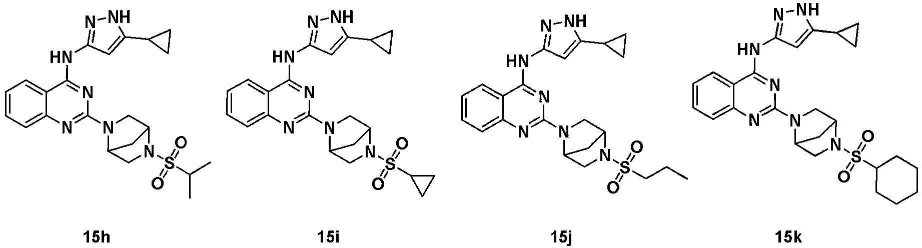

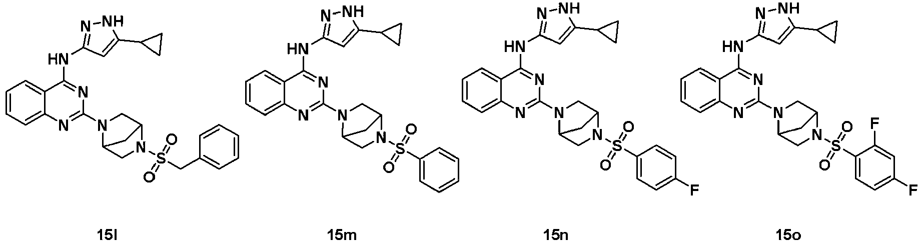

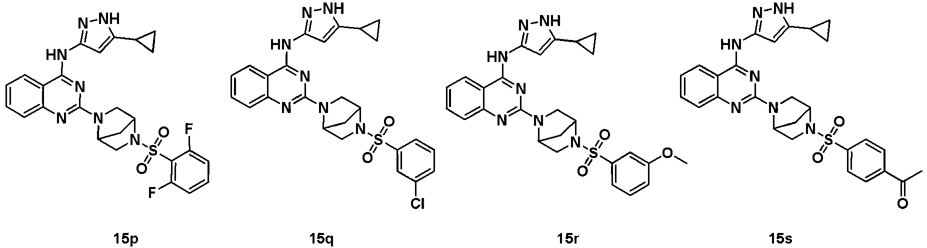

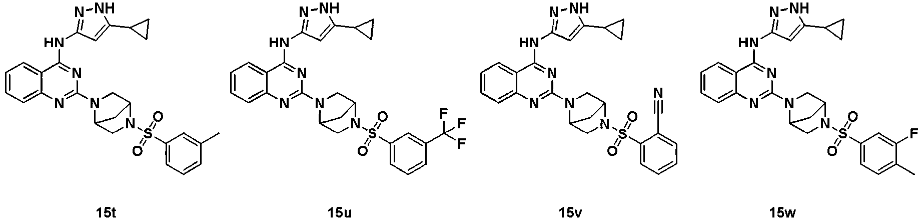

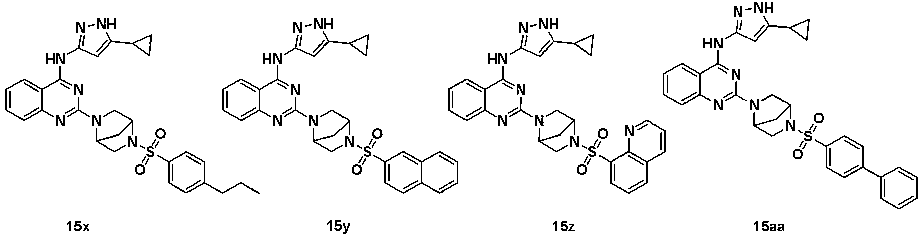

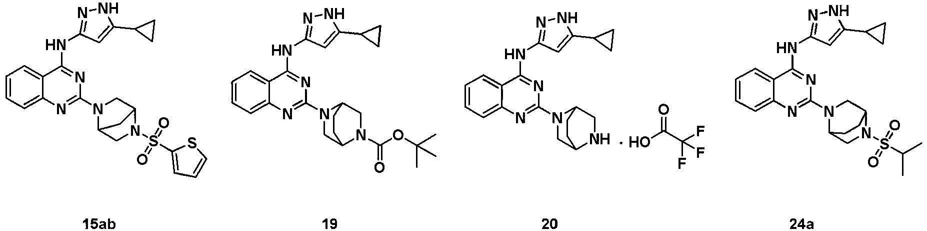

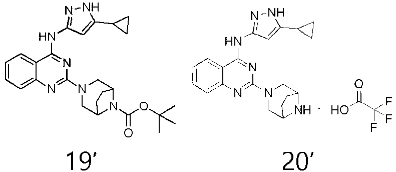

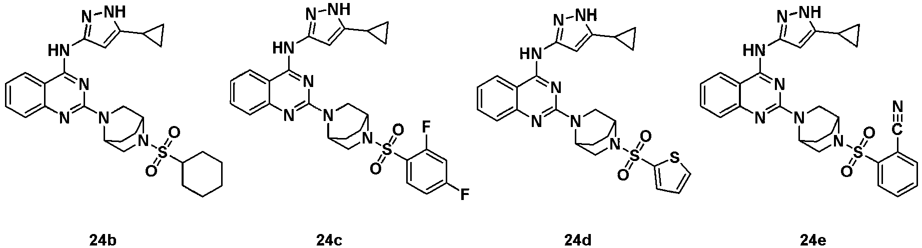

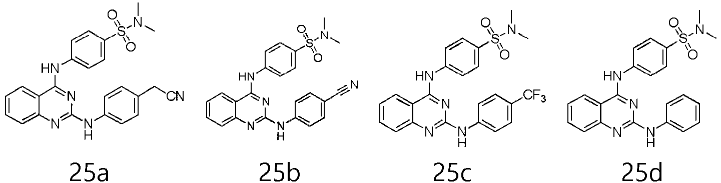

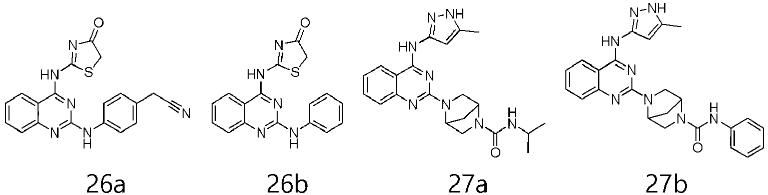

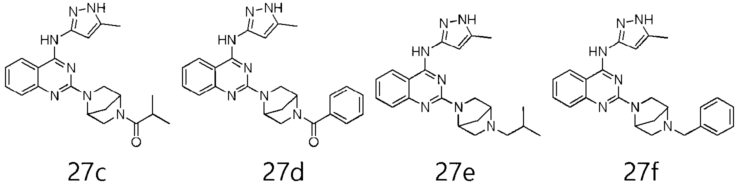

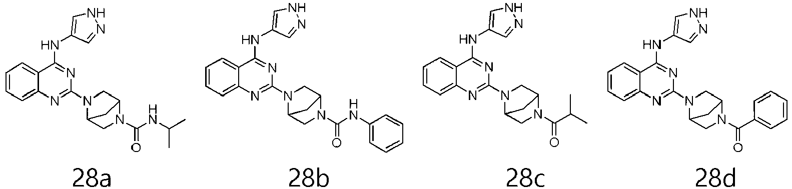

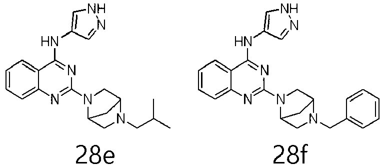

7. The compound, a stereoisomer thereof, a hydrate thereof, a solvate thereof, or a salt thereof, according to claim 1, wherein the compound, a stereoisomer thereof, a hydrate thereof, a solvate thereof, or a salt thereof inhibits MASTL (microtubule-associated serine/threonine kinase-like) activity.

8. A pharmaceutical composition for the prevention or treatment of cancer comprising a compound according to claim 1, a stereoisomer thereof, a hydrate thereof, a solvate thereof, or a salt thereof as an active ingredient.

9. The pharmaceutical composition for the prevention or treatment of cancer according to claim 8, wherein the composition inhibits MASTL (microtubule-associated serine/threonine kinase-like).

10. The pharmaceutical composition for the prevention or treatment of cancer according to claim 8, wherein the cancers comprise stomach cancer, breast cancer, uterine cancer, colon cancer, colorectal cancer, lung cancer, pancreatic cancer, liver cancer, or prostate cancer.

11. A method for the prevention or treatment of cancer comprising the step of administering a composition according to any one of claims 8 to 10 to a mammal.

12. A health functional food composition for the prevention or treatment of cancer comprising the compound according to claim 1, a stereoisomer thereof, a hydrate thereof, a solvate thereof, or a salt thereof as an active ingredient.

13. Use of the compound, a stereoisomer thereof, a hydrate thereof, a solvate thereof, or a salt thereof according to claim 1 for manufacturing a drug for the prevention or treatment of cancer
